# EUROPEAN PATENT APPLICATION

(11) **EP 3 581 201 A1**
(43) Date of publication of application: **18.12.2019**
(21) Application number: 18178128.7
(22) Date of filing: 15.06.2018
(51) Int. Cl.: A61K 39/00, C07K 14/195, C12Q 1/689

(54) **ESCHERICHIA COLI O157:H7 PROTEINS AND USES THEREOF**

(71) Applicant: GlaxoSmithKline Biologicals S.A., 1330 Rixensart (BE)
(72) Inventor: The designation of the inventor has not yet been filed
(74) Representative: Sanderson, Andrew John

(57) **Abstract**

The present invention is direct to isolated polypeptides comprising or consisting of: an amino acid sequence selected from the group consisting of SEQ ID NOs: 25, 26, 27, 28, 29, 30, 31, 32, 33, 34, 35, 36, 37, 38, 39, 40, 41, 42, 43, 44, 45, 46, 47 or 48; or (a) a variant and/or fragment of (a), for example: (i) a variant of (a); (ii) a fragment of (a); or (iii) a variant of a fragment of (a); together with corresponding isolated nucleic acid molecules, vectors, host cells, methods of production, vesicles, binding moieties, pharmaceutical compositions, kits, methods of treatment and medical uses.

SEQUENCE LISTING

<110>  GlaxoSmithKline Biologicals SA

<120>  NOVEL PROTEINS AND USES THEREOF

<130>  VB66511P

<160>  116

<170>  PatentIn version 3.5

<210>  1
<211>  960
<212>  DNA
<213>  Escherichia coli

<400>  1
atgaaaaaaa gtgtcatcgc tggcgtcttt attgctctgt catttaccac gtgttcagct      60

atcgcgaaca gccttgcatt atcattagca aatgatgatg cagggaagtt tcaaccaata     120

cttaatgata tttatggcaa taaacatgaa aacagagatg attactcaca aggcttattt     180

ctgggatata gccacgatat ctcagactcg agccaattat ctctccatat tgcgcaagat     240

atttactctc catcaggcag taataaaaga cacaacacag ctgtaactgg agacagagct     300

tttagtgcat acactcacac tggtattgaa tggaactccc ttgcgaatga ctggattcgc     360

tatcgattag gtactgacat aggtgttgtt ggccccgacg caggcggtca gaaagtacaa     420

aataaagctc atgagattat tggggcagaa aaatatcatg catgggatga tcaaatagag     480

aatcgctacg gttatactgt aaaagggatg ctatccatga caccaagtat ggatatttta     540

ggtgctaatg ttggattata ccctgaagtt tctgctgtta ctggaaactt atttcaatat     600

gtagcatatg gcgcaaccat tgccattggt aatgataaaa ccttcaattc ggataatggc     660

tttggtctgc tggctccccg tggtttaatg catatgtccg atacaagcgg attcaaatac     720

aagatttttg caggtatgga aagacgagat gtcaatcgca actatactct cgaaggaaaa     780

acaatacaga cgaaacaaac aacagtatcg ctaaacaaaa ctgttgatga atatcaagtt     840

ggcgcaacaa ttgggtatgc acctgtagcc ttcacactag catttaataa agtaacatca     900

gaattcaaga caggggatga ctattcattt ataaatggag caatcacctt ctttttttaa     960


<210>  2
<211>  801
<212>  DNA
<213>  Escherichia coli

<400>  2
atgaattcaa aaaagctttg ttgcatatgt gtgttattct cgctgcttgc aggatgtgcc      60

tctgaatctt ctattgatga aaagaagaaa aaagctcaag tcacacaaag taatattaat     120

aaaaacactc cccagcaact gacagacaaa gatttattcg gtaatgaaac cactctggcc 180

gtatccgaag aggatattca agcagcgctt gatggagatg agttccgcgt tccccttaat 240

tctcctgtaa tacttgttca atccggcaac cgcgcaccgg aaaccattat gcaggaggag 300

atgcgaaaat attatactgt ttccacattc tctggtatcc cggacaggca aaagcctctg 360

acttgtaaca aaaacaaaga taaaaatgag aacgaggatg ttgcccgtgc tgagaatatg 420

aactggatgc aggcactgcg ttttgtggct gcaaaaggac atcagaaagc gattattgtt 480

taccaggata tgttgcagac aggaaaatat gactctgcgc tgaaatcaac agtatggtcc 540

gactataaaa atgaaaaact cactgacgct atctccctgc gctacctggt acgtttcacg 600

ctggtggatg tggcaacagg tgagtgggct acctggtcgc cggtgaatta tgaatcgaga 660

gtaatttttc cgccggctgg aataacaaaa acgagcaata aagaactatc aaatgaccat 720

gttacggaag cgcaactgtt taatttgaaa caaaaaacat atgtatcgat ggtgaaagac 780

ttggtgagtc gattccagta a 801

<210> 3
<211> 4587
<212> DNA
<213> Escherichia coli

<400> 3
atgaacagga ccagtcccta ttattgtcgt cgctcagtac tttccttatt gatatctgcc 60

ttgatatatg ccccgcccgg gatggctgcc ttcactcctg atgttattgg tgtggtaaac 120

gatgagactg tagatggcag ccaacgagta gatgaacgag gtacaacaaa taacactcat 180

attatcaacc atggccagca gaatgtttat ggcggggtat ctaatggaag tcttattgaa 240

tctggtggat atcaagatgt aggaaggcat aacaattatg tggggcagtc taataatacc 300

accattaacg ggggcagaca gtcaattcat gacgggggta tttccacagg tacgataatc 360

gagagtggca atcaggacgt ttataaaggg ggtatcagca atggaacgac aattaagggc 420

ggtgcttcac gcgtagaggg agggagtgcg aatggaacac tcattgatgg tggtagccag 480

atagtaaaag ttcaagggca tgctgatggt acaacgataa ataagtctgg ctctcaggac 540

gtagtacaag gaagtctggc aacgaacaca accataaatg gtggtcgaca gtatgttgaa 600

cagagcacag tagaaacaac caccatcaaa aatggcggtg agcaaagagt atatgagagc 660

cgtgcgctgg acacgacgat tgaaggcgga actcagtctc tgaatagtaa gtcaacggca 720

aaaaatactc agatctattc tggtggtacg caaattattg ataacaccag ctcctcggat 780

gttattgaag tttattccgg tggcgtgctt gatgttagtg gtggtacggc aacaaatgtt 840

acccagcacg atggtgcaat tttaaaaact aacactaacg gtacgacggt gagcggtacg 900

aatagtgaag gtgcattctc catccacaat cacgtggcag acaatgtgtt gctggaaaac 960

ggtggtcatt tagacataaa cgcatatggt tcggcaaaca agacgattat taaagataaa 1020

ggaacaatgt cagttttaac caatgctaaa gctgatgcga cccgaataga taatggcggg 1080

gttatggatg ttgcaggaaa cgcgacaaat accataatta atggtggcac acagaatatt 1140

aataattatg gcatagccac aggcaccaat atcaacagcg gaacgcaaaa tatcaaaagc 1200

ggcgggaaag ctgacacaac aattatatcc tccgggagcc ggcaggttgt tgagaaagat 1260

ggtacggcaa ttggcagcaa tattagcgcc ggaggctcgc tgattgtcta taccggcggt 1320

attgcacatg gggttaacca ggagacgggc agtgctttag ttgccaacac gggtgcaggg 1380

actgatatcg aaggatacaa caagctctct cacttcacta ttaccggagg ggaggctaat 1440

tatgttgtgc tggaaaatac cggcgaactg acggtagtgg ctaaaacctc ggcgaaaaat 1500

actaccattg atgctggcgg taagctgatt gtccagaagg aggctaaaac agatagcacc 1560

agacttaata atggcggcgt tctggaggtt caggacggtg gtgaggctaa gcatgttgag 1620

caacaatccg gcggcgcatt aattgcttcc acgacctccg gaacacttat cgaaggaacc 1680

aacagttatg gtgatgcttt ctacatcagg aattcagaag ctaaaaatgt agtgctggaa 1740

aacgctggct cattaacagt cgtcactggt tcccgggcag ttgacacgat tattaatgcc 1800

aacggcaaaa tggatgttta tggaaaagat gttggcactg tactcaatag tgctggcacc 1860

caaacaatat atgccagtgc cacttctgat aaagcaaata tcaaaggtgg caagcaaacg 1920

gtatatggtt tagccactga agcaaatatc gaaagtggtg aacaaattgt tgatggtggg 1980

tcaacagaga aaacacacat caatggtggc acgcaaaccg ttcagaatta tggtaaggcg 2040

atcaataccg atatcgtctc tggcctacaa caaattatgg caaacgggac agcggaaggt 2100

tccattatta atggcggttc acagatagtt aatgagggcg gtctggctga aaactcggtg 2160

cttaatgatg gcggcacact cgatgtgcgg gagaaaggca gcgcaacggg gatacagcag 2220

agtagccagg gcgcgttggt tgcaaccacc agggcgacgc gggtcacagg aacacgcgcg 2280

gatggcgtcg cgttcagcat cgagcagggt gcggcgaaca atatcctgct ggcaaatggc 2340

ggagtgttaa ccgtggagtc agacacctct tctgacaaaa cacaggtcaa tacgggcgga 2400

cgggagatcg tcaaaacaaa agccactgcg acaggcacga cgctcaccgg cggtgaacaa 2460

attgtcgagg gtgtggcgaa tgagacaaca attaacgacg gcggaataca aacagtttca 2520

gctaacggag aggcaataaa aacaacgatc aatgaaggcg gtacgctgac agtcaacgat 2580

aatggcaaag cgacagatat cgtccagaac agcggtgccg ctctccagac gagcacggct 2640

aacggtattg aaatcagcgg tactcaccag tacggcactt tttccatttc cggcaattta 2700

gcgaccaata tgttgctgga aaatggcggt aatttattgg tattagcagg taccgaagct 2760

cgcgactcca cggttggcaa gggggggcga atgcaaaacc agggtcagga ctccgccaca 2820

aaggttaact ctggtgggca atataccctt gggcggtcaa aagatgagtt tcaggctctg 2880

gcccgggcag aagatctcca ggttgctggc gggacagcaa tcgtctacgc aggtacgctg 2940

gcggatgcat cggtcagtgg cgcgacagga agcctgtcgt taatgacgcc acgggataat 3000

gttacgccag ttaaactcga aggggcgatc cggattaccg atagcgcgac attaactatc 3060

ggcaatggcg ttgatacgac gcttgccgac ctgacggctg ccagccgggg cagtgtctgg 3120

cttaacagca ataattcctg tgcaggcacc agcaactgcg agtatagagt aaacagtttg 3180

ctacttaacg acggtaatgt ttatttatca gcacaaacag cagcgcctgc cacaactaac 3240

ggtatataca atacgctgac aaccaatgaa ctttccggta gcggtaattt ctacctgcat 3300

accaacgttg caggctctcg gggcgatcaa ctggtcgtca acaacaacgc cactggtaat 3360

tttaaaatct ttgttcagga taccggcgtc agtcctcagt ctgacgacgc gatgacgctg 3420

gtgaaaacag gggaggggga tgcttcgttt tcgctgggca atactggcgg tttcgttgat 3480

cttgggacct atgagtatgt cctgaaaagc gatggcaaca gcaactggaa cctgaccaat 3540

gatgtcaaac ccaacccgga tcccaaccca aatcccaacc caaatccgaa gccggatcca 3600

aaaccagacc caaaaccgga tccgaaacca gacccgactc ccgagccaac gccgacaccc 3660

gttccggaga aacgcatcac gccttctacc gcagccgtac tcaatatggc agcaacatta 3720

ccgttggtat ttgatgctga gctaaacagt attcgcgagc ggttgaacat aatgaaagcg 3780

agtccacaca acaataatgt ctggggggcg acgtataaca cccgtaataa tgtcaccacc 3840

gatgcggggg ccgggtttga gcagacgctg accggaatga cagtggggat cgacagccct 3900

aatgatattc ctgaggggat tgcgacgctg ggcgctttta tgggttattc ccattcacat 3960

atcggttttg atcgcggagg acatggcagt gtgggcagtt attctctggg cggctatgcc 4020

agttgggaac atgaaagtgg tttctatctg gacggtgtcg tgaagctgaa ccgttttgaa 4080

agtaacgtag ccggtaaaat gagcagcggt ggagccgcca atggcagtta ccacagcaac 4140

gggctgggcg gtcacattga aaccgggatg cgatttaccg atggtaactg gaacctgacg 4200

ccgtatgcat cgttaacggg gttcaccgct gataaccccg aatatcattt atccaatggc 4260

atggaatcga aatcagtcga tacccgcagt atatatcgtg aactgggcgc aacgctgagt 4320

tacaacatgc gtctggggaa cggtatggaa attgagccgt ggctgaaggc ggctgtgcgc 4380

aaagaatttg tcgatgataa ccgggtgaag gtgaataatg acggtaattt cgtcaatgat 4440

ttgtcgggca gacgtggaat ataccaggca ggtattaaag cctcattcag cagtacgtta 4500

agcgggcatc ttggggtggg gtatagccat ggtgccggtg tggaatcccc gtggaacgcg 4560

gtagctggtg tgaactggtc gttctga 4587

<210> 4
<211> 747
<212> DNA

<213>  Escherichia coli

<400>  4
atggcaggtt ttttaatatt cctgtcttct gctgcttatg ctgatatcaa tctgtatggt      60

cctggtggcc cgcatacagc cttgcttgat gcagccaaac tttatgccga aaaaacaggt     120

attatagtga acgttcatta cggcccacag aacaaatgga atgaagatgc caaaaaaaat     180

gcagatatct tgtttggcgc atcagaacaa tctgctctgg ctatcattcg ggaccataaa     240

gacagcttca gtgaaaaaga tattcagcct ctttatctgc gaaaaagtat tttactggta     300

aagaaaggta atcctaaaaa tatccggagt attgacgacc tgaccagacc tgggattggc     360

gtaattgtta atgatggtgg tggtaccagt aatacatcag gcactggcgt ctgggaagat     420

attgccggac gtaaagggaa tatagaaact gtcgccgcaa tccgaaaaaa tattatttta     480

tatgcgccca atagcggaac tgcacgtaag gctcttgaga atcagcctgg agcagatgtc     540

tggataacct gggctgactg ggcagccagt aatccagaaa ttggtgatgt cgtggaaata     600

gcgccagact acgtgatatg gcgtgatatg aacattacag tacgtcagga tgcaaatgat     660

gaaacccgtc gatttgcaga atggctacaa accgatgaag cggcgcctgc attcaaaaaa     720

tatggctgga ccaggaaagg cacttga                                         747

<210>  5
<211>  1101
<212>  DNA
<213>  Escherichia coli

<400>  5
atgaaattaa aaatagttgc ggtggttgta actggtttgt tagctgcgaa cgtagcacac      60

gctgccgaag tctataacaa ggatggtaat aaactcgacc tttatggcaa ggttaccgct     120

ctacgttatt ttactgatga taagcgtgac gatggtgata aaacttatgc ccgtctcggc     180

tttaaaggag aaacgcaaat caatgatcaa atgattggtt ttggtcactg ggaatatgat     240

tttaaaggct ataacgatga agccaacggc tcgcgcgaca acaagacccg tctggcctat     300

gctggtttaa aaattagtga atttggctct ctggactatg gccgtaacta cggtgtcggc     360

tatgacattg gttcatggac tgatatgttg ccagaatttg gtggcgatac ctggagtcag     420

aaagatgtct tcatgacata ccgtaccacc ggtgtagcaa cctatcgcaa ctacgatttc     480

tttggcttaa ttgaagggct gaactttgcc gcgcaatatc aaggcaaaaa tgaacgtact     540

gacaacagtc atctttatgg tgctgactac acgcgtgcca acggtgacgg tttcggtatc     600

tcctcaactt atgtttatga tggctttggt atcggagcgg tgtataccaa atccgatcgg     660

acaaatgcgc aggaaagagc cgctgctaat cctctcaatg cctccggtaa gaatgcagaa     720

ctgtgggcta caggtataaa atatgatgcc aacaacatct actttgcagc taattacgct     780

gaaacattaa acatgaccac ctatggcgat ggttatattt ctaacaaagc acaaagtttt     840

gaagtggtgg cgcaatatca attcgacttc ggcttgcgcc cctcactcgc ttacctgaaa 900

tcgaaaggca tagatctggg ccgctacggc gatcaggaca tgattgagta tatcgacgtt 960

ggtgcgacgt atttcttcaa caaaaatatg tcgacctatg ttgattataa aatcaacctg 1020

attgatgaaa gcgactttac ccgtgccgta gatattcgca ccgataacat cgtcgctacg 1080

ggcattacct atcagttcta a 1101

<210> 6
<211> 960
<212> DNA
<213> Escherichia coli

<400> 6
atgaagtgta tattatttaa atgggtactg tgcctgttac tgggtttttc ttcggtatcc 60

tattcccggg agtttacgat agacttttcg acccaacaaa gttatgtctc ttcgttaaat 120

agtatacgga cagagatatc gacccctctt gaacatatat ctcaggggac cacatcggtg 180

tctgttatta accacacccc accgggcagt tattttgctg tggatatacg agggcttgat 240

gtctatcagg cgcgttttga ccatcttcgt ctgattattg agcaaaataa tttatatgtg 300

gccgggttcg ttaatacggc aacaaatact ttctaccgtt tttcagattt tacacatata 360

tcagtgcccg gtgtgacaac ggtttccatg acaacggaca gcagttatac cactctgcaa 420

cgtgtcgcag cgctggaacg ttccggaatg caaatcagtc gtcactcact ggtttcatca 480

tatctggcgt taatggagtt cagtggtaat acaatgacca gagatgcatc cagagcagtt 540

ctgcgttttg tcactgtcac agcagaagcc ttacgcttca ggcagataca gagagaattt 600

cgtcaggcac tgtctgaaac tgctcctgtg tatacgatga cgccgggaga cgtggacctc 660

actctgaact gggggcgaat cagcaatgtg cttccggagt atcggggaga ggatggtgtc 720

agagtgggga gaatatcctt taataatata tcagcgatac tggggactgt ggccgttata 780

ctgaattgcc atcatcaggg ggcgcgttct gttcgcgccg tgaatgaaga gagtcaacca 840

gaatgtcaga taactggcga caggcctgtt ataaaaataa acaatacatt atgggaaagt 900

aatacagctg cagcgtttct gaacagaaag tcacagtttt tatatacaac gggtaaataa 960

<210> 7
<211> 948
<212> DNA
<213> Escherichia coli

<400> 7
atgaaaataa ttatttttag agtgctaact tttttctttg ttatcttttc agttaatgtg 60

gttgcgaagg aatttacctt agacttctcg actgcaaaga cgtatgtaga ttcgctgaat 120

gtcattcgct ctgcaatagg tactccatta cagactattt catcaggagg tacgtcttta 180

ctgatgattg atagtggcac aggggataat ttgtttgcag ttgatgtcag agggatagat 240

ccagaggaag ggcggtttaa taatctacgg cttattgttg aacgaaataa tttatatgtg 300

acaggatttg ttaacaggac aaataatgtt ttttatcgct ttgctgattt ttcacatgtt 360

acctttccag gtacaacagc ggttacattg tctggtgaca gtagctatac cacgttacag 420

cgtgttgcag ggatcagtcg tacggggatg cagataaatc gccattcgtt gactacttct 480

tatctggatt taatgtcgca tagtggaacc tcactgacgc agtctgtggc aagagcgatg 540

ttacggtttg ttactgtgac agctgaagct ttacgttttc ggcaaataca gaggggattt 600

cgtacaacac tggatgatct cagtgggcgt tcttatgtaa tgactgctga agatgttgat 660

cttacattga actggggaag gttgagtagt gtcctgcctg attatcatgg acaagactct 720

gttcgtgtag gaagaatttc ttttggaagc attaatgcaa ttctgggaag cgtggcatta 780

atactgaatt gtcatcatca tgcatcgcga gttgccagaa tggcatctga tgagtttcct 840

tctatgtgtc cggcagatgg aagagtccgt gggattacgc acaataaaat attgtgggat 900

tcatccactc tgggggcaat tctgatgcgc agaactatta gcagttga 948

<210> 8
<211> 1110
<212> DNA
<213> Escherichia coli

<400> 8
atgcaaagga aaggcaataa actgttgatt cagttatgca gtgtgatact gctatttttt 60

accacatcct ggtatgcatt ggcgaatgaa tgttatatag agagaaatgc tgaaggggat 120

tatcacatga agataagctc tactcagctt agtctggcgt cacaaatggt cgaggttccg 180

acagaaatag ccgaagctac atgggatgta aatattcaac taagaggcga tgccataggg 240

tgtaaatctc ttggggatag taaggcagtt cactttctta atacagctga cccaagttta 300

atatccacgt acaccacaac gaatggcgca gcgttattaa aaacaactgt tccaggcatt 360

gtgtattctg tcgagttatt atgccttagt tgtggtgccg cagatgaact tgatttatgg 420

ctacctgcac aaagtggcgc agataacttc ataccaagca cccagacgaa atgggcctat 480

gagtacagtg atcaaagttg gtatttacgt tttcgcttat tcataactcc tgaatttaaa 540

cccaagaatg gtgtttccag cggaacaacg atagcaggaa agattgcgtc atggtatata 600

ggtaccaatg accagccgtg gatcaacttt tacattgaca atgactcttt aaagtttttc 660

gtcgatgaac cgacctgtgc aacagttgcc ctggcacaag atcagggcaa cgtcagtggc 720

aatcaggtaa cgcttgggaa cagctatgtt tcggaagtga aaaatgggct tacgcgggaa 780

atcccttttt ctatccgtgc tgaatactgt tatgccagta aaattacggt taagttgaaa 840

gcggcaaata aacccagcga tgccacactg gtgggtaaaa cgactggctc ggcttcaggc 900

gtggctgtaa aagtaaattc aacttatgac aatagcaaag tattgttaaa agcagatggt 960

agcaacacgg ttgactacaa cttcgccgcc tggtcaaaca acctgctgtt tttacctttt 1020

acggcgcagc tggtaccgga tggtagcggt aatgctgtcg gtgttggaac attttcaggt 1080

aacgcgacct tctcctttac ctacgaataa 1110

<210> 9
<211> 1062
<212> DNA
<213> Escherichia coli

<400> 9
atgagattcg ccaaaggcgt gttgctcgcc atctgtctga tttttttgcc gctaaaagcg 60

gcgctggcgc tgaactgcta ttttggtacc gcaaacggcg ctgtagaaaa atcggaagct 120

attatgccct tcgccgttcc ggccaattcc aaacccggcg ataaaatctg ggaatctgac 180

gatataaaaa ttccggtcta ctgtgacaac aacacaaacg gtaattttga aagcgagcac 240

gtctacgcct gggtaaaccc ttaccccggt attcaggatc cctattatca attgggcgta 300

acctatgaag gcgtggacta tgacgcaagc cttgggaaaa gccgcatcga cacgaatcag 360

tgcatcgaca gtaagaatat tgatatttac accccggagc agatcattgc gatgggctgg 420

caaaataaac tttgctccgg cgatccaacc gttatgcaca aatcgcggac gtttgtggcg 480

cgtatgcggc tttacgttaa agttcgcgcc atgccgcccc atgattatca gagcaaactc 540

agcgattaca tcgtcgtaca gtttgatggc gcaggcagcg tgaacgaaga ccctaccgcg 600

aagaatctga aatatcacat taccggtctg gaaaatatcc gcgtgctgga ttgcagcgtc 660

aactttgcta tctcacctga aacccaggtc gttgattttg gccgatttaa cgtgctggat 720

attcgtcgcc atacgatgtc gcagcaattt aaaataacta caaccaaatc acaaaacgac 780

cagtgtaccg acggttttaa agtcagctct tcgttttata ccgatgaaac ccttatcgac 840

gaagataaat cactgctcat tggcaatggc ttgaagcttc gattgttgga tgaaaatgcc 900

tcaccctata ctttcaacaa atacagtgag tatgccgatt ttaccagcga cctgttagtg 960

tatgaaaaaa cctacacggc agaattgtca tccacgccgg gtacgcccat cgacgttggt 1020

ccctttgata cagtggtact tttcaaaatt aactacaact ga 1062

<210> 10
<211> 1086
<212> DNA
<213> Escherichia coli

<400> 10
ttgtgggaat gtgatgcaac agatttgcca aacatctatt ttctggtcgc taccaatggc 60

gacgaccgcg tgggggcttt ttacgatgca ggcgggcctg atggtctgag tgatgtctat 120

gccacctggt tcgcttttgt cggtctcaag cagaccatgg cgggcgtgac gcttggtcgt 180

tactggaaga aagtgcccat caccagttat gccactcagg gaactaaaat ccagattcgc 240

ttacaggata tccctcctct tcatgctgag ctttatcgca tcagtacgct acctgataca 300

tcagcaacaa caagttggtg cggtaataat aatacagata gtagtggagt cggattcgca 360

aaaccttccg gtacaatcta taactgtgtt cagcccaatg cctatattca gctttccggt 420

accagcggca ttttatttgg tcatgatgag cccggcgaag atagttctgt tcattgggat 480

ttctggggtg ctgataatgg ttttggttac ggaatgcgtt cggccaatcg actctacaac 540

aatgccacct gcgttgcccg cagcgccacg ccgttagtat tgctgccgac aattgcagaa 600

gcacaactga atgcgggcat ggaaagtacc ggtaatttta atgtccgcgt cgagtgtagt 660

aactcggttc aatcagggat tagcgatact cagacagcat taggaatcca ggtgtctgaa 720

ggtgcatata cagcggcgca aaaactgggg attatcaata gcaacggcgg cgtcagcgcc 780

ctggtctctg ataattatga cgcagcagag atggcaaagg gcgttgggat ctacatttct 840

aacagtgctc accccgatac ggcgatgacg ctggttggtc aaccgggcat cgcgaagtta 900

acccccggag gaaatgcagc ggggtggtat cctgtatttg aaggggcaac attagaaggt 960

gcgactcacc ccggatactc cagctatagt tactctttta tcgcccggtt gaagaaactg 1020

ccaaatcaga cagtcagtgc gggaaaagtg cgggcaacgg cttatatatt ggtgaaaatg 1080

caatga 1086

<210> 11
<211> 789
<212> DNA
<213> Escherichia coli

<400> 11
atgaatatgg ctttttacgg caagtggttt gcttgcctgt ggctcgctac gtcgtgcgtt 60

caggcggcga gcacagataa taaggcgctg gagataattc gtcgcgctga cgaaattcgt 120

tcgccgaata aaccgtttcg ttacacgctg acggtaacgg aatataaagc gggcgcaacc 180

cagccagaga ataaacaggt cctcgatatt tccatgcgct ttatgaaacc gcagggtaat 240

gaaaaagcgg atgcacgctc gctggtgcgt tttatttatc cgccgcgaga taaaggaaaa 300

atcatgcttt ccgactggta cgatttatgg ttttacacgc cggaactgcg acgtcctatg 360

cctatttcac ggcaacaaag attaattggg caaatctcta atggcgatgt cattgtcact 420

aactttgaat atgcctatga ctcgaccctg atgggggaag tcacgtgtgc tgaaaagcag 480

tgttacaagc tggcgctggt gcgtaaatcg gcggatatca cctggccgaa ggttatctat 540

tacgttgaaa aagacggtga taatcgcccg tggaaggcgg cctattattc gcaggatgac 600

cagttaataa aagaagtgtt gtatcaggat ttccagccgg tgttggggaa aacgcgaccc 660

atgaaaataa ccgttaccga tgttcgtcac ggcaataact attcagtgat ggagtacagc 720

gatgttcgtc tggaatcgct gccggagttt catttcacca aagagtatat tcagcgaggt 780

gcgaaatga 789

<210> 12
<211> 1389
<212> DNA
<213> Escherichia coli

<400> 12
atgaaacgta cgctgaagat ctcttcgttg ctgtgtgttg cgctgccgtt gacggtacag 60

gcggattgcc tgtcaggtga cgaggtggca caaaatagcg atgtgacgct ttacgagcag 120

gtgtcttatg tcaataaaca ggcttcggcc tggcagattg caggcaaaaa tccctacacc 180

cgtcacaacg gttatcagga ggcggggatt ggcatcaata gcggttgttc gataatcgac 240

aatacgctgg accttaagct caatctgtat ggaataaacg aatatgcgct taaacccgca 300

gggaaattcg aaacggatga tagccgcacg cgtgcgttaa ttaaccgcct cagtctggtc 360

tatagcgcct cggacagcgt tcaatttgaa gcaggcaaat ttgccgcgcc gtcaggaatg 420

ttctttttgc gttcgccttc tgatttgcaa acgcattatt acaccggatt ccagtcgacg 480

cgtttgcacg atcccaaaat gacatcggcg tatcaggcgt catcgtgggg cgcaaaaatg 540

agcgtggata cacgcgatta tgcgttttcg gcgagtgtca taccgaagtt agccaccatc 600

gacaaacgct atgtgacgtc tggcaactgg tcggctaatc agcagggaaa cagcgacgaa 660

gcctatctgc tgagcttcag cgatcatcgc tttggtgagc acacgccgac agtcaatgtg 720

cggctcggac catcgccgtc actcgcgctg tctgacagtt atcactacac cccgcagctt 780

acgctgaatg tcgacgcagc ctaccaccgc tcccagcaat ggcggcatct ttcccaccgt 840

gagacggcgc aggtcgagga atatcagttt ccggattcgc tctatgaaac gaaagatgaa 900

agcggcgtag agctggcgct gggtgggcag tacaccagtg ataatttcag cgtgtttggc 960

gttgagtatt actttcagag cgagggttat tcgcgcgctg aacagcgtca gcagcgcgag 1020

ttaattgact tcctcaatac cacaaccggc tatgcgccac tggatcaggc atttgattct 1080

tacaaatacc tgatggcttc agaaatcagc aataccgcca atcaggggat gctgcagggt 1140

aagcactatc tgaacgcctg ggccagtctg ccgctggcgg gagagtcgac gctgcagcct 1200

tcgctggtgg tgaatctcgt tgatggcagc acgttgctgg gcctgcatta ttccacgccg 1260

ctcagcgcta taagtaacca gcttgaagcc tatgccggtg gctatagcgc gctgggaagt 1320

cggtactccg aatttgcgct ttttggcgac acgttaggcc tttatttggg ctttaaatac 1380

tatctataa 1389

<210> 13
<211> 705

<212>  DNA
<213>  Escherichia coli

<400>  13
gtggggatcg acagccgtaa tgatattcct gaggggattg cgacgctggg cgcttttatg      60

ggttattccc attcacatat cggttttgat cgtggaggac atggcagtgt ggacagttat     120

tctctgggcg gctatgccag ttgggaacat gaaagtggtt tctatctgga cggtgtcgtg     180

aagctgaacc gttttgaaag taacgtagcc ggtaaaatga gcagcggtgg agccgccaat     240

ggcagttacc atagcaacgg gctgggcggt cacattgaaa ccgggatgcg atttaccgat     300

ggtaactgga acctgacgcc gtatgcctcg ttaacggggt tcaccgctga taaccccgaa     360

tatcatttat ccaatggcat ggaatcgaaa tcagtcgata cccgcagtat atatcgtgaa     420

ctgggtgcaa cgctgagtta caacatgcgt ctggggaacg gtatggaagt tgagccgtgg     480

ctgaaggcgg ctgtgcgcaa agaatttgtc gatgataacc gggtgaaagt gaatagtgac     540

ggtaatttcg tcaatgattt gtcgggcaga cgtggaatat accaggcagg tattaaagcc     600

tcattcagca gtacgttaag cgggcatctt ggggtggggt atagcaacgg tgctggtatg     660

gaatccccgt ggaacgcggt ggctggtgtg aactggtcgt tctga                     705


<210>  14
<211>  1260
<212>  DNA
<213>  Escherichia coli

<400>  14
   atgaaattcc cttcaatatt taacaaaata aaaccacaat ccatacagca acatccagaa      60
   aaaaatcaac ttaactggat gctcgaatta aataaatgga aagaagaacg tatacttaca     120
   ggtgaaatcc atcgtccgga atgtcgaaac gaagccgcta aaaggataaa ctgtgctttt     180
   ttgtcgaaac agaatgacat tgatttatca ggacttaatt tatctactca accaccaggg     240
   ctgcaaaact tcacctctat caatcttgat aataaccaac tcacacattt tgatgcaacc     300
   aactacgata gactcgtaaa acttagtctg aatagtaaca ctcttgagtc aataaatatt     360
   catcaaggca gaaatgtaag cattacacat atatctatga ataataattg tctcagaaat     420
   attgatatag ataggctttc atcaattact tattttagtg cggcacataa taaactagag     480
   tttgtgcaat tagaatcttg cgaatggctg caatacctga atctcagcca taatcaatta     540
   actgatattg ttacaggaaa taaagaagaa ctcttactgc tggatctatc ccataataaa     600
   ctagcaagtt tacacaatgc cttatttccc aacttaaata cgttacttat caacaacaac     660
   ttgctttctg aaattaaaat gttttatagc aacttctgca aagttcagac attaaacgct     720
   gctaacaatc agttggaaaa aataaacctt catttcctga cttatctttc atctatcaaa     780
   agtttaaggc tggacaataa taaaataact cgcattgata ctgagaacac atccgatatt     840
   agaagtttat tccccataat aaagaagagc gaaagcttaa attttttaaa tatttctggc 900
   gagaacaatt gccctactat ccagctcatg ttatttaatt tgttttcccc agcacttaag 960
   cttaatactg gcctggcaat tctttcgcct ggtgcatttg aagatcactc tgacggatta 1020
   gatgtggata acgaattgtt tcactatact attaataaag catatacccc atataatata 1080
   catacttata aaacagaaga agttgtaaac cagaggaata taaaaattaa aaatatgacc 1140
   ttagatgaaa taaacaatac ttattgtaat aacgattatt acaatgaggc aataagagag 1200
   gaaccgatag actttctgga cagatcgttt tcctccagct catggccttt ttatcactaa 1260

<210> 15
<211> 711
<212> DNA
<213> Escherichia coli

<400> 15
gtgaacaagg ccccctcttt aattgccgct atcgtcctcg gactggggat tagcgcctgt 60

ggttattttg ttggcgatgg tgttaaacat ttaaaaacca acaaccgtta tgtcaatgtg 120

cgcgggcttt ctgaaaaaga agttcgcgca gatacggcgg aattaacaat tgcgattaac 180

tttaagggta atgttcccgg cgaactgttc ccgaaactgg aagaagcgca gaaaaaaatt 240

gttgccgaac tcaatgctca ggggattaac gagaaagaga taattctggg ccaatggaca 300

agcaagcgaa cagattcctt ttatctgaaa gatgatccca caatgccacg ctataacgca 360

gacgggtcag tgacgataaa aacccataat gttgctgctg tcgaaaaagt cgtcgcgaaa 420

ttaaacgaac tgcaagtcgc tacggatggc gctatcgctg agagcaaagt cgcctatcgc 480

tttaatggta ttggcgcact gcgcgctgag atgattgctg ccgccaccaa agatgcacgc 540

aacgccgcac tacaatttgc aacggactca ggtagccagg tggggtcaat tagcgatgcc 600

tcacaaggcg tattccagat ctttgccagt ggcagtgatg aagatgatcc tacggcgatt 660

aataaaacgg tacgcgtagt cacgaccgta acctatgcgt tgcaggatta a 711

<210> 16
<211> 843
<212> DNA
<213> Escherichia coli

<400> 16
atgaaaaaat ggacgatatt tttgacaagt ctgatattac ttgctctcag tttagaaaca 60

ccaaagtgct atgccggtga taaactcatg agtgcaagct tttccagtac taaaatttat 120

tatgcgatga aaaacgttac tgcttccggc agtttatatt tctatgtcac ggtagtgacg 180

ccgggagaag taagctacgg acaatacaac tccaatgcaa gaaaaggcga caccctcaag 240

ttaatatcct ggagcggatc tggcccggca ccgacactgg ttttgactga ttatagacgt 300

acggacacct caaattgccc tggtatcaat actcgtgtct ttgtctgtgc atatatgact 360

tttaacgtca ctgtagaatc tgataattat ggttgtccgt ggattgcctc attttattca 420

gtatctgagg catttggatt tggtacgtat acaagcccaa cagttcatga ttctatttgc 480

ccgacaattc ccgtcgcttc gtttgatata tcctggagtg ataattatgt tagccataat 540

aaagcgctgc gattgcaatc cgatggttcg acaatcacga ctacgctttc gacctacctg 600

atggaaagcg gcaaactctg cgatggcagt atttttgatt ccagaggggc atattgtcgt 660

gctgtttctg acttattaac ctttacctcg tatggttgtg ataatgcaaa ggtgacagtc 720

accccctcac gtcaaccatt gacggataga aagctacacg acattgtggt acaggtgaat 780

accagtagcc gtgagccaat tgattctaca tgccgtttcc agtatgtact gaatgaactg 840

tag 843

<210> 17
<211> 912
<212> DNA
<213> Escherichia coli

<400> 17
atgggtacag cagctataag tcatttacga tatgatctta acaaatatgc cctttcttta 60

cgcaaaaccg caacacttgc ttcaacattt tttattgagt ccccattagt tagattcgag 120

tatctgcaag aaatagaaaa tactattaat gatattactc acagattcaa ttcttcatat 180

gacatcaatg aaaaagcacg acttatcaat gaattaaaaa tggagtcaga aacagcaaga 240

aaagaatatc aacttttcag acaaggaaat tatgataaat atatcactac tgatattttc 300

gaagaacatg gtttgataaa atatgtgaac ttaagtttcg atattgttgc atctgtagga 360

caggtcgtag gcggggttgg cgcgttgaaa tttggtaagg tagtgcattc aaaccgaata 420

aaaggtatag gcgtcacatt agttgctcat ggggcgaata atttttatga aagcttatca 480

cctttatttt ttaacgagta tgattcaggt ccaatacgtg aactctacag agtaatagca 540

aaaaaaatgg gaggtgatat taattctgct gattatgcat acagcatcgt ggacttttcg 600

attactgcat atggtggata ttcaggcatg aaaatcgtac ctaagtataa tagattgatt 660

cgtccttcat taggaaatag acctggtaca ggacgattat ttcattacac atcagttgat 720

tttaaaaata aattctcatt aaaacctacc cctttaaaaa tcatacaaat tagttctact 780

gtaaaaaaat tcaaggttac attttatgat gaaaatacaa atttgaggat aaaacatggc 840

ctcaataatc atatgccaca tcaattatta atgaatatat caggaactac aactggtgca 900

ggaatacaat aa 912

<210> 18
<211> 1065
<212> DNA
<213> Escherichia coli

<400>  18
ttgctgtcac tgacactgat gtctgcttta ttatcgcctt tatctcttca ggcagcggat      60

gtccggcgta gcggagatga agcatttatc attcagcagc agcgtcagga agcccttgag     120

caacaactga cgccttcagc ccctgatgtt cgcctttctg cacctggctc ttttgcccat     180

aagattaatt ttcctgttga aacgccctgt tttcagatta aacagacgga actgaagggg     240

gctgatgcgt taccacactg gctgccttta caaaaaatcg ccaacggggc ggtcgggcat     300

tgcctggggg cgaaaggaat taatctgctg atgagtacat tgcagaaccg tctggtcgat     360

catggttatg tcaccacccg tgttctggca ccttcgcagg atttaaaaag cggtatcctc     420

cggctggtta ttattcccgg tgttgtgcga catgtgcgtc tgacaccgga cagtgatgac     480

tatattcagt tgtattcctc attcccggca cacgaaggtt ctctgctgga tttacgggac     540

attgagcagg ggctggattt aggtaacagc cggatacagg gacaacatac tgagctgaat     600

gcaaccagtg gaaatctgtc tacacagaat gcgcaactga gtgccgatac gctttccgcc     660

cggactgccg ggcagttcag cagtaatggc ggtacgataa atgccgacac actgcagata     720

tcggcacaaa gcctgtcaaa tcgtaaaggc agtctgattc agacgggaac aggggatttt     780

tcgctgagtc tgccgggaag cgtggataac cgggaagggc tgcttgcggc aaatggcgcg     840

gtgcgtctgg atgcactgag ccttgataat cgcaagggga aagtgcaggc ggagcagtca     900

ccctcccttc agaaatcccc gcccacgttt ctgaaaccgt ttgtggctgg tgtctgtgcg     960

gcattgctgg cggtcagcgt ggctattccg ggatggcagt ttctgacaca gccatcaccg    1020

gaggagcagc attttacctg ggggaatggt tgtaaaaagc agtga                    1065

<210>  19
<211>  4200
<212>  DNA
<213>  Escherichia coli

<400>  19
atgagcggaa aaccggcggc gcgtcagggc gacatgacgc agtatggcgg tagcattgtt      60

cagggttcag ccggggtgcg cattggtgcc cccaccggcg tggcctgttc ggtgtgcccc     120

ggcggagtga cgtccggcca tccggtcaat cccctgctcg gtgcaaaggt ccttcccggt     180

gaaaccgaca tcgccctgcc cggcccgctg ccgttcatcc tctcccgcac ctacagcagt     240

taccggacaa aaacgcccgc gccggtgggg agcctcggcc ccggctggaa aatgcctgcg     300

gatatccgct tacagctgcg cgataacaca ctgatactca gtgataacgg cggcagaagc     360

ctgtattttg agcacctgtt tcccggtgag gacggttaca gccgcagcga gtcactctgg     420

ctggtgcgcg gcggcgtggc gaaactggat gaaggtcacc ggctggccgc actctggcag     480

gcgctgccgg aagaactccg cttaagtccg catcgttatc tggcgacaaa cagtccgcag     540

gggccgtggt ggctgctcgg ctggtgtgag cgggtgccgg aagcggatga ggtgctgcct 600

gcgccgctgc cgccgtaccg ggtactgacc gggctggtgg accgcttcgg gcgcacacag 660

acgttccacc gcgaagccgc cggtgaattc agcggcgaaa tcaccggcgt gacggatggt 720

gccgggcgtc acttccggct ggtactgacc acgcaggcgc agcgggcaga agaagcccgg 780

cagcaggcca tttccggcgg gacggaaccg tccgcttttc ctgataccct gccgggttac 840

accgaatatg gccgggacaa cggcatccgt ctgtctgccg tgtggctgac gcacgacccg 900

gaatacccgg agaatttacc tgccgcgccg ctggtgcgct atggctggac gccgcgcggc 960

gaactggcgg tggtgtatga ccgtagtggc aaacaggtgc gcagctttac ttacgatgat 1020

aaataccggg gccggatggt ggcgcaccgt cacacgggcc ggccggaaat ccgttaccgt 1080

tacgacagcg acgggcgggt gacagaacag ctaaacccgg caggcttaag ctacacgtat 1140

cagtatgaga aagaccgcat caccatcacc gacagcctga accgccgtga agtcctgcac 1200

acgcagggtg aaggcgggct gaagcgggtg gtgaaaaagg aacacgcgga cggcagcgtc 1260

acgcagagtc agtttgacgc ggtgggcagg ctcagggcac agacggatgc cgcaggcagg 1320

acaacagaat acagcccgga tgtggtgacg ggcctcatca cgcgcatcac cacgccggat 1380

ggcagggcat cggcgtttta ctataaccac cacagccagt taacgtcagc caccgggcct 1440

gacgggctgg aaatacgccg ggaatatgat gaatggggcc gtctgattca ggaaactgcc 1500

cctgacggcg atatcacccg ctaccgttat gataatccac acagtgactt accctgcgca 1560

acggaagatg ccaccggcag ccggaaaacc atgacgtgga gccgttacgg tcagttgctg 1620

agcttcaccg actgttccgg ttatgtaacc cgttatgacc atgaccgctt cgggcagatg 1680

acggcggtgc accgcgagga agggctgagt cagtaccgcg catacgacag ccgtggacag 1740

ttaattgccg tgaaagacac gcagggccat gaaacgcggt atgaatacaa cgccgccggt 1800

gacctgacca ccgtcattgc cccggacggc agcagaaacg ggacacagta cgatgcgtgg 1860

ggaaaagcca tctgtaccac gcagggcggt ctgacgcgca gtatggaata cgatgctgcc 1920

ggacgggtca tccgcctgac cagtgaaaac ggcagccaca ccaccttccg ttacgatgta 1980

ctcgaccggc tgatacagga aaccggcttt gacggccgca cacagcgtta tcaccacgac 2040

ctgaccggca aacttatccg cagcgaggat gaggggctgg tcacccactg gcactatgac 2100

gaagcagacc gcctcacgca ccgcaccgtg aagggtgaaa ccgcagagcg ctggcagtat 2160

gacgaacgcg gctggctgac agacatcagc catatcagcg aagggcaccg ggtgacggtg 2220

cattacgggt atgatgagaa aggccggctg accggtgagc gtcagacggt gcatcacccg 2280

cagacggaag cactgctctg gcagcatgag accagacacg cttacaacgc gcaggggctg 2340

gcgaaccgct gtataccgga cagcctgccc gccgtggaat ggctgaccta tggcagcggc 2400

tggctggcag gcatgaagct cggcgacaca ccgctggtgg atttcacccg cgaccgcctg 2460

caccggaaaa cgctgcgcag attcggccgt tatgaactca ccaccgctta tacccctgcc 2520

gggcagttac agagccagca cctgaacagc ctgcagtatg accgcgatta cacctggaac 2580

gacaacggcg aactcatccg catcagcagc ccgcgccaga cccggagtta cagctacagc 2640

gactccggca ggctgaccgg cgttcacacc accgcagcga atctggatat ccgcatcccg 2700

tatgccacgg acccggcagg taaccgcctg cccgacccgg agctgcaccc ggacagcacc 2760

ctcagcatgt ggccggataa ccgtatcgcc cgtgacgcgc actatcttta ccggtatgac 2820

cgtcacggca ggctgacaga gaaaaccgac ctcatcccgg aaggggttat ccgcacggat 2880

gatgagcgga ctcaccggta ccattacgac agtcagcacc ggctggtgca ctacacgcgg 2940

acacaatatg aagagccgct ggtcgaaagc cgctatcttt acgacccgct gggccgcagg 3000

gtggcaaaac gggtgtggcg acgtgaacgg gacctgacgg gctggatgtc gctgtcacgg 3060

aaaccgcaag tgacctggta cggctgggac ggcgaccggc tgaccacaat acagaacgac 3120

agaacccgca tccagacgat ttatcagccg gggagcttca cgccactcat cagggttgaa 3180

accgccaccg gtgagctggc gaaaacgcag cgccgcagcc tggcggatgc gcttcagcag 3240

tccggcggcg aagacggtgg cagtgtggtg ttcccgccgg tgctggtgca gatgctcgac 3300

cggctggaaa gtgaaatcct ggctgaccgg gtgagtgagg aaagccgccg ctggctggca 3360

tcgtgcggcc tgactgtggc gcagatgcaa agccagatgg acccggtata cacgccggcg 3420

cgaaaaatcc acctgtacca ctgcgaccat cgcggcctgc cgctggccct tatcagtaag 3480

gaaggggcaa cagaatggtg cgcagaatac gatgagtggg gcaacctgct gaatgaagag 3540

aacccgcatc agctgcagca gcttatccgc ctgccggggc agcagtatga tgaggagtcc 3600

ggcctgtatt acaaccgcca ccgctattat gacccgctgc aggggaggta tatcactcag 3660

gatccgattg gactgaaggg gggatggaat ttttatcagt atccgttgaa tccggttcag 3720

tatatagatt caatgggact ggcatcaaaa tatggacact taaataatgg cggatatgga 3780

gcgagaccca acaaaccgcc tacgcccgat ccaagtaaat ttccagacat agcgaaacaa 3840

ttaagactgc catatcctat tgaccaggcc agtagtgcgc ctaatgtttt caaaacattc 3900

ttcagagcat taagccctta cgactacaca ctgtattgca ggaagtgggt aaaaccaaat 3960

ctgacttgta cgccacagga tgatccccag tatccaggga tggatacaaa gacagcaagt 4020

gattacctgc cacagacaaa ttggccaaca actcaattac caccaggata tacttgtgca 4080

gaaccctatt tattccctga cattaataaa cccgatgggc cagcaacagc agggatagat 4140

gatttgggtg aaattttagc taaaatgaaa cagagaacat cgagaggaat aagaaaatga 4200

<210> 20
<211> 7863
<212> DNA

<213>  Escherichia coli

<400>  20
atggctgcgg cagcacaagg tgtggtaaac gccgcaaccc aacaaccagt tcctgcacaa      60

attgccattg caaatgccaa tacggtgccc tacacccttg gagcgctgga atcggcccaa     120

agcgttgccg aacgtttcgg tatttcggtg gctgagttac gcaaactcaa ccagtttcgt     180

acgtttgctc gaggttttga taatgtccgc cagggtgatg aactggatgt cccggcacaa     240

gttagtgaaa ataatttaac cccgccaccg ggtaatagca gtggcaacct tgagcaacag     300

atagccagta cttcacagca aatcgggtct ctgctcgccg aggatatgaa cagcgagcaa     360

gcggcaaata tggcgcgtgg atgggcctct tctcaggctt caggcgcaat gacagactgg     420

ttaagccgct tcggtaccgc aagaatcacg ctgggcgtgg atgaagattt tagcctgaag     480

aactcccagt tcgattttct ccatccgtgg tatgaaacgc ctgataatct ctttttcagt     540

cagcatactc tccatcgtac tgacgagcgt acgcagatta acaacggctt gggttggcgt     600

catttcactc ccacatggat gtcgggcatc aacttctttt tcgaccacga tcttagccgt     660

taccactccc gcgccggcat tggcgcggag tactggcgcg actatctaaa attaagcagt     720

aacggctatt tgcgactgac caactggcgc agcgcacctg aactggacaa cgattatgaa     780

gcacgcccgg ccaatggctg ggatgtacgc gcagaaggct ggctacccgc ctggccgcac     840

cttggcggta aactggtcta tgaacagtat tatggcgatg aagtggccct gttcgataaa     900

gatgatcggc aaagtaatcc tcatgccata accgctggac ttaactatac ccccttcccg     960

ctgatgacct tcagcgcgga gcaacgccag ggtaaacagg gcgaaaatga cacccgtttt    1020

gccgtcgatt ttacctggca acctggaagc gcgatgcaga aacagcttga cccgaatgaa    1080

gtcgatgcac ggcgtagcct tgcaggcagc cgttttgatc tggtggatcg caacaacaac    1140

atcgttctgg aatatcgcaa aaaagaactg gttcgcctga ccctgacaga ccccgtgaca    1200

gggaagtcag gagaagtgaa atcactggtt tcgtcgctac aaaccaaata tgccctgaaa    1260

ggctataacg tcgaagccac cgctctggaa gctgccggtg gtaaagtggt tacaacgggt    1320

aaagatattc tggttaccct gccggcgtac cggttcacca gtacgccaga aaccgataac    1380

acctggccga ttgaagtcac cgctgaagat gtcaaaggca atttttcgaa tcgtgaacag    1440

agcatggtag tcgttcaggc tcctacgcta agccagaaag attcctcggt atcgttaagt    1500

agccagacgt tgagcgcgga ttcccattca accgccacac tgacttttat tgcgcatgat    1560

gcagcaggta atcctgttat cgggctggtg ctctcgacgc gtcacgaagg tgttcaggac    1620

atcacccttt ctgactggaa agataatggt gacggaagct atacccagat cctgaccaca    1680

ggagcgatgt ctggcacgct gacgctgatg ccacagctga acggtgtgga tgcggctaaa    1740

gcccccgccg tggtgaatat catttctgtt tcgtcatccc ggactcactc gtcaattaag    1800

attgataagg accgttatct ctccgggaat cctatcgagg tgacggtaga actgagagat 1860

gaaaatgaca aacctgttaa ggagcaaaaa cagcaactga ataccgcagt cagcatcgac 1920

aacgtgaaac ctggtgtcac tacagactgg aaagaaaccg cagatggcgt ctataaggca 1980

acctataccg cctataccaa aggcagtggg cttactgcga agctgttaat gcaaaactgg 2040

aatgaagatt tgcataccgc tggatttatc atcgacgcca acccgcagtc agcgaaaatt 2100

gcgacattat ctgccagcaa taatggtgtg ctcgccaatg agaatgcagc aaacaccgtc 2160

tcggtcaatg tcgctgatga aggaagcaac ccaatcaatg atcataccgt cacgtttgcg 2220

gtattaagcg gatcggcaac ttcctttaac aatcaaaaca ccgcaaaaac ggatgttaat 2280

ggtctggcga cttttgatct gaaaagtagt aagcaggaag acaacacggt tgaagtcacc 2340

cttgaaaatg gcgtgaaaca aacgttaatc gtcagttttg tcggcgactc gagtaccgcg 2400

caggttgatc tgcagaagtc gaaaaatgaa gtggtcgctg acggcaatga cagtgccaca 2460

atgaccgcga cagttcggga tgcaaaaggc aacctgctca atgacgtcaa ggtcaccttc 2520

aatgtcaatt cagcagcagc gaaactgagc caaaccgaag tgaatagcca cgacgggatc 2580

gccacagcta cgctgaccag tttgaaaaat ggtgattata cggttacggc ctctgtgagc 2640

tctggttctc aggctaatca acaggtgatt tttatcggtg atcaaagtac tgctgccctg 2700

accctcagtg tgccttcagg tgatatcacc gtcaccaaca cagctccgct acatatgact 2760

gcaaccttgc aggataaaaa tggcaatcca ctaaaagata aagaaatcac cttctctgtg 2820

ccaaacgacg tcgcaagtcg gttctcgatt agcaacagcg gaaaaggcat gacggatagc 2880

aacgggactg caatcgcctc cctgaccggc acgttagcgg gcacgcatat gatcacggct 2940

cgtctggcta acagcaatgt cagcgataca cagccaatga cgtttgtggc ggataaagac 3000

agagcggttg tcgttctgca aacatcgaaa gcggaaatca ttgggaatgg cgtggatgag 3060

acgactctga cagcaacagt taaagatcct tttgataacg tggttaaaaa tctttcagta 3120

gtcttccgca cctcccccgc agacacgcaa ctgagtctga acgcgcgtaa tactaatgag 3180

aacggtattg ccgaagttac ccttaagggc acggttttgg gtgttcatac agccgaagcc 3240

atactgctta acggcaacag agatacgaaa atcgtcaata ttgcgcccga tgccagcaac 3300

gcgcaggtca ccctgaacat ccctgcacaa caggtggtga cgaataacag tgacagcgtg 3360

cagctgacgg cgacggtgaa agacccgtcg aatcatccgg tggcgggaat aacggtgaac 3420

ttcaccatgc cacaggacgt ggcggcaaac tttacccttg aaaataacgg tattgccatc 3480

actcaggcca atggcgaagc gcatgtcacc ctcaaaggca aaaaagcggg cacgcatact 3540

gtgaccgcca cgctgggtaa caataatgcc agcgatgcgc aaccagtcac cttcgtggcg 3600

gataaggaca gcgcggttgt cgttctgcaa acatcgaaag cggaaatcat tgggaatggc 3660

gtggatgaga cgactctgac ggcaacagtg aaagatcctt ttgataacgc agtaaaagat 3720

ctacaggtca ccttcagtac caaccccgca gatactcaac ttagtcagag caaaagcaat 3780

actaacgaca gtggtgtggc cgaagttacc tttaagggca cggttttggg tgttcataca 3840

gccgaagcca cactgcctaa cggcaacaac gatacgaaga tagtcaatat tgcgcccgat 3900

gccagcaacg cgcaggttac gctgaacatc cctgctcaac aggtggtgac gaataacagc 3960

gacagcgtgc agctgacggc gacggtgaaa gatccgtcga atcatccggt ggcgggaata 4020

acggtgaact tcaccatgcc acaggacgtg gcggcaaact ttaccctcga aaataacggt 4080

attgccatca cccaggccaa tggggaagcg catgtcacgc tcaaaggtaa aaaagcgggt 4140

acgcatacgg ttaccgcaac gctgagtaat aacaatacca gtgattcaca gccggtaacg 4200

tttgtggcgg acaaaacctc ggctctggtt gttcttcaga tatcaaaaaa tgagatcaca 4260

ggtaatggcg tcgatagcgc aacgctaact gcaacggtca aagatcagtt cgacaatgag 4320

gtgaacaatc ttccggtaac attcagcaca gcttcttcag gcctcaccct gaccccaggg 4380

gaaagtaata ccaatgagtc tggcatcgcg caggccactc tcgcaggcgt tgcctttggt 4440

gagcagacgg tcactgcatc actggctaat aatggtgcca gcgacaacaa aactgtgcat 4500

tttattggcg acacagcggc ggcaaaaatt atcgagttga cgcctgtccc agacagcata 4560

atcgcaggta ccccgcagaa cagctccggc agcgtcatca ccgccacagt cgttgataat 4620

aatggctttc cggtgaaagg tgtgactgtg aacttcacca gcaacgcagc gacagccgaa 4680

atgacgaatg gcggtcaagc cgtgacgaac gaacagggta aggctaccgt cacttatacc 4740

aatacccgct cctcgataga atcaggagcg agaccggata ccgttgaggc cagtctggaa 4800

aatggtagct ccacgcttag cacatcaatt aatgtcaacg ctgatgcgtc tacggcacat 4860

ctcaccttgc tacaggcact tttgatacaa gtctccgcag gcgacactac caatctgtat 4920

attgaggtga aggataatta cggcaacgga gtaccccagc aggaggtaac cctcagcgtt 4980

tcaccaagtg aaggtgtgac ccccagtaat aacgctatat atacgaccaa tcacgacggc 5040

aatttttacg caagctttac cgctacaaaa gccggggtat accaagtgac ggcaaccctc 5100

gaaaatggcg attcgatgca acaaacagtg acctatgtgc cgaacgtagc gaatgctgaa 5160

atctcgctgg cagcctcgaa ggatccggta attgccaaca ataacgatct cacgacacta 5220

acagcaacag tcgctgatac agagggcaat gcgatagcca acagtgaggt aacatttact 5280

ctgccggaag atgtgagggc gaacttcacg ctgggcgatg gcggtaaagt ggttactgat 5340

actgaaggca aagcgaaagt cacgctgaaa ggtacaaaag caggcgctca tactgttaca 5400

gcatcgatgg ctggcggtaa gagtgagcag ttggtggtga actttattgc ggatacactc 5460

actgcgcagg ttaatcttaa cgttaccgag gacaatttta tcgctaataa cgtcgggatg 5520

accaggctgc aggcaacagt gactgatgga aacggcaacc cgttagccaa tgaggcggtg 5580

acattcacgc taccggcaga tgtgagcgca agctttactc tcggacaagg cggttccgcc 5640

attactgaca tcaacggcaa ggctgaagtt acactgagcg gtacaaaatc cggcacctac 5700

cccgtgacag ttagcgtgaa caattatggt gtcagtgata cgaaacaggt gactttgatt 5760

gccgatgctg gtaccgcaaa actagcctcc ttaacctctg tatactcatt cgtcgtcagc 5820

acgaccgagg gcgcgaccat gactgcaagc gtcactgacg ctaacggcaa cccggtagaa 5880

ggtataaaag ttaatttccg cggaacttcc gtcacgctaa gcagcaccag cgttgaaacg 5940

gatgatcggg gtttcgctga aattcttgtg acaagcaccg aggtcggact gaaaacagtt 6000

tcagcctctc tggcagataa acctactgaa gtcatctcgc gattactgaa tgcaaaagca 6060

gatattaatt ctgcaacgat taccagtctg gagatacctg aaggtcaggt catggtcgca 6120

caagacgtag cagttaaagc tcacgtcaac gaccagtttg gcaatccgat tcttaatgaa 6180

tctgtaacat tcagtgcaga accaccagag cacatgacca tcagccaaaa tattgtctct 6240

actgatacgc atggtatagc cgaggtcact atgacgcccg aaagaaacgg ttcgtatatg 6300

gtgaaagcat ccctggcgaa tggatcctct tatgagaagg atctggtggt aatcgatcaa 6360

aaactgacac tctcggcgtc cagcccgctt atcggtgtca attccccaac aggtgcaact 6420

ctgacggcaa cgctaacttc tgcaaatggc actccagtgg agggtcaggt catcaacttt 6480

agcgtaacgc cagaaggtgc gacgttaagt ggcggaaaag tgagaaccaa ctcttcaggt 6540

caggctccag tcgttctgac cagcaataaa gtcggtacat atacggtgac tgcatcgttc 6600

cataacggcg taacaataca gacacagaca atcgtgaaag tcactggcaa ctcaagcacc 6660

gcccatgttg ctagctttat cgctgatcca tcgactatag ccgccaccaa cagtgattta 6720

agtaccttaa aggcaacggt tgaggatggc agtggtaacc tgatcgaagg tctcactgtg 6780

tacttcgcct taaaaagcgg ctctgccaca ttaacgtcat taacagcggt gacagatcaa 6840

aacggaatcg cgacaacaag cgtgagagga gcgataacgg ggagcgtcac ggtaagcgca 6900

gtcacgaccg ctggtggaat gcaaacagta gatataacgc tggtggcagg cccggcagac 6960

gcctcgcagt ccgtccttaa gaacaatcgg tcatcattga aaggagactt taccgatagt 7020

gctgagctac atcttgttct gcacgatata tcaggcaatc cgatcaaagt ttctgaaggg 7080

ctggaatttg tgcagtcagg taccaacgcg ccctatgtgc aagttagtgc aattgactac 7140

agtaaaaatt tctcaggcga gtacaaagcc actgttacag gcggcggaga gggtatcgca 7200

acgctgatcc ctgtattgaa tggtgttcat caagcgggtc tgagtaccac aatacaattc 7260

actcgcgcag aagacaaaat aatgagcggt acagtgttag tcaatggtgc taacctaccg 7320

acaactacat tcccttcgca ggggttcact ggggcgtatt atcagttgaa taatgacaac 7380

tttgccccag gaaaaacggc ggctgattat gagttttcaa gctctgcctc ctgggttgat 7440

gttgatgcta ccggtaaagt gacatttaaa aatgtcggca gcaaatggga gaggattacg 7500

gcgacgccaa aaacaggcgg ccctagctat atatacgaaa tccgagtgaa gagttggtgg 7560

gtgaacgccg gcgatgcttt catgatatac agccttgctg aaaatttttg cagtagcaat 7620

ggctacacac ttccccttgg agaccattta aaccatagtc gttcccgagg catcgggtca 7680

ctgtacagtg aatggggaga tatggggcat tacacgactg aagctggttt tcattcaaat 7740

atgtattggt catcgagtcc cgcaaactca aacgaacaat acgtagtttc cctggcaaca 7800

ggtgatcaaa gcgtatttga aaagcttggg tttgcttatg cgacatgtta taaaaacctc 7860

tga 7863

<210> 21
<211> 5037
<212> DNA
<213> Escherichia coli

<400> 21
   gtgccttaca cgcttggtgc gctggaatca gcgcaaagcg ttgctaatcg cttcggtatt 60
   tcactggagg agcttcgtcg tcttaatcag ttccgtactt ttgctcgcgg ctttgataac 120
   gtgcgccagg gtgaagaact ggatgttccg gcaacaacct cgcagaaaag ccatgagcaa 180
   caaaatgccg taccgcctgc gaatggcgaa aacacgctgg agaatcaaat agccagcacc 240
   tcgcagcgag ttggcactct gctttcacaa gatatgaata gtgagcaggc cagcggcatg 300
   gcgcgtggtt gggcgtcttc agaagcctca ggcgcgatga ctgattggtt aaataacttt 360
   ggtactgcga gaatctctct gggtgtggat gaagatttta gcctgaaaaa ttcgcaattc 420
   gacttcctgc atccgtggta tgacacacct gattatctgc tcttcagcca gcataccctt 480
   caccgaacag acgatcgtac ccagatcaac accggtttgg gctggcgtca tttcacctcc 540
   agctggatgt caggcatcaa cctttttttt gaccacgacc tgagccgcta tcactcccgc 600
   gcagggcttg gcgcagaata ctggcgtgat tatctgaagt tgagcagcaa cgcttatatc 660
   ggcctgaccg gctggcgtag cgcaccagaa ttggataacg acttcgaagc ccgcccggcc 720
   aacggctggg atttacgcgc ggaaggctgg ttacctgcct ggccacaact ggggggaaaa 780
   ctggtctatg aacaatacta tggcgatgaa gtggcgctgt ttgacaagaa tgatcgtcaa 840
   agtaaccccc atgctattac cgcaggcctc aactataccc ccttcccgct tctgactctc 900
   agtgcggaac agcgtcaggg gaagcaaggt gaaaatgaca cacgttttgc cgttgatctg 960
   acctggcaac ccagcagttc aatgcagaaa cagcttaatc cggacgaagt ggccggacgg 1020
   cgcagtctgg ccggtagtcg ttatgacctg attgatcgca acaacaacat cgttctggaa 1080
   taccgcaaga aagagctgat tcgcctgagt ctgctggatc cggtgaaagg gaagtctgga 1140
   gaaataaaac cgctggtttc ctcgctacag accaaatatg cccttaaagg ctataacatc 1200
   gaagccgctg cgctggaagc tgccggaggt aaagtcagca cgtctggaaa agatatcacg 1260
   gtcacgctgc caggttaccg cttcactaac accccagaaa ccgataatac atggtcgata 1320
   gacgttaccg ccgaggatgt aaaaggtaac ctgtcacggc atgaacaaag catggtagtt 1380
   attcaggctc cgacattaag ccagaaagat tctctgttat ccgtcaatcc gctaaccgtg 1440
   gctgcagata aaaaatcgac gaccacattg accgttactg cgcacgattc cgacggaact 1500
   ccggtgccgg ggctggcgct gcaaacccgc agtgaaggcg ttcaggatat caccctgtct 1560
   gactggacag ataacggtga tggtagttac acacagatac tgaccgccgg aacgacatca 1620
   ggttcagtaa cactgacgcc gcaaattaac ggtgagagtg cggtaaaaga atccatcgtc 1680
   gttaatatcg tccctgttgt ctcatcccgc gaccattcat caataacaat tgataacgta 1740
   tcgtattatg ccggagacga catcaaggtt agggtggaac tgaaagacga tagcaatcaa 1800
   ccggttgcat atcaaaaaga ggaattggta aaagccgtta ctgtcgaaaa cagcaaacct 1860
   ggcgccacga ttgtctggca cgaagagcag ccgggggttt atgccgcgaa ttatccggcc 1920
   tataagcaag ggactgcact aagggcacaa cttagccttc acaactggaa tgctccactg 1980
   caatcgcata tttataacat tgaggcaaac cagaataagg ctcgcgttgc cacattatca 2040
   gcgacaaata atgacgttta cgccgataaa aagacattta ataccctcac gatcaacgtc 2100
   actgatgaga gtgataatcc cctgacaaat catcaggtca cctttaagaa tgaaaaagga 2160
   agcgcggagt ttgtcgaacc gccgcagcaa aatacggatg catatggtgt tgccacaata 2220
   aacatggtaa gtcaggttgc ggaagaaaat acgattagcg ccacgctgcc aaatggtttt 2280
   tcacaacgga taattgcgaa attcgttagc gattcgagta cgccaaaatt caaacaactg 2340
   gttgccgatc cagataccat tattgctggc aacagccagg gcagtactct gaccgccatc 2400
   atcacagact ttcataacaa cccgttaaaa gatatgaaag tgaattttgt ggcacctggt 2460
   ggctcgcaac tggacaacac gaccgccaca acagaccagt ccggtattgt gcgggtgcac 2520
   ctgaccagtt caaaagctgg tagctattcc gtcgatgcct cgcttgaggt ggataaaaat 2580
   attcaccagt cggtcacgat caccgtggtc ccaaacaggg aacaatcggt aatgaccttg 2640
   aatgccgggt cgggcagtgc gatcgctaac aatacaaata tcgttaccct gactgccagt 2700
   gtgaaagatg tttatggaca cccgttgccg gatgaggatg tgaaatttac cttgccagcc 2760
   tccatgaccg ggaacttcac gctaagtagt gaaaccgccc gcaccgatgc aaacggtgat 2820
   gccgtggtca cattgcgagg cacaaaagcg ggtgagttta cagttacggc gacgctgacc 2880
   agaaataata ccgttgctta tcagcaagtc acttttattg gggatacaaa cagtgcgcag 2940
   ctccagccgc tgactgcctc attaaattcc attgttgcgg gtaacagtac ggggagtacc 3000
   ctgacggcaa cgatcctgga cgcttaccaa aatccgctta aagaccagtt ggtcactttc 3060
   cagagtaacg atgtcactct aagcgaaaca gaagtcacca ccaatacgct gggtcaggcg 3120
   acggtaacaa tgaccagcaa tattgccgga caacataacg tcgtggtgag ccggaaagcg 3180
   caagcttccg ataataaaac gtttagttta tcagtgctac cggatgaaag ttcggcgaag 3240
   gtaataagta taaccggagc cgaaaaaacg ataacggtgg gcgaaaacat cacgctacgg 3300
   atactcgtcc aggacgcgtt taacaatgta atcgcgggtc aacgcgtcag attaagtgcg 3360
   cagccaacaa ctaacattac gataggcgat acggcttaca ccgataataa cggttatgcg 3420
   tacgttaacc ttctcagcac ccaacctggg gtttatcagg tgacggcaac gctggacaat 3480
   aacagtagta gtaaggttga cgtgaatgtg gcaaatggca aactcgagtt aacatcatcg 3540
   aaaccagaaa ctacggtcca taatagtgag ggtattacgc tgaccgcaac ggcgagaaat 3600
   gcgcggggtg aattgatgcc agggcaaatt atcaccttta gcgtaacgcc tgaaggtgca 3660
   acgctaagca atacagggga agtccttact gaccagtcag gtcaggccaa agtgacgctg 3720
   accagtgaca aagtgaatgt ctataccgtt acggccataa tgggcaaaga tgttcccgtt 3780
   cagagccagg taacggttgc ggttaaggca gatgctaaaa cggcacatgt tgtgagcgtc 3840
   gtggcttctc ctgacaccat caccgccgac ggcatcgata gcagcaccat cacttcacga 3900
   gtagaagatg attacggatt cccggttgaa ggtgtcgata ttagtcatgg cttagacacc 3960
   aaaggcagcc cggtagttaa tattccaact acgcgtaccg atcagtccgg gcaagtcacg 4020
   gcgacaataa ccagtacatt ggcagaaacc ttaacagtca atgtgcaagt tcctggcaca 4080
   gccaaccaat ccgcaaccat tacattggtt gccggcacgg ccgatgaaag taagtcaatt 4140
   ttgaaatccg atgttgacac tctgaaggct gactaccagc agagcgcaaa acttacgcta 4200
   acattgcaag acaagtacgg taacccgata gtgacgtctg atcatctgga atttgtccag 4260
   tcaggcccct tcgtgaactt tctcaagttg agcgatattg attacagcca aagaaattat 4320
   ggcgagtaca ccgtgactgt cactggcgga aaagagggaa cagcgacact cattcccatg 4380
   ctgaacgggg ttcatcaggc aaacttaagc atatcgctga atctcatcca atcgataaaa 4440
   gaaatgtccg gtcatgtcac tgcaaacaac catacctcct ccacggctaa attcccgagc 4500
   gaaggctttg caggagcgta ttacacactc aacaatgata actttgaagc gggtaaaacc 4560
   gttgatgatt atatgttttc aagttcacag ggttgggtgt ctgtcgatgc ttcgggtaaa 4620
   gtttctttcg caaatatcgg cgatcaaacg tcagtcacaa taagcgctgt tccccgacaa 4680
   ggaggtacaa cctaccagac cttaattaag ctgaaaggct ggtgggtgaa taatggaaat 4740
   cataccaata tctggctagc tgccaatgcg ctctgtcatg ctaaaaatga tggatataat 4800
   cttcctggca tcacacattt gacgtctggc gaaaacaaac gcacgcaggg atcactgtat 4860
   ggtgaatggg ggaacgttgg agcgttttcc agtaattcgc aatttacacc gggagcttac 4920
   tggacaagtg aatctgatga ttacagtcgg cactactatg tgcagatgct aaccggtatg 4980
   accggaagcg acgctgattc cagcccccaa ctgaccgcct gccgtaaatc actttaa 5037

<210>  22
<211>  2409
<212>  DNA
<213>  Escherichia coli

<400>  22
atggtcgcta aattaaaacc agatgttttt gttaatacta atcctttttt agaagcaatg      60

tataaggaga ggcttagtca taaaggctat tctgataaaa ttgctttaag tattaatgat     120

aagaaatata atgtaaactc taaggatatt gaaaatattc ttgatggcaa aggggattta     180

tttaaaaaac gtacactgtg ggagtttgtt cgcgatcttt ttcctggctc tcatattaaa     240

gaagtcaaag gcttaattta cgaatttgtc acgaaagttg ataataaagc cgaggtgttc     300

gataagatta aatctctggc aaaaaaagaa caacaatggc gatttagtac taagacagac     360

tttaccacaa atgaaaataa tgaagttatt gtgagtagat cctttaatct ctacacgggg     420

gctacttcga atgataacga aaaaaaacag gtttcttcag agagattaac gcttgataat     480

tatctcgatg atttacattt tgataattct cccttggtga gattaacgtt tgataattat     540

tctgtaaaac ttgcgacgct gattaaaaat aaaattccta ttattaatac aacaatcaat     600

ttatcatcgt tgtctaagga tgttttaaat tcattaaaat attgtagttt taagaatgta     660

attttttcag gagtgataaa cagccctaac cttgaaggtc cggtttttga aaattgctat     720

ttcgatgatt gtaaattcaa caacatacag ctttatgaga ctaatgatga tactgtggaa     780

tcaggaaata aaaaaccaat aaaaggtatg tttaaaggtt gttttatttc taaatgcaaa     840

attgaaaatt atagatgtga gacgtctaaa atctacaccg ttacacaacc tgatagtatt     900

caagaaaaat taggttctta tctctttatg cagtctttcg ttcaggattg tataatccag     960

ggaggatgtt gtcctggctc gagtatactt ctgagtcatt tttataactg taatataata    1020

gggcttgatg ctcatggaat ggattttttg gcaaattcgt tcaacaagtc taattatgat    1080

gaaatcaggg gtccggatac ggggactgtg ttttataatt gtaatttgaa gcatattaaa    1140

ataaacgatg gtcttcgtga agatggttca gatagaacta tgtttaaccc caaagagtat    1200

ttttctgatt acattgaaag aagatcgaga aatcttaagt tagatgataa tttttcgaaa    1260

tgcgtagaga gtataaagaa agggaccgat tcttcattta gcaagaaaaa tacagagaac    1320

aataatgtct tctttaaaaa tagtaatttg attggggcaa atttaggcta ttattacagt    1380

ggagataggt gcaaagactg tgctattgat ccaaatacaa attattcaaa agatggtctt    1440

acagcgcaaa aatacatgta tatggatctg gacggagcta taagtaaaga acaaatacct    1500

gatttcttaa aaaaggtaag tgctatcaat atcgatgtaa taaattttta taattccgag    1560

tcaaagacta aggaaaaata taaaaacgca tttttagagt tagagtcgtt tttatcaaca    1620

ctttatgttg aaaataaatc gtacgaggaa aaatatcatt ttgataatag tagggttgag    1680

ttttttattt ttaaagatat gcaggaaaat gcacaaaata tcattaacaa tatgatagaa 1740

agtgaccgga ttaagtttgt tgaatctata ataaataaaa tgattccgcc gccagatgga 1800

acaatactta ctgaaaatcc aaaagaatat gtccaaaagc aaataaagga gtctcataaa 1860

gaatctgcta caaatgtaac atttgattat aaggaacttg cacccatttt tgaaggtgtt 1920

gaagaaaaac aaattaaagc actatcaaac caactggaac atataaaatc gtttaagaca 1980

gattacgact caaggctcaa taagtatgct cgcgattttt actatttttc cagtgcattt 2040

gcaattaact gggaggattt actaaaaaac tatcaggaaa taagagcttc cattaaagat 2100

tacgatgact tattgcgaga aattaaagag ttaatcataa gtcgtaataa atcattagag 2160

gataaaagga ctttatttga taacaaaagg gacaattgga atagtataga agtgcaggat 2220

gaagtaaatg cattgaatgc gaaaattgtc gattgtgatg ataaaatacg ttctaagctt 2280

actattgttc gaaataatag acttgagaat caatataaag acgataaaaa catcagtgac 2340

gcgatgcgta atattctgga ttggttcgaa cggtatcccg atattgtaca aaatataacc 2400

caggcttaa 2409

<210> 23
<211> 2451
<212> DNA
<213> Escherichia coli

<400> 23
atgttaagga cgacacgatg ggtcgctgct attatttttt tgtattcttt tccgggttat 60

gctgaagaaa cttttgatac gcactttatg atcggcggca tgaaaggaga aaaagtttcc 120

gaatatcatt tcgataataa acaaccgctt ccaggaaact acgaacttga tttttatgta 180

aataaccagt ggcgtggaaa acaagatatc acgatcccag agtcaccagt caaaccatgt 240

ctgcctaagg tgctactaac caaactgggg gtaaagaccg gtaacctcaa tacagaagat 300

aattgtattt tactggataa agcggttcat ggtggacaat atcagtggga tatcagtgaa 360

caccgactaa atttaacagt accacaggcg tatatcaatg aacttgaaag aggttacgtt 420

cccccggaaa gttgggatcg cggcattgat gctttttata cctcctataa tttaagtcag 480

tatcgctctt atgacagcaa taataacagt aatacagcca gttatggccg atttaacagt 540

ggattaaatt tatttagttg gcagttacac tctgatgcca gctatagcaa acccgatgat 600

atgaaaggca catggcaaag taatactctt tacctggaac atggctggtc gcagatatta 660

agcacagttc agataggaga aaattacaca tcctcactca tattcgactc tctgagattt 720

agcggaatac gcttatttcg cgatatgcaa atgctacctg attctatgca gagctttaca 780

cctttagtac aaggtgttgc gcaaagtaat gccttgataa cggtttcgca aaatggctac 840

atcatttacc aaaaagaggt tcccccggga ccatttacta tcgcagattt gcaactctcg 900

ggcagcggct ctgatctcga tgtcagcata aaggaagcag atggcagtgt tcgttccttt 960

ttggtgccct actcttccgt gcctaacatg ctgcaacctg gtatttcaaa tttcgatttt 1020

atcgccggac gcagtaagat atatggtgta aagaatcagg aggactttct cgaggcaaat 1080

tatatatatg gcctcaacaa tcttttgaca ctttatggcg gtacgatttt atccgataac 1140

tataatgcta taactctggg aaatggttgg aatacaccgc tgggtgccat ctcttttgac 1200

gctacacgat cgagtagtaa gttaaataat gatataacac acgaaggtac gagctatcag 1260

gtcgcctata ataaatatct agtacaaacg gccactcgat ttagcgtggc cgcctggcgt 1320

tatgcttcgc aggattacag gacattcagc gaccatcttt acgaaaatga taaaataaat 1380

catcagagtg actatgacga tttttatgat attggcagaa aaaatagcct ttctgccaat 1440

atcatgcaac ctttatccaa taatctggga aatgtatcat taagtgcgct ttggcggaat 1500

tactgggggc gaagtggaaa tgctaaagat taccaattca gttattccaa tagctggcaa 1560

cgcatcagtt atactttctc tgccagccaa tcctatgatg aaaacgacaa agaagaggag 1620

cgttttaatc tgtttatctc cattcctttc tactgggggg atgatattgc caaaacacgt 1680

caccaaatta acttatcgaa ttcgacctca ttttccaaag atggctattc ctccaacaat 1740

actggaatta ctggcatagc cggtgaacat gatcagttaa attatggtat atatgttaat 1800

cagcaacaac aaaataatga tacatcgctt ggtacgaatt taagctggag aactcccatc 1860

gccacaatag atggcagcta tagccattct aaaaacgcct ggcaaagtgg tggaagtatt 1920

agtagtggat tagttgtctg gcccggtggt attaatatca ctaaccagct atccgataca 1980

tttgcaattc tggatgcgcc tgggttagaa ggcgcgcata ttaatggaca aaaatacaac 2040

cgaacaaaca gcaaaggcca ggttgtttac gacctgatga tacctcatcg tgaaaaccat 2100

ctggtacttg atacagcaaa cagtgaaagt gaaacagaat tgcagggcaa tcgtcaaatt 2160

attgcgcctt accgtggagc agtttcttat gtgcagttta caactgacca acgtaagccc 2220

tggtatatac aggcactgcg tcccgatggt tcgccattaa cttttggcta tgacgtactg 2280

gatctccagg aaaacaatat tggagtcgtt ggccagggta gtcgcctttt tattcgcgta 2340

gatgaaattc caactggcat aaaagttgct ctcaatgatg aacagaattt attctgtact 2400

attacttttc aacacgttat cgatgaaaac aaaacatata tatgccagta a 2451

<210> 24
<211> 2166
<212> DNA
<213> Escherichia coli

<400> 24
atgacggcag atgagcgcaa cgtggttaaa gcggcaaccg acgatagtat ggaagctgct 60

tatatgctaa aagataatat ccgctggtat tatcataatg gtgacctatc attacccgct 120

aacttcagca ataaaaataa actggtggtg aacggtaatt taacgatttc cggcgattat 180

gatgattatc tgtcaggtaa tggtcatctg attgtattag gtaatgttat tgtcgataac 240

tttattaatc atgatttcgc gtatgtcaaa ggacagatga cagcgaaagg tttagtttat 300

gctgattaca atgatcataa ctttgaagtg atgaagggaa ttagtgcgcg aggcattatt 360

gtttccgata aagcaacgca atttgaagtc attaaagctg aattttatat caatgaagat 420

ggaagtggtg aaggatataa ctgggatgag aatattcaaa aagcctattc actggtgact 480

gccgatcttt atgatcacac tgaaatcgaa accgataaca tttccaatgc gtatcctgac 540

tatgatagcg tcgcagacaa catcgttcag ggtttaccgc tttttcgaga taaagccgct 600

cctgaaatta atgagaaatt aaaatggatt gaaacgggga aactcgataa tttcccagcg 660

aataaaataa agcatcaaga tccactggtg gcacgttttc tgactcatac agagagttta 720

tcacccgccg ttatgctgca actgttacag caccccgatg accagacgcg tgaatcgatg 780

gcgcagtcct ggcccgctca acagatgcat tggcttaccg atgaattaat taaagatgag 840

gctgttgcca gagggctggt taaaaacagt aatatttccg ccgatgtgaa taaaaaatta 900

atgtctgtac ccgtcgagag cgtacagctg gagcaggcca gacaggataa tctttctcca 960

gatattgttg cgtcgttaag tcatagcccc tttctcagtg tacgcaagac attactcagc 1020

cattacgatt acgcctggct ggttccgact gcagtggccg atgagttgat aaataacgaa 1080

gacccggagt tacgtgaaag aattaccgga gcagatttaa ccgcacaaca agcagtgatg 1140

ttgagtaaag ataaatcact gaaagttcgt gaagcattag cccggacgct tactgaatta 1200

aaaattaccc aactgtcagc aaccctgcgt acagaggata ttgaacgtat cgcagaacaa 1260

atgtatctgg ataataaaga aaataaaaat atcgtaaagg tgttattgat agcgttgccg 1320

gagatgcgtc aactgtctct ggcaaaagaa gatgtgcaca atttgcgcga aggtgcgcgt 1380

tatctgacat cgaaggacgt gattagttat ctattgactc agcatgatgt tcccaccgtc 1440

tgggatgagc tggcacgcga taaattattg ccgctggaat ataagaaaca gttatggcaa 1500

cgcacattaa atttaatgat gagcaaacgg caagaagatc aagagcaagc ctacgaagta 1560

cagctggcgc tgattgataa tggtgttgtt gacgaagaga tgttaaataa tgccattgat 1620

ttactggttg atttacccgc tgaataccgc tatcgaatgc gtaaccaatt atttgataat 1680

aaagaattac cttccggaat tatcaacaaa ctcgatcaac agtatcgctt caactctgac 1740

tgggcgctgg ctgttgtcag tatgaaaaat agcacccgta gacaaagtga aagaggatta 1800

caccgctgga accatgagga tagcgatatt tttgcggaac tggcgacgat aaaagataaa 1860

tcagatgatg aatggtggcg tgcattgctg caaagtcgca atgatcattt gcgccaaacg 1920

gcattacgca acgcacatac cccggcgtcg ttgttaacga cattgactga gtctcaggat 1980

cggtcgctgg ctattaataa tccacagctg gctgccgatg tgaaaacggt gtggctaaaa 2040

gaggatccat cattactttt atttgtcgat aaacccgatc tttcgcagtt acgggattta 2100

gtgaaaaccg gggcaacgcg gaaaattcgc aatgaagcgc gtcaccggct tgaggaaaaa 2160

caatga 2166

<210> 25
<211> 319
<212> PRT
<213> Escherichia coli

<400> 25

Met Lys Lys Ser Val Ile Ala Gly Val Phe Ile Ala Leu Ser Phe Thr
1               5                   10                  15

Thr Cys Ser Ala Ile Ala Asn Ser Leu Ala Leu Ser Leu Ala Asn Asp
            20                  25                  30

Asp Ala Gly Lys Phe Gln Pro Ile Leu Asn Asp Ile Tyr Gly Asn Lys
        35                  40                  45

His Glu Asn Arg Asp Asp Tyr Ser Gln Gly Leu Phe Leu Gly Tyr Ser
    50                  55                  60

His Asp Ile Ser Asp Ser Ser Gln Leu Ser Leu His Ile Ala Gln Asp
65                  70                  75                  80

Ile Tyr Ser Pro Ser Gly Ser Asn Lys Arg His Asn Thr Ala Val Thr
                85                  90                  95

Gly Asp Arg Ala Phe Ser Ala Tyr Thr His Thr Gly Ile Glu Trp Asn
            100                 105                 110

Ser Leu Ala Asn Asp Trp Ile Arg Tyr Arg Leu Gly Thr Asp Ile Gly
        115                 120                 125

Val Val Gly Pro Asp Ala Gly Gly Gln Lys Val Gln Asn Lys Ala His
    130                 135                 140

Glu Ile Ile Gly Ala Glu Lys Tyr His Ala Trp Asp Asp Gln Ile Glu
145                 150                 155                 160

Asn Arg Tyr Gly Tyr Thr Val Lys Gly Met Leu Ser Met Thr Pro Ser
                165                 170                 175

Met Asp Ile Leu Gly Ala Asn Val Gly Leu Tyr Pro Glu Val Ser Ala
            180                 185                 190

Val Thr Gly Asn Leu Phe Gln Tyr Val Ala Tyr Gly Ala Thr Ile Ala
        195                 200                 205

Ile Gly Asn Asp Lys Thr Phe Asn Ser Asp Asn Gly Phe Gly Leu Leu
    210                 215                 220

Ala Pro Arg Gly Leu Met His Met Ser Asp Thr Ser Gly Phe Lys Tyr
225                 230                 235                 240

Lys Ile Phe Ala Gly Met Glu Arg Arg Asp Val Asn Arg Asn Tyr Thr
                245                 250                 255

Leu Glu Gly Lys Thr Ile Gln Thr Lys Gln Thr Thr Val Ser Leu Asn
            260                 265                 270

Lys Thr Val Asp Glu Tyr Gln Val Gly Ala Thr Ile Gly Tyr Ala Pro
        275                 280                 285

Val Ala Phe Thr Leu Ala Phe Asn Lys Val Thr Ser Glu Phe Lys Thr
    290                 295                 300

Gly Asp Asp Tyr Ser Phe Ile Asn Gly Ala Ile Thr Phe Phe Phe
305                 310                 315

<210>  26
<211>  266
<212>  PRT
<213>  Escherichia coli

<400>  26

Met Asn Ser Lys Lys Leu Cys Cys Ile Cys Val Leu Phe Ser Leu Leu
1               5                   10                  15

Ala Gly Cys Ala Ser Glu Ser Ser Ile Asp Glu Lys Lys Lys Lys Ala
            20                  25                  30

Gln Val Thr Gln Ser Asn Ile Asn Lys Asn Thr Pro Gln Gln Leu Thr
        35                  40                  45

Asp Lys Asp Leu Phe Gly Asn Glu Thr Thr Leu Ala Val Ser Glu Glu
    50                  55                  60

Asp Ile Gln Ala Ala Leu Asp Gly Asp Glu Phe Arg Val Pro Leu Asn
65                  70                  75                  80

Ser Pro Val Ile Leu Val Gln Ser Gly Asn Arg Ala Pro Glu Thr Ile
                85                  90                  95

Met Gln Glu Glu Met Arg Lys Tyr Tyr Thr Val Ser Thr Phe Ser Gly
            100                 105                 110

Ile Pro Asp Arg Gln Lys Pro Leu Thr Cys Asn Lys Asn Lys Asp Lys
        115                 120                 125

Asn Glu Asn Glu Asp Val Ala Arg Ala Glu Asn Met Asn Trp Met Gln
    130                 135                 140

Ala Leu Arg Phe Val Ala Ala Lys Gly His Gln Lys Ala Ile Ile Val
145                 150                 155                 160

Tyr Gln Asp Met Leu Gln Thr Gly Lys Tyr Asp Ser Ala Leu Lys Ser
                165                 170                 175

Thr Val Trp Ser Asp Tyr Lys Asn Glu Lys Leu Thr Asp Ala Ile Ser
            180                 185                 190

Leu Arg Tyr Leu Val Arg Phe Thr Leu Val Asp Val Ala Thr Gly Glu
        195                 200                 205

Trp Ala Thr Trp Ser Pro Val Asn Tyr Glu Ser Arg Val Ile Phe Pro
    210                 215                 220

Pro Ala Gly Ile Thr Lys Thr Ser Asn Lys Glu Leu Ser Asn Asp His
225                 230                 235                 240

Val Thr Glu Ala Gln Leu Phe Asn Leu Lys Gln Lys Thr Tyr Val Ser
                245                 250                 255

Met Val Lys Asp Leu Val Ser Arg Phe Gln
            260                 265

<210>  27
<211>  1528
<212>  PRT
<213>  Escherichia coli

<400>  27

Met Asn Arg Thr Ser Pro Tyr Tyr Cys Arg Arg Ser Val Leu Ser Leu
1               5                   10                  15

Leu Ile Ser Ala Leu Ile Tyr Ala Pro Pro Gly Met Ala Ala Phe Thr
            20                  25                  30

Pro Asp Val Ile Gly Val Val Asn Asp Glu Thr Val Asp Gly Ser Gln
        35                  40                  45

Arg Val Asp Glu Arg Gly Thr Thr Asn Asn Thr His Ile Ile Asn His
    50                  55                  60

Gly Gln Gln Asn Val Tyr Gly Gly Val Ser Asn Gly Ser Leu Ile Glu
65                  70                  75                  80

Ser Gly Gly Tyr Gln Asp Val Gly Arg His Asn Asn Tyr Val Gly Gln
                85                  90                  95

Ser Asn Asn Thr Thr Ile Asn Gly Gly Arg Gln Ser Ile His Asp Gly
            100                 105                 110

Gly Ile Ser Thr Gly Thr Ile Ile Glu Ser Gly Asn Gln Asp Val Tyr
        115                 120                 125

Lys Gly Gly Ile Ser Asn Gly Thr Thr Ile Lys Gly Gly Ala Ser Arg
    130                 135                 140

Val Glu Gly Gly Ser Ala Asn Gly Thr Leu Ile Asp Gly Gly Ser Gln
145                 150                 155                 160

Ile Val Lys Val Gln Gly His Ala Asp Gly Thr Thr Ile Asn Lys Ser
                165                 170                 175

Gly Ser Gln Asp Val Val Gln Gly Ser Leu Ala Thr Asn Thr Thr Ile
            180                 185                 190

Asn Gly Gly Arg Gln Tyr Val Glu Gln Ser Thr Val Glu Thr Thr Thr
        195                 200                 205

Ile Lys Asn Gly Gly Glu Gln Arg Val Tyr Glu Ser Arg Ala Leu Asp
    210                 215                 220

Thr Thr Ile Glu Gly Gly Thr Gln Ser Leu Asn Ser Lys Ser Thr Ala
225                 230                 235                 240

Lys Asn Thr Gln Ile Tyr Ser Gly Gly Thr Gln Ile Ile Asp Asn Thr
                245                 250                 255

Ser Ser Ser Asp Val Ile Glu Val Tyr Ser Gly Gly Val Leu Asp Val
            260                 265                 270

Ser Gly Gly Thr Ala Thr Asn Val Thr Gln His Asp Gly Ala Ile Leu
        275                 280                 285

Lys Thr Asn Thr Asn Gly Thr Thr Val Ser Gly Thr Asn Ser Glu Gly
    290                 295                 300

Ala Phe Ser Ile His Asn His Val Ala Asp Asn Val Leu Leu Glu Asn
305                 310                 315                 320

Gly Gly His Leu Asp Ile Asn Ala Tyr Gly Ser Ala Asn Lys Thr Ile
                325                 330                 335

Ile Lys Asp Lys Gly Thr Met Ser Val Leu Thr Asn Ala Lys Ala Asp
            340                 345                 350

Ala Thr Arg Ile Asp Asn Gly Gly Val Met Asp Val Ala Gly Asn Ala
        355                 360                 365

Thr Asn Thr Ile Ile Asn Gly Gly Thr Gln Asn Ile Asn Asn Tyr Gly
    370                 375                 380

Ile Ala Thr Gly Thr Asn Ile Asn Ser Gly Thr Gln Asn Ile Lys Ser
385                 390                 395                 400

Gly Gly Lys Ala Asp Thr Thr Ile Ile Ser Ser Gly Ser Arg Gln Val
                405                 410                 415

Val Glu Lys Asp Gly Thr Ala Ile Gly Ser Asn Ile Ser Ala Gly Gly
            420                 425                 430

Ser Leu Ile Val Tyr Thr Gly Gly Ile Ala His Gly Val Asn Gln Glu
        435                 440                 445

Thr Gly Ser Ala Leu Val Ala Asn Thr Gly Ala Gly Thr Asp Ile Glu
    450                 455                 460

Gly Tyr Asn Lys Leu Ser His Phe Thr Ile Thr Gly Gly Glu Ala Asn
465                 470                 475                 480

Tyr Val Val Leu Glu Asn Thr Gly Glu Leu Thr Val Val Ala Lys Thr
                485                 490                 495

Ser Ala Lys Asn Thr Thr Ile Asp Ala Gly Gly Lys Leu Ile Val Gln
            500                 505                 510

Lys Glu Ala Lys Thr Asp Ser Thr Arg Leu Asn Asn Gly Gly Val Leu
        515                 520                 525

Glu Val Gln Asp Gly Gly Glu Ala Lys His Val Glu Gln Gln Ser Gly
    530                 535                 540

Gly Ala Leu Ile Ala Ser Thr Thr Ser Gly Thr Leu Ile Glu Gly Thr

545                 550                 555                 560

Asn Ser Tyr Gly Asp Ala Phe Tyr Ile Arg Asn Ser Glu Ala Lys Asn
                565                 570                 575

Val Val Leu Glu Asn Ala Gly Ser Leu Thr Val Val Thr Gly Ser Arg
            580                 585                 590

Ala Val Asp Thr Ile Ile Asn Ala Asn Gly Lys Met Asp Val Tyr Gly
        595                 600                 605

Lys Asp Val Gly Thr Val Leu Asn Ser Ala Gly Thr Gln Thr Ile Tyr
    610                 615                 620

Ala Ser Ala Thr Ser Asp Lys Ala Asn Ile Lys Gly Gly Lys Gln Thr
625                 630                 635                 640

Val Tyr Gly Leu Ala Thr Glu Ala Asn Ile Glu Ser Gly Glu Gln Ile
                645                 650                 655

Val Asp Gly Gly Ser Thr Glu Lys Thr His Ile Asn Gly Gly Thr Gln
            660                 665                 670

Thr Val Gln Asn Tyr Gly Lys Ala Ile Asn Thr Asp Ile Val Ser Gly
        675                 680                 685

Leu Gln Gln Ile Met Ala Asn Gly Thr Ala Glu Gly Ser Ile Ile Asn
    690                 695                 700

Gly Gly Ser Gln Ile Val Asn Glu Gly Gly Leu Ala Glu Asn Ser Val
705                 710                 715                 720

Leu Asn Asp Gly Gly Thr Leu Asp Val Arg Glu Lys Gly Ser Ala Thr
                725                 730                 735

Gly Ile Gln Gln Ser Ser Gln Gly Ala Leu Val Ala Thr Thr Arg Ala
            740                 745                 750

Thr Arg Val Thr Gly Thr Arg Ala Asp Gly Val Ala Phe Ser Ile Glu
        755                 760                 765

Gln Gly Ala Ala Asn Asn Ile Leu Leu Ala Asn Gly Gly Val Leu Thr
    770                 775                 780

Val Glu Ser Asp Thr Ser Ser Asp Lys Thr Gln Val Asn Thr Gly Gly
785                 790                 795                 800

Arg Glu Ile Val Lys Thr Lys Ala Thr Ala Thr Gly Thr Thr Leu Thr
                805                 810                 815

Gly Gly Glu Gln Ile Val Glu Gly Val Ala Asn Glu Thr Thr Ile Asn
            820                 825                 830

Asp Gly Gly Ile Gln Thr Val Ser Ala Asn Gly Glu Ala Ile Lys Thr
        835                 840                 845

Thr Ile Asn Glu Gly Gly Thr Leu Thr Val Asn Asp Asn Gly Lys Ala
    850                 855                 860

Thr Asp Ile Val Gln Asn Ser Gly Ala Ala Leu Gln Thr Ser Thr Ala
865                 870                 875                 880

Asn Gly Ile Glu Ile Ser Gly Thr His Gln Tyr Gly Thr Phe Ser Ile
                885                 890                 895

Ser Gly Asn Leu Ala Thr Asn Met Leu Leu Glu Asn Gly Gly Asn Leu
            900                 905                 910

Leu Val Leu Ala Gly Thr Glu Ala Arg Asp Ser Thr Val Gly Lys Gly
        915                 920                 925

Gly Ala Met Gln Asn Gln Gly Gln Asp Ser Ala Thr Lys Val Asn Ser
    930                 935                 940

Gly Gly Gln Tyr Thr Leu Gly Arg Ser Lys Asp Glu Phe Gln Ala Leu
945                 950                 955                 960

Ala Arg Ala Glu Asp Leu Gln Val Ala Gly Gly Thr Ala Ile Val Tyr
                965                 970                 975

Ala Gly Thr Leu Ala Asp Ala Ser Val Ser Gly Ala Thr Gly Ser Leu
            980                 985                 990

Ser Leu Met Thr Pro Arg Asp Asn Val Thr Pro Val Lys Leu Glu Gly
        995                 1000                1005

Ala Ile Arg Ile Thr Asp Ser Ala Thr Leu Thr Ile Gly Asn Gly
    1010                1015                1020

Val Asp Thr Thr Leu Ala Asp Leu Thr Ala Ala Ser Arg Gly Ser
    1025                1030                1035

Val Trp Leu Asn Ser Asn Asn Ser Cys Ala Gly Thr Ser Asn Cys
    1040                1045                1050

Glu Tyr Arg Val Asn Ser Leu Leu Leu Asn Asp Gly Asn Val Tyr
    1055                1060                1065

Leu Ser Ala Gln Thr Ala Ala Pro Ala Thr Thr Asn Gly Ile Tyr
    1070                1075                1080

Asn Thr Leu Thr Thr Asn Glu Leu Ser Gly Ser Gly Asn Phe Tyr
    1085                1090                1095

Leu His Thr Asn Val Ala Gly Ser Arg Gly Asp Gln Leu Val Val
    1100                1105                1110

Asn Asn Asn Ala Thr Gly Asn Phe Lys Ile Phe Val Gln Asp Thr
    1115                1120                1125

Gly Val Ser Pro Gln Ser Asp Asp Ala Met Thr Leu Val Lys Thr
    1130                1135                1140

Gly Gly Gly Asp Ala Ser Phe Ser Leu Gly Asn Thr Gly Gly Phe
    1145                1150                1155

Val Asp Leu Gly Thr Tyr Glu Tyr Val Leu Lys Ser Asp Gly Asn
    1160                1165                1170

Ser Asn Trp Asn Leu Thr Asn Asp Val Lys Pro Asn Pro Asp Pro
    1175                1180                1185

Asn Pro Asn Pro Asn Pro Asn Pro Lys Pro Asp Pro Lys Pro Asp
    1190                1195                1200

Pro Lys Pro Asp Pro Lys Pro Asp Pro Thr Pro Glu Pro Thr Pro
    1205                1210                1215

Thr Pro Val Pro Glu Lys Arg Ile Thr Pro Ser Thr Ala Ala Val
    1220                1225                1230

Leu Asn Met Ala Ala Thr Leu Pro Leu Val Phe Asp Ala Glu Leu
    1235                1240                1245

Asn Ser Ile Arg Glu Arg Leu Asn Ile Met Lys Ala Ser Pro His
    1250                1255                1260

Asn Asn Asn Val Trp Gly Ala Thr Tyr Asn Thr Arg Asn Asn Val
    1265                1270                1275

Thr Thr Asp Ala Gly Ala Gly Phe Glu Gln Thr Leu Thr Gly Met
    1280                1285                1290

Thr Val Gly Ile Asp Ser Pro Asn Asp Ile Pro Glu Gly Ile Ala
    1295                1300                1305

Thr Leu Gly Ala Phe Met Gly Tyr Ser His Ser His Ile Gly Phe
    1310                1315                1320

Asp Arg Gly Gly His Gly Ser Val Gly Ser Tyr Ser Leu Gly Gly
    1325                1330                1335

Tyr Ala Ser Trp Glu His Glu Ser Gly Phe Tyr Leu Asp Gly Val
    1340                1345                1350

Val Lys Leu Asn Arg Phe Glu Ser Asn Val Ala Gly Lys Met Ser
    1355                1360                1365

Ser Gly Gly Ala Ala Asn Gly Ser Tyr His Ser Asn Gly Leu Gly
    1370                1375                1380

Gly His Ile Glu Thr Gly Met Arg Phe Thr Asp Gly Asn Trp Asn
    1385                1390                1395

Leu Thr Pro Tyr Ala Ser Leu Thr Gly Phe Thr Ala Asp Asn Pro
    1400                1405                1410

Glu Tyr His Leu Ser Asn Gly Met Glu Ser Lys Ser Val Asp Thr
    1415                1420                1425

Arg Ser Ile Tyr Arg Glu Leu Gly Ala Thr Leu Ser Tyr Asn Met
    1430                1435                1440

Arg Leu Gly Asn Gly Met Glu Ile Glu Pro Trp Leu Lys Ala Ala
    1445                1450                1455

Val Arg Lys Glu Phe Val Asp Asp Asn Arg Val Lys Val Asn Asn
    1460                1465                1470

Asp Gly Asn Phe Val Asn Asp Leu Ser Gly Arg Arg Gly Ile Tyr
    1475                1480                1485

Gln Ala Gly Ile Lys Ala Ser Phe Ser Ser Thr Leu Ser Gly His
    1490                1495                1500

Leu Gly Val Gly Tyr Ser His Gly Ala Gly Val Glu Ser Pro Trp
    1505                1510                1515

Asn Ala Val Ala Gly Val Asn Trp Ser Phe

1520                1525

<210>  28
<211>  248
<212>  PRT
<213>  Escherichia coli

<400>  28

Met Ala Gly Phe Leu Ile Phe Leu Ser Ser Ala Ala Tyr Ala Asp Ile
1               5                   10                  15

Asn Leu Tyr Gly Pro Gly Gly Pro His Thr Ala Leu Leu Asp Ala Ala
            20                  25                  30

Lys Leu Tyr Ala Glu Lys Thr Gly Ile Ile Val Asn Val His Tyr Gly
        35                  40                  45

Pro Gln Asn Lys Trp Asn Glu Asp Ala Lys Lys Asn Ala Asp Ile Leu
    50                  55                  60

Phe Gly Ala Ser Glu Gln Ser Ala Leu Ala Ile Ile Arg Asp His Lys
65                  70                  75                  80

Asp Ser Phe Ser Glu Lys Asp Ile Gln Pro Leu Tyr Leu Arg Lys Ser
                85                  90                  95

Ile Leu Leu Val Lys Lys Gly Asn Pro Lys Asn Ile Arg Ser Ile Asp
            100                 105                 110

Asp Leu Thr Arg Pro Gly Ile Gly Val Ile Val Asn Asp Gly Gly Gly
        115                 120                 125

Thr Ser Asn Thr Ser Gly Thr Gly Val Trp Glu Asp Ile Ala Gly Arg
    130                 135                 140

Lys Gly Asn Ile Glu Thr Val Ala Ala Ile Arg Lys Asn Ile Ile Leu
145                 150                 155                 160

Tyr Ala Pro Asn Ser Gly Thr Ala Arg Lys Ala Leu Glu Asn Gln Pro
                165                 170                 175

Gly Ala Asp Val Trp Ile Thr Trp Ala Asp Trp Ala Ala Ser Asn Pro
            180                 185                 190

Glu Ile Gly Asp Val Val Glu Ile Ala Pro Asp Tyr Val Ile Trp Arg
        195                 200                 205

Asp Met Asn Ile Thr Val Arg Gln Asp Ala Asn Asp Glu Thr Arg Arg

210                 215                 220

Phe Ala Glu Trp Leu Gln Thr Asp Glu Ala Ala Pro Ala Phe Lys Lys
225                 230                 235                 240

Tyr Gly Trp Thr Arg Lys Gly Thr
                245

<210>  29
<211>  366
<212>  PRT
<213>  Escherichia coli

<400>  29

Met Lys Leu Lys Ile Val Ala Val Val Val Thr Gly Leu Leu Ala Ala
1               5                   10                  15

Asn Val Ala His Ala Ala Glu Val Tyr Asn Lys Asp Gly Asn Lys Leu
            20                  25                  30

Asp Leu Tyr Gly Lys Val Thr Ala Leu Arg Tyr Phe Thr Asp Asp Lys
        35                  40                  45

Arg Asp Asp Gly Asp Lys Thr Tyr Ala Arg Leu Gly Phe Lys Gly Glu
    50                  55                  60

Thr Gln Ile Asn Asp Gln Met Ile Gly Phe Gly His Trp Glu Tyr Asp
65                  70                  75                  80

Phe Lys Gly Tyr Asn Asp Glu Ala Asn Gly Ser Arg Asp Asn Lys Thr
                85                  90                  95

Arg Leu Ala Tyr Ala Gly Leu Lys Ile Ser Glu Phe Gly Ser Leu Asp
            100                 105                 110

Tyr Gly Arg Asn Tyr Gly Val Gly Tyr Asp Ile Gly Ser Trp Thr Asp
        115                 120                 125

Met Leu Pro Glu Phe Gly Gly Asp Thr Trp Ser Gln Lys Asp Val Phe
    130                 135                 140

Met Thr Tyr Arg Thr Thr Gly Val Ala Thr Tyr Arg Asn Tyr Asp Phe
145                 150                 155                 160

Phe Gly Leu Ile Glu Gly Leu Asn Phe Ala Ala Gln Tyr Gln Gly Lys
                165                 170                 175

Asn Glu Arg Thr Asp Asn Ser His Leu Tyr Gly Ala Asp Tyr Thr Arg

180                 185                 190

Ala Asn Gly Asp Gly Phe Gly Ile Ser Ser Thr Tyr Val Tyr Asp Gly
        195                 200                 205

Phe Gly Ile Gly Ala Val Tyr Thr Lys Ser Asp Arg Thr Asn Ala Gln
    210                 215                 220

Glu Arg Ala Ala Ala Asn Pro Leu Asn Ala Ser Gly Lys Asn Ala Glu
225                 230                 235                 240

Leu Trp Ala Thr Gly Ile Lys Tyr Asp Ala Asn Asn Ile Tyr Phe Ala
                245                 250                 255

Ala Asn Tyr Ala Glu Thr Leu Asn Met Thr Thr Tyr Gly Asp Gly Tyr
            260                 265                 270

Ile Ser Asn Lys Ala Gln Ser Phe Glu Val Val Ala Gln Tyr Gln Phe
        275                 280                 285

Asp Phe Gly Leu Arg Pro Ser Leu Ala Tyr Leu Lys Ser Lys Gly Ile
    290                 295                 300

Asp Leu Gly Arg Tyr Gly Asp Gln Asp Met Ile Glu Tyr Ile Asp Val
305                 310                 315                 320

Gly Ala Thr Tyr Phe Phe Asn Lys Asn Met Ser Thr Tyr Val Asp Tyr
                325                 330                 335

Lys Ile Asn Leu Ile Asp Glu Ser Asp Phe Thr Arg Ala Val Asp Ile
            340                 345                 350

Arg Thr Asp Asn Ile Val Ala Thr Gly Ile Thr Tyr Gln Phe
        355                 360                 365

<210>  30
<211>  319
<212>  PRT
<213>  Escherichia coli

<400>  30

Met Lys Cys Ile Leu Phe Lys Trp Val Leu Cys Leu Leu Leu Gly Phe
1               5                   10                  15

Ser Ser Val Ser Tyr Ser Arg Glu Phe Thr Ile Asp Phe Ser Thr Gln
            20                  25                  30

Gln Ser Tyr Val Ser Ser Leu Asn Ser Ile Arg Thr Glu Ile Ser Thr

35                  40                  45

Pro Leu Glu His Ile Ser Gln Gly Thr Thr Ser Val Ser Val Ile Asn
    50                  55                  60

His Thr Pro Pro Gly Ser Tyr Phe Ala Val Asp Ile Arg Gly Leu Asp
65                  70                  75                  80

Val Tyr Gln Ala Arg Phe Asp His Leu Arg Leu Ile Ile Glu Gln Asn
                85                  90                  95

Asn Leu Tyr Val Ala Gly Phe Val Asn Thr Ala Thr Asn Thr Phe Tyr
            100                 105                 110

Arg Phe Ser Asp Phe Thr His Ile Ser Val Pro Gly Val Thr Thr Val
        115                 120                 125

Ser Met Thr Thr Asp Ser Ser Tyr Thr Thr Leu Gln Arg Val Ala Ala
    130                 135                 140

Leu Glu Arg Ser Gly Met Gln Ile Ser Arg His Ser Leu Val Ser Ser
145                 150                 155                 160

Tyr Leu Ala Leu Met Glu Phe Ser Gly Asn Thr Met Thr Arg Asp Ala
                165                 170                 175

Ser Arg Ala Val Leu Arg Phe Val Thr Val Thr Ala Glu Ala Leu Arg
            180                 185                 190

Phe Arg Gln Ile Gln Arg Glu Phe Arg Gln Ala Leu Ser Glu Thr Ala
        195                 200                 205

Pro Val Tyr Thr Met Thr Pro Gly Asp Val Asp Leu Thr Leu Asn Trp
    210                 215                 220

Gly Arg Ile Ser Asn Val Leu Pro Glu Tyr Arg Gly Glu Asp Gly Val
225                 230                 235                 240

Arg Val Gly Arg Ile Ser Phe Asn Asn Ile Ser Ala Ile Leu Gly Thr
                245                 250                 255

Val Ala Val Ile Leu Asn Cys His His Gln Gly Ala Arg Ser Val Arg
            260                 265                 270

Ala Val Asn Glu Glu Ser Gln Pro Glu Cys Gln Ile Thr Gly Asp Arg
        275                 280                 285

Pro Val Ile Lys Ile Asn Asn Thr Leu Trp Glu Ser Asn Thr Ala Ala
    290                 295                 300

Ala Phe Leu Asn Arg Lys Ser Gln Phe Leu Tyr Thr Thr Gly Lys
305                 310                 315

<210>  31
<211>  315
<212>  PRT
<213>  Escherichia coli

<400>  31

<210>  32
<211>  369
<212>  PRT
<213>  Escherichia coli

<400>  32

Met Gln Arg Lys Gly Asn Lys Leu Leu Ile Gln Leu Cys Ser Val Ile
1               5                   10                  15

Leu Leu Phe Phe Thr Thr Ser Trp Tyr Ala Leu Ala Asn Glu Cys Tyr
            20                  25                  30

Ile Glu Arg Asn Ala Glu Gly Asp Tyr His Met Lys Ile Ser Ser Thr
        35                  40                  45

Gln Leu Ser Leu Ala Ser Gln Met Val Glu Val Pro Thr Glu Ile Ala
    50                  55                  60

Glu Ala Thr Trp Asp Val Asn Ile Gln Leu Arg Gly Asp Ala Ile Gly
65                  70                  75                  80

Cys Lys Ser Leu Gly Asp Ser Lys Ala Val His Phe Leu Asn Thr Ala
                85                  90                  95

Asp Pro Ser Leu Ile Ser Thr Tyr Thr Thr Thr Asn Gly Ala Ala Leu
            100                 105                 110

Leu Lys Thr Thr Val Pro Gly Ile Val Tyr Ser Val Glu Leu Leu Cys
        115                 120                 125

Leu Ser Cys Gly Ala Ala Asp Glu Leu Asp Leu Trp Leu Pro Ala Gln
    130                 135                 140

Ser Gly Ala Asp Asn Phe Ile Pro Ser Thr Gln Thr Lys Trp Ala Tyr
145                 150                 155                 160

Glu Tyr Ser Asp Gln Ser Trp Tyr Leu Arg Phe Arg Leu Phe Ile Thr
                165                 170                 175

Pro Glu Phe Lys Pro Lys Asn Gly Val Ser Ser Gly Thr Thr Ile Ala
            180                 185                 190

Gly Lys Ile Ala Ser Trp Tyr Ile Gly Thr Asn Asp Gln Pro Trp Ile
        195                 200                 205

Asn Phe Tyr Ile Asp Asn Asp Ser Leu Lys Phe Phe Val Asp Glu Pro
    210                 215                 220

Thr Cys Ala Thr Val Ala Leu Ala Gln Asp Gln Gly Asn Val Ser Gly
225                 230                 235                 240

Asn Gln Val Thr Leu Gly Asn Ser Tyr Val Ser Glu Val Lys Asn Gly
                245                 250                 255

Leu Thr Arg Glu Ile Pro Phe Ser Ile Arg Ala Glu Tyr Cys Tyr Ala
            260                 265                 270

Ser Lys Ile Thr Val Lys Leu Lys Ala Ala Asn Lys Pro Ser Asp Ala
        275                 280                 285

Thr Leu Val Gly Lys Thr Thr Gly Ser Ala Ser Gly Val Ala Val Lys
    290                 295                 300

Val Asn Ser Thr Tyr Asp Asn Ser Lys Val Leu Leu Lys Ala Asp Gly
305                 310                 315                 320

Ser Asn Thr Val Asp Tyr Asn Phe Ala Ala Trp Ser Asn Asn Leu Leu
                325                 330                 335

Phe Leu Pro Phe Thr Ala Gln Leu Val Pro Asp Gly Ser Gly Asn Ala
            340                 345                 350

Val Gly Val Gly Thr Phe Ser Gly Asn Ala Thr Phe Ser Phe Thr Tyr
        355                 360                 365

Glu

<210>  33
<211>  353
<212>  PRT
<213>  Escherichia coli

<400>  33

Met Arg Phe Ala Lys Gly Val Leu Leu Ala Ile Cys Leu Ile Phe Leu
1               5                   10                  15

Pro Leu Lys Ala Ala Leu Ala Leu Asn Cys Tyr Phe Gly Thr Ala Asn
            20                  25                  30

Gly Ala Val Glu Lys Ser Glu Ala Ile Met Pro Phe Ala Val Pro Ala
        35                  40                  45

Asn Ser Lys Pro Gly Asp Lys Ile Trp Glu Ser Asp Asp Ile Lys Ile
    50                  55                  60

Pro Val Tyr Cys Asp Asn Asn Thr Asn Gly Asn Phe Glu Ser Glu His
65                  70                  75                  80

Val Tyr Ala Trp Val Asn Pro Tyr Pro Gly Ile Gln Asp Pro Tyr Tyr
                85                  90                  95

Gln Leu Gly Val Thr Tyr Glu Gly Val Asp Tyr Asp Ala Ser Leu Gly
            100                 105                 110

Lys Ser Arg Ile Asp Thr Asn Gln Cys Ile Asp Ser Lys Asn Ile Asp
        115                 120                 125

Ile Tyr Thr Pro Glu Gln Ile Ile Ala Met Gly Trp Gln Asn Lys Leu
    130                 135                 140

Cys Ser Gly Asp Pro Thr Val Met His Lys Ser Arg Thr Phe Val Ala
145                 150                 155                 160

Arg Met Arg Leu Tyr Val Lys Val Arg Ala Met Pro Pro His Asp Tyr
                165                 170                 175

Gln Ser Lys Leu Ser Asp Tyr Ile Val Val Gln Phe Asp Gly Ala Gly
            180                 185                 190

Ser Val Asn Glu Asp Pro Thr Ala Lys Asn Leu Lys Tyr His Ile Thr
        195                 200                 205

Gly Leu Glu Asn Ile Arg Val Leu Asp Cys Ser Val Asn Phe Ala Ile
    210                 215                 220

Ser Pro Glu Thr Gln Val Val Asp Phe Gly Arg Phe Asn Val Leu Asp
225                 230                 235                 240

Ile Arg Arg His Thr Met Ser Gln Gln Phe Lys Ile Thr Thr Thr Lys
                245                 250                 255

Ser Gln Asn Asp Gln Cys Thr Asp Gly Phe Lys Val Ser Ser Ser Phe
            260                 265                 270

Tyr Thr Asp Glu Thr Leu Ile Asp Glu Asp Lys Ser Leu Leu Ile Gly
        275                 280                 285

Asn Gly Leu Lys Leu Arg Leu Leu Asp Glu Asn Ala Ser Pro Tyr Thr
    290                 295                 300

Phe Asn Lys Tyr Ser Glu Tyr Ala Asp Phe Thr Ser Asp Leu Leu Val
305                 310                 315                 320

Tyr Glu Lys Thr Tyr Thr Ala Glu Leu Ser Ser Thr Pro Gly Thr Pro
                325                 330                 335

Ile Asp Val Gly Pro Phe Asp Thr Val Val Leu Phe Lys Ile Asn Tyr
            340                 345                 350

Asn

<210> 34
<211> 361
<212> PRT
<213> Escherichia coli

<400> 34

Met Trp Glu Cys Asp Ala Thr Asp Leu Pro Asn Ile Tyr Phe Leu Val
1               5                   10                  15

Ala Thr Asn Gly Asp Asp Arg Val Gly Gly Phe Tyr Asp Ala Gly Gly
            20                  25                  30

Pro Asp Gly Leu Ser Asp Val Tyr Ala Thr Trp Phe Ala Phe Val Gly
        35                  40                  45

Leu Lys Gln Thr Met Ala Gly Val Thr Leu Gly Arg Tyr Trp Lys Lys
    50                  55                  60

Val Pro Ile Thr Ser Tyr Ala Thr Gln Gly Thr Lys Ile Gln Ile Arg
65                  70                  75                  80

Leu Gln Asp Ile Pro Pro Leu His Ala Glu Leu Tyr Arg Ile Ser Thr
                85                  90                  95

Leu Pro Asp Thr Ser Ala Thr Thr Ser Trp Cys Gly Asn Asn Asn Thr
            100                 105                 110

Asp Ser Ser Gly Val Gly Phe Ala Lys Pro Ser Gly Thr Ile Tyr Asn
        115                 120                 125

Cys Val Gln Pro Asn Ala Tyr Ile Gln Leu Ser Gly Thr Ser Gly Ile
    130                 135                 140

Leu Phe Gly His Asp Glu Pro Gly Glu Asp Ser Ser Val His Trp Asp
145                 150                 155                 160

Phe Trp Gly Ala Asp Asn Gly Phe Gly Tyr Gly Met Arg Ser Ala Asn
                165                 170                 175

Arg Leu Tyr Asn Asn Ala Thr Cys Val Ala Arg Ser Ala Thr Pro Leu
            180                 185                 190

Val Leu Leu Pro Thr Ile Ala Glu Ala Gln Leu Asn Ala Gly Met Glu
        195                 200                 205

Ser Thr Gly Asn Phe Asn Val Arg Val Glu Cys Ser Asn Ser Val Gln
    210                 215                 220

Ser Gly Ile Ser Asp Thr Gln Thr Ala Leu Gly Ile Gln Val Ser Glu
225                 230                 235                 240

Gly Ala Tyr Thr Ala Ala Gln Lys Leu Gly Ile Ile Asn Ser Asn Gly
                245                 250                 255

Gly Val Ser Ala Leu Val Ser Asp Asn Tyr Asp Ala Ala Glu Met Ala
            260                 265                 270

Lys Gly Val Gly Ile Tyr Ile Ser Asn Ser Ala His Pro Asp Thr Ala
        275                 280                 285

Met Thr Leu Val Gly Gln Pro Gly Ile Ala Lys Leu Thr Pro Gly Gly
    290                 295                 300

Asn Ala Ala Gly Trp Tyr Pro Val Phe Glu Gly Ala Thr Leu Glu Gly
305                 310                 315                 320

Ala Thr His Pro Gly Tyr Ser Ser Tyr Ser Tyr Ser Phe Ile Ala Arg
                325                 330                 335

Leu Lys Lys Leu Pro Asn Gln Thr Val Ser Ala Gly Lys Val Arg Ala
            340                 345                 350

Thr Ala Tyr Ile Leu Val Lys Met Gln
        355                 360

<210>  35
<211>  262
<212>  PRT
<213>  Escherichia coli

<400>  35

Met Asn Met Ala Phe Tyr Gly Lys Trp Phe Ala Cys Leu Trp Leu Ala
1               5                   10                  15

Thr Ser Cys Val Gln Ala Ala Ser Thr Asp Asn Lys Ala Leu Glu Ile
            20                  25                  30

Ile Arg Arg Ala Asp Glu Ile Arg Ser Pro Asn Lys Pro Phe Arg Tyr
        35                  40                  45

Thr Leu Thr Val Thr Glu Tyr Lys Ala Gly Ala Thr Gln Pro Glu Asn
    50                  55                  60

Lys Gln Val Leu Asp Ile Ser Met Arg Phe Met Lys Pro Gln Gly Asn
65                  70                  75                  80

Glu Lys Ala Asp Ala Arg Ser Leu Val Arg Phe Ile Tyr Pro Pro Arg
                85                  90                  95

Asp Lys Gly Lys Ile Met Leu Ser Asp Trp Tyr Asp Leu Trp Phe Tyr
            100                 105                 110

Thr Pro Glu Leu Arg Arg Pro Met Pro Ile Ser Arg Gln Gln Arg Leu
        115                 120                 125

Ile Gly Gln Ile Ser Asn Gly Asp Val Ile Val Thr Asn Phe Glu Tyr
    130                 135                 140

Ala Tyr Asp Ser Thr Leu Met Gly Glu Val Thr Cys Ala Glu Lys Gln
145                 150                 155                 160

Cys Tyr Lys Leu Ala Leu Val Arg Lys Ser Ala Asp Ile Thr Trp Pro
                165                 170                 175

Lys Val Ile Tyr Tyr Val Glu Lys Asp Gly Asp Asn Arg Pro Trp Lys
            180                 185                 190

Ala Ala Tyr Tyr Ser Gln Asp Asp Gln Leu Ile Lys Glu Val Leu Tyr
        195                 200                 205

Gln Asp Phe Gln Pro Val Leu Gly Lys Thr Arg Pro Met Lys Ile Thr
    210                 215                 220

Val Thr Asp Val Arg His Gly Asn Asn Tyr Ser Val Met Glu Tyr Ser
225                 230                 235                 240

Asp Val Arg Leu Glu Ser Leu Pro Glu Phe His Phe Thr Lys Glu Tyr
                245                 250                 255

Ile Gln Arg Gly Ala Lys
            260

<210>  36
<211>  462
<212>  PRT
<213>  Escherichia coli

<400>  36

Met Lys Arg Thr Leu Lys Ile Ser Ser Leu Leu Cys Val Ala Leu Pro
1               5                   10                  15

Leu Thr Val Gln Ala Asp Cys Leu Ser Gly Asp Glu Val Ala Gln Asn
            20                  25                  30

Ser Asp Val Thr Leu Tyr Glu Gln Val Ser Tyr Val Asn Lys Gln Ala
        35                  40                  45

Ser Ala Trp Gln Ile Ala Gly Lys Asn Pro Tyr Thr Arg His Asn Gly
    50                  55                  60

Tyr Gln Glu Ala Gly Ile Gly Ile Asn Ser Gly Cys Ser Ile Ile Asp
65                  70                  75                  80

Asn Thr Leu Asp Leu Lys Leu Asn Leu Tyr Gly Ile Asn Glu Tyr Ala
                85                  90                  95

Leu Lys Pro Ala Gly Lys Phe Glu Thr Asp Asp Ser Arg Thr Arg Ala
            100                 105                 110

Leu Ile Asn Arg Leu Ser Leu Val Tyr Ser Ala Ser Asp Ser Val Gln
        115                 120                 125

Phe Glu Ala Gly Lys Phe Ala Ala Pro Ser Gly Met Phe Phe Leu Arg
    130                 135                 140

Ser Pro Ser Asp Leu Gln Thr His Tyr Tyr Thr Gly Phe Gln Ser Thr
145                 150                 155                 160

Arg Leu His Asp Pro Lys Met Thr Ser Ala Tyr Gln Ala Ser Ser Trp
                165                 170                 175

Gly Ala Lys Met Ser Val Asp Thr Arg Asp Tyr Ala Phe Ser Ala Ser
            180                 185                 190

Val Ile Pro Lys Leu Ala Thr Ile Asp Lys Arg Tyr Val Thr Ser Gly
        195                 200                 205

Asn Trp Ser Ala Asn Gln Gln Gly Asn Ser Asp Glu Ala Tyr Leu Leu
    210                 215                 220

Ser Phe Ser Asp His Arg Phe Gly Glu His Thr Pro Thr Val Asn Val
225                 230                 235                 240

Arg Leu Gly Pro Ser Pro Ser Leu Ala Leu Ser Asp Ser Tyr His Tyr
                245                 250                 255

Thr Pro Gln Leu Thr Leu Asn Val Asp Ala Ala Tyr His Arg Ser Gln
            260                 265                 270

Gln Trp Arg His Leu Ser His Arg Glu Thr Ala Gln Val Glu Glu Tyr
        275                 280                 285

Gln Phe Pro Asp Ser Leu Tyr Glu Thr Lys Asp Glu Ser Gly Val Glu
    290                 295                 300

Leu Ala Leu Gly Gly Gln Tyr Thr Ser Asp Asn Phe Ser Val Phe Gly
305                 310                 315                 320

Val Glu Tyr Tyr Phe Gln Ser Glu Gly Tyr Ser Arg Ala Glu Gln Arg
                325                 330                 335

Gln Gln Arg Glu Leu Ile Asp Phe Leu Asn Thr Thr Thr Gly Tyr Ala
            340                 345                 350

Pro Leu Asp Gln Ala Phe Asp Ser Tyr Lys Tyr Leu Met Ala Ser Glu

355                 360                 365

Ile Ser Asn Thr Ala Asn Gln Gly Met Leu Gln Gly Lys His Tyr Leu
    370                 375                 380

Asn Ala Trp Ala Ser Leu Pro Leu Ala Gly Glu Ser Thr Leu Gln Pro
385                 390                 395                 400

Ser Leu Val Val Asn Leu Val Asp Gly Ser Thr Leu Leu Gly Leu His
                405                 410                 415

Tyr Ser Thr Pro Leu Ser Ala Ile Ser Asn Gln Leu Glu Ala Tyr Ala
            420                 425                 430

Gly Gly Tyr Ser Ala Leu Gly Ser Arg Tyr Ser Glu Phe Ala Leu Phe
        435                 440                 445

Gly Asp Thr Leu Gly Leu Tyr Leu Gly Phe Lys Tyr Tyr Leu
    450                 455                 460

<210>  37
<211>  234
<212>  PRT
<213>  Escherichia coli

<400>  37

Met Gly Ile Asp Ser Arg Asn Asp Ile Pro Glu Gly Ile Ala Thr Leu
1               5                   10                  15

Gly Ala Phe Met Gly Tyr Ser His Ser His Ile Gly Phe Asp Arg Gly
            20                  25                  30

Gly His Gly Ser Val Asp Ser Tyr Ser Leu Gly Gly Tyr Ala Ser Trp
        35                  40                  45

Glu His Glu Ser Gly Phe Tyr Leu Asp Gly Val Val Lys Leu Asn Arg
    50                  55                  60

Phe Glu Ser Asn Val Ala Gly Lys Met Ser Ser Gly Gly Ala Ala Asn
65                  70                  75                  80

Gly Ser Tyr His Ser Asn Gly Leu Gly Gly His Ile Glu Thr Gly Met
                85                  90                  95

Arg Phe Thr Asp Gly Asn Trp Asn Leu Thr Pro Tyr Ala Ser Leu Thr
            100                 105                 110

Gly Phe Thr Ala Asp Asn Pro Glu Tyr His Leu Ser Asn Gly Met Glu

115                 120                 125

Ser Lys Ser Val Asp Thr Arg Ser Ile Tyr Arg Glu Leu Gly Ala Thr
    130                 135                 140

Leu Ser Tyr Asn Met Arg Leu Gly Asn Gly Met Glu Val Glu Pro Trp
145                 150                 155                 160

Leu Lys Ala Ala Val Arg Lys Glu Phe Val Asp Asp Asn Arg Val Lys
                165                 170                 175

Val Asn Ser Asp Gly Asn Phe Val Asn Asp Leu Ser Gly Arg Arg Gly
            180                 185                 190

Ile Tyr Gln Ala Gly Ile Lys Ala Ser Phe Ser Ser Thr Leu Ser Gly
        195                 200                 205

His Leu Gly Val Gly Tyr Ser Asn Gly Ala Gly Met Glu Ser Pro Trp
    210                 215                 220

Asn Ala Val Ala Gly Val Asn Trp Ser Phe
225                 230

<210>  38
<211>  419
<212>  PRT
<213>  Escherichia coli

<400>  38

Met Lys Phe Pro Ser Ile Phe Asn Lys Ile Lys Pro Gln Ser Ile Gln
1               5                   10                  15

Gln His Pro Glu Lys Asn Gln Leu Asn Trp Met Leu Glu Leu Asn Lys
            20                  25                  30

Trp Lys Glu Glu Arg Ile Leu Thr Gly Glu Ile His Arg Pro Glu Cys
        35                  40                  45

Arg Asn Glu Ala Ala Lys Arg Ile Asn Cys Ala Phe Leu Ser Lys Gln
    50                  55                  60

Asn Asp Ile Asp Leu Ser Gly Leu Asn Leu Ser Thr Gln Pro Pro Gly
65                  70                  75                  80

Leu Gln Asn Phe Thr Ser Ile Asn Leu Asp Asn Asn Gln Leu Thr His
                85                  90                  95

Phe Asp Ala Thr Asn Tyr Asp Arg Leu Val Lys Leu Ser Leu Asn Ser

            100                 105                 110

Asn Thr Leu Glu Ser Ile Asn Ile His Gln Gly Arg Asn Val Ser Ile
        115                 120                 125

Thr His Ile Ser Met Asn Asn Asn Cys Leu Arg Asn Ile Asp Ile Asp
    130                 135                 140

Arg Leu Ser Ser Ile Thr Tyr Phe Ser Ala Ala His Asn Lys Leu Glu
145                 150                 155                 160

Phe Val Gln Leu Glu Ser Cys Glu Trp Leu Gln Tyr Leu Asn Leu Ser
                165                 170                 175

His Asn Gln Leu Thr Asp Ile Val Thr Gly Asn Lys Glu Glu Leu Leu
            180                 185                 190

Leu Leu Asp Leu Ser His Asn Lys Leu Ala Ser Leu His Asn Ala Leu
        195                 200                 205

Phe Pro Asn Leu Asn Thr Leu Leu Ile Asn Asn Asn Leu Leu Ser Glu
    210                 215                 220

Ile Lys Met Phe Tyr Ser Asn Phe Cys Lys Val Gln Thr Leu Asn Ala
225                 230                 235                 240

Ala Asn Asn Gln Leu Glu Lys Ile Asn Leu His Phe Leu Thr Tyr Leu
                245                 250                 255

Ser Ser Ile Lys Ser Leu Arg Leu Asp Asn Asn Lys Ile Thr Arg Ile
            260                 265                 270

Asp Thr Glu Asn Thr Ser Asp Ile Arg Ser Leu Phe Pro Ile Ile Lys
        275                 280                 285

Lys Ser Glu Ser Leu Asn Phe Leu Asn Ile Ser Gly Glu Asn Asn Cys
    290                 295                 300

Pro Thr Ile Gln Leu Met Leu Phe Asn Leu Phe Ser Pro Ala Leu Lys
305                 310                 315                 320

Leu Asn Thr Gly Leu Ala Ile Leu Ser Pro Gly Ala Phe Glu Asp His
                325                 330                 335

Ser Asp Gly Leu Asp Val Asp Asn Glu Leu Phe His Tyr Thr Ile Asn
            340                 345                 350

Lys Ala Tyr Thr Pro Tyr Asn Ile His Thr Tyr Lys Thr Glu Glu Val
        355                 360                 365

Val Asn Gln Arg Asn Ile Lys Ile Lys Asn Met Thr Leu Asp Glu Ile
    370                 375                 380

Asn Asn Thr Tyr Cys Asn Asn Asp Tyr Tyr Asn Glu Ala Ile Arg Glu
385                 390                 395                 400

Glu Pro Ile Asp Phe Leu Asp Arg Ser Phe Ser Ser Ser Ser Trp Pro
                405                 410                 415

Phe Tyr His

<210>  39
<211>  236
<212>  PRT
<213>  Escherichia coli

<400>  39

Met Asn Lys Ala Pro Ser Leu Ile Ala Ala Ile Val Leu Gly Leu Gly
1               5                   10                  15

Ile Ser Ala Cys Gly Tyr Phe Val Gly Asp Gly Val Lys His Leu Lys
            20                  25                  30

Thr Asn Asn Arg Tyr Val Asn Val Arg Gly Leu Ser Glu Lys Glu Val
        35                  40                  45

Arg Ala Asp Thr Ala Glu Leu Thr Ile Ala Ile Asn Phe Lys Gly Asn
    50                  55                  60

Val Pro Gly Glu Leu Phe Pro Lys Leu Glu Glu Ala Gln Lys Lys Ile
65                  70                  75                  80

Val Ala Glu Leu Asn Ala Gln Gly Ile Asn Glu Lys Glu Ile Ile Leu
                85                  90                  95

Gly Gln Trp Thr Ser Lys Arg Thr Asp Ser Phe Tyr Leu Lys Asp Asp
            100                 105                 110

Pro Thr Met Pro Arg Tyr Asn Ala Asp Gly Ser Val Thr Ile Lys Thr
        115                 120                 125

His Asn Val Ala Ala Val Glu Lys Val Val Ala Lys Leu Asn Glu Leu
    130                 135                 140

Gln Val Ala Thr Asp Gly Ala Ile Ala Glu Ser Lys Val Ala Tyr Arg
145                 150                 155                 160

Phe Asn Gly Ile Gly Ala Leu Arg Ala Glu Met Ile Ala Ala Ala Thr
                165                 170                 175

Lys Asp Ala Arg Asn Ala Ala Leu Gln Phe Ala Thr Asp Ser Gly Ser
            180                 185                 190

Gln Val Gly Ser Ile Ser Asp Ala Ser Gln Gly Val Phe Gln Ile Phe
        195                 200                 205

Ala Ser Gly Ser Asp Glu Asp Asp Pro Thr Ala Ile Asn Lys Thr Val
    210                 215                 220

Arg Val Val Thr Thr Val Thr Tyr Ala Leu Gln Asp
225                 230                 235

<210>  40
<211>  280
<212>  PRT
<213>  Escherichia coli

<400>  40

Met Lys Lys Trp Thr Ile Phe Leu Thr Ser Leu Ile Leu Leu Ala Leu
1               5                   10                  15

Ser Leu Glu Thr Pro Lys Cys Tyr Ala Gly Asp Lys Leu Met Ser Ala
            20                  25                  30

Ser Phe Ser Ser Thr Lys Ile Tyr Tyr Ala Met Lys Asn Val Thr Ala
        35                  40                  45

Ser Gly Ser Leu Tyr Phe Tyr Val Thr Val Val Thr Pro Gly Glu Val
    50                  55                  60

Ser Tyr Gly Gln Tyr Asn Ser Asn Ala Arg Lys Gly Asp Thr Leu Lys
65                  70                  75                  80

Leu Ile Ser Trp Ser Gly Ser Gly Pro Ala Pro Thr Leu Val Leu Thr
                85                  90                  95

Asp Tyr Arg Arg Thr Asp Thr Ser Asn Cys Pro Gly Ile Asn Thr Arg
            100                 105                 110

Val Phe Val Cys Ala Tyr Met Thr Phe Asn Val Thr Val Glu Ser Asp
        115                 120                 125

Asn Tyr Gly Cys Pro Trp Ile Ala Ser Phe Tyr Ser Val Ser Glu Ala
    130                 135                 140

Phe Gly Phe Gly Thr Tyr Thr Ser Pro Thr Val His Asp Ser Ile Cys
145                 150                 155                 160

Pro Thr Ile Pro Val Ala Ser Phe Asp Ile Ser Trp Ser Asp Asn Tyr
                165                 170                 175

Val Ser His Asn Lys Ala Leu Arg Leu Gln Ser Asp Gly Ser Thr Ile
            180                 185                 190

Thr Thr Thr Leu Ser Thr Tyr Leu Met Glu Ser Gly Lys Leu Cys Asp
        195                 200                 205

Gly Ser Ile Phe Asp Ser Arg Gly Ala Tyr Cys Arg Ala Val Ser Asp
    210                 215                 220

Leu Leu Thr Phe Thr Ser Tyr Gly Cys Asp Asn Ala Lys Val Thr Val
225                 230                 235                 240

Thr Pro Ser Arg Gln Pro Leu Thr Asp Arg Lys Leu His Asp Ile Val
                245                 250                 255

Val Gln Val Asn Thr Ser Ser Arg Glu Pro Ile Asp Ser Thr Cys Arg
            260                 265                 270

Phe Gln Tyr Val Leu Asn Glu Leu
        275                 280

<210>  41
<211>  303
<212>  PRT
<213>  Escherichia coli

<400>  41

Met Gly Thr Ala Ala Ile Ser His Leu Arg Tyr Asp Leu Asn Lys Tyr
1               5                   10                  15

Ala Leu Ser Leu Arg Lys Thr Ala Thr Leu Ala Ser Thr Phe Phe Ile
            20                  25                  30

Glu Ser Pro Leu Val Arg Phe Glu Tyr Leu Gln Glu Ile Glu Asn Thr
        35                  40                  45

Ile Asn Asp Ile Thr His Arg Phe Asn Ser Ser Tyr Asp Ile Asn Glu
    50                  55                  60

Lys Ala Arg Leu Ile Asn Glu Leu Lys Met Glu Ser Glu Thr Ala Arg
65                  70                  75                  80

Lys Glu Tyr Gln Leu Phe Arg Gln Gly Asn Tyr Asp Lys Tyr Ile Thr
                85                  90                  95

Thr Asp Ile Phe Glu Glu His Gly Leu Ile Lys Tyr Val Asn Leu Ser
            100                 105                 110

Phe Asp Ile Val Ala Ser Val Gly Gln Val Val Gly Gly Val Gly Ala
        115                 120                 125

Leu Lys Phe Gly Lys Val Val His Ser Asn Arg Ile Lys Gly Ile Gly
    130                 135                 140

Val Thr Leu Val Ala His Gly Ala Asn Asn Phe Tyr Glu Ser Leu Ser
145                 150                 155                 160

Pro Leu Phe Phe Asn Glu Tyr Asp Ser Gly Pro Ile Arg Glu Leu Tyr
                165                 170                 175

Arg Val Ile Ala Lys Lys Met Gly Gly Asp Ile Asn Ser Ala Asp Tyr
            180                 185                 190

Ala Tyr Ser Ile Val Asp Phe Ser Ile Thr Ala Tyr Gly Gly Tyr Ser
        195                 200                 205

Gly Met Lys Ile Val Pro Lys Tyr Asn Arg Leu Ile Arg Pro Ser Leu
    210                 215                 220

Gly Asn Arg Pro Gly Thr Gly Arg Leu Phe His Tyr Thr Ser Val Asp
225                 230                 235                 240

Phe Lys Asn Lys Phe Ser Leu Lys Pro Thr Pro Leu Lys Ile Ile Gln
                245                 250                 255

Ile Ser Ser Thr Val Lys Lys Phe Lys Val Thr Phe Tyr Asp Glu Asn
            260                 265                 270

Thr Asn Leu Arg Ile Lys His Gly Leu Asn Asn His Met Pro His Gln
        275                 280                 285

Leu Leu Met Asn Ile Ser Gly Thr Thr Thr Gly Ala Gly Ile Gln
    290                 295                 300

<210>  42
<211>  354
<212>  PRT

<213>  Escherichia coli

<400>  42

Met Leu Ser Leu Thr Leu Met Ser Ala Leu Leu Ser Pro Leu Ser Leu
1               5                   10                  15

Gln Ala Ala Asp Val Arg Arg Ser Gly Asp Glu Ala Phe Ile Ile Gln
            20                  25                  30

Gln Gln Arg Gln Glu Ala Leu Glu Gln Gln Leu Thr Pro Ser Ala Pro
        35                  40                  45

Asp Val Arg Leu Ser Ala Pro Gly Ser Phe Ala His Lys Ile Asn Phe
    50                  55                  60

Pro Val Glu Thr Pro Cys Phe Gln Ile Lys Gln Thr Glu Leu Lys Gly
65                  70                  75                  80

Ala Asp Ala Leu Pro His Trp Leu Pro Leu Gln Lys Ile Ala Asn Gly
                85                  90                  95

Ala Val Gly His Cys Leu Gly Ala Lys Gly Ile Asn Leu Leu Met Ser
            100                 105                 110

Thr Leu Gln Asn Arg Leu Val Asp His Gly Tyr Val Thr Thr Arg Val
        115                 120                 125

Leu Ala Pro Ser Gln Asp Leu Lys Ser Gly Ile Leu Arg Leu Val Ile
    130                 135                 140

Ile Pro Gly Val Val Arg His Val Arg Leu Thr Pro Asp Ser Asp Asp
145                 150                 155                 160

Tyr Ile Gln Leu Tyr Ser Ser Phe Pro Ala His Glu Gly Ser Leu Leu
                165                 170                 175

Asp Leu Arg Asp Ile Glu Gln Gly Leu Asp Leu Gly Asn Ser Arg Ile
            180                 185                 190

Gln Gly Gln His Thr Glu Leu Asn Ala Thr Ser Gly Asn Leu Ser Thr
        195                 200                 205

Gln Asn Ala Gln Leu Ser Ala Asp Thr Leu Ser Ala Arg Thr Ala Gly
    210                 215                 220

Gln Phe Ser Ser Asn Gly Gly Thr Ile Asn Ala Asp Thr Leu Gln Ile
225                 230                 235                 240

Ser Ala Gln Ser Leu Ser Asn Arg Lys Gly Ser Leu Ile Gln Thr Gly
                245                 250                 255

Thr Gly Asp Phe Ser Leu Ser Leu Pro Gly Ser Val Asp Asn Arg Glu
            260                 265                 270

Gly Leu Leu Ala Ala Asn Gly Ala Val Arg Leu Asp Ala Leu Ser Leu
        275                 280                 285

Asp Asn Arg Lys Gly Lys Val Gln Ala Glu Gln Ser Pro Ser Leu Gln
    290                 295                 300

Lys Ser Pro Pro Thr Phe Leu Lys Pro Phe Val Ala Gly Val Cys Ala
305                 310                 315                 320

Ala Leu Leu Ala Val Ser Val Ala Ile Pro Gly Trp Gln Phe Leu Thr
                325                 330                 335

Gln Pro Ser Pro Glu Glu Gln His Phe Thr Trp Gly Asn Gly Cys Lys
            340                 345                 350

Lys Gln

<210>  43
<211>  1399
<212>  PRT
<213>  Escherichia coli

<400>  43

Met Ser Gly Lys Pro Ala Ala Arg Gln Gly Asp Met Thr Gln Tyr Gly
1               5                   10                  15

Gly Ser Ile Val Gln Gly Ser Ala Gly Val Arg Ile Gly Ala Pro Thr
            20                  25                  30

Gly Val Ala Cys Ser Val Cys Pro Gly Gly Val Thr Ser Gly His Pro
        35                  40                  45

Val Asn Pro Leu Leu Gly Ala Lys Val Leu Pro Gly Glu Thr Asp Ile
    50                  55                  60

Ala Leu Pro Gly Pro Leu Pro Phe Ile Leu Ser Arg Thr Tyr Ser Ser
65                  70                  75                  80

Tyr Arg Thr Lys Thr Pro Ala Pro Val Gly Ser Leu Gly Pro Gly Trp
                85                  90                  95

Lys Met Pro Ala Asp Ile Arg Leu Gln Leu Arg Asp Asn Thr Leu Ile
            100                 105                 110

Leu Ser Asp Asn Gly Gly Arg Ser Leu Tyr Phe Glu His Leu Phe Pro
        115                 120                 125

Gly Glu Asp Gly Tyr Ser Arg Ser Glu Ser Leu Trp Leu Val Arg Gly
    130                 135                 140

Gly Val Ala Lys Leu Asp Glu Gly His Arg Leu Ala Ala Leu Trp Gln
145                 150                 155                 160

Ala Leu Pro Glu Glu Leu Arg Leu Ser Pro His Arg Tyr Leu Ala Thr
                165                 170                 175

Asn Ser Pro Gln Gly Pro Trp Trp Leu Leu Gly Trp Cys Glu Arg Val
            180                 185                 190

Pro Glu Ala Asp Glu Val Leu Pro Ala Pro Leu Pro Pro Tyr Arg Val
        195                 200                 205

Leu Thr Gly Leu Val Asp Arg Phe Gly Arg Thr Gln Thr Phe His Arg
    210                 215                 220

Glu Ala Ala Gly Glu Phe Ser Gly Glu Ile Thr Gly Val Thr Asp Gly
225                 230                 235                 240

Ala Gly Arg His Phe Arg Leu Val Leu Thr Thr Gln Ala Gln Arg Ala
                245                 250                 255

Glu Glu Ala Arg Gln Gln Ala Ile Ser Gly Gly Thr Glu Pro Ser Ala
            260                 265                 270

Phe Pro Asp Thr Leu Pro Gly Tyr Thr Glu Tyr Gly Arg Asp Asn Gly
        275                 280                 285

Ile Arg Leu Ser Ala Val Trp Leu Thr His Asp Pro Glu Tyr Pro Glu
    290                 295                 300

Asn Leu Pro Ala Ala Pro Leu Val Arg Tyr Gly Trp Thr Pro Arg Gly
305                 310                 315                 320

Glu Leu Ala Val Val Tyr Asp Arg Ser Gly Lys Gln Val Arg Ser Phe
                325                 330                 335

Thr Tyr Asp Asp Lys Tyr Arg Gly Arg Met Val Ala His Arg His Thr
            340                 345                 350

Gly Arg Pro Glu Ile Arg Tyr Arg Tyr Asp Ser Asp Gly Arg Val Thr
        355                 360                 365

Glu Gln Leu Asn Pro Ala Gly Leu Ser Tyr Thr Tyr Gln Tyr Glu Lys
    370                 375                 380

Asp Arg Ile Thr Ile Thr Asp Ser Leu Asn Arg Arg Glu Val Leu His
385                 390                 395                 400

Thr Gln Gly Glu Gly Gly Leu Lys Arg Val Val Lys Lys Glu His Ala
                405                 410                 415

Asp Gly Ser Val Thr Gln Ser Gln Phe Asp Ala Val Gly Arg Leu Arg
            420                 425                 430

Ala Gln Thr Asp Ala Ala Gly Arg Thr Thr Glu Tyr Ser Pro Asp Val
        435                 440                 445

Val Thr Gly Leu Ile Thr Arg Ile Thr Thr Pro Asp Gly Arg Ala Ser
    450                 455                 460

Ala Phe Tyr Tyr Asn His His Ser Gln Leu Thr Ser Ala Thr Gly Pro
465                 470                 475                 480

Asp Gly Leu Glu Ile Arg Arg Glu Tyr Asp Glu Trp Gly Arg Leu Ile
                485                 490                 495

Gln Glu Thr Ala Pro Asp Gly Asp Ile Thr Arg Tyr Arg Tyr Asp Asn
            500                 505                 510

Pro His Ser Asp Leu Pro Cys Ala Thr Glu Asp Ala Thr Gly Ser Arg
        515                 520                 525

Lys Thr Met Thr Trp Ser Arg Tyr Gly Gln Leu Leu Ser Phe Thr Asp
    530                 535                 540

Cys Ser Gly Tyr Val Thr Arg Tyr Asp His Asp Arg Phe Gly Gln Met
545                 550                 555                 560

Thr Ala Val His Arg Glu Glu Gly Leu Ser Gln Tyr Arg Ala Tyr Asp
                565                 570                 575

Ser Arg Gly Gln Leu Ile Ala Val Lys Asp Thr Gln Gly His Glu Thr
            580                 585                 590

Arg Tyr Glu Tyr Asn Ala Ala Gly Asp Leu Thr Thr Val Ile Ala Pro

595                 600                 605

Asp Gly Ser Arg Asn Gly Thr Gln Tyr Asp Ala Trp Gly Lys Ala Ile
    610                 615                 620

Cys Thr Thr Gln Gly Gly Leu Thr Arg Ser Met Glu Tyr Asp Ala Ala
625                 630                 635                 640

Gly Arg Val Ile Arg Leu Thr Ser Glu Asn Gly Ser His Thr Thr Phe
                645                 650                 655

Arg Tyr Asp Val Leu Asp Arg Leu Ile Gln Glu Thr Gly Phe Asp Gly
            660                 665                 670

Arg Thr Gln Arg Tyr His His Asp Leu Thr Gly Lys Leu Ile Arg Ser
        675                 680                 685

Glu Asp Glu Gly Leu Val Thr His Trp His Tyr Asp Glu Ala Asp Arg
    690                 695                 700

Leu Thr His Arg Thr Val Lys Gly Glu Thr Ala Glu Arg Trp Gln Tyr
705                 710                 715                 720

Asp Glu Arg Gly Trp Leu Thr Asp Ile Ser His Ile Ser Glu Gly His
                725                 730                 735

Arg Val Thr Val His Tyr Gly Tyr Asp Glu Lys Gly Arg Leu Thr Gly
            740                 745                 750

Glu Arg Gln Thr Val His His Pro Gln Thr Glu Ala Leu Leu Trp Gln
        755                 760                 765

His Glu Thr Arg His Ala Tyr Asn Ala Gln Gly Leu Ala Asn Arg Cys
    770                 775                 780

Ile Pro Asp Ser Leu Pro Ala Val Glu Trp Leu Thr Tyr Gly Ser Gly
785                 790                 795                 800

Trp Leu Ala Gly Met Lys Leu Gly Asp Thr Pro Leu Val Asp Phe Thr
                805                 810                 815

Arg Asp Arg Leu His Arg Lys Thr Leu Arg Arg Phe Gly Arg Tyr Glu
            820                 825                 830

Leu Thr Thr Ala Tyr Thr Pro Ala Gly Gln Leu Gln Ser Gln His Leu
        835                 840                 845

Asn Ser Leu Gln Tyr Asp Arg Asp Tyr Thr Trp Asn Asp Asn Gly Glu
    850                 855                 860

Leu Ile Arg Ile Ser Ser Pro Arg Gln Thr Arg Ser Tyr Ser Tyr Ser
865                 870                 875                 880

Asp Ser Gly Arg Leu Thr Gly Val His Thr Thr Ala Ala Asn Leu Asp
                885                 890                 895

Ile Arg Ile Pro Tyr Ala Thr Asp Pro Ala Gly Asn Arg Leu Pro Asp
            900                 905                 910

Pro Glu Leu His Pro Asp Ser Thr Leu Ser Met Trp Pro Asp Asn Arg
        915                 920                 925

Ile Ala Arg Asp Ala His Tyr Leu Tyr Arg Tyr Asp Arg His Gly Arg
    930                 935                 940

Leu Thr Glu Lys Thr Asp Leu Ile Pro Glu Gly Val Ile Arg Thr Asp
945                 950                 955                 960

Asp Glu Arg Thr His Arg Tyr His Tyr Asp Ser Gln His Arg Leu Val
                965                 970                 975

His Tyr Thr Arg Thr Gln Tyr Glu Glu Pro Leu Val Glu Ser Arg Tyr
            980                 985                 990

Leu Tyr Asp Pro Leu Gly Arg Arg Val Ala Lys Arg Val Trp Arg Arg
        995                 1000                1005

Glu Arg Asp Leu Thr Gly Trp Met Ser Leu Ser Arg Lys Pro Gln
    1010                1015                1020

Val Thr Trp Tyr Gly Trp Asp Gly Asp Arg Leu Thr Thr Ile Gln
    1025                1030                1035

Asn Asp Arg Thr Arg Ile Gln Thr Ile Tyr Gln Pro Gly Ser Phe
    1040                1045                1050

Thr Pro Leu Ile Arg Val Glu Thr Ala Thr Gly Glu Leu Ala Lys
    1055                1060                1065

Thr Gln Arg Arg Ser Leu Ala Asp Ala Leu Gln Gln Ser Gly Gly
    1070                1075                1080

Glu Asp Gly Gly Ser Val Val Phe Pro Pro Val Leu Val Gln Met
    1085                1090                1095

Leu Asp Arg Leu Glu Ser Glu Ile Leu Ala Asp Arg Val Ser Glu
    1100                1105                1110

Glu Ser Arg Arg Trp Leu Ala Ser Cys Gly Leu Thr Val Ala Gln
    1115                1120                1125

Met Gln Ser Gln Met Asp Pro Val Tyr Thr Pro Ala Arg Lys Ile
    1130                1135                1140

His Leu Tyr His Cys Asp His Arg Gly Leu Pro Leu Ala Leu Ile
    1145                1150                1155

Ser Lys Glu Gly Ala Thr Glu Trp Cys Ala Glu Tyr Asp Glu Trp
    1160                1165                1170

Gly Asn Leu Leu Asn Glu Glu Asn Pro His Gln Leu Gln Gln Leu
    1175                1180                1185

Ile Arg Leu Pro Gly Gln Gln Tyr Asp Glu Glu Ser Gly Leu Tyr
    1190                1195                1200

Tyr Asn Arg His Arg Tyr Tyr Asp Pro Leu Gln Gly Arg Tyr Ile
    1205                1210                1215

Thr Gln Asp Pro Ile Gly Leu Lys Gly Gly Trp Asn Phe Tyr Gln
    1220                1225                1230

Tyr Pro Leu Asn Pro Val Gln Tyr Ile Asp Ser Met Gly Leu Ala
    1235                1240                1245

Ser Lys Tyr Gly His Leu Asn Asn Gly Gly Tyr Gly Ala Arg Pro
    1250                1255                1260

Asn Lys Pro Pro Thr Pro Asp Pro Ser Lys Phe Pro Asp Ile Ala
    1265                1270                1275

Lys Gln Leu Arg Leu Pro Tyr Pro Ile Asp Gln Ala Ser Ser Ala
    1280                1285                1290

Pro Asn Val Phe Lys Thr Phe Phe Arg Ala Leu Ser Pro Tyr Asp
    1295                1300                1305

Tyr Thr Leu Tyr Cys Arg Lys Trp Val Lys Pro Asn Leu Thr Cys
    1310                1315                1320

Thr Pro Gln Asp Asp Pro Gln Tyr Pro Gly Met Asp Thr Lys Thr
    1325                1330                1335

Ala Ser Asp Tyr Leu Pro Gln Thr Asn Trp Pro Thr Thr Gln Leu
    1340                1345                1350

Pro Pro Gly Tyr Thr Cys Ala Glu Pro Tyr Leu Phe Pro Asp Ile
    1355                1360                1365

Asn Lys Pro Asp Gly Pro Ala Thr Ala Gly Ile Asp Asp Leu Gly
    1370                1375                1380

Glu Ile Leu Ala Lys Met Lys Gln Arg Thr Ser Arg Gly Ile Arg
    1385                1390                1395

Lys

<210> 44
<211> 2620
<212> PRT
<213> Escherichia coli

<400> 44

Met Ala Ala Ala Ala Gln Gly Val Val Asn Ala Ala Thr Gln Gln Pro
1               5                   10                  15

Val Pro Ala Gln Ile Ala Ile Ala Asn Ala Asn Thr Val Pro Tyr Thr
            20                  25                  30

Leu Gly Ala Leu Glu Ser Ala Gln Ser Val Ala Glu Arg Phe Gly Ile
        35                  40                  45

Ser Val Ala Glu Leu Arg Lys Leu Asn Gln Phe Arg Thr Phe Ala Arg
    50                  55                  60

Gly Phe Asp Asn Val Arg Gln Gly Asp Glu Leu Asp Val Pro Ala Gln
65                  70                  75                  80

Val Ser Glu Asn Asn Leu Thr Pro Pro Pro Gly Asn Ser Ser Gly Asn
                85                  90                  95

Leu Glu Gln Gln Ile Ala Ser Thr Ser Gln Gln Ile Gly Ser Leu Leu
            100                 105                 110

Ala Glu Asp Met Asn Ser Glu Gln Ala Ala Asn Met Ala Arg Gly Trp
        115                 120                 125

Ala Ser Ser Gln Ala Ser Gly Ala Met Thr Asp Trp Leu Ser Arg Phe
    130                 135                 140

Gly Thr Ala Arg Ile Thr Leu Gly Val Asp Glu Asp Phe Ser Leu Lys
145                 150                 155                 160

Asn Ser Gln Phe Asp Phe Leu His Pro Trp Tyr Glu Thr Pro Asp Asn
                165                 170                 175

Leu Phe Phe Ser Gln His Thr Leu His Arg Thr Asp Glu Arg Thr Gln
            180                 185                 190

Ile Asn Asn Gly Leu Gly Trp Arg His Phe Thr Pro Thr Trp Met Ser
        195                 200                 205

Gly Ile Asn Phe Phe Phe Asp His Asp Leu Ser Arg Tyr His Ser Arg
    210                 215                 220

Ala Gly Ile Gly Ala Glu Tyr Trp Arg Asp Tyr Leu Lys Leu Ser Ser
225                 230                 235                 240

Asn Gly Tyr Leu Arg Leu Thr Asn Trp Arg Ser Ala Pro Glu Leu Asp
                245                 250                 255

Asn Asp Tyr Glu Ala Arg Pro Ala Asn Gly Trp Asp Val Arg Ala Glu
            260                 265                 270

Gly Trp Leu Pro Ala Trp Pro His Leu Gly Gly Lys Leu Val Tyr Glu
        275                 280                 285

Gln Tyr Tyr Gly Asp Glu Val Ala Leu Phe Asp Lys Asp Asp Arg Gln
    290                 295                 300

Ser Asn Pro His Ala Ile Thr Ala Gly Leu Asn Tyr Thr Pro Phe Pro
305                 310                 315                 320

Leu Met Thr Phe Ser Ala Glu Gln Arg Gln Gly Lys Gln Gly Glu Asn
                325                 330                 335

Asp Thr Arg Phe Ala Val Asp Phe Thr Trp Gln Pro Gly Ser Ala Met
            340                 345                 350

Gln Lys Gln Leu Asp Pro Asn Glu Val Asp Ala Arg Arg Ser Leu Ala
        355                 360                 365

Gly Ser Arg Phe Asp Leu Val Asp Arg Asn Asn Asn Ile Val Leu Glu
    370                 375                 380

Tyr Arg Lys Lys Glu Leu Val Arg Leu Thr Leu Thr Asp Pro Val Thr

385                 390                 395                 400

Gly Lys Ser Gly Glu Val Lys Ser Leu Val Ser Ser Leu Gln Thr Lys
                405                 410                 415

Tyr Ala Leu Lys Gly Tyr Asn Val Glu Ala Thr Ala Leu Glu Ala Ala
            420                 425                 430

Gly Gly Lys Val Val Thr Thr Gly Lys Asp Ile Leu Val Thr Leu Pro
        435                 440                 445

Ala Tyr Arg Phe Thr Ser Thr Pro Glu Thr Asp Asn Thr Trp Pro Ile
    450                 455                 460

Glu Val Thr Ala Glu Asp Val Lys Gly Asn Phe Ser Asn Arg Glu Gln
465                 470                 475                 480

Ser Met Val Val Val Gln Ala Pro Thr Leu Ser Gln Lys Asp Ser Ser
                485                 490                 495

Val Ser Leu Ser Ser Gln Thr Leu Ser Ala Asp Ser His Ser Thr Ala
            500                 505                 510

Thr Leu Thr Phe Ile Ala His Asp Ala Ala Gly Asn Pro Val Ile Gly
        515                 520                 525

Leu Val Leu Ser Thr Arg His Glu Gly Val Gln Asp Ile Thr Leu Ser
    530                 535                 540

Asp Trp Lys Asp Asn Gly Asp Gly Ser Tyr Thr Gln Ile Leu Thr Thr
545                 550                 555                 560

Gly Ala Met Ser Gly Thr Leu Thr Leu Met Pro Gln Leu Asn Gly Val
                565                 570                 575

Asp Ala Ala Lys Ala Pro Ala Val Val Asn Ile Ile Ser Val Ser Ser
            580                 585                 590

Ser Arg Thr His Ser Ser Ile Lys Ile Asp Lys Asp Arg Tyr Leu Ser
        595                 600                 605

Gly Asn Pro Ile Glu Val Thr Val Glu Leu Arg Asp Glu Asn Asp Lys
    610                 615                 620

Pro Val Lys Glu Gln Lys Gln Gln Leu Asn Thr Ala Val Ser Ile Asp
625                 630                 635                 640

Asn Val Lys Pro Gly Val Thr Thr Asp Trp Lys Glu Thr Ala Asp Gly
            645                 650                 655

Val Tyr Lys Ala Thr Tyr Thr Ala Tyr Thr Lys Gly Ser Gly Leu Thr
        660                 665                 670

Ala Lys Leu Leu Met Gln Asn Trp Asn Glu Asp Leu His Thr Ala Gly
    675                 680                 685

Phe Ile Ile Asp Ala Asn Pro Gln Ser Ala Lys Ile Ala Thr Leu Ser
    690                 695                 700

Ala Ser Asn Asn Gly Val Leu Ala Asn Glu Asn Ala Ala Asn Thr Val
705                 710                 715                 720

Ser Val Asn Val Ala Asp Glu Gly Ser Asn Pro Ile Asn Asp His Thr
            725                 730                 735

Val Thr Phe Ala Val Leu Ser Gly Ser Ala Thr Ser Phe Asn Asn Gln
        740                 745                 750

Asn Thr Ala Lys Thr Asp Val Asn Gly Leu Ala Thr Phe Asp Leu Lys
    755                 760                 765

Ser Ser Lys Gln Glu Asp Asn Thr Val Glu Val Thr Leu Glu Asn Gly
    770                 775                 780

Val Lys Gln Thr Leu Ile Val Ser Phe Val Gly Asp Ser Ser Thr Ala
785                 790                 795                 800

Gln Val Asp Leu Gln Lys Ser Lys Asn Glu Val Val Ala Asp Gly Asn
            805                 810                 815

Asp Ser Ala Thr Met Thr Ala Thr Val Arg Asp Ala Lys Gly Asn Leu
        820                 825                 830

Leu Asn Asp Val Lys Val Thr Phe Asn Val Asn Ser Ala Ala Ala Lys
    835                 840                 845

Leu Ser Gln Thr Glu Val Asn Ser His Asp Gly Ile Ala Thr Ala Thr
    850                 855                 860

Leu Thr Ser Leu Lys Asn Gly Asp Tyr Thr Val Thr Ala Ser Val Ser
865                 870                 875                 880

Ser Gly Ser Gln Ala Asn Gln Gln Val Ile Phe Ile Gly Asp Gln Ser
            885                 890                 895

Thr Ala Ala Leu Thr Leu Ser Val Pro Ser Gly Asp Ile Thr Val Thr
            900                 905                 910

Asn Thr Ala Pro Leu His Met Thr Ala Thr Leu Gln Asp Lys Asn Gly
        915                 920                 925

Asn Pro Leu Lys Asp Lys Glu Ile Thr Phe Ser Val Pro Asn Asp Val
    930                 935                 940

Ala Ser Arg Phe Ser Ile Ser Asn Ser Gly Lys Gly Met Thr Asp Ser
945                 950                 955                 960

Asn Gly Thr Ala Ile Ala Ser Leu Thr Gly Thr Leu Ala Gly Thr His
                965                 970                 975

Met Ile Thr Ala Arg Leu Ala Asn Ser Asn Val Ser Asp Thr Gln Pro
            980                 985                 990

Met Thr Phe Val Ala Asp Lys Asp Arg Ala Val Val Val Leu Gln Thr
        995                 1000                1005

Ser Lys Ala Glu Ile Ile Gly Asn Gly Val Asp Glu Thr Thr Leu
    1010                1015                1020

Thr Ala Thr Val Lys Asp Pro Phe Asp Asn Val Val Lys Asn Leu
    1025                1030                1035

Ser Val Val Phe Arg Thr Ser Pro Ala Asp Thr Gln Leu Ser Leu
    1040                1045                1050

Asn Ala Arg Asn Thr Asn Glu Asn Gly Ile Ala Glu Val Thr Leu
    1055                1060                1065

Lys Gly Thr Val Leu Gly Val His Thr Ala Glu Ala Ile Leu Leu
    1070                1075                1080

Asn Gly Asn Arg Asp Thr Lys Ile Val Asn Ile Ala Pro Asp Ala
    1085                1090                1095

Ser Asn Ala Gln Val Thr Leu Asn Ile Pro Ala Gln Gln Val Val
    1100                1105                1110

Thr Asn Asn Ser Asp Ser Val Gln Leu Thr Ala Thr Val Lys Asp
    1115                1120                1125

Pro Ser Asn His Pro Val Ala Gly Ile Thr Val Asn Phe Thr Met
    1130                1135                1140

Pro Gln Asp Val Ala Ala Asn Phe Thr Leu Glu Asn Asn Gly Ile
    1145                1150                1155

Ala Ile Thr Gln Ala Asn Gly Glu Ala His Val Thr Leu Lys Gly
    1160                1165                1170

Lys Lys Ala Gly Thr His Thr Val Thr Ala Thr Leu Gly Asn Asn
    1175                1180                1185

Asn Ala Ser Asp Ala Gln Pro Val Thr Phe Val Ala Asp Lys Asp
    1190                1195                1200

Ser Ala Val Val Val Leu Gln Thr Ser Lys Ala Glu Ile Ile Gly
    1205                1210                1215

Asn Gly Val Asp Glu Thr Thr Leu Thr Ala Thr Val Lys Asp Pro
    1220                1225                1230

Phe Asp Asn Ala Val Lys Asp Leu Gln Val Thr Phe Ser Thr Asn
    1235                1240                1245

Pro Ala Asp Thr Gln Leu Ser Gln Ser Lys Ser Asn Thr Asn Asp
    1250                1255                1260

Ser Gly Val Ala Glu Val Thr Phe Lys Gly Thr Val Leu Gly Val
    1265                1270                1275

His Thr Ala Glu Ala Thr Leu Pro Asn Gly Asn Asn Asp Thr Lys
    1280                1285                1290

Ile Val Asn Ile Ala Pro Asp Ala Ser Asn Ala Gln Val Thr Leu
    1295                1300                1305

Asn Ile Pro Ala Gln Gln Val Val Thr Asn Asn Ser Asp Ser Val
    1310                1315                1320

Gln Leu Thr Ala Thr Val Lys Asp Pro Ser Asn His Pro Val Ala
    1325                1330                1335

Gly Ile Thr Val Asn Phe Thr Met Pro Gln Asp Val Ala Ala Asn
    1340                1345                1350

Phe Thr Leu Glu Asn Asn Gly Ile Ala Ile Thr Gln Ala Asn Gly
    1355                1360                1365

Glu Ala His Val Thr Leu Lys Gly Lys Lys Ala Gly Thr His Thr

1370                1375                1380

Val Thr Ala Thr Leu Ser Asn Asn Asn Thr Ser Asp Ser Gln Pro
    1385                1390                1395

Val Thr Phe Val Ala Asp Lys Thr Ser Ala Leu Val Val Leu Gln
    1400                1405                1410

Ile Ser Lys Asn Glu Ile Thr Gly Asn Gly Val Asp Ser Ala Thr
    1415                1420                1425

Leu Thr Ala Thr Val Lys Asp Gln Phe Asp Asn Glu Val Asn Asn
    1430                1435                1440

Leu Pro Val Thr Phe Ser Thr Ala Ser Ser Gly Leu Thr Leu Thr
    1445                1450                1455

Pro Gly Glu Ser Asn Thr Asn Glu Ser Gly Ile Ala Gln Ala Thr
    1460                1465                1470

Leu Ala Gly Val Ala Phe Gly Glu Gln Thr Val Thr Ala Ser Leu
    1475                1480                1485

Ala Asn Asn Gly Ala Ser Asp Asn Lys Thr Val His Phe Ile Gly
    1490                1495                1500

Asp Thr Ala Ala Ala Lys Ile Ile Glu Leu Thr Pro Val Pro Asp
    1505                1510                1515

Ser Ile Ile Ala Gly Thr Pro Gln Asn Ser Ser Gly Ser Val Ile
    1520                1525                1530

Thr Ala Thr Val Val Asp Asn Asn Gly Phe Pro Val Lys Gly Val
    1535                1540                1545

Thr Val Asn Phe Thr Ser Asn Ala Ala Thr Ala Glu Met Thr Asn
    1550                1555                1560

Gly Gly Gln Ala Val Thr Asn Glu Gln Gly Lys Ala Thr Val Thr
    1565                1570                1575

Tyr Thr Asn Thr Arg Ser Ser Ile Glu Ser Gly Ala Arg Pro Asp
    1580                1585                1590

Thr Val Glu Ala Ser Leu Glu Asn Gly Ser Ser Thr Leu Ser Thr
    1595                1600                1605

Ser Ile Asn Val Asn Ala Asp Ala Ser Thr Ala His Leu Thr Leu
    1610                1615                1620

Leu Gln Ala Leu Phe Asp Thr Val Ser Ala Gly Asp Thr Thr Asn
    1625                1630                1635

Leu Tyr Ile Glu Val Lys Asp Asn Tyr Gly Asn Gly Val Pro Gln
    1640                1645                1650

Gln Glu Val Thr Leu Ser Val Ser Pro Ser Glu Gly Val Thr Pro
    1655                1660                1665

Ser Asn Asn Ala Ile Tyr Thr Thr Asn His Asp Gly Asn Phe Tyr
    1670                1675                1680

Ala Ser Phe Thr Ala Thr Lys Ala Gly Val Tyr Gln Val Thr Ala
    1685                1690                1695

Thr Leu Glu Asn Gly Asp Ser Met Gln Gln Thr Val Thr Tyr Val
    1700                1705                1710

Pro Asn Val Ala Asn Ala Glu Ile Ser Leu Ala Ala Ser Lys Asp
    1715                1720                1725

Pro Val Ile Ala Asn Asn Asn Asp Leu Thr Thr Leu Thr Ala Thr
    1730                1735                1740

Val Ala Asp Thr Glu Gly Asn Ala Ile Ala Asn Ser Glu Val Thr
    1745                1750                1755

Phe Thr Leu Pro Glu Asp Val Arg Ala Asn Phe Thr Leu Gly Asp
    1760                1765                1770

Gly Gly Lys Val Val Thr Asp Thr Glu Gly Lys Ala Lys Val Thr
    1775                1780                1785

Leu Lys Gly Thr Lys Ala Gly Ala His Thr Val Thr Ala Ser Met
    1790                1795                1800

Ala Gly Gly Lys Ser Glu Gln Leu Val Val Asn Phe Ile Ala Asp
    1805                1810                1815

Thr Leu Thr Ala Gln Val Asn Leu Asn Val Thr Glu Asp Asn Phe
    1820                1825                1830

Ile Ala Asn Asn Val Gly Met Thr Arg Leu Gln Ala Thr Val Thr
    1835                1840                1845

Asp Gly Asn Gly Asn Pro Leu Ala Asn Glu Ala Val Thr Phe Thr
    1850                1855                1860

Leu Pro Ala Asp Val Ser Ala Ser Phe Thr Leu Gly Gln Gly Gly
    1865                1870                1875

Ser Ala Ile Thr Asp Ile Asn Gly Lys Ala Glu Val Thr Leu Ser
    1880                1885                1890

Gly Thr Lys Ser Gly Thr Tyr Pro Val Thr Val Ser Val Asn Asn
    1895                1900                1905

Tyr Gly Val Ser Asp Thr Lys Gln Val Thr Leu Ile Ala Asp Ala
    1910                1915                1920

Gly Thr Ala Lys Leu Ala Ser Leu Thr Ser Val Tyr Ser Phe Val
    1925                1930                1935

Val Ser Thr Thr Glu Gly Ala Thr Met Thr Ala Ser Val Thr Asp
    1940                1945                1950

Ala Asn Gly Asn Pro Val Glu Gly Ile Lys Val Asn Phe Arg Gly
    1955                1960                1965

Thr Ser Val Thr Leu Ser Ser Thr Ser Val Glu Thr Asp Asp Arg
    1970                1975                1980

Gly Phe Ala Glu Ile Leu Val Thr Ser Thr Glu Val Gly Leu Lys
    1985                1990                1995

Thr Val Ser Ala Ser Leu Ala Asp Lys Pro Thr Glu Val Ile Ser
    2000                2005                2010

Arg Leu Leu Asn Ala Lys Ala Asp Ile Asn Ser Ala Thr Ile Thr
    2015                2020                2025

Ser Leu Glu Ile Pro Glu Gly Gln Val Met Val Ala Gln Asp Val
    2030                2035                2040

Ala Val Lys Ala His Val Asn Asp Gln Phe Gly Asn Pro Ile Leu
    2045                2050                2055

Asn Glu Ser Val Thr Phe Ser Ala Glu Pro Pro Glu His Met Thr
    2060                2065                2070

Ile Ser Gln Asn Ile Val Ser Thr Asp Thr His Gly Ile Ala Glu
    2075                2080                2085

Val Thr Met Thr Pro Glu Arg Asn Gly Ser Tyr Met Val Lys Ala
    2090                2095                2100

Ser Leu Ala Asn Gly Ser Ser Tyr Glu Lys Asp Leu Val Val Ile
    2105                2110                2115

Asp Gln Lys Leu Thr Leu Ser Ala Ser Ser Pro Leu Ile Gly Val
    2120                2125                2130

Asn Ser Pro Thr Gly Ala Thr Leu Thr Ala Thr Leu Thr Ser Ala
    2135                2140                2145

Asn Gly Thr Pro Val Glu Gly Gln Val Ile Asn Phe Ser Val Thr
    2150                2155                2160

Pro Glu Gly Ala Thr Leu Ser Gly Gly Lys Val Arg Thr Asn Ser
    2165                2170                2175

Ser Gly Gln Ala Pro Val Val Leu Thr Ser Asn Lys Val Gly Thr
    2180                2185                2190

Tyr Thr Val Thr Ala Ser Phe His Asn Gly Val Thr Ile Gln Thr
    2195                2200                2205

Gln Thr Ile Val Lys Val Thr Gly Asn Ser Ser Thr Ala His Val
    2210                2215                2220

Ala Ser Phe Ile Ala Asp Pro Ser Thr Ile Ala Ala Thr Asn Ser
    2225                2230                2235

Asp Leu Ser Thr Leu Lys Ala Thr Val Glu Asp Gly Ser Gly Asn
    2240                2245                2250

Leu Ile Glu Gly Leu Thr Val Tyr Phe Ala Leu Lys Ser Gly Ser
    2255                2260                2265

Ala Thr Leu Thr Ser Leu Thr Ala Val Thr Asp Gln Asn Gly Ile
    2270                2275                2280

Ala Thr Thr Ser Val Arg Gly Ala Ile Thr Gly Ser Val Thr Val
    2285                2290                2295

Ser Ala Val Thr Thr Ala Gly Gly Met Gln Thr Val Asp Ile Thr
    2300                2305                2310

Leu Val Ala Gly Pro Ala Asp Ala Ser Gln Ser Val Leu Lys Asn

2315                2320                2325

Asn Arg Ser Ser Leu Lys Gly Asp Phe Thr Asp Ser Ala Glu Leu
    2330                2335                2340

His Leu Val Leu His Asp Ile Ser Gly Asn Pro Ile Lys Val Ser
    2345                2350                2355

Glu Gly Leu Glu Phe Val Gln Ser Gly Thr Asn Ala Pro Tyr Val
    2360                2365                2370

Gln Val Ser Ala Ile Asp Tyr Ser Lys Asn Phe Ser Gly Glu Tyr
    2375                2380                2385

Lys Ala Thr Val Thr Gly Gly Gly Glu Gly Ile Ala Thr Leu Ile
    2390                2395                2400

Pro Val Leu Asn Gly Val His Gln Ala Gly Leu Ser Thr Thr Ile
    2405                2410                2415

Gln Phe Thr Arg Ala Glu Asp Lys Ile Met Ser Gly Thr Val Leu
    2420                2425                2430

Val Asn Gly Ala Asn Leu Pro Thr Thr Thr Phe Pro Ser Gln Gly
    2435                2440                2445

Phe Thr Gly Ala Tyr Tyr Gln Leu Asn Asn Asp Asn Phe Ala Pro
    2450                2455                2460

Gly Lys Thr Ala Ala Asp Tyr Glu Phe Ser Ser Ser Ala Ser Trp
    2465                2470                2475

Val Asp Val Asp Ala Thr Gly Lys Val Thr Phe Lys Asn Val Gly
    2480                2485                2490

Ser Lys Trp Glu Arg Ile Thr Ala Thr Pro Lys Thr Gly Gly Pro
    2495                2500                2505

Ser Tyr Ile Tyr Glu Ile Arg Val Lys Ser Trp Trp Val Asn Ala
    2510                2515                2520

Gly Asp Ala Phe Met Ile Tyr Ser Leu Ala Glu Asn Phe Cys Ser
    2525                2530                2535

Ser Asn Gly Tyr Thr Leu Pro Leu Gly Asp His Leu Asn His Ser
    2540                2545                2550

Arg Ser Arg Gly Ile Gly Ser Leu Tyr Ser Glu Trp Gly Asp Met
    2555                2560                2565

Gly His Tyr Thr Thr Glu Ala Gly Phe His Ser Asn Met Tyr Trp
    2570                2575                2580

Ser Ser Ser Pro Ala Asn Ser Asn Glu Gln Tyr Val Val Ser Leu
    2585                2590                2595

Ala Thr Gly Asp Gln Ser Val Phe Glu Lys Leu Gly Phe Ala Tyr
    2600                2605                2610

Ala Thr Cys Tyr Lys Asn Leu
    2615                2620

<210> 45
<211> 1678
<212> PRT
<213> Escherichia coli

<400> 45

Met Pro Tyr Thr Leu Gly Ala Leu Glu Ser Ala Gln Ser Val Ala Asn
1               5                   10                  15

Arg Phe Gly Ile Ser Leu Glu Glu Leu Arg Arg Leu Asn Gln Phe Arg
            20                  25                  30

Thr Phe Ala Arg Gly Phe Asp Asn Val Arg Gln Gly Glu Glu Leu Asp
        35                  40                  45

Val Pro Ala Thr Thr Ser Gln Lys Ser His Glu Gln Gln Asn Ala Val
    50                  55                  60

Pro Pro Ala Asn Gly Glu Asn Thr Leu Glu Asn Gln Ile Ala Ser Thr
65                  70                  75                  80

Ser Gln Arg Val Gly Thr Leu Leu Ser Gln Asp Met Asn Ser Glu Gln
                85                  90                  95

Ala Ser Gly Met Ala Arg Gly Trp Ala Ser Ser Glu Ala Ser Gly Ala
            100                 105                 110

Met Thr Asp Trp Leu Asn Asn Phe Gly Thr Ala Arg Ile Ser Leu Gly
        115                 120                 125

Val Asp Glu Asp Phe Ser Leu Lys Asn Ser Gln Phe Asp Phe Leu His
    130                 135                 140

Pro Trp Tyr Asp Thr Pro Asp Tyr Leu Leu Phe Ser Gln His Thr Leu
145                 150                 155                 160

His Arg Thr Asp Asp Arg Thr Gln Ile Asn Thr Gly Leu Gly Trp Arg
                165                 170                 175

His Phe Thr Ser Ser Trp Met Ser Gly Ile Asn Leu Phe Phe Asp His
            180                 185                 190

Asp Leu Ser Arg Tyr His Ser Arg Ala Gly Leu Gly Ala Glu Tyr Trp
        195                 200                 205

Arg Asp Tyr Leu Lys Leu Ser Ser Asn Ala Tyr Ile Gly Leu Thr Gly
    210                 215                 220

Trp Arg Ser Ala Pro Glu Leu Asp Asn Asp Phe Glu Ala Arg Pro Ala
225                 230                 235                 240

Asn Gly Trp Asp Leu Arg Ala Glu Gly Trp Leu Pro Ala Trp Pro Gln
                245                 250                 255

Leu Gly Gly Lys Leu Val Tyr Glu Gln Tyr Tyr Gly Asp Glu Val Ala
            260                 265                 270

Leu Phe Asp Lys Asn Asp Arg Gln Ser Asn Pro His Ala Ile Thr Ala
        275                 280                 285

Gly Leu Asn Tyr Thr Pro Phe Pro Leu Leu Thr Leu Ser Ala Glu Gln
    290                 295                 300

Arg Gln Gly Lys Gln Gly Glu Asn Asp Thr Arg Phe Ala Val Asp Leu
305                 310                 315                 320

Thr Trp Gln Pro Ser Ser Ser Met Gln Lys Gln Leu Asn Pro Asp Glu
                325                 330                 335

Val Ala Gly Arg Arg Ser Leu Ala Gly Ser Arg Tyr Asp Leu Ile Asp
            340                 345                 350

Arg Asn Asn Asn Ile Val Leu Glu Tyr Arg Lys Lys Glu Leu Ile Arg
        355                 360                 365

Leu Ser Leu Leu Asp Pro Val Lys Gly Lys Ser Gly Glu Ile Lys Pro
    370                 375                 380

Leu Val Ser Ser Leu Gln Thr Lys Tyr Ala Leu Lys Gly Tyr Asn Ile
385                 390                 395                 400

Glu Ala Ala Ala Leu Glu Ala Ala Gly Gly Lys Val Ser Thr Ser Gly
                405                 410                 415

Lys Asp Ile Thr Val Thr Leu Pro Gly Tyr Arg Phe Thr Asn Thr Pro
            420                 425                 430

Glu Thr Asp Asn Thr Trp Ser Ile Asp Val Thr Ala Glu Asp Val Lys
        435                 440                 445

Gly Asn Leu Ser Arg His Glu Gln Ser Met Val Val Ile Gln Ala Pro
    450                 455                 460

Thr Leu Ser Gln Lys Asp Ser Leu Leu Ser Val Asn Pro Leu Thr Val
465                 470                 475                 480

Ala Ala Asp Lys Lys Ser Thr Thr Thr Leu Thr Val Thr Ala His Asp
                485                 490                 495

Ser Asp Gly Thr Pro Val Pro Gly Leu Ala Leu Gln Thr Arg Ser Glu
            500                 505                 510

Gly Val Gln Asp Ile Thr Leu Ser Asp Trp Thr Asp Asn Gly Asp Gly
        515                 520                 525

Ser Tyr Thr Gln Ile Leu Thr Ala Gly Thr Thr Ser Gly Ser Val Thr
    530                 535                 540

Leu Thr Pro Gln Ile Asn Gly Glu Ser Ala Val Lys Glu Ser Ile Val
545                 550                 555                 560

Val Asn Ile Val Pro Val Val Ser Ser Arg Asp His Ser Ser Ile Thr
                565                 570                 575

Ile Asp Asn Val Ser Tyr Tyr Ala Gly Asp Asp Ile Lys Val Arg Val
            580                 585                 590

Glu Leu Lys Asp Asp Ser Asn Gln Pro Val Ala Tyr Gln Lys Glu Glu
        595                 600                 605

Leu Val Lys Ala Val Thr Val Glu Asn Ser Lys Pro Gly Ala Thr Ile
    610                 615                 620

Val Trp His Glu Glu Gln Pro Gly Val Tyr Ala Ala Asn Tyr Pro Ala
625                 630                 635                 640

Tyr Lys Gln Gly Thr Ala Leu Arg Ala Gln Leu Ser Leu His Asn Trp
                645                 650                 655

Asn Ala Pro Leu Gln Ser His Ile Tyr Asn Ile Glu Ala Asn Gln Asn
            660                 665                 670

Lys Ala Arg Val Ala Thr Leu Ser Ala Thr Asn Asn Asp Val Tyr Ala
        675                 680                 685

Asp Lys Lys Thr Phe Asn Thr Leu Thr Ile Asn Val Thr Asp Glu Ser
    690                 695                 700

Asp Asn Pro Leu Thr Asn His Gln Val Thr Phe Lys Asn Glu Lys Gly
705                 710                 715                 720

Ser Ala Glu Phe Val Glu Pro Pro Gln Gln Asn Thr Asp Ala Tyr Gly
                725                 730                 735

Val Ala Thr Ile Asn Met Val Ser Gln Val Ala Glu Glu Asn Thr Ile
            740                 745                 750

Ser Ala Thr Leu Pro Asn Gly Phe Ser Gln Arg Ile Ile Ala Lys Phe
        755                 760                 765

Val Ser Asp Ser Ser Thr Pro Lys Phe Lys Gln Leu Val Ala Asp Pro
    770                 775                 780

Asp Thr Ile Ile Ala Gly Asn Ser Gln Gly Ser Thr Leu Thr Ala Ile
785                 790                 795                 800

Ile Thr Asp Phe His Asn Asn Pro Leu Lys Asp Met Lys Val Asn Phe
                805                 810                 815

Val Ala Pro Gly Gly Ser Gln Leu Asp Asn Thr Thr Ala Thr Thr Asp
            820                 825                 830

Gln Ser Gly Ile Val Arg Val His Leu Thr Ser Ser Lys Ala Gly Ser
        835                 840                 845

Tyr Ser Val Asp Ala Ser Leu Glu Val Asp Lys Asn Ile His Gln Ser
    850                 855                 860

Val Thr Ile Thr Val Val Pro Asn Arg Glu Gln Ser Val Met Thr Leu
865                 870                 875                 880

Asn Ala Gly Ser Gly Ser Ala Ile Ala Asn Asn Thr Asn Ile Val Thr
                885                 890                 895

Leu Thr Ala Ser Val Lys Asp Val Tyr Gly His Pro Leu Pro Asp Glu

            900                 905                 910

Asp Val Lys Phe Thr Leu Pro Ala Ser Met Thr Gly Asn Phe Thr Leu
        915                 920                 925

Ser Ser Glu Thr Ala Arg Thr Asp Ala Asn Gly Asp Ala Val Val Thr
    930                 935                 940

Leu Arg Gly Thr Lys Ala Gly Glu Phe Thr Val Thr Ala Thr Leu Thr
945                 950                 955                 960

Arg Asn Asn Thr Val Ala Tyr Gln Gln Val Thr Phe Ile Gly Asp Thr
                965                 970                 975

Asn Ser Ala Gln Leu Gln Pro Leu Thr Ala Ser Leu Asn Ser Ile Val
            980                 985                 990

Ala Gly Asn Ser Thr Gly Ser Thr Leu Thr Ala Thr Ile Leu Asp Ala
        995                 1000                1005

Tyr Gln Asn Pro Leu Lys Asp Gln Leu Val Thr Phe Gln Ser Asn
    1010                1015                1020

Asp Val Thr Leu Ser Glu Thr Glu Val Thr Thr Asn Thr Leu Gly
    1025                1030                1035

Gln Ala Thr Val Thr Met Thr Ser Asn Ile Ala Gly Gln His Asn
    1040                1045                1050

Val Val Val Ser Arg Lys Ala Gln Ala Ser Asp Asn Lys Thr Phe
    1055                1060                1065

Ser Leu Ser Val Leu Pro Asp Glu Ser Ser Ala Lys Val Ile Ser
    1070                1075                1080

Ile Thr Gly Ala Glu Lys Thr Ile Thr Val Gly Glu Asn Ile Thr
    1085                1090                1095

Leu Arg Ile Leu Val Gln Asp Ala Phe Asn Asn Val Ile Ala Gly
    1100                1105                1110

Gln Arg Val Arg Leu Ser Ala Gln Pro Thr Thr Asn Ile Thr Ile
    1115                1120                1125

Gly Asp Thr Ala Tyr Thr Asp Asn Asn Gly Tyr Ala Tyr Val Asn
    1130                1135                1140

Leu Leu Ser Thr Gln Pro Gly Val Tyr Gln Val Thr Ala Thr Leu
    1145                1150                1155

Asp Asn Asn Ser Ser Ser Lys Val Asp Val Asn Val Ala Asn Gly
    1160                1165                1170

Lys Leu Glu Leu Thr Ser Ser Lys Pro Glu Thr Thr Val His Asn
    1175                1180                1185

Ser Glu Gly Ile Thr Leu Thr Ala Thr Ala Arg Asn Ala Arg Gly
    1190                1195                1200

Glu Leu Met Pro Gly Gln Ile Ile Thr Phe Ser Val Thr Pro Glu
    1205                1210                1215

Gly Ala Thr Leu Ser Asn Thr Gly Glu Val Leu Thr Asp Gln Ser
    1220                1225                1230

Gly Gln Ala Lys Val Thr Leu Thr Ser Asp Lys Val Asn Val Tyr
    1235                1240                1245

Thr Val Thr Ala Ile Met Gly Lys Asp Val Pro Val Gln Ser Gln
    1250                1255                1260

Val Thr Val Ala Val Lys Ala Asp Ala Lys Thr Ala His Val Val
    1265                1270                1275

Ser Val Val Ala Ser Pro Asp Thr Ile Thr Ala Asp Gly Ile Asp
    1280                1285                1290

Ser Ser Thr Ile Thr Ser Arg Val Glu Asp Asp Tyr Gly Phe Pro
    1295                1300                1305

Val Glu Gly Val Asp Ile Ser His Gly Leu Asp Thr Lys Gly Ser
    1310                1315                1320

Pro Val Val Asn Ile Pro Thr Thr Arg Thr Asp Gln Ser Gly Gln
    1325                1330                1335

Val Thr Ala Thr Ile Thr Ser Thr Leu Ala Glu Thr Leu Thr Val
    1340                1345                1350

Asn Val Gln Val Pro Gly Thr Ala Asn Gln Ser Ala Thr Ile Thr
    1355                1360                1365

Leu Val Ala Gly Thr Ala Asp Glu Ser Lys Ser Ile Leu Lys Ser
    1370                1375                1380

Asp Val Asp Thr Leu Lys Ala Asp Tyr Gln Gln Ser Ala Lys Leu
    1385                1390                1395

Thr Leu Thr Leu Gln Asp Lys Tyr Gly Asn Pro Ile Val Thr Ser
    1400                1405                1410

Asp His Leu Glu Phe Val Gln Ser Gly Pro Phe Val Asn Phe Leu
    1415                1420                1425

Lys Leu Ser Asp Ile Asp Tyr Ser Gln Arg Asn Tyr Gly Glu Tyr
    1430                1435                1440

Thr Val Thr Val Thr Gly Gly Lys Glu Gly Thr Ala Thr Leu Ile
    1445                1450                1455

Pro Met Leu Asn Gly Val His Gln Ala Asn Leu Ser Ile Ser Leu
    1460                1465                1470

Asn Leu Ile Gln Ser Ile Lys Glu Met Ser Gly His Val Thr Ala
    1475                1480                1485

Asn Asn His Thr Phe Ser Thr Ala Lys Phe Pro Ser Glu Gly Phe
    1490                1495                1500

Ala Gly Ala Tyr Tyr Thr Leu Asn Asn Asp Asn Phe Glu Ala Gly
    1505                1510                1515

Lys Thr Val Asp Asp Tyr Met Phe Ser Ser Ser Gln Gly Trp Val
    1520                1525                1530

Ser Val Asp Ala Ser Gly Lys Val Ser Phe Ala Asn Ile Gly Asp
    1535                1540                1545

Gln Thr Ser Val Thr Ile Ser Ala Val Pro Arg Gln Gly Gly Thr
    1550                1555                1560

Thr Tyr Gln Thr Leu Ile Lys Leu Lys Gly Trp Trp Val Asn Asn
    1565                1570                1575

Gly Asn His Thr Asn Ile Trp Leu Ala Ala Asn Ala Leu Cys His
    1580                1585                1590

Ala Lys Asn Asp Gly Tyr Asn Leu Pro Gly Ile Thr His Leu Thr
    1595                1600                1605

Ser Gly Glu Asn Lys Arg Thr Gln Gly Ser Leu Tyr Gly Glu Trp
    1610                1615                1620

Gly Asn Val Gly Ala Phe Ser Ser Asn Ser Gln Phe Thr Pro Gly
    1625                1630                1635

Ala Tyr Trp Thr Ser Glu Ser Asp Asp Tyr Ser Arg His Tyr Tyr
    1640                1645                1650

Val Gln Met Leu Thr Gly Met Thr Gly Ser Asp Ala Asp Ser Ser
    1655                1660                1665

Pro Gln Leu Thr Ala Cys Arg Lys Ser Leu
    1670                1675

<210> 46
<211> 802
<212> PRT
<213> Escherichia coli

<400> 46

Met Val Ala Lys Leu Lys Pro Asp Val Phe Val Asn Thr Asn Pro Phe
1               5                   10                  15

Leu Glu Ala Met Tyr Lys Glu Arg Leu Ser His Lys Gly Tyr Ser Asp
            20                  25                  30

Lys Ile Ala Leu Ser Ile Asn Asp Lys Lys Tyr Asn Val Asn Ser Lys
        35                  40                  45

Asp Ile Glu Asn Ile Leu Asp Gly Lys Gly Asp Leu Phe Lys Lys Arg
    50                  55                  60

Thr Leu Trp Glu Phe Val Arg Asp Leu Phe Pro Gly Ser His Ile Lys
65                  70                  75                  80

Glu Val Lys Gly Leu Ile Tyr Glu Phe Val Thr Lys Val Asp Asn Lys
                85                  90                  95

Ala Glu Val Phe Asp Lys Ile Lys Ser Leu Ala Lys Lys Glu Gln Gln
            100                 105                 110

Trp Arg Phe Ser Thr Lys Thr Asp Phe Thr Thr Asn Glu Asn Asn Glu
        115                 120                 125

Val Ile Val Ser Arg Ser Phe Asn Leu Tyr Thr Gly Ala Thr Ser Asn
    130                 135                 140

Asp Asn Glu Lys Lys Gln Val Ser Ser Glu Arg Leu Thr Leu Asp Asn
145                 150                 155                 160

Tyr Leu Asp Asp Leu His Phe Asp Asn Ser Pro Leu Val Arg Leu Thr
                165                 170                 175

Phe Asp Asn Tyr Ser Val Lys Leu Ala Thr Leu Ile Lys Asn Lys Ile
            180                 185                 190

Pro Ile Ile Asn Thr Thr Ile Asn Leu Ser Ser Leu Ser Lys Asp Val
        195                 200                 205

Leu Asn Ser Leu Lys Tyr Cys Ser Phe Lys Asn Val Ile Phe Ser Gly
    210                 215                 220

Val Ile Asn Ser Pro Asn Leu Glu Gly Pro Val Phe Glu Asn Cys Tyr
225                 230                 235                 240

Phe Asp Asp Cys Lys Phe Asn Asn Ile Gln Leu Tyr Glu Thr Asn Asp
                245                 250                 255

Asp Thr Val Glu Ser Gly Asn Lys Lys Pro Ile Lys Gly Met Phe Lys
            260                 265                 270

Gly Cys Phe Ile Ser Lys Cys Lys Ile Glu Asn Tyr Arg Cys Glu Thr
        275                 280                 285

Ser Lys Ile Tyr Thr Val Thr Gln Pro Asp Ser Ile Gln Glu Lys Leu
    290                 295                 300

Gly Ser Tyr Leu Phe Met Gln Ser Phe Val Gln Asp Cys Ile Ile Gln
305                 310                 315                 320

Gly Gly Cys Cys Pro Gly Ser Ser Ile Leu Leu Ser His Phe Tyr Asn
                325                 330                 335

Cys Asn Ile Ile Gly Leu Asp Ala His Gly Met Asp Phe Leu Ala Asn
            340                 345                 350

Ser Phe Asn Lys Ser Asn Tyr Asp Glu Ile Arg Gly Pro Asp Thr Gly
        355                 360                 365

Thr Val Phe Tyr Asn Cys Asn Leu Lys His Ile Lys Ile Asn Asp Gly
    370                 375                 380

Leu Arg Glu Asp Gly Ser Asp Arg Thr Met Phe Asn Pro Lys Glu Tyr
385                 390                 395                 400

Phe Ser Asp Tyr Ile Glu Arg Arg Ser Arg Asn Leu Lys Leu Asp Asp

                405                 410                 415

Asn Phe Ser Lys Cys Val Glu Ser Ile Lys Lys Gly Thr Asp Ser Ser
            420                 425                 430

Phe Ser Lys Lys Asn Thr Glu Asn Asn Asn Val Phe Phe Lys Asn Ser
        435                 440                 445

Asn Leu Ile Gly Ala Asn Leu Gly Tyr Tyr Tyr Ser Gly Asp Arg Cys
    450                 455                 460

Lys Asp Cys Ala Ile Asp Pro Asn Thr Asn Tyr Ser Lys Asp Gly Leu
465                 470                 475                 480

Thr Ala Gln Lys Tyr Met Tyr Met Asp Leu Asp Gly Ala Ile Ser Lys
                485                 490                 495

Glu Gln Ile Pro Asp Phe Leu Lys Lys Val Ser Ala Ile Asn Ile Asp
            500                 505                 510

Val Ile Asn Phe Tyr Asn Ser Glu Ser Lys Thr Lys Glu Lys Tyr Lys
        515                 520                 525

Asn Ala Phe Leu Glu Leu Glu Ser Phe Leu Ser Thr Leu Tyr Val Glu
    530                 535                 540

Asn Lys Ser Tyr Glu Glu Lys Tyr His Phe Asp Asn Ser Arg Val Glu
545                 550                 555                 560

Phe Phe Ile Phe Lys Asp Met Gln Glu Asn Ala Gln Asn Ile Ile Asn
                565                 570                 575

Asn Met Ile Glu Ser Asp Arg Ile Lys Phe Val Glu Ser Ile Ile Asn
            580                 585                 590

Lys Met Ile Pro Pro Pro Asp Gly Thr Ile Leu Thr Glu Asn Pro Lys
        595                 600                 605

Glu Tyr Val Gln Lys Gln Ile Lys Glu Ser His Lys Glu Ser Ala Thr
    610                 615                 620

Asn Val Thr Phe Asp Tyr Lys Glu Leu Ala Pro Ile Phe Glu Gly Val
625                 630                 635                 640

Glu Glu Lys Gln Ile Lys Ala Leu Ser Asn Gln Leu Glu His Ile Lys
                645                 650                 655

Ser Phe Lys Thr Asp Tyr Asp Ser Arg Leu Asn Lys Tyr Ala Arg Asp
            660                 665                 670

Phe Tyr Tyr Phe Ser Ser Ala Phe Ala Ile Asn Trp Glu Asp Leu Leu
        675                 680                 685

Lys Asn Tyr Gln Glu Ile Arg Ala Ser Ile Lys Asp Tyr Asp Asp Leu
    690                 695                 700

Leu Arg Glu Ile Lys Glu Leu Ile Ile Ser Arg Asn Lys Ser Leu Glu
705                 710                 715                 720

Asp Lys Arg Thr Leu Phe Asp Asn Lys Arg Asp Asn Trp Asn Ser Ile
                725                 730                 735

Glu Val Gln Asp Glu Val Asn Ala Leu Asn Ala Lys Ile Val Asp Cys
            740                 745                 750

Asp Asp Lys Ile Arg Ser Lys Leu Thr Ile Val Arg Asn Asn Arg Leu
        755                 760                 765

Glu Asn Gln Tyr Lys Asp Asp Lys Asn Ile Ser Asp Ala Met Arg Asn
    770                 775                 780

Ile Leu Asp Trp Phe Glu Arg Tyr Pro Asp Ile Val Gln Asn Ile Thr
785                 790                 795                 800

Gln Ala

<210> 47
<211> 816
<212> PRT
<213> Escherichia coli

<400> 47

Met Leu Arg Thr Thr Arg Trp Val Ala Ala Ile Ile Phe Leu Tyr Ser
1               5                   10                  15

Phe Pro Gly Tyr Ala Glu Glu Thr Phe Asp Thr His Phe Met Ile Gly
            20                  25                  30

Gly Met Lys Gly Glu Lys Val Ser Glu Tyr His Phe Asp Asn Lys Gln
        35                  40                  45

Pro Leu Pro Gly Asn Tyr Glu Leu Asp Phe Tyr Val Asn Asn Gln Trp
    50                  55                  60

Arg Gly Lys Gln Asp Ile Thr Ile Pro Glu Ser Pro Val Lys Pro Cys
65                  70                  75                  80

Leu Pro Lys Val Leu Leu Thr Lys Leu Gly Val Lys Thr Gly Asn Leu
                85                  90                  95

Asn Thr Glu Asp Asn Cys Ile Leu Leu Asp Lys Ala Val His Gly Gly
            100                 105                 110

Gln Tyr Gln Trp Asp Ile Ser Glu His Arg Leu Asn Leu Thr Val Pro
        115                 120                 125

Gln Ala Tyr Ile Asn Glu Leu Glu Arg Gly Tyr Val Pro Pro Glu Ser
    130                 135                 140

Trp Asp Arg Gly Ile Asp Ala Phe Tyr Thr Ser Tyr Asn Leu Ser Gln
145                 150                 155                 160

Tyr Arg Ser Tyr Asp Ser Asn Asn Asn Ser Asn Thr Ala Ser Tyr Gly
                165                 170                 175

Arg Phe Asn Ser Gly Leu Asn Leu Phe Ser Trp Gln Leu His Ser Asp
            180                 185                 190

Ala Ser Tyr Ser Lys Pro Asp Asp Met Lys Gly Thr Trp Gln Ser Asn
        195                 200                 205

Thr Leu Tyr Leu Glu His Gly Trp Ser Gln Ile Leu Ser Thr Val Gln
    210                 215                 220

Ile Gly Glu Asn Tyr Thr Ser Ser Leu Ile Phe Asp Ser Leu Arg Phe
225                 230                 235                 240

Ser Gly Ile Arg Leu Phe Arg Asp Met Gln Met Leu Pro Asp Ser Met
                245                 250                 255

Gln Ser Phe Thr Pro Leu Val Gln Gly Val Ala Gln Ser Asn Ala Leu
            260                 265                 270

Ile Thr Val Ser Gln Asn Gly Tyr Ile Ile Tyr Gln Lys Glu Val Pro
        275                 280                 285

Pro Gly Pro Phe Thr Ile Ala Asp Leu Gln Leu Ser Gly Ser Gly Ser
    290                 295                 300

Asp Leu Asp Val Ser Ile Lys Glu Ala Asp Gly Ser Val Arg Ser Phe
305                 310                 315                 320

Leu Val Pro Tyr Ser Ser Val Pro Asn Met Leu Gln Pro Gly Ile Ser
                325                 330                 335

Asn Phe Asp Phe Ile Ala Gly Arg Ser Lys Ile Tyr Gly Val Lys Asn
            340                 345                 350

Gln Glu Asp Phe Leu Glu Ala Asn Tyr Ile Tyr Gly Leu Asn Asn Leu
        355                 360                 365

Leu Thr Leu Tyr Gly Gly Thr Ile Leu Ser Asp Asn Tyr Asn Ala Ile
    370                 375                 380

Thr Leu Gly Asn Gly Trp Asn Thr Pro Leu Gly Ala Ile Ser Phe Asp
385                 390                 395                 400

Ala Thr Arg Ser Ser Ser Lys Leu Asn Asn Asp Ile Thr His Glu Gly
                405                 410                 415

Thr Ser Tyr Gln Val Ala Tyr Asn Lys Tyr Leu Val Gln Thr Ala Thr
            420                 425                 430

Arg Phe Ser Val Ala Ala Trp Arg Tyr Ala Ser Gln Asp Tyr Arg Thr
        435                 440                 445

Phe Ser Asp His Leu Tyr Glu Asn Asp Lys Ile Asn His Gln Ser Asp
    450                 455                 460

Tyr Asp Asp Phe Tyr Asp Ile Gly Arg Lys Asn Ser Leu Ser Ala Asn
465                 470                 475                 480

Ile Met Gln Pro Leu Ser Asn Asn Leu Gly Asn Val Ser Leu Ser Ala
                485                 490                 495

Leu Trp Arg Asn Tyr Trp Gly Arg Ser Gly Asn Ala Lys Asp Tyr Gln
            500                 505                 510

Phe Ser Tyr Ser Asn Ser Trp Gln Arg Ile Ser Tyr Thr Phe Ser Ala
        515                 520                 525

Ser Gln Ser Tyr Asp Glu Asn Asp Lys Glu Glu Glu Arg Phe Asn Leu
    530                 535                 540

Phe Ile Ser Ile Pro Phe Tyr Trp Gly Asp Asp Ile Ala Lys Thr Arg
545                 550                 555                 560

His Gln Ile Asn Leu Ser Asn Ser Thr Ser Phe Ser Lys Asp Gly Tyr
                565                 570                 575

Ser Ser Asn Asn Thr Gly Ile Thr Gly Ile Ala Gly Glu His Asp Gln
            580                 585                 590

Leu Asn Tyr Gly Ile Tyr Val Asn Gln Gln Gln Gln Asn Asn Asp Thr
        595                 600                 605

Ser Leu Gly Thr Asn Leu Ser Trp Arg Thr Pro Ile Ala Thr Ile Asp
    610                 615                 620

Gly Ser Tyr Ser His Ser Lys Asn Ala Trp Gln Ser Gly Gly Ser Ile
625                 630                 635                 640

Ser Ser Gly Leu Val Val Trp Pro Gly Gly Ile Asn Ile Thr Asn Gln
                645                 650                 655

Leu Ser Asp Thr Phe Ala Ile Leu Asp Ala Pro Gly Leu Glu Gly Ala
            660                 665                 670

His Ile Asn Gly Gln Lys Tyr Asn Arg Thr Asn Ser Lys Gly Gln Val
        675                 680                 685

Val Tyr Asp Leu Met Ile Pro His Arg Glu Asn His Leu Val Leu Asp
    690                 695                 700

Thr Ala Asn Ser Glu Ser Glu Thr Glu Leu Gln Gly Asn Arg Gln Ile
705                 710                 715                 720

Ile Ala Pro Tyr Arg Gly Ala Val Ser Tyr Val Gln Phe Thr Thr Asp
                725                 730                 735

Gln Arg Lys Pro Trp Tyr Ile Gln Ala Leu Arg Pro Asp Gly Ser Pro
            740                 745                 750

Leu Thr Phe Gly Tyr Asp Val Leu Asp Leu Gln Glu Asn Asn Ile Gly
        755                 760                 765

Val Val Gly Gln Gly Ser Arg Leu Phe Ile Arg Val Asp Glu Ile Pro
    770                 775                 780

Thr Gly Ile Lys Val Ala Leu Asn Asp Glu Gln Asn Leu Phe Cys Thr
785                 790                 795                 800

Ile Thr Phe Gln His Val Ile Asp Glu Asn Lys Thr Tyr Ile Cys Gln
                805                 810                 815

<210>  48

<211>  721
<212>  PRT
<213>  Escherichia coli

<400>  48

Met Thr Ala Asp Glu Arg Asn Val Val Lys Ala Ala Thr Asp Asp Ser
1               5                   10                  15

Met Glu Ala Ala Tyr Met Leu Lys Asp Asn Ile Arg Trp Tyr Tyr His
            20                  25                  30

Asn Gly Asp Leu Ser Leu Pro Ala Asn Phe Ser Asn Lys Asn Lys Leu
        35                  40                  45

Val Val Asn Gly Asn Leu Thr Ile Ser Gly Asp Tyr Asp Asp Tyr Leu
    50                  55                  60

Ser Gly Asn Gly His Leu Ile Val Leu Gly Asn Val Ile Val Asp Asn
65                  70                  75                  80

Phe Ile Asn His Asp Phe Ala Tyr Val Lys Gly Gln Met Thr Ala Lys
                85                  90                  95

Gly Leu Val Tyr Ala Asp Tyr Asn Asp His Asn Phe Glu Val Met Lys
            100                 105                 110

Gly Ile Ser Ala Arg Gly Ile Ile Val Ser Asp Lys Ala Thr Gln Phe
        115                 120                 125

Glu Val Ile Lys Ala Glu Phe Tyr Ile Asn Glu Asp Gly Ser Gly Glu
    130                 135                 140

Gly Tyr Asn Trp Asp Glu Asn Ile Gln Lys Ala Tyr Ser Leu Val Thr
145                 150                 155                 160

Ala Asp Leu Tyr Asp His Thr Glu Ile Glu Thr Asp Asn Ile Ser Asn
                165                 170                 175

Ala Tyr Pro Asp Tyr Asp Ser Val Ala Asp Asn Ile Val Gln Gly Leu
            180                 185                 190

Pro Leu Phe Arg Asp Lys Ala Ala Pro Glu Ile Asn Glu Lys Leu Lys
        195                 200                 205

Trp Ile Glu Thr Gly Lys Leu Asp Asn Phe Pro Ala Asn Lys Ile Lys
    210                 215                 220

His Gln Asp Pro Leu Val Ala Arg Phe Leu Thr His Thr Glu Ser Leu

225                 230                 235                 240

Ser Pro Ala Val Met Leu Gln Leu Leu Gln His Pro Asp Asp Gln Thr
                245                 250                 255

Arg Glu Ser Met Ala Gln Ser Trp Pro Ala Gln Gln Met His Trp Leu
            260                 265                 270

Thr Asp Glu Leu Ile Lys Asp Glu Ala Val Ala Arg Gly Leu Val Lys
        275                 280                 285

Asn Ser Asn Ile Ser Ala Asp Val Asn Lys Lys Leu Met Ser Val Pro
    290                 295                 300

Val Glu Ser Val Gln Leu Glu Gln Ala Arg Gln Asp Asn Leu Ser Pro
305                 310                 315                 320

Asp Ile Val Ala Ser Leu Ser His Ser Pro Phe Leu Ser Val Arg Lys
                325                 330                 335

Thr Leu Leu Ser His Tyr Asp Tyr Ala Trp Leu Val Pro Thr Ala Val
            340                 345                 350

Ala Asp Glu Leu Ile Asn Asn Glu Asp Pro Glu Leu Arg Glu Arg Ile
        355                 360                 365

Thr Gly Ala Asp Leu Thr Ala Gln Gln Ala Val Met Leu Ser Lys Asp
    370                 375                 380

Lys Ser Leu Lys Val Arg Glu Ala Leu Ala Arg Thr Leu Thr Glu Leu
385                 390                 395                 400

Lys Ile Thr Gln Leu Ser Ala Thr Leu Arg Thr Glu Asp Ile Glu Arg
                405                 410                 415

Ile Ala Glu Gln Met Tyr Leu Asp Asn Lys Glu Asn Lys Asn Ile Val
            420                 425                 430

Lys Val Leu Leu Ile Ala Leu Pro Glu Met Arg Gln Leu Ser Leu Ala
        435                 440                 445

Lys Glu Asp Val His Asn Leu Arg Glu Gly Ala Arg Tyr Leu Thr Ser
    450                 455                 460

Lys Asp Val Ile Ser Tyr Leu Leu Thr Gln His Asp Val Pro Thr Val
465                 470                 475                 480

Trp Asp Glu Leu Ala Arg Asp Lys Leu Leu Pro Leu Glu Tyr Lys Lys
                485                 490                 495

Gln Leu Trp Gln Arg Thr Leu Asn Leu Met Met Ser Lys Arg Gln Glu
            500                 505                 510

Asp Gln Glu Gln Ala Tyr Glu Val Gln Leu Ala Leu Ile Asp Asn Gly
        515                 520                 525

Val Val Asp Glu Glu Met Leu Asn Asn Ala Ile Asp Leu Leu Val Asp
    530                 535                 540

Leu Pro Ala Glu Tyr Arg Tyr Arg Met Arg Asn Gln Leu Phe Asp Asn
545                 550                 555                 560

Lys Glu Leu Pro Ser Gly Ile Ile Asn Lys Leu Asp Gln Gln Tyr Arg
                565                 570                 575

Phe Asn Ser Asp Trp Ala Leu Ala Val Val Ser Met Lys Asn Ser Thr
            580                 585                 590

Arg Arg Gln Ser Glu Arg Gly Leu His Arg Trp Asn His Glu Asp Ser
        595                 600                 605

Asp Ile Phe Ala Glu Leu Ala Thr Ile Lys Asp Lys Ser Asp Asp Glu
    610                 615                 620

Trp Trp Arg Ala Leu Leu Gln Ser Arg Asn Asp His Leu Arg Gln Thr
625                 630                 635                 640

Ala Leu Arg Asn Ala His Thr Pro Ala Ser Leu Leu Thr Thr Leu Thr
                645                 650                 655

Glu Ser Gln Asp Arg Ser Leu Ala Ile Asn Asn Pro Gln Leu Ala Ala
            660                 665                 670

Asp Val Lys Thr Val Trp Leu Lys Glu Asp Pro Ser Leu Leu Leu Phe
        675                 680                 685

Val Asp Lys Pro Asp Leu Ser Gln Leu Arg Asp Leu Val Lys Thr Gly
    690                 695                 700

Ala Thr Arg Lys Ile Arg Asn Glu Ala Arg His Arg Leu Glu Glu Lys
705                 710                 715                 720

Gln

<210>  49
<211>  36
<212>  DNA
<213>  Artificial

<220>
<223>  Primer MCRL-34Fw

<400>  49
ctgtacttcc agggctttca accaatactt aatgat                                36


<210>  50
<211>  33
<212>  DNA
<213>  Artificial

<220>
<223>  Primer MCRL-34Rv

<400>  50
aattaagtcg cgttaaaaaa agaaggtgat tgc                                   33


<210>  51
<211>  35
<212>  DNA
<213>  Artificial

<220>
<223>  Primer MCRL-24Fw

<400>  51
ctgtacttcc agggcgatat caatctgtat ggtcc                                 35


<210>  52
<211>  30
<212>  DNA
<213>  Artificial

<220>
<223>  Primer MCRL-24Rv

<400>  52
aattaagtcg cgttaagtgc ctttcctggt                                       30


<210>  53
<211>  31
<212>  DNA
<213>  Artificial

<220>
<223>  Primer MCRL-40Fw

<400>  53
ctgtacttcc agggcgacct ttatggcaag g                                     31


<210>  54
<211>  33

<212>  DNA
<213>  Artificial

<220>
<223>  Primer MCRL-40Rv

<400>  54
aattaagtcg cgttagaact gataggtaat gcc                                    33


<210>  55
<211>  36
<212>  DNA
<213>  Artificial

<220>
<223>  Primer MCRL-35Fw

<400>  55
ctgtacttcc agggcgagtt tacgatagac ttttcg                                 36


<210>  56
<211>  37
<212>  DNA
<213>  Artificial

<220>
<223>  Primer MCRL-35Rv

<400>  56
aattaagtcg cgttatttac ccgttgtata taaaaac                                37


<210>  57
<211>  33
<212>  DNA
<213>  Artificial

<220>
<223>  Primer MCRL-32Fw

<400>  57
ctgtacttcc agggcacgta tgtagattcg ctg                                    33


<210>  58
<211>  33
<212>  DNA
<213>  Artificial

<220>
<223>  Primer MCRL-32Rv

<400>  58
aattaagtcg cgttaactgc taatagttct gcg                                    33


<210>  59
<211>  33
<212>  DNA
<213>  Artificial

<220>
<223>  Primer MCRL-41Fw

<400>  59
ctgtacttcc agggcatgaa gataagctct act                                    33


<210>  60
<211>  30
<212>  DNA
<213>  Artificial

<220>
<223>  Primer MCRL-41Rv

<400>  60
aattaagtcg cgttattcgt aggtaaagga                                        30


<210>  61
<211>  32
<212>  DNA
<213>  Artificial

<220>
<223>  Primer MCRL-28Fw

<400>  61
ctgtacttcc agggccagca actgacagac aa                                     32


<210>  62
<211>  31
<212>  DNA
<213>  Artificial

<220>
<223>  Primer MCRL-28Rv

<400>  62
aattaagtcg cgttactgga atcgactcac c                                      31


<210>  63
<211>  33
<212>  DNA
<213>  Artificial

<220>
<223>  Primer MCRL-36Fw

<400>  63
ctgtacttcc agggcaactg ctattttggt acc                                    33


<210>  64
<211>  36
<212>  DNA
<213>  Artificial

<220>
<223>  Primer MCRL-36Rv

<400>  64
aattaagtcg cgttagttgt agttaatttt gaaaag                                36


<210>  65
<211>  31
<212>  DNA
<213>  Artificial

<220>
<223>  Primer MCRL-38Fw

<400>  65
ctgtacttcc agggcatgtg ggaatgtgat g                                     31


<210>  66
<211>  30
<212>  DNA
<213>  Artificial

<220>
<223>  Primer MCRL-38Rv

<400>  66
aattaagtcg cgttattgca ttttcaccaa                                       30


<210>  67
<211>  31
<212>  DNA
<213>  Artificial

<220>
<223>  Primer MCRL-26Fw

<400>  67
ctgtacttcc agggcacgct gacggtaacg g                                     31


<210>  68
<211>  30
<212>  DNA
<213>  Artificial

<220>
<223>  Primer MCRL-26Rv

<400>  68
aattaagtcg cgttatttcg cacctcgctg                                       30


<210>  69
<211>  30
<212>  DNA
<213>  Artificial

<220>
<223>  Primer MCRL-43Fw

<400>  69
ctgtacttcc agggcacgct ttacgagcag                                       30

<210>  70
<211>  35
<212>  DNA
<213>  Artificial

<220>
<223>  Primer MCRL-43Rv

<400>  70
aattaagtcg cgttatagat agtatttaaa gccgt                                35


<210>  71
<211>  30
<212>  DNA
<213>  Artificial

<220>
<223>  Primer MCRL-22Fw

<400>  71
ctgtacttcc agggcgtggg gatcgacagc                                      30


<210>  72
<211>  32
<212>  DNA
<213>  Artificial

<220>
<223>  Primer MCRL-22Rv

<400>  72
aattaagtcg cgttagaacg accagttcac ac                                   32


<210>  73
<211>  30
<212>  DNA
<213>  Artificial

<220>
<223>  Primer MCRL-42Fw

<400>  73
ctgtacttcc agggcatgaa attcccttca                                      30


<210>  74
<211>  30
<212>  DNA
<213>  Artificial

<220>
<223>  Primer MCRL-42Rv

<400>  74
aattaagtcg cgttagtgat aaaaaggcca                                      30


<210>  75
<211>  31

<212>  DNA
<213>  Artificial

<220>
<223>  Primer MCRL-23Fw

<400>  75
ctgtacttcc agggcggtta ttttgttggc g                                    31


<210>  76
<211>  30
<212>  DNA
<213>  Artificial

<220>
<223>  Primer MCRL-23Rv

<400>  76
aattaagtcg cgttaatcct gcaacgcata                                      30


<210>  77
<211>  33
<212>  DNA
<213>  Artificial

<220>
<223>  Primer MCRL-29Fw

<400>  77
ctgtacttcc agggcattta ttatgcgatg aaa                                  33


<210>  78
<211>  36
<212>  DNA
<213>  Artificial

<220>
<223>  Primer MCRL-29Rv

<400>  78
aattaagtcg cgttacagtt cattcagtac atactg                               36


<210>  79
<211>  33
<212>  DNA
<213>  Artificial

<220>
<223>  Primer MCRL-30Fw

<400>  79
ctgtacttcc agggcatggg tacagcagct ata                                  33


<210>  80
<211>  31
<212>  DNA
<213>  Artificial

<220>
<223>  Primer

<400>  80
aattaagtcg cgttattgta ttcctgcacc a                                    31


<210>  81
<211>  30
<212>  DNA
<213>  Artificial

<220>
<223>  Primer MCRL-37Fw

<400>  81
ctgtacttcc agggcgatgt ccggcgtagc                                      30


<210>  82
<211>  34
<212>  DNA
<213>  Artificial

<220>
<223>  Primer MCRL-37Rv

<400>  82
aattaagtcg cgttactgct ttttacaacc attc                                 34


<210>  83
<211>  30
<212>  DNA
<213>  Artificial

<220>
<223>  Primer MCRL-48Fw

<400>  83
ctgtacttcc agggcatgag cggaaaaccg                                      30


<210>  84
<211>  35
<212>  DNA
<213>  Artificial

<220>
<223>  Primer MCRL-48Rv

<400>  84
aattaagtcg cgttattttc ttattcctct cgatg                                35


<210>  85
<211>  30
<212>  DNA
<213>  Artificial

<220>
<223>  Primer MCRL-52Fw

<400>  85
ctgtacttcc agggcatggc tgcggcagca                                        30


<210>  86
<211>  30
<212>  DNA
<213>  Artificial

<220>
<223>  Primer MCRL-52Rv

<400>  86
aattaagtcg cgttacgcaa tattgacgat                                        30


<210>  87
<211>  30
<212>  DNA
<213>  Artificial

<220>
<223>  Primer MCRL-49Fw

<400>  87
ctgtacttcc agggcgtggg gcagtctaat                                        30


<210>  88
<211>  33
<212>  DNA
<213>  Artificial

<220>
<223>  Primer MCRL-49Rv

<400>  88
aattaagtcg cgttaactcg ctttcattat gtt                                    33


<210>  89
<211>  33
<212>  DNA
<213>  Artificial

<220>
<223>  Primer MCRL-51Fw

<400>  89
ctgtacttcc agggcgtgcc ttacacgctt ggt                                    33


<210>  90
<211>  33
<212>  DNA
<213>  Artificial

<220>
<223>  Primer MCRL-51Rv

<400>  90
aattaagtcg cgttaaagtg atttacggca ggc                                    33

<210>  91
<211>  34
<212>  DNA
<213>  Artificial

<220>
<223>  Primer MCRL-45Fw

<400>  91
ctgtacttcc agggcatggt cgctaaatta aaac                                    34


<210>  92
<211>  33
<212>  DNA
<213>  Artificial

<220>
<223>  Primer MCRL-45Rv

<400>  92
aattaagtcg cgttaagcct gggttatatt aac                                     33


<210>  93
<211>  34
<212>  DNA
<213>  Artificial

<220>
<223>  Primer MCRL-45Fw

<400>  93
ctgtacttcc agggcatggt cgctaaatta aaac                                    34


<210>  94
<211>  33
<212>  DNA
<213>  Artificial

<220>
<223>  Primer MCRL-45Rv

<400>  94
aattaagtcg cgttaagcct gggttatatt aac                                     33


<210>  95
<211>  30
<212>  DNA
<213>  Artificial

<220>
<223>  Primer MCRL-46Fw

<400>  95
ctgtacttcc agggcagtta tggccgattt                                         30


<210>  96
<211>  33

<212>  DNA
<213>  Artificial

<220>
<223>  Primer MCRL-46Rv

<400>  96
aattaagtcg cgttatgtaa actgcacata aga                                    33


<210>  97
<211>  26
<212>  DNA
<213>  Artificial

<220>
<223>  Primer pET-TEV-Fw

<400>  97
taacgcgact taattctagc ataacc                                            26


<210>  98
<211>  21
<212>  DNA
<213>  Artificial

<220>
<223>  Primer pET-TEV-Rv

<400>  98
gccctggaag tacaggtttt c                                                 21


<210>  99
<211>  103
<212>  DNA
<213>  Artificial

<220>
<223>  Primer TolR-Cat-Fw

<400>  99
tggaggtcga tttgccagac gctactgaat cacaggcggt gagcagtaac gataatccgc      60

cagtgattgt tgacatatga atatcctcct tagttcctat tcc                        103


<210>  100
<211>  96
<212>  DNA
<213>  Artificial

<220>
<223>  Primer TolR-Cat-Rv

<400>  100
ccttgaaacg gctggacact tccgccacca cctgctctgg tggtaaacgc tccaggcgat      60

ctttctcaac cagtgtaggc tggagctgct tcgaag                                96


<210>  101

<211>  39
<212>  DNA
<213>  Artificial

<220>
<223>  Primer GMMA-MC001Fw

<400>  101
caggaggaat taaccatgaa aaaaagtgtc atcgctggc                              39


<210>  102
<211>  39
<212>  DNA
<213>  Artificial

<220>
<223>  Primer GMMA-MC001Rv-NONhis

<400>  102
atgatgatga tgatgatgaa aaaagaaggt gattgctcc                              39


<210>  103
<211>  38
<212>  DNA
<213>  Artificial

<220>
<223>  Primer GMMA-MC007Fw

<400>  103
caggaggaat taaccatggg gcagtctaat aataccac                               38


<210>  104
<211>  39
<212>  DNA
<213>  Artificial

<220>
<223>  Primer GMMA-MC007Rv-NONhis

<400>  104
atgatgatga tgatgatgga acgaccagtt cacaccagc                              39


<210>  105
<211>  39
<212>  DNA
<213>  Artificial

<220>
<223>  Primer GMMA-MC020Fw

<400>  105
caggaggaat taaccatggg gcagtctaat aataccacc                              39


<210>  106
<211>  38
<212>  DNA
<213>  Artificial

<220>
<223>  Primer GMMA-MC020Rv-NONhis

<400>  106
atgatgatga tgatgatgga acgaccagtt cacaccag                              38


<210>  107
<211>  68
<212>  DNA
<213>  Artificial

<220>
<223>  Primer MC001tagFw

<400>  107
gattacaaag acgatgatga caaggattac aaagacgatg atgacaagaa cagccttgca      60

ttatcatt                                                               68


<210>  108
<211>  68
<212>  DNA
<213>  Artificial

<220>
<223>  Primer MC001tagRv

<400>  108
cttgtcatca tcgtctttgt aatccttgtc atcatcgtct ttgtaatccg cgatagctga      60

acacgtgg                                                               68


<210>  109
<211>  68
<212>  DNA
<213>  Artificial

<220>
<223>  Primer MC007tagFw

<400>  109
gattacaaag acgatgatga caaggattac aaagacgatg atgacaagtc tgaatcttct      60

attgatga                                                               68


<210>  110
<211>  68
<212>  DNA
<213>  Artificial

<220>
<223>  Primer MC007tagRv

<400>  110
cttgtcatca tcgtctttgt aatccttgtc atcatcgtct ttgtaatcgg cacatcctgc      60

aagcagcg                                                               68

<210>  111
<211>  68
<212>  DNA
<213>  Artificial

<220>
<223>  Primer MC020tagFw

<400>  111
gattacaaag acgatgatga caaggattac aaagacgatg atgacaaggc cttcactcct      60

gatgttat                                                                68


<210>  112
<211>  68
<212>  DNA
<213>  Artificial

<220>
<223>  Primer MC020tagRv

<400>  112
cttgtcatca tcgtctttgt aatccttgtc atcatcgtct ttgtaatcag ccatcccggg      60

cggggcat                                                                68


<210>  113
<211>  25
<212>  DNA
<213>  Artificial

<220>
<223>  Primer pBADRv

<400>  113
catggttaat tcctcctgtt agccc                                             25


<210>  114
<211>  25
<212>  DNA
<213>  Artificial

<220>
<223>  Primer pBADFwNonHIS

<400>  114
tgagtttaaa cggtctccag cttgg                                             25


<210>  115
<211>  296
<212>  PRT
<213>  Salmonella enterica

<400>  115

Ser Ser Leu Ala Ile Ser Val Ala Asn Asp Asp Ala Gly Ile Phe Gln
1               5                   10                  15

Pro Ser Leu Asn Ala Leu Tyr Gly His Pro Ala Ala Asp Arg Gly Asp
            20                  25                  30

Tyr Thr Ala Gly Leu Phe Leu Gly Tyr Ser His Asp Leu Thr Asp Ala
        35                  40                  45

Ser Gln Leu Ser Phe His Ile Ala Gln Asp Ile Tyr Ser Pro Ser Gly
    50                  55                  60

Ala Asn Lys Arg Lys Pro Glu Ala Val Lys Gly Asp Arg Ala Phe Ser
65                  70                  75                  80

Ala Phe Leu His Thr Gly Leu Glu Trp Asn Ser Leu Ala Thr Asn Trp
                85                  90                  95

Leu Arg Tyr Arg Leu Gly Thr Asp Ile Gly Val Ile Gly Pro Asp Ala
            100                 105                 110

Gly Gly Gln Glu Val Gln Asn Arg Ala His Arg Ile Ile Gly Ala Glu
        115                 120                 125

Lys Tyr Pro Ala Trp Gln Asp Gln Ile Glu Asn Arg Tyr Gly Tyr Thr
    130                 135                 140

Ala Lys Gly Met Val Ser Leu Thr Pro Ala Ile Asp Ile Leu Gly Val
145                 150                 155                 160

Asn Val Gly Phe Tyr Pro Glu Val Ser Ala Val Gly Gly Asn Leu Phe
                165                 170                 175

Gln Tyr Leu Gly Tyr Gly Ala Thr Val Ala Leu Gly Asn Asp Lys Thr
            180                 185                 190

Phe Asn Ser Asp Asn Gly Phe Gly Leu Leu Ser Arg Arg Gly Leu Ile
        195                 200                 205

His Thr Gln Lys Glu Gly Leu Ile Tyr Lys Val Phe Ala Gly Val Glu
    210                 215                 220

Arg Arg Glu Val Asp Lys Asn Tyr Thr Leu Gln Gly Lys Thr Leu Gln
225                 230                 235                 240

Thr Lys Met Glu Thr Val Asp Ile Asn Lys Thr Val Asp Glu Tyr Arg
                245                 250                 255

Val Gly Ala Thr Ile Gly Tyr Ser Pro Val Ala Phe Ser Leu Ser Leu
            260                 265                 270

Asn Lys Val Thr Ser Glu Phe Arg Thr Gly Asp Asp Tyr Ser Tyr Ile
        275                 280                 285

Asn Gly Asp Ile Thr Phe Phe Phe
    290                 295

<210>  116
<211>  8
<212>  PRT
<213>  Artificial

<400>  8

Asp Tyr Lys Asp Asp Asp Asp Lys
1               5

## Description

### FIELD OF THE INVENTION

The present invention relates to novel enterohaemorrhagic *Escherichia coli* (EHEC) antigens and their use in treating disease.

### BACKGROUND TO THE INVENTION

*Escherichia coli* enterohemorrhagic (EHEC) is an etiologic agent for anthropozoonotic diarrheal disease and hemorrhagic colitis. EHEC infections occur mainly in developed countries; the most common O157:H7 serotype and other non-157 serogroups often have been implicated in human outbreaks (Kaper et al., 2004; Moriel et al., 2012; Croxen et al., 2013). The main reservoir of EHEC is ruminants, and anthropozoonotic infection mainly occurs from direct or indirect contamination of food products with animal faeces (Rivas et al., 2016). EHEC strains are characterized for the expression of shiga-like toxin (Stx), this being the hallmark of the pathotype. Some strains also carry the Locus of Enterocyte Effacement (LEE) that encodes the type three secretion system (T3SS) responsible for the attachment and effacing (A/E) lesions on the intestinal microvilli (Kaper et al., 2004).

Symptoms of infection include the development of the hemolytic uremic syndrome (HUS) and later renal failure, due mainly to the Stx (Tarr et al., 2005). Although the use of antibiotic remains a key intervention strategy to treat many bacterial infections, this therapy is not recommended in diseases caused by EHEC (Goldwater and Bettelheim, 2012; Rivas et al., 2016).

Furthermore, the increasing burden of these *E. coli* diarrheal diseases, the emergence of hybrid strains (genomic plasticity), and the increasing annual cost for the health care systems point out the need to develop effective therapeutic and preventive strategies. Among these, vaccination is a promising strategy to control disease (Croxen and Finlay, 2010; Moriel et al., 2012; Croxen et al., 2013; Rappuoli et al., 2014).

A number of antigen candidates for potential EHEC vaccine use have been identified. Virulence factors expressed as recombinant proteins (such as Shiga-toxin (Stx), Intimin and *E. coli* secreted protein A (EspA)) and avirulent ghost cells of EHEC O157:H7 have been tested using different routes of immunization and adjuvant combinations in several animal models providing encouraging results (Rojas-Lopez et al., 2018, Front. Microbiol., 2018; 9: 440, doi: 10.3389/fmicb.2018.00440).

To date, no human EHEC vaccine has been brought to market. Hence, there is a continued need to identify novel antigens for vaccine development against infections caused by EHEC.

### DESCRIPTION OF THE INVENTION

Novel antigens have been identified from the EHEC O157:H7 genome.

A first aspect of the invention provides an isolated polypeptide comprising or consisting of:
(a) Group A polypeptides: an amino acid sequence selected from the group consisting of SEQ ID NOs: 25, 26, 27, 28, 29, 30, 31, 32, 33, 34, 35, 36, 37, 38, 39, 40, 41, 42, 43, 44, 45, 46, 47 or 48; or
(b) Group B polypeptides: a variant and/or fragment of (Group A), for example:
   (i) a variant of (Group A);
   (ii) a fragment of (Group A);
   (iii) a variant of a fragment of (Group A).

The term 'amino acid' as used herein includes the standard twenty genetically-encoded amino acids and their corresponding stereoisomers in the 'D' form (as compared to the natural 'L' form), omega-amino acids and other naturally-occurring amino acids, unconventional amino acids (e.g. α,α-disubstituted amino acids, N-alkyl amino acids, etc.) and chemically derivatised amino acids (see below).

Thus, when an amino acid is being specifically enumerated, such as 'alanine' or 'Ala' or 'A', the term refers to both L-alanine and D-alanine unless explicitly stated otherwise. Other unconventional amino acids may also be suitable components for polypeptides of the present invention, as long as the desired functional property is retained by the polypeptide. For the peptides shown, each encoded amino acid residue, where appropriate, is represented by a single letter designation, corresponding to the trivial name of the conventional amino acid.

By "isolated" we mean that the feature (e.g., the polypeptide) of the invention is provided in a context other than that in which it may be found naturally. One of skill in the art would understand that "isolated" means altered "by the hand of man" from its natural state, i.e., if it occurs in nature, it has been changed or removed from its original environment, or both. For example, a polynucleotide or a polypeptide naturally present in a living organism is not "isolated" when in such living organism, but the same polynucleotide or polypeptide separated from the coexisting materials of its natural state is "isolated," as the term is used in this disclosure. Further, a polynucleotide or polypeptide that is introduced into an organism by transformation, genetic manipulation or by any other recombinant method would be understood to be "isolated" even if it is still present in said organism, which organism may be living or non-living, except where such transformation, genetic manipulation or other recombinant method produces an organism that is otherwise indistinguishable from the naturally-occurring organism.

By 'variant' of the polypeptide we include insertions, deletions and/or substitutions, either conservative or non-conservative. In particular, the variant polypeptide may be a non-naturally occurring variant (i.e., does not, or is not known to, occur in nature).

"Sequence identity" can be determined by the Smith Waterman homology search algorithm as implemented in the MPSRCH program (Oxford Molecular), using an affine gap search with parameters gap open penalty=12 and gap extension penalty=1, orby the Needleman-Wunsch global alignment algorithm (see e.g. Rubin (2000) Pediatric. Clin. North Am. 47:269-285), using default parameters (e.g. with Gap opening penalty = 10.0, and with Gap extension penalty = 0.5, using the EBLOSUM62 scoring matrix). This algorithm is conveniently implemented in the needle tool in the EMBOSS package. Unless specified otherwise, where the application refers to sequence identity to a particular sequence, the identity is intended to be calculated over the entire length of that sequence. Alternatively, percent identity can be determined by methods well known in the art, for example using the LALIGN program (Huang and Miller, Adv. Appl. Math. (1991) 12:337-357, the disclosures of which are incorporated herein by reference) at the ExPASy facility website www.ch.embnet.org/software/LALIGN_form.html using as parameters the global alignment option, scoring matrix BLOSUM62, opening gap penalty -14, extending gap penalty -4. Alternatively, the percent sequence identity between two polypeptides may be determined using suitable computer programs, for example AlignX, Vector NTI Advance 10 (from Invitrogen Corporation) or the GAP program (from the University of Wisconsin Genetic Computing Group).

It will be appreciated that percent identity is calculated in relation to polymers (e.g., polypeptide or polynucleotide) whose sequence has been aligned.

Fragments and variants may be made using the methods of protein engineering and site-directed mutagenesis well known in the art (for example, see Molecular Cloning: a Laboratory Manual, 3rd edition, Sambrook & Russell, 2001, Cold Spring Harbor Laboratory Press, the disclosures of which are incorporated herein by reference).

It will be appreciated by skilled persons that the polypeptide of the invention, or fragment, variant or fusion thereof, may comprise one or more amino acids that are modified or derivatised. Thus, the polypeptide may comprise or consist of a derivative of the amino acid sequences of Group A or of a fragment or variant thereof.

Chemical derivatives of one or more amino acids may be achieved by reaction with a functional side group. Such derivatised molecules include, for example, those molecules in which free amino groups have been derivatised to form amine hydrochlorides, p-toluene sulphonyl groups, carboxybenzoxy groups, t-butyloxycarbonyl groups, chloroacetyl groups or formyl groups. Free carboxyl groups may be derivatised to form salts, methyl and ethyl esters or other types of esters and hydrazides. Free hydroxyl groups may be derivatised to form O-acyl or O-alkyl derivatives. Also included as chemical derivatives are those peptides which contain naturally occurring amino acid derivatives of the twenty standard amino acids. For example: 4-hydroxyproline may be substituted for proline; 5-hydroxylysine may be substituted for lysine; 3-methylhistidine may be substituted for histidine; homoserine may be substituted for serine and ornithine for lysine. Derivatives also include peptides containing one or more additions or deletions as long as the requisite activity is maintained. Other included modifications are amidation, amino terminal acylation (e.g. acetylation or thioglycolic acid amidation), terminal carboxylamidation (e.g. with ammonia or methylamine), and the like terminal modifications.

It will be further appreciated by persons skilled in the art that peptidomimetic compounds may also be useful. Thus, by 'polypeptide' we include peptidomimetic compounds which exhibit endolysin activity. The term 'peptidomimetic' refers to a compound that mimics the conformation and desirable features of a particular polypeptide as a therapeutic agent.

For example, the polypeptides described herein include not only molecules in which amino acid residues are joined by peptide (-CO-NH-) linkages but also molecules in which the peptide bond is reversed. Such retro-inverso peptidomimetics may be made using methods known in the art, for example such as those described in Meziere et al. (1997) J. Immunol. 159, 3230-3237, the disclosures of which are incorporated herein by reference. Such retro-inverse peptides, which contain NH-CO bonds instead of CO-NH peptide bonds, are much more resistant to proteolysis. Alternatively, the polypeptide of the invention may be a peptidomimetic compound wherein one or more of the amino acid residues are linked by a -γ(CH2NH)-bond in place of the conventional amide linkage.

It will be appreciated that the polypeptide may conveniently be blocked at its N- or C-terminus so as to help reduce susceptibility to exoproteolytic digestion, e.g., by amidation.

As discussed herein, a variety of uncoded or modified amino acids such as D-amino acids and N-methyl amino acids may be used to modify polypeptides of the invention. In addition, a presumed bioactive conformation may be stabilised by a covalent modification, such as cyclisation or by incorporation of lactam or other types of bridges. Methods of synthesis of cyclic homodetic peptides and cyclic heterodetic peptides, including disulphide, sulphide and alkylene bridges, are disclosed in US 5,643,872. Other examples of cyclisation methods are discussed and disclosed in US 6,008,058, the relevant disclosures in which documents are hereby incorporated by reference. A further approach to the synthesis of cyclic stabilised peptidomimetic compounds is ring-closing metathesis (RCM).

In summary, terminal modifications are useful, as is well known, to reduce susceptibility by proteinase digestion and therefore to prolong the half-life of the peptides in solutions, particularly in biological fluids where proteases may be present. Polypeptide cyclisation is also a useful modification and is preferred because of the stable structures formed by cyclisation and in view of the biological activities observed for cyclic peptides.

Thus, in one embodiment the polypeptide, or fragment, variant, fusion or derivative thereof, is cyclic. However, in a preferred embodiment, the polypeptide, or fragment, variant, fusion or derivative thereof, is linear.

In a further embodiment of the first aspect of the invention, the polypeptide comprises or consists of a fusion of the amino acid sequence of Group A or Group B polypeptides, or of a fragment, variant or derivative thereof.

By 'fusion' of a polypeptide we include a polypeptide which is fused to any other polypeptide. For example, the polypeptide may comprise one or more additional amino acids, inserted internally and/or at the N- and/or C-termini of the amino acid sequence of Group A or Group B polypeptides, or of a fragment, variant or derivative thereof.

Thus, as described herein, in one embodiment the polypeptide of the first aspect of the invention comprises a polypeptide of the invention to which is fused an enzymatic domain from a different source (e.g., from a source other than the polypeptide of the first aspect of the invention). Examples of suitable enzymatic domains include:
L-alanoyl-D-glutamate endopeptidase; D-glutamyl-m-DAP endopeptidase; interpeptide bridge-specific endopeptidase; N-acetyl-β-D-glucosaminidase (=muramoylhydrolase); N-acetyl-β-D-muramidase (=lysozyme); lytic transglycosylase.

Also N-acetylmuramoyl-L-alanine amidase from other sources could be utilised (see Loessner, 2005, Current Opinion in Microbiology 8: 480-487, the disclosures of which are incorporated herein by reference).

For example, the said polypeptide may be fused to a polypeptide such as glutathione-S-transferase (GST) or protein A in order to facilitate purification of said polypeptide. Examples of such GST fusions are well known to those skilled in the art. Similarly, the said polypeptide may be fused to an oligo-histidine tag such as His6 or to an epitope recognised by an antibody such as the well-known Myc tag epitope. Fusions to any fragment, variant or derivative of said polypeptide are also included in the scope of the invention. It will be appreciated that fusions (or variants or derivatives thereof) which retain desirable properties, e.g., antigenic activity, are preferred. It is also particularly preferred if the fusions are ones which are suitable for use in the methods described herein.

For example, the fusion may comprise a further portion which confers a desirable feature on the said polypeptide of the invention; for example, the portion may be useful in detecting or isolating the polypeptide, promoting cellular uptake of the polypeptide, or directing secretion of the protein from a cell. The portion may be, for example, a biotin moiety, a radioactive moiety, a fluorescent moiety, for example a small fluorophore or a green fluorescent protein (GFP) fluorophore, as well known to those skilled in the art. The moiety may be an immunogenic tag, for example a Myc tag, as known to those skilled in the art or may be a lipophilic molecule or polypeptide domain that is capable of promoting cellular uptake of the polypeptide, as known to those skilled in the art.

It will be appreciated by persons skilled in the art that the polypeptides of the invention also include pharmaceutically acceptable acid or base addition salts of the herein described polypeptides. The acids which are used to prepare the pharmaceutically acceptable acid addition salts of the aforementioned base compounds useful in this invention are those which form non-toxic acid addition salts, i.e., salts containing pharmacologically acceptable anions, such as the hydrochloride, hydrobromide, hydroiodide, nitrate, sulphate, bisulphate, phosphate, acid phosphate, acetate, lactate, citrate, acid citrate, tartrate, bitartrate, succinate, maleate, fumarate, gluconate, saccharate, benzoate, methanesulphonate, ethanesulphonate, benzenesulphonate, p-toluenesulphonate and pamoate [i.e. 1,1'-methylene-bis-(2-hydroxy-3 naphthoate)] salts, among others.

Pharmaceutically acceptable base addition salts may also be used to produce pharmaceutically acceptable salt forms of the polypeptides. The chemical bases that may be used as reagents to prepare pharmaceutically acceptable base salts of the present compounds that are acidic in nature are those that form non-toxic base salts with such compounds. Such non-toxic base salts include, but are not limited to those derived from such pharmacologically acceptable cations such as alkali metal cations (e.g. potassium and sodium) and alkaline earth metal cations (e.g. calcium and magnesium), ammonium or water-soluble amine addition salts such as N-methylglucamine-(meglumine), and the lower alkanolammonium and other base salts of pharmaceutically acceptable organic amines, among others.

The polypeptide, or fragment, variant, fusion or derivative thereof, may also be lyophilised for storage and reconstituted in a suitable carrier prior to use. Any suitable lyophilisation method (e.g. spray drying, cake drying) and/or reconstitution techniques can be employed. It will be appreciated by those skilled in the art that lyophilisation and reconstitution can lead to varying degrees of activity loss and that use levels may have to be adjusted upward to compensate. Preferably, the lyophilised (freeze dried) polypeptide loses no more than about 20%, or no more than about 25%, or no more than about 30%, or no more than about 35%, or no more than about 40%, or no more than about 45%, or no more than about 50% of its activity (prior to lyophilisation) when rehydrated.

Alternatively or additionally, the polypeptide of the invention is selected from the group consisting of SEQ ID NOs: 25, 26 and 27. Alternatively or additionally, the polypeptide of the invention is SEQ ID NO: 25.

Alternatively or additionally, a polypeptide of Group B exhibits at least 60% sequence identity to an amino acid sequence listed in (Group A), for example, at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 99.5% sequence identity to an amino acid sequence of Group A;
(i) wherein the at least 60% sequence identity is exhibited over at least 60% of the amino acid sequence listed in (Group A), for example, a contiguous amino acid sequence spanning at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of the amino acid sequence of Group A; or
(ii) wherein the at least 60% sequence identity is exhibited over at least 10 contiguous amino acids of the amino acid sequence listed in (Group A), for example, at least 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 350, 400, 450, 500, 550, 600, 650, 700, 750, 800, 850, 900, 950, 1000, 1500, 2000, 2500, 2600, 2601, 2602, 2603, 2604, 2605, 2606, 2607, 2608, 2609, 2610, 2611, 2612, 2613, 2614, 2615, 2616, 2617, 2618 or 2619 contiguous amino acids of the amino acid sequence of Group A.

Alternatively or additionally, the polypeptide comprises or consists of a fragment comprising at least 10 contiguous amino acids, for example, at least 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233 contiguous amino acids, and/or, where present, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 350, 400, 450, 500, 550, 600, 650, 700, 750, 800, 850, 900, 950, 1000, 1500, 2000, 2500 or 2600 contiguous amino acids of an amino acid sequence selected from Group A.

Alternatively or additionally, the polypeptide comprises or consists of a fragment wherein 1, or at least, 1 amino acid, is truncated from the N-terminus with respect to an amino acid sequence listed in Group A, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300 amino acids are truncated from the N-terminus with respect to the amino acid sequence listed in Group A

Alternatively or additionally, the polypeptide comprises or consists of a fragment wherein 1, or at least 1 amino acid, is truncated from the C-terminus with respect to the reference sequence, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300 amino acids are truncated from the C-terminus with respect to the reference sequence.

Alternatively or additionally, the polypeptide is fused to one or more additional polypeptide, for example:
(a) N-terminal fusion;
(b) C-terminal fusion; or
(c) N-terminal and C-terminal fusions.

Alternatively or additionally, the polypeptide is conjugated to one or more additional moiety, for example:
(a) one or more lipid (to form a lipoprotein);
(b) one or more saccharide or polysaccharide (to form a glycoprotein);
(c) one or more phosphate group (to form a phosphoprotein);
(d) one or more heme group (to form a hemoprotein);
(e) one or more the flavin adenine dinucleotide (FAD) or flavin mononucleotide (FMN) (to form a flavoprotein); and
(f) one or more metal ion cofactor (to form a metalloprotein).

The conjugation may be by means of one or more ionic or covalent bond, but is preferably conjugation by covalent bond.

Conjugation of antigens to carriers and the like is an established procedure for improving immunogenicity, particularly for polysaccharides. For instance, bacterial capsular polysaccharides (CPS) are naturally T cell independent antigens which give rise to an immune response that lacks several important properties. Conjugation to a carrier converts these saccharides to T-cell dependent antigens which can then produce an immunological memory effect, and also elicit a protective immune responses in young children. One known carrier in such conjugates is the 'OMPC' outer membrane protein complex, formed from *N. meningitidis* vesicles (e.g. see EP-0467714), which has been included as the carrier in approved *H. influenzae* B conjugate vaccines. OMPC has also been used as the carrier in protein conjugates. For example, Wu et al. (PNAS USA 2006; 103(48): 18243-18248) report that conjugation of Pfs25H to OMPC resulted in a Pfs25H-OMPC conjugate vaccine that was >1,000 times more potent in generating anti-Pfs25H ELISA reactivity in mice than a similar dose of Pfs25H alone. Conjugation of OMPC to Pfs25H protein can be achieved by reacting maleimide-activated Pfs25H with thiolated outer membrane proteins within OMPC (see e.g. WO2006/124712).

Polypeptides of the invention are preferably provided in purified or substantially purified form i.e., substantially free from other polypeptides (e.g. free from naturally-occurring polypeptides), particularly from other *E. coli* or host cell polypeptides, and are generally at least about 50% pure (by weight), for example at least 70%, 80%, 90%, 95%, 96%, 97%, 98% 99%, 99.5%, 99.5% or 100% pure by weight (i.e., less than 50% of a composition is made up of other expressed polypeptides). Thus, the antigens in the compositions are separated from the whole organism with which the antigen molecule is expressed.

Alternatively or additionally, the polypeptide is not naturally occurring. Alternatively or additionally, the polypeptide is recombinant (i.e., expressed from nucleic acid [e.g., a gene] that has been inserted or edited using recombinant nucleic acid technology).

Alternatively or additionally, the polypeptide is a fragment, variant, fusion and/or derivative capable of inducing a specific immune response to an amino acid sequence listed in Group A.

By "specific immune response" to an amino acid sequence or polypeptide listed in Group A, we mean or include the capability to induce an immune response in a subject that generates (e.g., stimulates the release of) antibody capable of binding to an amino acid sequence listed in Group A. It is preferred that the binding moiety is capable of binding *in vivo,* i.e., under the physiological conditions in which the amino acid sequence or polypeptide exists on or inside of a subject's body. Such binding specificity may be determined by methods well known in the art, such as e.g. ELISA, immunohistochemistry, immunoprecipitation, Western blots and flow cytometry using transfected cells expressing the/a polypeptide of the invention (e.g., any one of SEQ ID NOs: 25-28) (see the accompanying Examples).

In another embodiment, the binding moiety is capable of binding selectively to amino acid sequences or polypeptides of Group A and/or Group B.. By 'capable of binding selectively' we mean and/or include such binding moieties which bind at least 10-fold more strongly to said polypeptide or amino acid sequence than to another protein (i.e., proteins that are not variants or fragments of the test protein as claimed, or as defined herein); for example at least 50-fold more strongly or at least 100-fold more strongly. The binding moiety may be capable of binding selectively said polypeptide or amino acid sequence under physiological conditions, e.g. *in vivo.* Suitable methods for measuring relative binding strengths include immunoassays, for example where the binding moiety is an antibody (see Harlow & Lane, "Antibodies: A Laboratory", Cold Spring Habor Laboratory Press, New York, which is incorporated herein by reference). Alternatively, binding may be assessed using competitive assays or using BIACORE analysis (Biacore International AB, Sweden).

Alternatively or additionally, the immune response is an immune-activating response, for example, a protective immune response. The polypeptide may be capable of eliciting an *in vitro* protective immune response and/or an *in vivo* protective immune response when administered to a subject.

In the presence of co-stimulatory signals, T cells differentiate into specific phenotypic subtypes. Several of these subtypes are involved in suppressing or terminating natural inflammatory signals. By "immune-activating response" we mean and/or include that polypeptide induces or is capable of inducing an immune response in a subject that does not result in suppressing or terminating inflammation or inflammatory signals and, preferably, results in the activation or enhancement of inflammation or inflammatory signals (e.g., cytokines).

The *in vivo* protective immune response may be elicited in a mammal. Alternatively or additionally, the mammal is selected from the group consisting of armadillo (*dasypus novemcinctus*)*,* baboon (*papio anubis; papio cynocephalus*)*,* camel (*camelus bactrianus, camelus dromedarius, camelus ferus*)*,* cat (*felis catus*)*,* dog (*canis lupus familiaris*)*,* horse (*equus ferus caballus*)*,* ferret (*mustela putorius furo),* goat (*capra aegagrus hircus*)*,* guinea pig (*cavia porcellus*)*,* golden hamster (*mesocricetus auratus*)*,* kangeroo (*macropus rufus*)*,* llama (*lama glama*)*,* mouse (*mus musculus*)*,* pig (*sus scrofa domesticus*)*,* rabbit (*oryctolagus cuniculus),* rat (*rattus norvegicus*)*,* rhesus macaque (*macaca mulatta*)*,* sheep (*ovis aries*), non-human primates, and human (*Homo sapiens*)*.*

Alternatively or additionally, the protective immune response is protective against a disease or condition caused by an organism selected from the group consisting of: bacteria, Gram negative bacteria; *proteobacteria, enterobacteriales, enterobacteriaceae* (for example, *Salmonella, Escherichia* (*E. albertii, E. coli, E. fergusonii, E. hermannii, E. marmotae,* and *E. vulneris), Yersinia, Klebsiella, Proteus, Enterobacter, Serratia, and Citrobacter).*

Alternatively or additionally, the disease or condition is caused, wholly or in part, by *Escherichia coli,* for example, extraintestinal pathogenic *E. coli* (ExPEC), or intestinal pathogenic *E. coli* (InPEC).

Alternatively or additionally, the *Escherichia coli* is from a pathotype selected from the group consisting of: (i) enteropathogenic *E. coli* (EPEC); (ii) enterohemorrhagic *E. coli* (EHEC); (iii) enterotoxigenic *E. coli* (ETEC); (iv) enteroaggregative *E. coli* (EAEC); (v) diffusely adherent *E. coli* (DAEC); (vi) enteroinvasive *E. coli* (EIEC); (vii) uropathogenic *E. coli* (UPEC); (viii) neonatal meningitis *E. coli* (NMEC); (ix) Shiga Toxin (Stx) producing enteroaggregative *E. coli* (STEAEC); (x) adherent Invasive *E. coli* (AIEC); (xi) amoxicillin-resistant *E. coli* (AREC); (xii) asymptomatic bacteriuria *E. coli* (ABU); (xiii) Avian pathogenic *E. coli* (APEC).

Alternatively or additionally, the *Escherichia coli* is an enterohaemorrhagic *E. coli* (EHEC) selected from the group consisting of: O157:H7 e.g., EHEC O157:H7 EDL933 strain; EHEC O157:H7 Sakai stain; EHEC O26:H11 (e.g., strain 11368); EHEC O103:H2 (e.g., strain 12009); and EHEC O111:H- (e.g., strain 11128).

Alternatively or additionally, the *Escherichia coli* is an enteropathogenic *E. coli* (EPEC) selected from the group consisting of: O55:H7 (e.g., CB9615); and O127:H6 (e.g., strain E2348/69).

Alternatively or additionally, the *Escherichia coli* is an enterotoxigenic *E. coli* (ETEC) selected from the group consisting of: H10407; E24377A; and Porcine ETEC.

Alternatively or additionally, the *Escherichia coli* is an adherent Invasive *E. coli* (AIEC) selected from the group consisting of: LF82; O83:H1 NR G857C; and UM146.

Alternatively or additionally, the *Escherichia coli* is an enteroaggregative *E. coli* (EAEC), for example, 042 or 55989.

Alternatively or additionally, the *Escherichia coli* is a neonatal meningitis *E. coli* (NMEC) selected from the group consisting of: O7:K1 CE10; S88; and 1H E3034.

Alternatively or additionally, the *Escherichia coli* is an uropathogenic *E. coli* (UPEC) selected from the group consisting of: UMN026, CLONEDi14; CLONE Di2; CFT073; IA139; 536; NA114; and UTI89.

Alternatively or additionally, the *Escherichia coli* is AREC SMS-3-5; APEC 01; or ABU 83972.

The acidic capsular polysaccharide (CPS) is a thick, mucous-like, layer of polysaccharide that surrounds some pathogenic *E. coli,* and is referred to as 'K-antigen'. There are two separate groups of K-antigen groups, named group I and group II (while a small in-between subset (K3, K10, and K54/K96) has been classified as group III). The former (I) consist of 100 kDa (large) capsular polysaccharides, while the latter (II), associated with extraintestinal diseases, are under 50 kDa in size. Group I K antigens are only found with certain O-antigens (O8, O9, O20, and O101 groups), and are further subdivided on the basis of absence (IA, similar to that of *Klebsiella* species in structure) or presence (IB) of amino sugars. Some group I K-antigens are attached to the lipid A-core of the lipopolysaccharide (KLPS), in a similar way to O antigens (and being structurally identical to O antigens in some instances are only considered as K antigens when co-expressed with another authentic O antigen). Group II K antigens closely resemble those in Gram-positive bacteria and greatly differ in composition and are further subdivided according to their acidic components, generally 20-50% of the CPS chains are bound to phospholipids. In total there are 60 different K antigens that have been recognised.

Alternatively or additionally, the *Escherichia coli* is a strain with a K antigen selected from the group consisting of K1, K2a/ac, K3, K4, K5, K6, K7 (=K56), K8, K9 (=O104), K10, K11, K12 (K82), K13(=K20 and =K23), K14, K15, K16, K18a, K18ab (=K22), K19,K24, K26, K27, K28, K29, K30, K31, K34, K37, K39, K40, K41,K42, K43, K44, K45, K46, K47, K49 (O46), K50, K51, K52, K53, K54 (=K96), K55, K74, K84, K85ab/ac (=O141), K87 (=O32), K92, K93, K95, K97, K98, K100, K101, K102, K103, KX104, KX105, and KX106).

The outer membrane of an *E. coli* cell contains millions of lipopolysaccharide (LPS) molecules, which comprise:
O antigen, a polymer of immunogenic repeating oligosaccharides (1-40 units);
Core region of phosphorylated nonrepeating oligosaccharides; and
Lipid A (endotoxin).

The O antigen is used for serotyping *E. coli* and these O group designations go from O1 to O181, with the exception of some groups which have been historically removed, namely O31, O47, O67, O72, O93 (now K84), O94, and O122; groups 174 to 181 are provisional (O174=OX3 and O175=OX7) or are under investigation (176 to 181 are STEC/VTEC). Additionally subtypes exist for many O groups (e.g. O128ab and O128ac).

A repetitive glycan polymer contained within an LPS is referred to as the O antigen, O polysaccharide, or O side-chain of the bacteria. The O antigen is attached to the core oligosaccharide, and comprises the outermost domain of the LPS molecule. The composition of the O chain varies from strain to strain. For example, there are over 160 different O antigen structures produced by different *E. coli* strains. O antigen is exposed on the very outer surface of the bacterial cell, and, as a consequence, is a target for recognition by host antibodies.

Alternatively or additionally, the *Escherichia coli* is a strain with an O antigen selected from the group consisting of O1 A, O1 A1, O1 B, O1 C, O2, O3, O4, O4, O5 ab, O5 ac, O6, O6, O6, O7, O8, O9, O9 a, O10, O11, O12, O13, O15, O16, O16, O16, O17, O18 A, O18 A1, O18 A1A2, O18 ab, O18 ac, O18 B, O18 B1, O19 ab, O20 ab, O20 ac, O21, O22, O23 A, O24, O25, O26, O27, O28 ab, O28 ac, O29, O30, O32, O33, O34, O35, O36, O37, O38, O39, O40, O41, O42, O43, O44, O45, O45 rel, O46, O48, O49, O50, O51, O52, O53, O54, O55, O55, O56, O58, O59, O60, O61, O62, O63, O64, O65, O66, O68, O69, O70, O71, O73, O73 ab, O74, O75, O76, O77, O78, O79, O80, O81, O82, O83, O84, O85, O86, O86, O86, O87, O88, O89, O90, O91, O92, O95, O96, O97, O98, O99, O100, O101, O102, O103, O104, O105, O106, O107, O108, O109, O110, O111, O112 ab, O112 ac, O113, O114, O115, O116, O117, O118, O119, O120, O121, O123, O124, O125 ab, O125 ac, O126, O126, O127, O128 ab, O128 ab, O128 ac, O129, O130, O131, O132, O133, O134, O135, O136, O137, O138, O139, O140, O141, O142, O143, O144, O145, O146, O147, O148, O149, O150, O151, O152, O153, O154, O155, O156, O157, O158 ab, O158 ac, O159, O160, O161, O163, O164, O165, O166, O167, O168, O169, O170, O171, O172, O173, O174 ab, O174 ac, O175, O176, O177, O178, O179, O180, O181, O182, O183, O184, O185, O186 and O187.

The H antigen is a major component of flagella, involved in *E. coli* movement. It is generally encoded by the fliC gene. There are 53 identified H antigens, numbered from H1 to H56 (H13 and H22 were not *E. coli* antigens but from *Citrobacter freundii,* and H50 was found to be the same as H10).

Alternatively or additionally, the *Escherichia coli* is a strain with a H antigen selected from the group consisting of: H1, H2, H3, H4, H5, H6, H7, H8, H9, H10 (=H50), H11, H12, H14, H15, H16, H17, H18, H19, H20, H21, H23, H24, H25, H26, H27, H28, H29, H30, H31, H32, H33, H34, H35, H36, H37, H38, H39, H40, H41, H42, H43, H44, H45, H46, H47, H48, H49, H50 (=H10), H51, H52, H53, H54, H55 and H56.

Alternatively or additionally, the protective immune response is protective against a disease or condition selected from the group consisting of: gastroenteritis; urinary tract infection; neonatal meningitis; haemorrhagic colitis; and Crohn's disease.

Alternatively or additionally, the protective immune response is protective against one or more symptom of bacterial (e.g., EHEC) infection. Alternatively or additionally, the one or more symptom of bacterial infection is selected from the group consisting of diarrhoea (e.g., non-bloody), abdominal cramps and/or pain, fever, vomiting and haemolytic uremic syndrome (HUS). Alternatively or additionally, the one or more symptom of bacterial infection is prevented, abolished or reduced, for example, reduced by at least by at least 5%, for example, at least 10%, 15%, 20%, 25%, 30%, 35%, 40%, 45%, 50 %, 55%, 60%, 65%, 70%, 75%, 80%, 85%, 90%, 95%, 96%, 97%, 98%, 99% or at least 100%. Diarrhoea severity may be measured by evacuation volume and/or frequency. Abdominal cramps and/or pain severity may be measured by frequency and/or by the abdominal pain index (see Van Slyke et al., 1997, Development and validation of the pain response inventory for children, Psychological Assessment, 9:392-405, which is incorporated by reference herein). Vomiting severity may be measured by evacuation volume and/or frequency. HUS severity may be measured by thrombocyte (platelet) count and/or erythrocyte (red blood cell) count (i.e., reduced HUS severity correlates with increased thrombocyte and/or erythrocyte counts).

Alternatively or additionally, the protective immune response is an immune response that results in an increase in the level of one or more serum cytokine; for example, one or more cytokines selected from the group consisting of IL-1α, IL-1β, IL-1RA, IL-18, IL-2, IL-4, IL-7, IL-9, IL-13, IL-15, IL-3, IL-5, GM-CSF, IL-6, IL-11, G-CSF, IL-12, LIF, OSM, IL-10, IL-20, IL-14, IL-16, IL-17, IFN-α, IFN-β, IFN-γ, CD154, LT-β, TNF-α, TNF-β, 4-1BBL, APRIL, CD70, CD153, CD178, GITRL, LIGHT, OX40L, TALL-1, TRAIL, TWEAK, TRANCE, TGF-β1, TGF-β2, TGF-β3, Epo, Tpo, Flt-3L, SCF, M-CSF and MSP. The one or more cytokine is preferably a pro-inflammatory cytokines. The one or more cytokine may be measured using any suitable method known to the skilled person, for example, ELISA, or the Bio-Plex 200 system Luminex xMAP technology (Bio-Rad, USA), Meso Scale Discovery multiplex assay (Meso Scale Diagnostics, LLC, USA), TNF-α quantification; or equivalent (e.g., see Thorpe et al., 1992, Detection and measurement of cytokines, Blood Rev. 6(3):133-48, which is incorporated by reference herein).

Alternatively or additionally, the protective immune response is an immune response that results in, or is capable of resulting in bactericidal activity and/or opsonophagocytosis. Serum bactericidal activity may be measured, for example, by the method of Crokaert et al., 1988, Determination of serum bactericidal activity against Escherichia coli by an automated photometric method, J. Clin. Microbiol., 1988 Oct; 26(10): 2069-2076, or the method of Necchi, Saul and Rondini, 2017, Development of a high-throughput method to evaluate serum bactericidal activity using bacterial ATP measurement as survival readout, PLoS One, 12(2):e0172163, both of which are incorporated by reference herein. Opsonophagocytosis may be measured by the method of Abbanat et al., 2017, Development and Qualification of an Opsonophagocytic Killing Assay To Assess Immunogenicity of a Bioconjugated Escherichia coli Vaccine., Clin. Vaccine. Immunol., 24(12). pii: e00123-17 which is incorporated by reference herein, Phagocytosis assay; Phagolysosome formation assay (e.g., see Ogawa et al., 2017, Activation of IpxR gene through enterohaemorrhagic Escherichia coli virulence regulators mediates lipid A modification to attenuate innate immune response., Cell Microbiol., doi: 10.1111/cmi.12806, which is incorporated by reference herein).

Alternatively or additionally, the bactericidal activity and/or opsonophagocytosis reduces, or is capable of reducing, colony forming units (CFUs):
(a) by at least 1 log, for example, reduced by at least 2, 3, 4, 5, 6, 7, 8, 9 or 10 logs; or
(b) by at least 5%, for example at least 10%, 15%, 20%, 25%, 30%, 35%, 40%, 45%, 50 %, 55%, 60%, 65%, 70%, 75%, 80%, 85%, 90%, 95%, 96%, 97%, 98%, 99% or at least 100%.

CFUs can be measured using any suitable means known to the skilled person (e.g., Miller, 1972. In: Experiments in Molecular Genetics. Miller JH, editor. New York: Cold Spring Harbor; Determination of viable cell counts: bacterial growth curves; pp. 31-36).

### Adjuvants

Vaccines and immunogenic compositions of the invention may also comprise an adjuvant in addition to the antigen. Adjuvants are used in vaccines in order to enhance and modulate the immune response to the antigen. The adjuvants described herein may be combined with any of the antigen(s) herein described.

The adjuvant may be any adjuvant known to the skilled person, but adjuvants include (but are not limited to) oil-in-water emulsions (for example MF59 or AS03), liposomes, saponins, TLR2 agonists, TLR3 agonists, TLR4 agonists, TLR5 agonists, TLR6 agonists, TLR7 agonists, TLR8 agonists, TLR9 agonists, aluminium salts, nanoparticles, microparticles, ISCOMS, calcium fluoride and organic compound composites or combinations thereof.

### Oil-in-water emulsions

In an embodiment of the present invention, there is provided a vaccine or immunogenic composition for use in the invention comprising an oil-in-water emulsion. Oil-in-water emulsions of the present invention comprise a metabolisable oil and an emulsifying agent. In order for any oil-in-water composition to be suitable for human administration, the oil phase of the emulsion system has to comprise a metabolisable oil. The meaning of the term metabolisable oil is well known in the art. Metabolisable can be defined as "being capable of being transformed by metabolism" (Dorland's Illustrated Medical Dictionary, W.B. Sanders Company, 25th edition, 1974). A particularly suitable metabolisable oil is squalene. Squalene (2,6,10,15,19,23-Hexamethyl-2,6,10,14,18,22-tetracosahexaene) is an unsaturated oil which is found in large quantities in shark-liver oil, and in lower quantities in olive oil, wheat germ oil, rice bran oil, and yeast, and is a particularly preferred oil for use in an oil-in-water emulsion of the invention. Squalene is a metabolisable oil by virtue of the fact that it is an intermediate in the biosynthesis of cholesterol (Merck index, 10th Edition, entry no. 8619). In some embodiments, wherein the vaccines or immunogenic compositions of the invention comprise an oil-in-water emulsion, the metabolisable oil is present in the vaccine or in the immunogenic composition in an amount of 0.5% to 10% (v/v) of the total volume of the composition. The oil-in-water emulsion further comprises an emulsifying agent. The emulsifying agent may suitably be polyoxyethylene sorbitan monooleate (POLYSORBATE 80). Further, said emulsifying agent is suitably present in the vaccine or immunogenic composition in an amount of 0.125 to 4% (v/v) of the total volume of the composition. The oil-in-water emulsion may optionally comprise a tocol. Tocols are well known in the art and are described in EP0382271 B1. Suitably, the tocol may be alpha-tocopherol or a derivative thereof such as alpha-tocopherol succinate (also known as vitamin E succinate). Said tocol is suitably present in the adjuvant composition in an amount of 0.25% to 10% (v/v) of the total volume of the immunogenic composition. The oil-in-water emulsion may also optionally comprise sorbitan trioleate (SPAN 85).

The method of producing oil-in-water emulsions is well known to the person skilled in the art. Commonly, the method comprises mixing the oil phase (optionally comprising a tocol) with a surfactant such as a PBS/TWEEN80 solution, followed by homogenisation using a homogenizer; it would be clear to a person skilled in the art that a method comprising passing the mixture twice through a syringe needle is suitable for homogenising small volumes of liquid. Equally, the emulsification process in microfluidiser (e.g., M110S Microfluidics machine, maximum of 50 passes, for a period of 2 minutes at maximum pressure input of 6 bar (output pressure of about 850 bar)) could be adapted by the person skilled in the art to produce smaller or larger volumes of emulsion. The adaptation could be achieved by routine experimentation comprising the measurement of the resultant emulsion until a preparation was achieved with oil droplets of the required diameter.

In an oil-in-water emulsion, the oil and emulsifier should be in an aqueous carrier. The aqueous carrier may be, for example, phosphate buffered saline or citrate.

In particular, the oil-in-water emulsion systems used in the present invention have a small oil droplet size in the sub-micron range. Suitably the droplet sizes will be in the range 120 to 750 nm, more particularly sizes from 120 to 600 nm in diameter. Even more particularly, the oil-in water emulsion contains oil droplets of which at least 70% by intensity are less than 500 nm in diameter, more particular at least 80% by intensity are less than 300 nm in diameter, more particular at least 90% by intensity are in the range of 120 to 200 nm in diameter.

The oil droplet size, *i.e.* diameter, according to the present invention is given by intensity. There are several ways of determining the diameter of the oil droplet size by intensity. Intensity is measured by use of a sizing instrument, suitably by dynamic light scattering such as the Malvern Zetasizer 4000 or preferably the Malvern Zetasizer 3000HS. A first possibility is to determine the z average diameter ZAD by dynamic light scattering (PCS-Photon correlation spectroscopy); this method additionally gives the polydispersity index (PDI), and both the ZAD and PDI are calculated with the cumulants algorithm. These values do not require the knowledge of the particle refractive index. A second means is to calculate the diameter of the oil droplet by determining the whole particle size distribution by another algorithm, either the Contin, or NNLS, or the automatic "Malvern" one (the default algorithm provided for by the sizing instrument). Most of the time, as the particle refractive index of a complex composition is unknown, only the intensity distribution is taken into consideration, and if necessary the intensity mean originating from this distribution.

### ISCOMs

In some embodiments of the present invention, there are provided vaccines or immunogenic compositions of the invention comprising ISCOMs. ISCOMs are well known in the art (see Kersten & Crommelin, 1995, Biochimica et Biophysica Acta 1241: 117-138). ISCOMs comprise a saponin, cholesterol and phospholipids and form an open-cage-like structure of typically about 40 nm in size. ISCOMs result from the interaction of saponins, cholesterol and further phospholipids. A typical reaction mixture for the preparation of ISCOM is 5 mg/ml saponin and 1 mg/ml each for cholesterol and phospholipid. Phospholipids suitable for use in ISCOMs include, but are not limited, to phosphocholine (didecanoyl-L-α-phosphatidylcholine [DDPC], dilauroylphosphatidylcholine [DLPC], dimyristoylphosphatidylcholine [DMPC], dipalmitoyl phosphatidylcholine [DPPC], Distearoyl phosphatidylcholine [DSPC], Dioleoyl phosphatidylcholine [DOPC], 1-palmitoyl, 2-oleoylphosphatidylcholine [POPC], Dielaidoyl phosphatidylcholine [DEPC]), phosphoglycerol (1,2-Dimyristoyl-sn-glycero-3-phosphoglycerol [DMPG], 1,2-dipalmitoyl-sn-glycero-3-phosphoglycerol [DPPG], 1,2-distearoyl-sn-glycero-3-phosphoglycerol [DSPG], 1-palmitoyl-2-oleoyl-*sn*-glycero-3-phosphoglycerol [POPG]), phosphatidic acid (1,2-dimyristoyl-sn-glycero-3-phosphatidic acid [DMPA], dipalmitoyl phosphatidic acid [DPPA], distearoyl-phosphatidic acid [DSPA]), phosphoethanolamine (1,2-dimyristoyl-*sn*-glycero-3-phosphoethanolamine [DMPE], 1,2-Dipalmitoyl-sn-glycero-3-phosphoethanolamine [DPPE], 1,2-distearoyl-sn-glycero-3-phosphoethanolamine DSPE 1,2-Dioleoyl-sn-Glycero-3-Phosphoethanolamine [DOPE]), phoshoserine, polyethylene glycol [PEG] phospholipid (mPEG-phospholipid, polyglycerin-phospholipid, functionalized-phospholipid, terminal activated-phosholipid). In particular embodiments, ISCOMs comprise 1-palmitoyl-2-oleoyl-glycero-3-phosphoethanolamine. In further particular embodiments, highly purified phosphatidylcholine is used and can be selected from the group consisting of: Phosphatidylcholine (from egg), Phosphatidylcholine Hydrogenated (from egg) Phosphatidylcholine (from soy), Phosphatidylcholine Hydrogenated (from soy). In further particular embodiments, ISCOMs comprise phosphatidylethanolamine [POPE] or a derivative thereof. A number of saponins are suitable for use in ISCOMs. The adjuvant and haemolytic activity of individual saponins has been extensively studied in the art. For example, Quil A (derived from the bark of the South American tree Quillaja Saponaria Molina), and fractions thereof, are described in US 5,057,540 and "Saponins as vaccine adjuvants", Kensil, C. R., Crit. Rev. Ther. Drug. Carrier Syst., 1996, 12 (1-2): 1-55; and EP0362279 B1. ISCOMs comprising fractions of Quil A have been used in the manufacture of vaccines (EP0109942 B1). These structures have been reported to have adjuvant activity (EP0109942 B1; WO 96/11711). Fractions of QuilA, derivatives of QuilA and/or combinations thereof are suitable saponin preparations for use in ISCOMs. The haemolytic saponins QS21 and QS17 (HPLC purified fractions of Quil A) have been described as potent adjuvants, and the method of their production is disclosed in US 5,057,540 and EP0362279 B1. Also described in these references is the use of QS7 (a non-haemolytic fraction of Quil-A) which acts as a potent adjuvant for systemic vaccines. Use of QS21 is further described in Kensil et al. (1991. J. Immunology vol 146, 431-437). Combinations of QS21 and polysorbate or cyclodextrin are also known (WO 99/10008). Particulate adjuvant systems comprising fractions of QuilA, such as QS21 and QS7 are described in WO 96/33739 and WO 96/11711 and these are incorporated herein. Other particular QuilA fractions designated QH-A, QH-B, QH-C and a mixture of QH-A and QH-C designated QH-703 are disclosed in WO 96/011711 in the form of ISCOMs and are incorporated herein.

### Microparticles

In some embodiments of the present invention, there is provided a vaccine or immunogenic composition of the invention comprising microparticles. Microparticles, compositions comprising microparticles, and methods of producing microparticles are well known in the art (see Singh et al. [2007 Expert Rev. Vaccines 6(5): 797-808] and WO 98/033487). The term "microparticle" as used herein, refers to a particle of about 10 nm to about 10,000 µm in diameter or length, derived from polymeric materials which have a variety of molecular weights and, in the case of the copolymers such as PLG, a variety of lactide:glycolide ratios. In particular, the microparticles will be of a diameter that permits parenteral administration to a subject without occluding the administrating device and/or the subject's capillaries. Microparticles are also known as microspheres. Microparticle size is readily determined by techniques well known in the art, such as photon correlation spectroscopy, laser diffractometry and/or scanning electron microscopy. Microparticles for use herein will be formed from materials that are sterilizable, non-toxic and biodegradable. Such materials include, without limitation, poly(a-hydroxy acid), polyhydroxybutyric acid, polycaprolactone, polyorthoester, polyanhydride.

### Liposomes

In some embodiments of the present invention, there is provided a vaccine or immunogenic composition of the invention comprising liposomes. The term "liposomes" generally refers to uni- or multilamellar (particularly 2, 3, 4, 5, 6, 7, 8, 9, or 10 lamellar depending on the number of lipid membranes formed) lipid structures enclosing an aqueous interior. Liposomes and liposome formulations are well known in the art. Lipids, which are capable of forming liposomes, include all substances having fatty or fat-like properties. Lipids which can make up the lipids in the liposomes can be selected from the group comprising of glycerides, glycerophospholipides, glycerophosphinolipids, glycerophosphonolipids, sulfolipids, sphingolipids, phospholipids, isoprenolides, steroids, stearines, sterols, archeolipids, synthetic cationic lipids and carbohydrate containing lipids. Liposome size may vary from 30 nm to several µm depending on the phospholipid composition and the method used for their preparation. In particular embodiments of the invention, the liposome size will be in the range of 50 nm to 500 nm, and in further embodiments, 50 nm to 200 nm. Dynamic laser light scattering is a method used to measure the size of liposomes well known to those skilled in the art. The liposomes suitably contain a neutral lipid, for example phosphatidylcholine, which is suitably non-crystalline at room temperature, for example egg yolk phosphatidylcholine, dioleoyl phosphatidylcholine (DOPC) or dilauryl phosphatidylcholine. In a particular embodiment, the liposomes of the present invention contain DOPC. The liposomes may also contain a charged lipid which increases the stability of the liposome-saponin structure for liposomes composed of saturated lipids. In these cases the amount of charged lipid is suitably 1 to 20% (w/w), preferably 5 to 10%. The ratio of sterol to phospholipid is 1 to 50% (mol/mol), suitably 20 to 25% (mol/mol).

### Saponins

In some embodiments of the invention, the vaccine or immunogenic composition of the invention comprises a saponin. A particularly suitable saponin for use in the present invention is Quil A and its derivatives. Quil A is a saponin preparation isolated from the South American tree *Quillaja Saponaria Molina* and was first described by Dalsgaard et al. in 1974 ("Saponin adjuvants", Archiv. für die gesamte Virusforschung, Vol. 44, Springer Verlag, Berlin, p243-254) to have adjuvant activity. Purified fragments of Quil A have been isolated by HPLC which retain adjuvant activity without the toxicity associated with Quil A (EP0362278), for example QS7 and QS21 (also known as QA7 and QA21). QS-21 is a natural saponin derived from the bark of *Quillaja saponaria* Molina, which induces CD8+ cytotoxic T cells (CTLs), Th1 cells and a predominant IgG2a antibody response and is a particular saponin in the context of the present invention. The saponin adjuvant within the immunogenic compositions of the invention in particular are immunologically active fractions of Quil A, such as QS-7 or QS-21, suitably QS-21. In particular embodiments, the vaccines and/or immunogenic compositions of the invention contain the immunologically active saponin fraction in substantially pure form. In particular, the vaccines or immunogenic compositions of the invention contain QS21 in substantially pure form, that is to say, the QS21 is at least 75%, 80%, 85%, 90% pure, for example at least 95% pure, or at least 98% pure.

In a particular embodiment, QS21 is provided with an exogenous sterol, such as cholesterol for example. Suitable sterols include β-sitosterol, stigmasterol, ergosterol, ergocalciferol and cholesterol. In a further particular embodiment, the adjuvant composition comprises cholesterol as sterol. These sterols are well known in the art, for example cholesterol is disclosed in the Merck Index, 11th Edition, page 341, as a naturally occurring sterol found in animal fat.

In one embodiment, the liposomes of the invention that comprise a saponin suitably contain a neutral lipid, for example phosphatidylcholine, which is suitably non-crystalline at room temperature, for example egg yolk phosphatidylcholine, dioleoyl phosphatidylcholine (DOPC) or dilauryl phosphatidylcholine. The liposomes may also contain a charged lipid which increases the stability of the liposome-QS21 structure for liposomes composed of saturated lipids. In these cases the amount of charged lipid is suitably 1 to 20% (w/w), particularly 5 to 10% (w/w). The ratio of sterol to phospholipid is 1 to 50% (mol/mol), suitably 20 to 25% (mol/mol).

Where the active saponin fraction is QS21, the ratio of QS21:sterol will typically be in the order of 1:100 to 1:1 (w/w), suitably between 1:10 to 1:1 (w/w), and preferably 1:5 to 1:1 (w/w). Suitably, excess sterol is present, the ratio of QS21:sterol being at least 1:2 (w/w). In one embodiment, the ratio of QS21:sterol is 1:5 (w/w). The sterol is suitably cholesterol.

Other useful saponins are derived from the plants *Aesculus hippocastanum* or *Gyophilla struthium.* Other saponins which have been described in the literature include Escin, which has been described in the Merck index (12th Edition: entry 3737) as a mixture of saponins occuring in the seed of the horse chestnut tree, Lat: *Aesculus hippocastanum.* Its isolation is described by chromatography and purification (Fiedler, Arzneimittel-Forsch. 4, 213 (1953)), and by ion-exchange resins (Erbring et al., US 3,238,190). Fractions of Escin have been purified and shown to be biologically active (Yoshikawa et al., 1996, Chem Pharm Bull (Tokyo), 44(8): 1454-1464). Sapoalbin from *Gypsophilla struthium* (R. Vochten et al., 1968, J. Pharm. Belg. 42: p213-226) has also been described in relation to ISCOM production for example.

A saponin, such as QS21, can be used at amounts between 1 and 100µg per human dose of the adjuvant composition. QS21 may be used at a level of about 50µg, for example between 40 to 60 µg, suitably between 45 to 55 µg or between 49 and 51 µg or 50µg. In a further embodiment, the human dose of the adjuvant composition comprises QS21 at a level of about 25µg, for example between 20 to 30µg, suitably between 21 to 29µg or between 22 to 28µg or between 28 and 27µg or between 24 and 26µg, or 25µg.

### TLR4 agonist

In some embodiments, the vaccine or immunogenic composition of the invention comprises a TLR4 agonist. By "TLR agonist" it is meant a component which is capable of causing a signalling response through a TLR signalling pathway, either as a direct ligand or indirectly through generation of endogenous or exogenous ligand (Sabroe et al, 2003, JI p1630-5). A TLR4 agonist is capable of causing a signalling response through a TLR-4 signalling pathway. A suitable example of a TLR-4 agonist is a lipopolysaccharide, suitably a non-toxic derivative of lipid A, particularly monophosphoryl lipid A or more particularly 3-Deacylated monophoshoryl lipid A (3D - MPL).

3D-MPL is sold under the name MPL by GlaxoSmithKline Biologicals and is referred throughout the document as MPL or 3D-MPL. See, for example, US 4,436,727; US 4,877,611; US 4,866,034 and US 4,912,094. 3D-MPL primarily promotes CD4+ T cell responses with an IFN-gamma (Th1) phenotype. 3D-MPL can be produced according to the methods disclosed in GB 2 220 211 A. Chemically, it is a mixture of 3-deacylated monophosphoryl lipid A with 4, 5 or 6 acylated chains. In the compositions of the present invention, small particle 3D-MPL may be used to prepare the aqueous adjuvant composition. Small particle 3D-MPL has a particle size such that it may be sterile-filtered through a 0.22µm filter. Such preparations are described in WO 94/21292. Preferably, powdered 3D-MPL is used to prepare the aqueous adjuvant compositions of the present invention.

Other TLR-4 agonists which can be used are alkyl glucosaminide phosphates (AGPs) such as those disclosed in WO 98/50399 or US 6,303, 347 (processes for preparation of AGPs are also disclosed), suitably RC527 or RC529 or pharmaceutically acceptable salts of AGPs as disclosed in US 6,764,840.

Other suitable TLR-4 agonists are as described in WO 03/011223 and in WO 03/099195, such as compound I, compound II and compound III disclosed on pages 4-5 of WO 03/011223 or on pages 3 to 4 of WO 03/099195 and in particular those compounds disclosed in WO 03/011223, as ER803022, ER803058, ER803732, ER804053, ER804057m ER804058, ER804059, ER804442, ER804680 and ER804764. For example, one suitable TLR-4 agonist is ER804057.

A TLR-4 agonist, such as a lipopolysaccharide, such as 3D-MPL, can be used at amounts between 1 and 100 µg per human dose of the adjuvant composition. 3D-MPL may be used at a level of about 50 µg, for example between 40 to 60 µg, suitably between 45 to 55 µg or between 49 to 51 µg or 50 µg per human dose. In a further embodiment, the human dose of the adjuvant composition comprises 3D-MPL at a level of about 25 µg, for example between 20 to 30 µg, suitably between 21 to 29 µg or between 22 to 28 µg or between 28 to 27 µg or between 24 to 26 µg, or 25 µg.

Synthetic derivatives of lipid A are known and thought to be TLR 4 agonists including, but not limited to:
**OM174** (2-deoxy-6-o-[2-deoxy-2-[(R)-3-dodecanoyloxytetra-decanoylamino]-4-o-phosphono-β-D-glucopyranosyl]-2-[(R)-3-hydroxytetradecanoylamino]-α-D-glucopyranosyldihydrogenphosphate), (WO 95/14026)
**OM294** DP (3S, 9 R) -3--[(R)-dodecanoyloxytetradecanoylamino]-4-oxo-5-aza-9(R)-[(R)-3-hydroxytetradecanoylamino]decan-1,10-diol,1,10-bis(dihydrogenophosphate) (WO 99/64301 and WO 00/0462)
**OM197** MP-Ac DP (3S-, 9R) -3-[(R)-dodecanoyloxytetradecanoylamino]-4-oxo-5-aza-9-[(R)-3-hydroxytetradecanoylamino]decan-1,10-diol,1-dihydrogenophosphate 10-(6-aminohexanoate) (WO 01/46127).

Other suitable TLR-4 ligands, capable of causing a signalling response through TLR-4 (Sabroe et al, JI 2003 p1630-5) are, for example, lipopolysaccharide from gram-negative bacteria and its derivatives, or fragments thereof, in particular a non-toxic derivative of LPS (such as 3D-MPL). Other suitable TLR agonist are: heat shock protein (HSP) 10, 60, 65, 70, 75 or 90; surfactant Protein A, hyaluronan oligosaccharides, heparan sulphate fragments, fibronectin fragments, fibrinogen peptides and b-defensin-2, muramyl dipeptide (MDP) or F protein of respiratory syncytial virus (RSV). In one embodiment, the TLR agonist is HSP 60, 70 or 90.

### TLR agonists

Rather than a TLR4 agonist, other natural or synthetic agonists of TLR molecules may be used in vaccines or immunogenic composition of the invention. These include, but are not limited to, agonists for TLR2, TLR3, TLR5, TLR6, TLR7, TLR8 and TLR9.

In one embodiment of the present invention, a TLR agonist is used that is capable of causing a signalling response through TLR-1 (Sabroe et al, JI 2003 p1630-5). Suitably, the TLR agonist capable of causing a signalling response through TLR-1 is selected from: Tri-acylated lipopeptides (LPs); phenol-soluble modulin; Mycobacterium tuberculosis LP; S-(2,3-bis(palmitoyloxy)-(2-RS)-propyl)-N-palmitoyl-(R)-Cys-(S)-Ser-(S)-Lys(4)-OH, trihydrochloride (Pam3Cys) LP which mimics the acetylated amino terminus of a bacterial lipoprotein and OspA LP from *Borrelia burgdorfei.*

In a further embodiment, a TLR agonist is used that is capable of causing a signalling response through TLR-2 (Sabroe et al, JI 2003 p1630-5). Suitably, the TLR agonist capable of causing a signalling response through TLR-2 is one or more of a lipoprotein, a peptidoglycan, a bacterial lipopeptide from *M. tuberculosis, B. burgdorferi, T. pallidum,* peptidoglycans from species including *Staphylococcus aureus,* lipoteichoic acids, mannuronic acids, Neisseria porins, bacterial fimbriae, Yersinia virulence factors, CMV virions, measles haemagglutinin, and zymosan from yeast.

In a further embodiment, a TLR agonist is used that is capable of causing a signalling response through TLR-3 (Sabroe et al, JI 2003 p1630-5). Suitably, the TLR agonist capable of causing a signalling response through TLR-3 is double stranded RNA (dsRNA), or polyinosinic-polycytidylic acid (Poly IC), a molecular nucleic acid pattern associated with viral infection.

In an alternative embodiment, a TLR agonist is used that is capable of causing a signalling response through TLR-5 (Sabroe et al, JI 2003 p1630-5). Suitably, the TLR agonist capable of causing a signalling response through TLR-5 is bacterial flagellin. Said TLR-5 agonist may be flagellin or may be a fragment of flagellin which retains TLR-5 agonist activity. The flagellin can include a polypeptide selected from the group consisting of *H. pylori, S. typhimurium, V. cholera, S. marcesens, S. flexneri, T. pallidum, L. pneumophilia, B. burgdorferi; C. difficile, R. meliloti, A. tumefaciens; R. lupine; B. clarridgeiae, P. mirabilis, B. subtilus, L. moncytogenes, P. aeruginoa* and *E. coli.*

In a particular embodiment, the flagellin is selected from the group consisting of *S*. *typhimurium* flagellin B (Genbank Accession number AF045151), a fragment of *S*. *typhimurium* flagellin B, *E. coli* FliC. (Genbank Accession number AB028476); fragment of *E. coli* FliC; *S*. *typhimurium* flagellin FliC (ATCC14028) and a fragment of *S*. *typhimurium* flagellin FliC

In a further particular embodiment, said TLR-5 agonist is a truncated flagellin, as described in WO 09/156405 *i.e.* one in which the hypervariable domain has been deleted. In one aspect of this embodiment, said TLR-5 agonist is selected from the group consisting of: FliC_{Δ174-400}; FliC_{Δ161-405} and FliC_{Δ138-405}.

In a further particular embodiment, said TLR-5 agonist is a flagellin, as described in WO 09/128950.

In a further embodiment, a TLR agonist is used that is capable of causing a signalling response through TLR-6 (Sabroe et al, JI 2003 p1630-5). Suitably, the TLR agonist capable of causing a signalling response through TLR-6 is mycobacterial lipoprotein, di-acylated LP, and phenol-soluble modulin. Further TLR6 agonists are described in WO 03/043572.

In a further embodiment, a TLR agonist is used that is capable of causing a signalling response through TLR-7 (Sabroe et al, JI 2003 p1630-5). Suitably, the TLR agonist capable of causing a signalling response through TLR-7 is a single stranded RNA (ssRNA), loxoribine, a guanosine analogue at positions N7 and C8, or an imidazoquinoline compound, or derivative thereof. In a particular embodiment, the TLR agonist is imiquimod. Further TLR7 agonists are described in WO 02/085905.

In a further embodiment, a TLR agonist is used that is capable of causing a signalling response through TLR-8 (Sabroe et al, JI 2003 p1630-5). Suitably, the TLR agonist capable of causing a signalling response through TLR-8 is a single stranded RNA (ssRNA), an imidazoquinoline molecule with anti-viral activity, for example resiquimod (R848); resiquimod is also capable of recognition by TLR-7. Other TLR-8 agonists which may be used include those described in WO 04/071459.

In a further embodiment, a TLR agonist is used that is capable of causing a signalling response, such as one that comprises a CpG motif. The term "immunostimulatory oligonucleotide" is used herein to mean an oligonucleotide that is capable of activating a component of the immune system. In one embodiment, the immunostimulatory oligonucleotide comprises one or more unmethylated cytosine-guanosine (CpG) motifs. In a further embodiment, the immunostimulatory oligonucleotide comprises one or more unmethylated thymidine-guanosine (TG) motif or may be T-rich. By T-rich, it is meant that the nucleotide composition of the oligonucleotide comprises greater than 50, 60, 70 or 80% thymidine. In one embodiment, the oligonucleotide is not an immunostimulatory oligonucleotide and does not comprise an unmethylated CpG motif. In a further embodiment the immunostimulatory oligonucleotide is not T-rich and/or does not comprise an unmethylated TG motif.

The oligonucleotide may be modified in order to improve *in vitro* and/or *in vivo* stability. For example, in one embodiment, the oligonucleotides are modified so as to comprise a phosphorothioate backbone, *i.e.* internucleotide linkages. Other suitable modifications including diphosphorothioate, phosphoroamidate and methylphosphonate modifications as well as alternative internucleotide linkages to oligonucleotides are well known to those skilled in the art and are encompassed by the invention.

In another embodiment, the vaccines or immunogenic compositions of the invention further comprise an immunostimulant selected from the group consisting of: a TLR-1 agonist, a TLR-2 agonist, TLR-3 agonist, a TLR-4 agonist, a TLR-5 agonist, a TLR-6 agonist, a TLR-7 agonist, a TLR-8 agonist, TLR-9 agonist, or a combination thereof.

### Calcium composites

In some embodiments, the vaccine or immunogenic composition of the invention comprises a calcium fluoride composite, the composite comprising Ca, F, and Z. "Z" as used herein refers to an organic molecule. As used herein, a "composite" is a material that exists as a solid when dry, and that is insoluble, or poorly soluble, in pure water. In some aspects, Z comprises a functional group that forms an anion when ionized. Such functional groups include without limitation one or more functional groups selected from the group consisting of: hydroxyl, hydroxylate, hydroxo, oxo, N-hydroxylate, hydroaxamate, N-oxide, bicarbonate, carbonate, carboxylate, fatty acid, thiolate, organic phosphate, dihydrogenophosphate, monohydrogenophosphate, monoesters of phosphoric acid, diesters of phosphoric acid, esters of phospholipid, phosphorothioate, sulphates, hydrogen sulphates, enolate, ascorbate, phosphoascorbate, phenolate, and imine-olates.

In some aspects, the calcium fluoride composites herein described comprise Z, where Z is an anionic organic molecule possessing an affinity for calcium and forming a water insoluble composite with calcium and fluoride. In further aspects, the calcium fluoride composites herein described comprise Z, where Z may be categorized as comprising a member of a chemical category selected from the group consisting of: hydroxyl, hydroxylates, hydroxo, oxo, N-hydroxylate, hydroaxamate, N-oxide, bicarbonates, carbonates, carboxylates and dicarboxylate, salts of carboxylic-acids, salts of QS21, extract of bark of *Quillaja saponaria,* extract of immunological active saponine, salts of satured or unsaturated fatty acid, salts of oleic acid, salts of amino-acids, thiolates, thiolactate, salt of thiol-compounds, salts of cysteine, salts of N-acetyl-cysteine, L-2-Oxo-4-thiazolidinecarboxylate, phosphates, dihydrogenophosphates, monohydrogenophosphate, salts of phosphoric-acids, monoesters of phosphoric acids and their salts, diesters of phosphoric acids and their salts, esters of 3-O-desacyl-4'-monophophoryl lipid A, esters of 3D-MLA, MPL, esters of phospholipids, DOPC, dioleolyphosphatidic derivatives, phosphates from CpG motifs, phosphorothioates from CpG family, sulphates, hydrogen sulphates, salts of sulphuric acids, enolates, ascorbates, phosphoascorbate, phenolate, α-tocopherol, imine-olates, cytosine, methyl-cytosine, uracyl, thymine, barbituric acid, hypoxanthine, inosine, guanine, guanosine, 8-oxo-adenine, xanthine, uric acid, pteroic acid, pteroylglutamic acid, folic acid, riboflavin, and lumiflavin. In further aspects, the calcium fluoride composites herein described comprise Z, where Z is selected from the group consisting of: N-acetyl cysteine; thiolactate; adipate; carbonate; folic acid; glutathione; and uric acid. In some aspects, the calcium fluoride composites herein comprise Z, where Z is selected from the group consisting of: N-acetyl cysteine; adipate; carbonate; and folic acid. In further aspects, the calcium fluoride composites herein comprise Z, where Z is N-acetyl cysteine, and the composite comprises between 51% Ca, 48% F, no more than 1% N-acetyl cysteine (w/w) and 37% Ca, 26% F, and 37% N-acetyl cysteine (w/w). In further aspects, the calcium fluoride composites herein comprise Z, where Z is thiolactate, and the composite comprises between 51% Ca, 48% F, no more than 1% thiolactate (w/w) and 42% Ca, 30% F, 28% thiolactate (w/w). In further aspects, the calcium fluoride composites herein comprise Z, where Z is adipate, and the composite comprises between 51% Ca, 48% F, no more than 1% adipate (w/w) and 38% Ca, 27% F, 35% adipate (w/w). In further aspects, the calcium fluoride composites herein comprise Z, where Z is carbonate, and the composite comprises between 51% Ca, 48% F, no more than 1% carbonate (w/w) and 48% Ca, 34% F, 18% carbonate (w/w). In further aspects, the calcium fluoride composites herein comprise Z, where Z is folic acid, and the composite comprises between 51% Ca, 48% F, no more than 1% folic acid (w/w) and 22% Ca, 16% F, 62% folic acid (w/w). In further aspects, the calcium fluoride composites herein comprise Z, where Z is glutathione, and the composite comprises between 51% Ca, 48% F, no more than 1% glutathione (w/w) and 28% Ca, 20% F, 52% glutathione (w/w). In further aspects, the calcium fluoride composites herein comprise Z, where Z is uric acid, and the composite comprises between 51% Ca, 48% F, and no more than 1% uric acid (w/w) and 36% Ca, 26% F, and 38% uric acid (w/w).

### Aluminium salts

In one embodiment, the vaccine or immunogenic composition of the invention comprises an aluminium salt. Suitable aluminium salt adjuvants are well known to the skilled person and include but are not limited to aluminium phosphate, aluminium hydroxide or a combination thereof. Suitable aluminium salt adjuvants include but are not limited to REHYDRAGEL HS, ALHYDROGEL 85, REHYDRAGEL PM, REHYDRAGEL AB, REHYDRAGEL HPA, REHYDRAGEL LV, ALHYDROGEL or a combination thereof.

In particular, the aluminium salts may have a protein adsorption capacity of between 2.5 and 3.5, 2.6 and 3.4, 2.7 and 3.3 or 2.9 and 3.2, 2.5 and 3.7, 2.6 and 3.6, 2.7 and 3.5, or 2.8 and 3.4 protein (BSA)/ml aluminium salt. In a particular embodiment of the invention, the aluminium salt has a protein adsorption capacity of between 2.9 and 3.2 mg BSA/mg aluminium salt. Protein adsorption capacity of the aluminium salt can be measured by any means known to the skilled person. The protein adsorption capacity of the aluminium salt may be measured using the method as described in Example 1 of WO 12/136823 (which utilises BSA) or variations thereof.

Aluminium salts described herein *(i.e.* having the protein adsorption capacity described herein) may have a crystal size of between 2.8 and 5.7nm as measured by X-ray diffraction, for example 2.9 to 5.6nm, 2.8 to 3.5nm, 2.9 to 3.4nm or 3.4 to 5.6nm or 3.3 and 5.7nm as measured by X-ray diffraction. X-ray diffraction is well known to the skilled person. In a particular embodiment of the invention the crystal size is measured using the method described in Example 1 of WO 12/136823 or variations thereof.

The polypeptide(s) and/or nucleic acid(s) described herein may be administered to a subject by any route of administration, for example, orally, nasally, sublingually, intravenously, intramuscularly, intradermally (e.g. a skin patch with microprojections) or transdermally (e.g. an ointment or cream).

A second aspect of the invention provides an isolated nucleic acid molecule comprising or consisting of:
(Group C) a nucleic acid sequence selected from the group consisting of selected from the group consisting of SEQ ID NO: 1, 2, 3, 4, 5, 6, 7, 8, 9, 10, 11, 12, 13, 14, 15, 16, 17, 18, 19, 20, 21, 22, 23 or 24; or
(Group D) a fragment, variant and/or fusion of (Group C).

Alternatively or additionally, wherein the nucleic acid sequence of Group C is selected from the group consisting of SEQ ID NOs: 1, 2 and 3, for example, the nucleic acid sequence may be SEQ ID NO: 1.

Alternatively or additionally, the nucleic acid of Group D exhibits at least 60% sequence identity to a nucleic acid sequence listed in Group C, for example, at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to the nucleic acid sequence listed in (Group C); and
(I) wherein the at least 60% sequence identity is exhibited over at least 60% of the nucleic acid sequence listed in (Group C), for example, a contiguous amino acid sequence spanning at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of the nucleic acid sequence listed in (Group C); or
(II) wherein the at least 60% sequence identity is exhibited over at least 30 contiguous nucleic acids of the nucleic acid sequence listed in (Group C), for example, at least 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, or 7500 contiguous amino acids of the nucleic acid sequence listed in (Group C).

Alternatively or additionally, the nucleic acid encodes a polypeptide according to the first aspect of the invention.

The terms "polynucleotide" and "oligonucleotide" are used interchangeably herein, and refer to a heteropolymer of nucleotides or the sequence of these nucleotides. These phrases also refer to DNA or RNA of genomic or synthetic origin which may be single-stranded or double-stranded and may represent the sense or the antisense strand, to peptide nucleic acid (PNA) or to any DNA-like or RNA-like material. In the sequences herein A is adenine, C is cytosine, T is thymine, G is guanine and N is A, C, G or T (U). Where the polynucleotide is RNA, the T (thymine) in the sequences provided herein is substituted with U (uracil). Generally, nucleic acid segments provided by this invention may be assembled from fragments of the genome and short oligonucleotide linkers, or from a series of oligonucleotides, or from individual nucleotides, to provide a synthetic nucleic acid which is capable of being expressed in a recombinant transcriptional unit comprising regulatory elements derived from a microbial or viral operon, or a eukaryotic gene.

A third aspect of the invention provides a vector comprising a nucleic acid molecule as defined in the second aspect of the invention.

Suitable prokaryotic vector plasmids are: pUC18, pUC19, pBR322 and pBR329 available from Biorad Laboratories (Richmond, CA, USA); pTrc99A, pKK223-3, pKK233-3, pDR540 and pRIT5 available from Pharmacia (Piscataway, NJ, USA); pBS vectors, Phagescript vectors, Bluescript vectors, pNH8A, pNH16A, pNH18A, pNH46A available from Stratagene Cloning Systems (La Jolla, CA 92037, USA), and pBAD-A (Thermo Fisher).

A suitable mammalian cell vector plasmid is pSVL available from Pharmacia (Piscataway, NJ, USA). This vector uses the SV40 late promoter to drive expression of cloned genes, the highest level of expression being found in T antigen-producing cells, such as COS-1 cells. An example of an inducible mammalian expression vector is pMSG, also available from Pharmacia (Piscataway, NJ, USA). This vector uses the glucocorticoid-inducible promoter of the mouse mammary tumour virus long terminal repeat to drive expression of the cloned gene.

Useful yeast plasmid vectors are pRS403-406 and pRS413-416 and are generally available from Stratagene Cloning Systems (La Jolla, CA 92037, USA). Plasmids pRS403, pRS404, pRS405 and pRS406 are Yeast Integrating plasmids (Ylps) and incorporate the yeast selectable markers HIS3, TRP1, LEU2 and URA3. Plasmids pRS413-416 are Yeast Centromere plasmids (YCps).

Methods well known to those skilled in the art can be used to construct expression vectors containing the coding sequence and, for example appropriate transcriptional or translational controls. One such method involves ligation via homopolymer tails. Homopolymer polydA (or polydC) tails are added to exposed 3' OH groups on the DNA fragment to be cloned by terminal deoxynucleotidyl transferases. The fragment is then capable of annealing to the polydT (or polydG) tails added to the ends of a linearised plasmid vector. Gaps left following annealing can be filled by DNA polymerase and the free ends joined by DNA ligase.

Another method involves ligation via cohesive ends. Compatible cohesive ends can be generated on the DNA fragment and vector by the action of suitable restriction enzymes. These ends will rapidly anneal through complementary base pairing and remaining nicks can be closed by the action of DNA ligase.

A further method uses synthetic molecules called linkers and adaptors. DNA fragments with blunt ends are generated by bacteriophage T4 DNA polymerase or *E. coli* DNA polymerase I which remove protruding 3' termini and fill in recessed 3' ends. Synthetic linkers, pieces of blunt-ended double-stranded DNA which contain recognition sequences for defined restriction enzymes, can be ligated to blunt-ended DNA fragments by T4 DNA ligase. They are subsequently digested with appropriate restriction enzymes to create cohesive ends and ligated to an expression vector with compatible termini. Adaptors are also chemically synthesised DNA fragments which contain one blunt end used for ligation but which also possess one preformed cohesive end.

Synthetic linkers containing a variety of restriction endonuclease sites are commercially available from a number of sources including International Biotechnologies Inc, New Haven, CN, USA.

A desirable way to modify the DNA encoding the polypeptide of the invention is to use the polymerase chain reaction as disclosed by Saiki et al (1988) Science 239, 487-491. In this method the DNA to be enzymatically amplified is flanked by two specific oligonucleotide primers which themselves become incorporated into the amplified DNA. The said specific primers may contain restriction endonuclease recognition sites which can be used for cloning into expression vectors using methods known in the art.

### Self-Replicating RNA

Nucleic acid immunisation may be achieved by delivering a self-replicating RNA (or self-amplifying RNA) encapsulated within and/or adsorbed to a small particle. The RNA encodes a polypeptide antigen of interest, and the particle may deliver this RNA by mimicking the delivery function of a natural virus. After *in vivo* administration of the particles, RNA is released from the particles and is translated inside a cell to provide the antigen *in situ.*

Any of the polypeptide antigens described herein as suitable to be included in the vaccines or immunogenic compositions in accordance with the invention may be expressed in the form of a self-replicating RNA molecule encoding said antigen, as described in WO 12/006376 which is incorporated herein by reference. Accordingly, in particular embodiments where antigens in the vaccines or immunogenic compositions for use in the invention are polypeptides, such polypeptides are encoded by a self-replicating RNA. In such cases, said self-replicating RNA is suitably coupled with a delivery system, in particular lipid-based delivery systems, such as a cationic nanoemulsion (CNE), or a liposome. Suitably, when the lipid-based system is a CNE the self-replicating RNA is adsorbed to the outer surface of the CNE, while when said lipid-based system is a liposome the self-amplifyfing RNA is encapsulated into the liposome.

By "self-replicating RNA molecule" (or "self-amplifying RNA"), it is meant that, when delivered to a vertebrate cell, even without any proteins, the molecule leads to the production of multiple daughter RNAs by transcription from itself as explained in WO 12/006376, which ultimately results into the expression of the encoded antigen, becoming a major polypeptide of the cells.

One suitable system for achieving self-replication in this manner is to use an alphavirus-based replicon, as further described in WO 12/006376. Suitably, said replicon encodes (i) a RNA-dependent RNA polymerase which can transcribe RNA from the self-replicating RNA molecule and (ii) an antigen of interest. The polymerase can be an alphavirus replicase e.g. comprising one or more alphavirus proteins nsP1, nsP2, nsP3 and nsP4. Appropriate features of self-replicating RNA molecules and methods for preparing them are also described in WO 12/006376.

In some embodiments, the vaccines or immunogenic compositions for use in the present invention comprise a liposome and a self-replicating RNA encoding any of the polypeptide antigens herein described encapsulated into the liposome. In further embodiments, the vaccines or immunogenic compositions for use in the present invention comprise a CNE and a self-replicating RNA encoding any of the polypeptide antigens herein described adsorbed to the outer surface of the CNE. In particular embodiments, the self-replicating RNA molecule encodes polypeptide antigens derived from the group consisting of: HCMV, RSV and HIV.

Exemplary CNE for use in the present invention, as well as methods for their preparation are disclosed in WO 12/006380 which is incorporated herein by reference.

Various amphiphilic lipids can form bilayers in an aqueous environment to encapsulate a RNA-containing aqueous core as a liposome. These lipids can have an anionic, cationic or zwitterionic hydrophilic head group. Some phospholipids are anionic whereas others are zwitterionic and others are cationic. Suitable classes of phospholipids include, but are not limited to, phosphatidylethanolamines, phosphatidylcholines, phosphatidylserines, and phosphatidyl-glycerols. Useful cationic lipids include, but are not limited to, dioleoyl trimethylammonium propane (DOTAP), 1,2-distearyloxy-N,N-dimethyl-3-aminopropane (DSDMA), 1,2-dioleyloxy-N,N dimethyl-3-aminopropane (DODMA), 1,2-dilinoleyloxy-N,N-dimethyl-3-aminopropane (DLinDMA), 1,2-dilinolenyloxy-N,N-dimethyl-3-aminopropane (DLenDMA). Further useful cationic lipids are described in WO 15/095340, for example the lipids as claimed in any of claims 1 to 8 of WO 15/095340 incorporated herein by reference. Zwitterionic lipids include, but are not limited to, acyl zwitterionic lipids and ether zwitterionic lipids. Examples of useful zwitterionic lipids are DPPC, DOPC and dodecylphosphocholine. Liposomal particles of the invention can be formed from a single lipid or from a mixture of lipids. A mixture may comprise (i) a mixture of anionic lipids, (ii) a mixture of cationic lipids, (iii) a mixture of zwitterionic lipids, (iv) a mixture of anionic lipids and cationic lipids, (v) a mixture of anionic lipids and zwitterionic lipids, (vi) a mixture of zwitterionic lipids and cationic lipids or (vii) a mixture of anionic lipids, cationic lipids and zwitterionic lipids. Where a mixture of lipids is used, not all of the component lipids in the mixture need to be amphiphilic *e.g*. one or more amphiphilic lipids can be mixed with cholesterol. The hydrophilic portion of a lipid can be PEGylated *(i.e.* modified by covalent attachment of a polyethylene glycol). This modification can increase stability and prevent non-specific adsorption of the liposomes. Liposomal particles are usually divided into three groups: multilamellar vesicles (MLV); small unilamellar vesicles (SUV); and large unilamellar vesicles (LUV). MLVs have multiple bilayers in each vesicle, forming several separate aqueous compartments. SUVs and LUVs have a single bilayer encapsulating an aqueous core; SUVs typically have a diameter ≤50nm, and LUVs have a diameter >50nm. Liposomal particles useful in this aspect of the invention are ideally LUVs with a diameter in the range of 50-220nm. Techniques for preparing suitable liposomes are well known in the art. One useful method is described in Jeffs et al. (Pharmaceutical Research, 2005, 22(3): 362-372) and involves mixing (i) an ethanolic solution of the lipids (ii) an aqueous solution of the nucleic acid and (iii) buffer, followed by mixing, equilibration, dilution and purification.

### Viral Vectors

Alternatively, nucleic acid immunisation may be achieved by using a replicating or replication-defective vector, such as a viral vector. Numerous viral vectors suitable for introducing nucleic acids encoding antigens of interest into a subject are known in the art, and include both DNA and RNA viruses. Suitable examples are for instance: adenovirus vectors (replication or replication deficient), pox virus vectors, including vaccinia virus vectors, such as modified vaccinia Ankara virus (MVA), NYVAC, avipox vectors, canarypox (ALVAC) and fowl pox virus (FPV), Alphavirus vectors (such as Sindbis virus, Semlike Forest virus, Ross River virus, and Venezuelan equine encephalitis virus) and chimeras and replicons thereof, herpes virus vectors (*e.g*. cytomegalovirus-derived vectors), arena virus vectors, such as lymphocytic choriomeningitis virus (LCMV) retrovirus, lentivirus, viral like particles, and many others. In one embodiment, the polypeptide antigen in the vaccines or immunogenic compositions for use in the present invention is encoded by an adenoviral vector. In particular embodiments, the polypeptide antigen encoded by an adenoviral vector derives from HIV, Malaria, Ebola or RSV. The production and use of adenovirus vectors are well known to those of ordinary skill in the art. Suitable examples of disclosure of the design, production and use of adenovirus vectors can be found, for instance, in WO 05/071093, and WO 10/086189 which are incorporated herein by reference. Adenoviral vectors for use in the present invention may be derived from a range of mammalian hosts. Adenoviral vectors may be derived from a human adenovirus. Examples of such human-derived adenoviruses are Ad1, Ad2, Ad4, Ad5, Ad6, Ad11, Ad24, Ad34, Ad35, particularly Ad5, Ad11 and Ad35.

Alternatively, adenoviral vectors may be derived from a non-human primate adenovirus e.g. a chimpanzee adenovirus, such as one selected from serotypes ChAd3, ChAd63, ChAd83, ChAd155, Pan5, Pan6, Pan7 and Pan9. Specifically, the virus may be a non-human adenovirus, such as a simian adenovirus and in particular a chimpanzee adenovirus such as ChAd155, Pan 5, 6, 7 or 9. Examples of such strains are described in WO 03/000283 which is incorporated herein by reference and are available from the American Type Culture Collection, 10801 University Boulevard, Manassas, Virginia 20110-2209, and other sources. Desirable chimpanzee adenovirus strains include Pan 5 [ATCC VR-591], Pan 6 [ATCC VR-592], and Pan 7 [ATCC VR-593].

The adenoviral vectors for use in the present invention may be derived from replication-defective adenovirus, for example, comprising a functional E1 deletion. Adenoviral vectors for use in the present invention include PanAd3 (WO 10/086189) and ChAd155 (GB1510357.5). In some embodiments, the antigen of the vaccines or immunogenic compositions for use in the invention is recombinantly expressed in the adenoviral vector ChAd155. The adenoviral vectors can be produced on any suitable cell line in which the virus is capable of replication. Without limitation, such a cell line may be HeLa [ATCC Accession No. CCL 2], A549 [ATCC Accession No. CCL 185], HEK 293, KB [CCL 17], Detroit [e.g., Detroit 510, CCL 72] and WI-38 [CCL 75] cells, among others.

A fourth aspect of the invention provides host cell comprising a nucleic acid molecule defined the second aspect or a vector as defined in the third aspect of the invention.

Host cells that have been transformed by the nucleotide sequences or recombinant DNA of the invention are then cultured for a sufficient time and under appropriate conditions known to those skilled in the art in view of the teachings disclosed herein to permit the expression of the polypeptide, which can then be recovered.

The polypeptide of the invention can be recovered and purified from recombinant cell cultures by well-known methods including ammonium sulphate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Many expression systems are known, including (but not limited to) systems employing: bacteria (eg. *E. coli* and *Bacillus subtilis)* transformed with, for example, recombinant bacteriophage, plasmid or cosmid DNA expression vectors; yeasts (eg. *Saccharomyces cerevisiae)* transformed with, for example, yeast expression vectors; insect cell systems transformed with, for example, viral expression vectors (eg. baculovirus); plant cell systems transfected with, for example viral or bacterial expression vectors; animal cell systems transfected with, for example, adenovirus expression vectors.

The vectors can include a prokaryotic replicon, such as the Col E1 ori, for propagation in a prokaryote, even if the vector is to be used for expression in other, non-prokaryotic cell types. The vectors can also include an appropriate promoter such as a prokaryotic promoter capable of directing the expression (transcription and translation) of the genes in a bacterial host cell, such as *E. coli,* transformed therewith.

A promoter is an expression control element formed by a DNA sequence that permits binding of RNA polymerase and transcription to occur. Promoter sequences compatible with exemplary bacterial hosts are typically provided in plasmid vectors containing convenient restriction sites for insertion of a nucleotide sequence or DNA segment of the present invention.

As used herein, the term "subject" means all animals including humans (e.g., mammals as defined herein). Examples of subjects include humans, non-human primates, cows, dogs, cats, goats, sheep, and pigs. The term "patient" means a subject having a disorder in need of treatment.

A fifth aspect of the invention provides a method for producing a polypeptide according to the first aspect of the invention comprising or consisting of the steps of culturing a population of host cells according to the fourth aspect of the invention under conditions in which the peptide is expressed, and isolating the peptide therefrom.

Alternatively or additionally, the polypeptide of the first aspect of the invention may be produced by *in vitro* translation, for example, using a nucleic acid of the first aspect of the invention or a vector of the third aspect of the invention.

In addition to the available technologies, new vaccine development strategies have been explored. These innovations ideally serve to make vaccine production simpler, more cost effective, improve antigen presentation and immune response (MacLennan and Saul, 2014). For instance, the outer membrane vesicles are one of these systems employed in the vaccine development. Naturally, Gram-negative bacteria release native outer membrane vesicles (NOMV) that are rich in outer membrane lipids, outer membrane and periplasmic proteins, representing antigens in a natural conformation (Ellis and Kuehn, 2010). Therefore, NOMV based vaccines have been largely employed against the organism from they are recovered (Nieves et al., 2014; Petersen et al., 2014) or to express and deliver heterologous antigens (Bartolini et al., 2013; Daleke-Schermerhorn et al., 2014; Fantappie et al., 2014). However, in native conditions, blebs are recovered in small quantity, thus, *E. coli* strains can be genetically modified by deletion the *tolR* gene to enhance the level of vesicle production (Bernadac et al., 1998). This system has been successfully used for expression of properly folded membrane associated recombinant antigens and to induce functional immune responses (Bartolini et al., 2013). Recently, this antigen delivery approach, also known as Generalized Modules for Membrane Antigens (GMMA), has been successfully implemented and largely used (Berlanda Scorza et al., 2012; Gerke et al., 2015; De Benedetto et al., 2017).

A sixth aspect of the invention provides a vesicle comprising one or more polypeptide as defined in the first aspect of the invention.

Alternatively or additionally, the vesicle is derived from the membrane of a cell, for example, a Gram positive bacteria membrane vesicle or a Gram negative bacteria outer membrane vesicle (OMV).

OMVs are naturally-occurring membrane vesicles that form spontaneously during bacterial growth and are released into culture medium. They can be obtained e.g. by culturing bacteria in broth culture medium, separating whole cells from the smaller OMVs in the broth culture medium (e.g. by filtration or by low-speed centrifugation to pellet only the cells and not the smaller vesicles), and then collecting the OMVs from the cell-depleted medium (*e.g*. by filtration, by differential precipitation or aggregation, by high-speed centrifugation to pellet the vesicles). Strains for use in production of OMVs can generally be selected on the basis of the amount of OMVs produced in culture. The present OMVs are characterised by the fact of being collected and isolated following a detergent-free procedure. Preferably, the present OMV are released into the fermentation broth and are purified using a centrifugation and subsequent filtration step (for a general reference see *e.g*. Clin Vaccine Immunol. 2016 Apr; 23(4): 304-314). Still preferably, the present OMV are released into the fermentation broth and are purified using the following two consecutive Tangential Flow Filtration (TFF) steps: (i) a microfiltration in which the culture supernatant containing the OMV is separated from the bacteria, and (ii) an ultrafiltration in which the OMV are separated from soluble proteins (for a general reference see *e.g*. PLoS One. 2015; 10(8): e0134478). The thus obtained OMV can then directly be used within the present invention without additional purification/isolation steps. The presently considered OMVs have a preferred size distribution comprised from 20 to 250 nm, measured by Dynamic Light Scattering DLS technique.

According to some embodiments, the OMVs are prepared from wild-type bacteria or from bacteria which have been genetically manipulated generally to increase immunogenicity (*e.g*. to hyper-express immunogens), to reduce toxicity, to inhibit capsular saccharide synthesis, to down-regulate immunodominant antigen expression, and the like. They also may be prepared from hyperblebbing strains. The OMVs of the invention may also express exogenous proteins on their surface and they may be endotoxin-depleted.

Preferably, the OMVs to be used in the present invention are produced from genetically-modified bacterial strains that are mutated to enhance vesicles production, and optionally also to remove or modify antigens *(e.g.* lipid A) and/or to over-express homologous antigens or antigens from other organisms. Said preferred OMV are also known as Generalized Modules of Membrane Antigens (GMMA) as e.g. described in PLoS One. 2015; 10(8): e0134478.

Enhanced spontaneous generation of vesicles can be achieved, for example, by targeted deletion of proteins involved in maintenance of membrane integrity. It has been observed that the outer surface of OMVs substantially corresponds to the outer surface of the bacterium from which they are derived, preserving the membrane antigens (including *e.g*. lipopolysaccharides, lipooligosaccharides and lipoproteins) in the context of the membrane. Advantageously, the OMVs used in the invention (unlike detergent-extracted dOMVs) retain these outer membrane components in their native conformation and correct orientation, better preserving immunogenicity against the bacterial strain from which they are derived.

Generally, the OMVs for use in the present invention may be prepared from any suitable bacterium, where preferred bacteria include, but are not limited to: *Neisseria (e.g.* in particular *N. meningitidis* of any serogroup including A, B, C, Y, X or W135, or from a non-pathogenic *Neisseria*)*, Salmonella enterica serovars* (such as *Salmonella* Paratyphi, *Salmonella* Enteritidis, *Salmonella* Typhi or *Salmonella* Typhimurium), *Haemophilus influenzae (e.g.* non-typable *H. influenzae), Vibrio cholerae, Bordetella pertussis, Mycobacterium smegmatis, Mycobacterium bovis BCG, Escherichia coli, Bacteroides* (including *Porphyromonas), Pseudomonas aeruginosa, Helicobacter pylori, Brucella melitensis Campylobacter jejuni, Actinobacillus actinomycetemcomitans, Xenorhabdus nematophilus, Moraxella catarrhalis,* or *Borrelia burgdorferi.*

Particularly preferred bacteria are selected from at least one of: *S*. *sonnei, S. flexneri, Salmonella* bacterium, and *meningococcus,* particularly *meningococcus serogroup B.*

As far as *Salmonella* bacterium is concerned, a particularly preferred strain is selected from: *Salmonella* Typhimurium, *Salmonella* Enteritidis and *Salmonella* Paratyphi A.

*Meningococcus* bacteria OMVs are also preferred. Such vesicles can be prepared from any *meningococcal* strain. The vesicles are preferably prepared from a serogroup B strain, but it is also suitable to prepare them from serogroups other than B, such as one of: A, C, W135, Y or X, according to procedures known in the art. The strain may be of any serotype *(e.g.* 1, 2a, 2b, 4, 14, 15, 16, etc.), any serosubtype *(e.g.* P1.4), and any immunotype *(e.g.* L1; L2; L3; L3,7; L3,7,9; L10; etc.). The *meningococci* may be from any suitable lineage, including hyperinvasive and hypervirulent lineages, preferably any of the following seven hypervirulent lineages: subgroup I; subgroup III; subgroup IV-1; ET-5 complex; ET-37 complex; A4 cluster; lineage 3. Most preferably, OMVs are prepared from the strain NZ98/254, or another strain with the P1.4 PorA serosubtype.

In another embodiment, bacteria for preparing OMVs useful for the invention may be mutant strains which have been manipulated e.g. to enhance vesicles production, to express one or more desired antigen(s), and/or to knockout or modify an undesired gene (e.g. one which encodes a toxin or which encodes an enzyme involved in generating a toxic product, such as endotoxin).

In this direction, other preferred OMVs for the invention are produced by a *Salmonella* bacterium, particularly a *S*. Typhimurium (also known as *Salmonella enterica serovar* Typhimurium) which does not express a functional TolR protein.

Where the vesicles are prepared from *E. coli* or *Salmonella* the bacterium may express no more than 4 of TolA, TolB, TolQ, TolR and PaI proteins. Thus at least one protein from the natural five-protein ToI-PaI system may be absent, resulting in a bacterium which, during growth in culture medium, releases greater quantities of outer membrane vesicles into the medium than the same bacterium expressing all 5 ToI-PaI proteins. Preferably TolR is not expressed, but the other four proteins may be expressed (*i.e.* a ΔTolR strain).

In preferred embodiments, at least one of the five ToI-PaI proteins in *E.coli* or *Salmonella* is removed *e.g.* by deletion or inactivation of the gene encoding the protein. Thus the bacterium may express 0, 1, 2, 3 or 4 of TolA, TolB, TolQ, TolR and PaI proteins. Removal of one of the five proteins can suffice, in which case the bacterium expresses only 4 of these proteins. Preferably the TolR protein is removed *e.g.* by inactivation of a starting strain's tolR gene. Thus a preferred bacterium may be tolA+ tolB+ tolQ+ TolR- Pal+.

In some embodiments, the bacterium expresses all five ToI-PaI proteins, but at least one is mutated to cause hyperblebbing. For instance, the TolA, TolQ, TolR and/or PaI protein may be mutated such that the protein retains its membrane localisation but its interactions with other members of the ToI-Pal system are disrupted. The bacterium will thus retain TolA, TolQ and TolR as transmembrane proteins in the inner membrane, and PaI protein as a periplasm-facing lipoprotein in the outer membrane, but at least one of the TolA, TolQ, TolR and/or PaI proteins is mutated and not fully functional.

In addition other mutations may also be present *e.g*. to give lipopolysaccharide O antigen (OAg) deficient strains, such as DgaIU, DgaIE or DwbaP in *E.coli* or *Salmonella* strains.

In one further preferred embodiment, a meningococcus does not express a functional MltA protein. Knockout of MltA (the membrane-bound lytic transglycosylase, also known as GNA33) in meningococcus provides bacteria which spontaneously release large amounts of OMVs into culture medium, from which they can be readily purified. For instance, the vesicles can be purified using the two stage size filtration process, comprising: (i) a first filtration step in which vesicles are separated from the bacteria based on their different sizes, with the vesicles passing into the filtrate; and (ii) a second filtration step in which the vesicles are retained in the retentate.

In the present invention, it is preferred that -OAg is present on the OMVs because it has been observed (*e.g.* OMVs from *Salmonella*) that, the presence of the -OAg on the surface of said OMVs is advantageous in providing a bivalent vaccine, as the -OAg can act as a protective antigen. Some preferred strains have penta- or tetra-acylated less toxic LPS, which includes attached -OAg, after the mutation of msbB, htrB, ddg and/or PagP (see *e.g*. Rossi O et al, Clin Vaccine Immunol. 2016 Apr 4;23(4):304-14 and Rossi O et al, J Biol Chem. 2014 Sep 5;289(36):24922-35).

Where the vesicles are prepared from *Neisseria,* the strain has preferably a modified fur gene. According to this embodiment, mutant *Neisseria* are engineered to reduce or switch off expression of at least one gene involved in rendering toxic the lipid A portion of LPS, in particular of Ipxl1 gene. In this way, the resulting OMVs present a reduced toxicity with respect to the wild type strain, since the conversion of acylated lipid A in a less acylated form.

Similarly, preferred mutant *Neisseria* for the invention are engineered to reduce or switch off expression of at least one gene involved in the capsular saccharide synthesis or export, in particular of synX and/or ctrA genes. In this way, the resulting OMVs may present a cross protection versus different serotypes, particularly appreciated by the skilled in the art.

In preferred embodiments a strain may include one or more of the knockout and/or hyper-expression mutations disclosed *e.g.* in Fukusawa et al. (1999), Vaccine 17:2951-2958. For instance, following the therein guidance and nomenclature, useful genes for down-regulation and/or knockout include: (a) Cps, CtrA, CtrB, CtrC, CtrD, FrpB, GalE, HtrB/MsbB, LbpA, LbpB, LpxK, Opa, Opc, PilC, PorB, SiaA, SiaB, SiaC, SiaD, TbpA, and/or TbpB; (b) CtrA, CtrB, CtrC, CtrD, FrpB, GalE, HtrB/MsbB, LbpA, LbpB, LpxK, Opa, Opc, PhoP, PilC, PmrE, PmrF, SiaA, SiaB, SiaC, SiaD, TbpA, and/or TbpB; (c) ExbB, ExbD, rmpM, CtrA, CtrB, CtrD, GalE, LbpA, LpbB, Opa, Opc, PilC, PorB, SiaA, SiaB, SiaC, SiaD, TbpA, and/or TbpB; or (d) CtrA, CtrB, CtrD, FrpB, OpA, OpC, PilC, PorB, SiaD, SynA, SynB, SynX and/or SynC.

Alternatively or additionally, the vesicle is a Gram negative bacteria outer membrane vesicle (OMV) of a bacterium defined in the first aspect of the invention.

Alternatively or additionally, the polypeptide is:
(a) homologous to the cell; or
(b) heterologous to the cell.

Alternatively or additionally, the polypeptide is:
(a) located in the lumen of the vesicle (for example, where the vesicle is a cell, in the cytoplasm);
(b) located in the/a inter-membrane space of the vesicle (for example, where the vesicle is a cell, the periplasm]); and/or
(c) located on the outer surface of the outer membrane (for example, where the vesicle is a cell, the outer membrane).

Alternatively or additionally, the polypeptide is expressed by the cell.

Alternatively or additionally, the polypeptide is chemically conjugated to the vesicle (for example, to a lipid, protein or polysaccharide component of the vesicle membrane). Hence, the invention may comprise vesicle conjugates prepared by a process comprising the steps of:
i) reacting at least a vesicle surface protein or lipopolysaccharide residue with the first terminal portion of a divalent Linker to obtain a vesicle-linker derivative; and
ii) connecting the vesicle-linker derivative of (i) to one or more polypeptide or nucleic acid of the invention via the second terminal portion of the divalent Linker, thus obtaining a vesicle-linker-antigen.

The linker may be a divalent homobifunctional linker, *i.e.* having the same first and second terminal functionalities or a divalent heterobifunctional linker, *i.e.* having different terminal functionalities.

A seventh aspect of the invention provides a binding moiety capable of specifically binding to one or more polypeptide defined in the first aspect or a nucleic acid defined in the second aspect of the invention.

As used herein, the term "binding moiety" includes a region or regions of the agent of the invention capable of reversibly and/or irreversibly associating with a region or regions of another molecule or molecules by covalent and/or ionic interaction.

Alternatively or additionally, the binding moiety comprises an antibody or an antigen-binding fragment thereof, or a variant, fusion or derivative of said antibody or an antigen-binding fragment, or a fusion of a said variant or derivative thereof, which retains the binding specificity for a polypeptide of the invention (e.g., any one of SEQ ID Nos: 25-48).

By "antibody" we include substantially intact antibody molecules, as well as chimaeric antibodies, humanised antibodies, human antibodies (wherein at least one amino acid is mutated relative to the naturally occurring human antibodies), single chain antibodies, bispecific antibodies, antibody heavy chains, antibody light chains, homodimers and heterodimers of antibody heavy and/or light chains, and antigen binding fragments and derivatives of the same.

For example, the antibody or antigen-binding fragment, or variant, fusion or derivative thereof, may comprise, consist or consist essentially of an intact antibody. By "consist essentially of" we mean that the antibody or antigen-binding fragment, variant, fusion or derivative thereof consists of a portion of an intact antibody sufficient to retain binding specificity for a polypeptide of the invention (e.g., any one of SEQ ID NOs: 25-48).

The term 'antibody' also includes all classes of antibodies, including IgG, IgA, IgM, IgD and IgE. Thus, the antibody may be an IgG molecule, such as an IgG1, IgG2, IgG3, or IgG4 molecule.

Preferably, the antibody is an IgG antibody, for example, an IgG2 or IgG4 antibody.

In a preferred embodiment, the antibody, antigen-binding fragment, variant, fusion or derivative thereof is in an isolated and/or purified form.

In one embodiment, the antibody is a non-naturally occurring antibody. Where the antibody is a naturally occurring antibody, it is provided in an isolated form *(i.e.* separated from the milieu in which it is found in nature).

It will be appreciated by persons skilled in the art that the binding specificity of an antibody or antigen binding fragment thereof is conferred by the presence of Complementarity Determining Regions (CDRs) within the variable regions of the constituent heavy and light chains.

The variable heavy (V_{H}) and variable light (V_{L}) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent-parented antibody (Morrison et al (1984) Proc. Natl. Acad. Sci. USA 81, 6851-6855).

Antigenic specificity is conferred by variable domains and is independent of the constant domains, as known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544). A general review of the techniques involved in the synthesis of antibody fragments which retain their specific binding sites is to be found in Winter & Milstein (1991) Nature 349, 293-299.

As used herein, the term "antigen-binding fragment" refers to a functional fragment of an antibody that is capable of binding to a polypeptide of the invention (e.g., any one of SEQ ID NOs: 25-48).

Exemplary antigen-binding fragments of the invention may be selected from the group consisting of Fv fragments *(e.g.* single chain Fv and disulphide-bonded Fv), and Fab-like fragments *(e.g.* Fab fragments, Fab' fragments and F(ab)₂ fragments).

In a preferred embodiment, the antigen-binding fragment is an scFv.

The advantages of using antibody fragments, rather than whole antibodies, are several-fold. The smaller size of the fragments may lead to improved pharmacological properties, such as better penetration of solid tissue. Moreover, antigen-binding fragments such as Fab, Fv, ScFv and dAb antibody fragments can be expressed in and secreted from *E. coli or yeast,* thus allowing the facile production of large amounts of the said fragments.

Also included within the scope of the invention are modified versions of antibodies and antigen-binding fragments thereof, e.g., modified by the covalent attachment of polyethylene glycol or other suitable polymer.

Methods of generating antibodies and antibody fragments are well known in the art. For example, antibodies may be generated via any one of several methods which employ induction of *in vivo* production of antibody molecules, screening of immunoglobulin libraries (Orlandi et al, 1989. Proc. Natl. Acad. Sci. U.S.A. 86:3833-3837; Winter et al., 1991, Nature 349:293-299) or generation of monoclonal antibody molecules by cell lines in culture. These include, but are not limited to, the hybridoma technique, the human B-cell hybridoma technique, and the Epstein-Barr virus (EBV)-hybridoma technique (Kohler et al., 1975. Nature 256:4950497; Kozbor et al., 1985. J. Immunol. Methods 81:31-42; Cote et al., 1983. Proc. Natl. Acad. Sci. USA 80:2026-2030; Cole et al., 1984. Mol. Cell. Biol. 62:109-120).

The antibody or antigen-binding fragment or derivative thereof may be produced by recombinant means.

Preferably, the antibody is a monoclonal antibody.

Suitable monoclonal antibodies to selected antigens may be prepared by known techniques, for example those disclosed in *"*Monoclonal Antibodies: A manual of techniques", H Zola (CRC Press, 1988) and in *"*Monoclonal Hybridoma Antibodies: Techniques and Applications", J G R Hurrell (CRC Press, 1982), which are incorporated herein by reference.

Antibody fragments can also be obtained using methods well known in the art (see, for example, Harlow & Lane, 1988, "Antibodies: A Laboratory Manual", Cold Spring Harbor Laboratory, New York, which is incorporated herein by reference). For example, antibody fragments according to the present invention can be prepared by proteolytic hydrolysis of the antibody or by expression in *E. coli* or mammalian cells (*e.g*. Chinese hamster ovary cell culture or other protein expression systems) of DNA encoding the fragment. Alternatively, antibody fragments can be obtained by pepsin or papain digestion of whole antibodies by conventional methods, or by cell free *in vitro* expression.

As defined herein, the binding moiety may be a variant, fusion or derivative thereof of an antibody or antigen-binding fragment, provided such variants, fusions and derivatives retain binding specificity for a polypeptide of the invention (e.g., any one of SEQ ID NOs: 25-48).

Variants may be made using the methods of protein engineering and site-directed mutagenesis well known in the art using the recombinant polynucleotides (see example, see Molecular Cloning: a Laboratory Manual, 3rd edition, Sambrook & Russell, 2001, Cold Spring Harbor Laboratory Press, which is incorporated herein by reference).

In other preferred embodiments, the antibody variant may be a single-domain antibody, such as a nanobody. Such antibodies are known to exist in camelids (Curr. Opin. Pharmacol., 8, (2008), 600-608) and sharks *(e.g.* IgNAR; Curr. Opin. Pharmacol., 8, (2008), 600-608). Other preferred antibody variants include isolated heavy-chain variable (V_{H}) regions or isolated light-chain (V_{L}) regions, for example from human antibodies (Curr. Opin. Pharmacol., 8, (2008), 600-608), and iMabs (WO 03/050283).

In a preferred embodiment, the invention provides an agent wherein the antibody, antigen-binding fragment, variant, fusion or derivative thereof is capable of competing for binding to a polypeptide of the invention (e.g., any one of SEQ ID NOs: 25-48) with an antibody molecule as defined herein, or a variant, fusion or derivative of said antibody or antigen-binding fragment, or a fusion of a said variant or derivative thereof, which retains the binding specificity for a polypeptide of the invention (e.g., any one of SEQ ID NOs: 25-48).

By "capable of competing" for binding to a polypeptide of the invention (e.g., any one of SEQ ID NOs: 24-48) with an antibody molecule as defined herein (or a variant, fusion or derivative of said antibody or antigen-binding fragment, or a fusion of a said variant or derivative thereof, which retains the binding specificity for a polypeptide of the invention (e.g., any one of SEQ ID NOs: 25-48)) we mean that the tested antibody, antigen-binding fragment, variant, fusion or derivative thereof is capable of inhibiting or otherwise interfering, at least in part, with the binding of an antibody molecule as defined herein (or a variant, fusion or derivative of said antibody or antigen-binding fragment, or a fusion of a said variant or derivative thereof).

For example, the antibody or antigen-binding fragment, variant, fusion or derivative thereof, or fusion of a said variant or derivative thereof, may be capable of inhibiting the binding of an antibody molecule defined herein by at least 10%, for example at least 20%, 30%, 40%, 50%, 60%, 70%, 80%, 90%, 35% or even by 100%.

Competitive binding may be determined by methods well known to those skilled in the art, such as ELISA (as described herein) and/or SPR (as described in the accompanying Examples).

In a "Sandwich ELISA", a suitable amount of, e.g. a polyclonal antibody, such as 10 µg/ml of a polyclonal rabbit antibody, directed against the/a polypeptide of the invention (e.g., any one of SEQ ID NOs: 25-48) is used as a capture antibody when coated in a 96-well plate, such as e.g. MAXISORP NUNC. Coating is done according to standard procedures known in the art. Wells may be blocked for 1h with 3% bovine serum albumin (BSA) in tris-buffered saline-TWEEN 20 (TBS-T) at e.g. room temperature. Cell extract from HEK-cells stably transfected with an expression vector expressing the/a polypeptide of the invention (e.g., any one of SEQ ID NOs: 25-48), or from cells in which a/the polypeptide of the invention (e.g., any one of SEQ ID NOs: 25-48), is then diluted in assay buffer (e.g. TBS-T supplemented with 0.1% BSA, 1 mM MgCl2 and 10 µM CaCl₂). Suitable amount of diluted cell extract, such as e.g. 50 µl is then added per well and incubated to allow binding to the coated antibody, e.g. for 1h at room temperature. Plates are then washed three times with TBS-T. Primary antibody conjugated with biotin is then added in a suitable amount, such as e.g. at 2 µg/ml in assay buffer. Plates are then incubated for a sufficient time to allow binding of primary and control antibody where possible, e.g. for 1h at room temperature followed by three washes in TBS-T. In case where biotinylated primary antibodies are used, Streptavidin-HRP antibody (DAKO) may be used and diluted accordingly in assay buffer (1:5000), added to the wells. Plates are then incubated enough for the streptavidin-biotin complex to form, e.g. for 1h at room temperature. Following washing, e.g. three times with TBS-T and the plates are developed with peroxidase substrate (e.g. OPD SigmaFast, Sigma). The absorbance of the colorimetric change is determined at suitable wavelength, in this case 490 nm.

If primary antibodies are not conjugated, the same ELISA can instead be incubated with a secondary antibody against the human IgG4 directly conjugated with HRP, e.g. mouse anti-human IgG4-HRP, from e.g. Serotec, or, if not conjugated, followed by a HRP-conjugated anti-mouse antibody from e.g. DAKO. Plates are then washed and developed as outlined herein.

Further methods include reversing the sandwich ELISA outlined herein, and instead using the/a antibody against the/a polypeptide of the invention (e.g., any one of SEQ ID NOs: 25-48) as a capture antibody, as will appreciated by those in the art.

These ELISA assays can be used to evaluate epitope-modifying or blocking antibodies. Additional methods suitable for identifying competing antibodies are disclosed in Antibodies: A Laboratory Manual, Harlow & Lane, which is incorporated herein by reference (for example, see pages 567 to 569, 574 to 576, 583 and 590 to 612, 1988, CSHL, NY, ISBN 0-87969-314-2).

In a preferred embodiment, the invention provides an agent wherein the antibody, antigen-binding fragment, variant, fusion or derivative thereof is capable of binding to the same epitope as an antibody molecule as defined herein.

In an alternative embodiment, the invention provides an agent wherein the antibody, antigen-binding fragment, variant, fusion or derivative thereof is capable of binding to an epitope distinct from that to which an antibody molecule as defined herein.

As used herein, the term "epitope refers to a site of a molecule to which an antibody binds, *i.e.* a molecular region of an antigen. An epitope may be a linear epitope, which is determined by *e.g.* the amino acid sequence, *i.e.* the primary structure, or a three-dimensional epitope, defined by the secondary structure, *e.g.* folding of a peptide chain into beta sheet or alpha helical, or by the tertiary structure, *e.g.* way which helices or sheets are folded or arranged to give a three-dimensional structure, of an antigen.

Alternatively or additionally, the binding moiety is selected from the group consisting of: antibodies; antigen-binding fragments; and antibody mimetics. Alternatively or additionally, the binding moiety is an antibody. Alternatively or additionally, the antibody is polyclonal or monoclonal. Alternatively or additionally, the binding moiety is an antigen-binding fragment selected from the group consisting of: Fab (fragment antigen binding); F(ab')₂; Fab'; scFv (single chain variable fragment); di-scFv; sdAb (single domain antibody / domain antibody); trifunctional antibody; chemically-linked F(ab')₂; and BiTE (bi-specific T-cell engager). Alternatively or additionally, the antibody or antigen binding fragment thereof is an antigen binding fragment selected from the group consisting of affibodies molecules; affilins; affimers; affitins; alphabodies; anticalins; avimers; DARPins; fynomers; kunitz domain peptides; monobodies and nanoCLAMPs.

An eighth aspect of the invention provides a pharmaceutical composition comprising a polypeptide defined in the first aspect, a nucleic acid molecule defined in the second aspect, a vector as defined in the third aspect, a host cell as defined in the fourth aspect and/or a binding moiety as defined in the fifth aspect of the invention.

A ninth aspect of the invention provides a kit comprising or consisting of a polypeptide defined in the first aspect, a nucleic acid molecule defined in the second aspect, a vector as defined in the third aspect, a host cell as defined in the fourth aspect and/or a binding moiety as defined in the fifth aspect and/or a pharmaceutical composition as defined in the eighth aspect of the invention; and (optionally) instructions for use.

The antigen(s) and adjuvants may be combined extemporaneously, at the time of delivery. Thus, the invention provides kits including the antigen(s) and adjuvant ready for mixing. The kits allow the antigen(s) and adjuvant to be kept separately until the time of use.

The components may be physically separate from each other within a kit, and this separation can be achieved in various ways. For instance, the two components may be in two separate containers, such as vials. The contents of the two vials can then be mixed e.g. by removing the contents of one vial and adding them to the other vial, or by separately removing the contents of both vials and mixing them in a third container (for example vial). One or more of the kit components may be in a syringe.

The kit components may be in aqueous form. In some embodiments, a component such as the antigen(s) is in dry form (e.g., in a lyophilised form), with the other component being in aqueous form. The two components can be mixed in order to reactivate the dry component and give an aqueous composition for administration to a subject or patient. A lyophilised component will typically be located within a vial rather than a syringe.

Dried components may include stabilizers such as lactose, sucrose or mannitol, as well as mixtures thereof e.g. lactose/sucrose mixtures, sucrose/mannitol mixtures, etc. One possible arrangement uses an aqueous adjuvant component in a pre-filled syringe and a lyophilised antigen component in a vial.

A tenth aspect of the invention provides a polypeptide as defined in the first aspect, a nucleic acid molecule as defined in the second aspect, a vector as defined in the third aspect, a host cell as defined in the fourth aspect and/or a binding moiety as defined in the fifth aspect and/or a pharmaceutical composition as defined in the eighth aspect of the invention, for use in medicine.

An eleventh aspect of the invention provides a polypeptide as defined in the first aspect, a nucleic acid molecule as defined in the second aspect, a vector as defined in the third aspect, a host cell as defined in the fourth aspect and/or a binding moiety as defined in the fifth aspect and/or a pharmaceutical composition as defined in the eighth aspect of the invention, for use in preventing or treating bacterial infection and/or symptoms thereof.

Alternatively or additionally, the bacterial infection is, wholly or in part, infection with one or more bacterium defined in the first aspect of the invention.

Alternatively or additionally, the use comprises the consecutive or concurrent use of:
(a) a polypeptide defined in the first aspect;
(b) a nucleic acid molecule defined in the second aspect;
(c) a vector as defined in the third aspect;
(d) a vesicle as defined in the fourth aspect;
(e) a host cell as defined in the fifth aspect;
(f) a binding moiety as defined in the sixth aspect; and/or
(g) a pharmaceutical composition as defined in the eighth aspect of the invention.

Alternatively or additionally, the use comprises the consecutive or concurrent use of a binding moiety as defined in the sixth aspect of the invention with:
(a) a nucleic acid molecule of the second aspect and/or a vector as defined the third aspect of the invention; and/or
(b) a binding moiety as defined in the sixth aspect and/or a vesicle as defined in the fourth aspect of the invention.

An twelfth aspect of the invention provides the use of a polypeptide as defined in the first aspect, a nucleic acid molecule as defined in the second aspect, a vector as defined in the third aspect, a host cell as defined in the fourth aspect and/or a binding moiety as defined in the fifth aspect and/or a pharmaceutical composition as defined in the eighth aspect of the invention, in preventing or treating bacterial infection and/or symptoms thereof in a subject or patient, for example, wherein the bacterial infection is, wholly or in part, infection with one or more bacterium defined in the first aspect.

A thirteenth aspect of the invention provides the use of a polypeptide as defined in the first aspect, a nucleic acid molecule as defined in the second aspect, a vector as defined in the third aspect, a host cell as defined in the fourth aspect and/or a binding moiety as defined in the fifth aspect and/or a pharmaceutical composition as defined in the eighth aspect of the invention, in the manufacture of a medicament for preventing or treating bacterial infection and/or symptoms thereof, for example, wherein the bacterial infection is, wholly or in part, infection with one or more bacterium defined in the first aspect.

A fourteenth aspect of the invention provides a method for preventing or treating bacterial infection and/or symptoms thereof comprising or consisting of administering to a subject or patient an effective amount of a polypeptide as defined in the first aspect of the invention, a nucleic acid molecule as defined in the second aspect, a vector as defined in the third aspect, a host cell as defined in the fourth aspect and/or a binding moiety as defined in the fifth aspect and/or a pharmaceutical composition as defined in the eighth aspect of the invention, for example, wherein the bacterial infection is, wholly or in part, infection with one or more bacterium defined in the first aspect.

A fifteenth aspect of the invention provides the use of a nucleic acid molecule as defined in the second aspect of the invention, or a binding moiety as defined in the fifth aspect, for detecting the presence of bacteria, for example, wherein the bacteria are one or more bacterium defined in the first aspect of the invention. Alternatively or additionally, the detection is *in vitro* and/or *in vivo.*

**Sequences of the antigen candidates are as follows:**

| Antigen Candidate | Polynucleotide | Polypeptide |
|---|---|---|
| MC001 | SEQ ID NO: 1 | SEQ ID NO:25 |
| MC007 | SEQ ID NO: 2 | SEQ ID NO: 26 |
| MC020 | SEQ ID NO: 3 | SEQ ID NO: 27 |
| MC002 | SEQ ID NO: 4 | SEQ ID NO: 28 |
| MC003 | SEQ ID NO: 5 | SEQ ID NO: 29 |
| MC004 | SEQ ID NO: 6 | SEQ ID NO: 30 |
| MC005 | SEQ ID NO: 7 | SEQ ID NO: 31 |
| MC006 | SEQ ID NO: 8 | SEQ ID NO: 32 |
| MC008 | SEQ ID NO: 9 | SEQ ID NO: 33 |
| MC009 | SEQ ID NO: 10 | SEQ ID NO: 34 |
| MC010 | SEQ ID NO: 11 | SEQ ID NO: 35 |
| MC011 | SEQ ID NO: 12 | SEQ ID NO: 36 |
| MC012 | SEQ ID NO: 13 | SEQ ID NO: 37 |
| MC013 | SEQ ID NO: 14 | SEQ ID NO: 38 |
| MC014 | SEQ ID NO: 15 | SEQ ID NO: 39 |
| MC015 | SEQ ID NO: 16 | SEQ ID NO: 40 |
| MC016 | SEQ ID NO: 17 | SEQ ID NO: 41 |
| MC017 | SEQ ID NO: 18 | SEQ ID NO: 42 |
| MC018 | SEQ ID NO: 19 | SEQ ID NO: 43 |
| MC019 | SEQ ID NO: 20 | SEQ ID NO: 44 |
| MC021 | SEQ ID NO: 21 | SEQ ID NO: 45 |
| MC022 | SEQ ID NO: 22 | SEQ ID NO: 46 |
| MC023 | SEQ ID NO: 23 | SEQ ID NO: 47 |
| MC024 | SEQ ID NO: 24 | SEQ ID NO: 48 |

Preferred, non-limiting examples which embody certain aspects of the invention will now be described, with reference to the following figures:

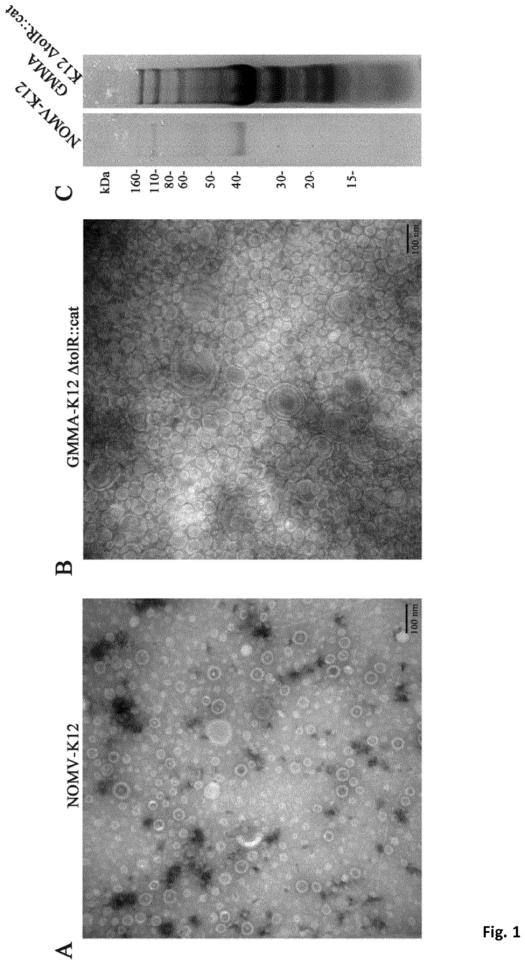

**Figure 1****. *E. coli* K12 engineering to generate Generalized Modules for Membrane Antigens (GMMA).** NOMV and GMMA were isolated by ultracentrifugation from supernatants of *E. coli* K12 WT and K12 Δ*tolR::cat* (A) Negative staining of native NOMV released from a wild-type E. coli K12 observed by transmission electron microscopy (TEM). NOMV from K12 WT strain appeared as closed spherical particles and homogeneous in shape with a size ranging from 20 to 100 nm. (B) Negative staining of GMMA produced by the K12 *tolR::cat* (K12*tolR::cat*) strain analyzed by TEM GMMA from *tolR* mutant were released in higher amount (size ranging from 20 to 200 nm), and some of them showed an atypical shape characterized by more than one membrane layer (magnification 120,000x). (C) SDS Page (4-12% bis-tris polyacrylamide) of membrane vesicles (NOMV and GMMA) purified from 75 ml of culture supernatants. Total protein content was quantified and 50 ug of GMMA obtained from K12 Δ*tolR::cat* sample was loaded into the gel. An equivalent volumetric amount of NOMV from K12 WT obtained from 75 mL of supernatant was loaded and separated by SDS-PAGE. The *tolR* mutant showed an extensive protein profile in the supernatant compared to wild type. SDS-PAGE comparison of these preparations showed that the *tolR* mutant was able to yield 25-fold more vesicles than wild-type strain in terms of total protein content.

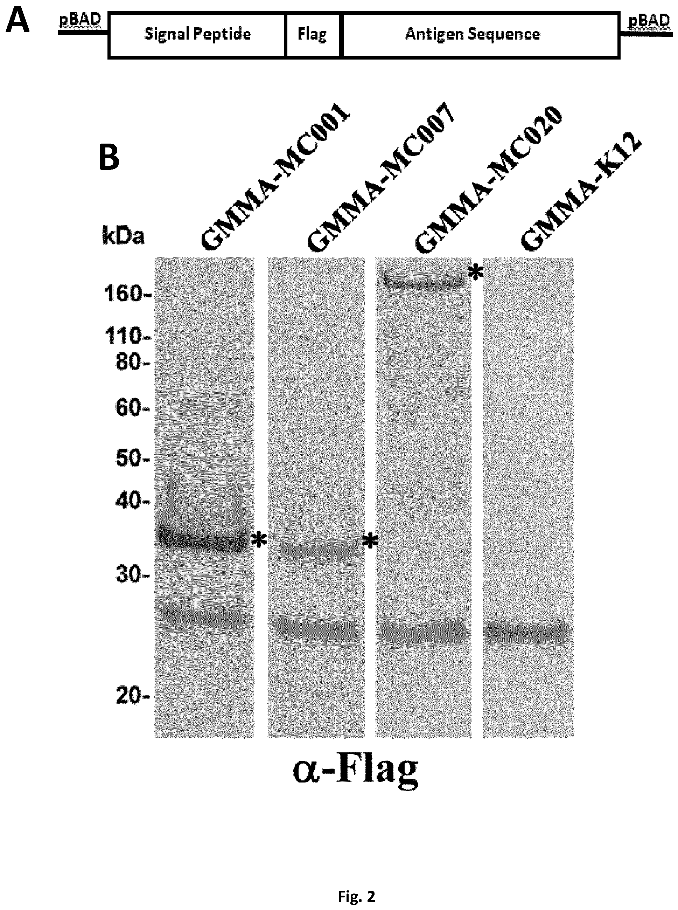

**Figure 2****. Antigen delivery into GMMA.** (A) Scheme of the antigens cloned into pBAD with flag-tag. The three selected candidates were cloned into a pBAD vector using their own signal peptide. A flag-tag was inserted after the signal peptide of each protein. (B) Western blot of MC001, MC007 and MC020 independently fused to a flag-tag. Each construct was independently transformed in the K12*tolR::cat* mutant and induced with arabinose. The western blots show the expression of the flag-fused antigens (*) on GMMA recovered from these 16h cultures, in comparison to the GMMA-K12.

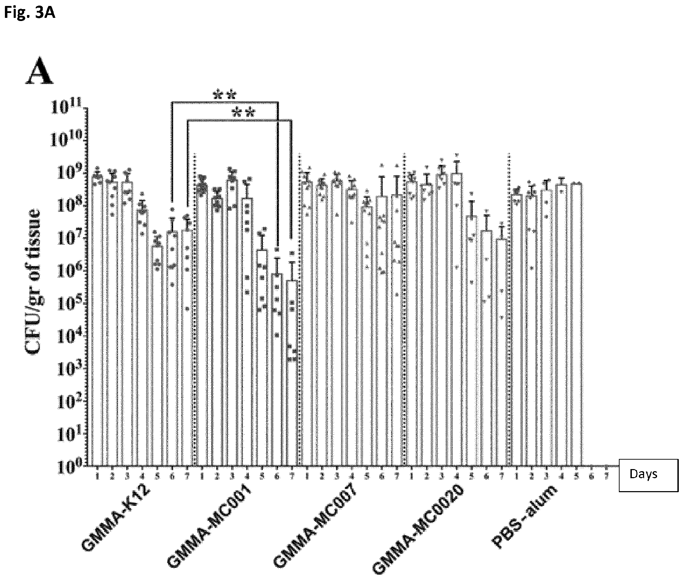

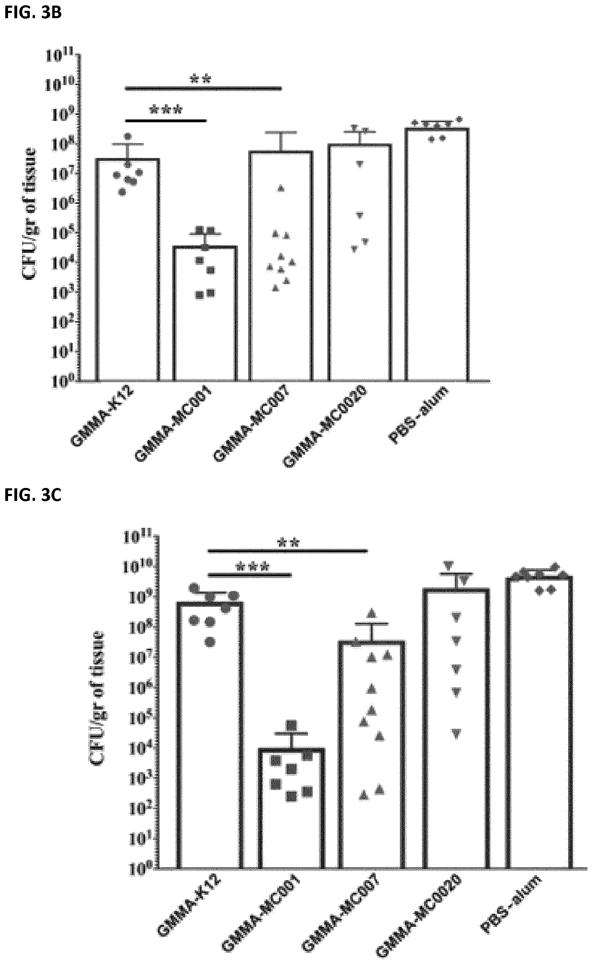

**Figure 3****. Vaccination with GMMA-MC001 reduces bacteria colonization feces, colon and caecum.** (A) Graphic representation of bacterial counts in feces from challenged mice at Days 1 -7 post-challenge. The mice immunized with GMMA-MC001 showed significant reduction in bacterial shedding at day 6 (≈2-logs, ** *P* value = 0.0033) and day 7 (≈4-logs reduction, ***P* value = 0.0037) compared to the mice immunized with GMMA-K12. (B) The bacterial colonization in colon was reduced in mice immunized with GMMA-MC001 (≈3-logs, *** *P* = 0.0003) and GMMA-MC007 (≈2-logs, ** *P* = 0.0068) compared to the mice immunized with GMMA-K12. (C) As for bacteria counts in caecum at day-7 post-challenge a significant reduction of bacteria was observed in mice immunized with GMMA-MC001 (≈5-logs, *** *P* = 0.0006) and GMMA-MC007 (≈3-logs, ** *P* = 0.0012), compared to mice immunized with GMMA-K12. The plots represent individual average and standard error of the mean (SEM) of individual mice. Significant differences in colonization were calculated by Mann-Whitney.

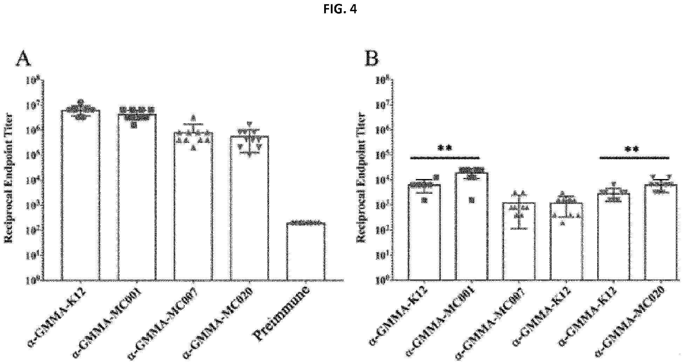

**Figure 4****. Specific total IgG response after immunization.** Serum was collected from vaccinated animals previous the first immunization and 2 weeks post third immunization. Specific IgG antibody relative titers of GMMA over expressing the antigens or recombinant protein were measured by ELISA assays. Data are shown as mean of each group. (A) The GMMA vaccines were able to trigger the immune response, compared to pre-immune sera/PBS-alum group. However, the total IgG showed no significant difference among the GMMA groups. (B) The further test of specific IgG against the recombinant proteins form of each antigen, showed a significant difference of GMMA-MC001 (** *P* = 0.0076) and GMMA-020 (** *P* = 0.0075) against GMMA-K12 group. The endpoint titer of a sample is defined as the reciprocal of the highest dilution that has a reading above the cut-off. (C) The vaccines candidates were also able to generate specific antibodies against the antigens. By western blot assays, using recombinant proteins forms of each antigens as target, allowed the recognition only of the MC001 and MC020 recombinant proteins challenged with GMMA-MC001 and GMMA-MC020 sera respectively.

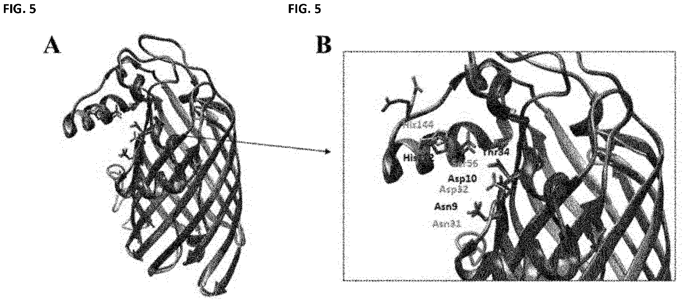

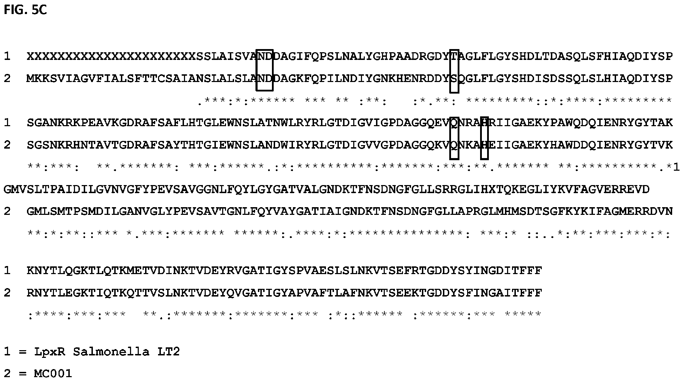

**Figure 5****. MC001 comparative protein modeling.** (A) Structural model of MC001 obtained with SWISS-MODEL (depicted in green) using LpxR from Salmonella (3FID) as template (depicted in blue). (B) A detail of figure 9a show the conservation of the active site residues. (C) Sequence alignment between MC001 and LpxR from Salmonella in which the residues of the active site are boxed.

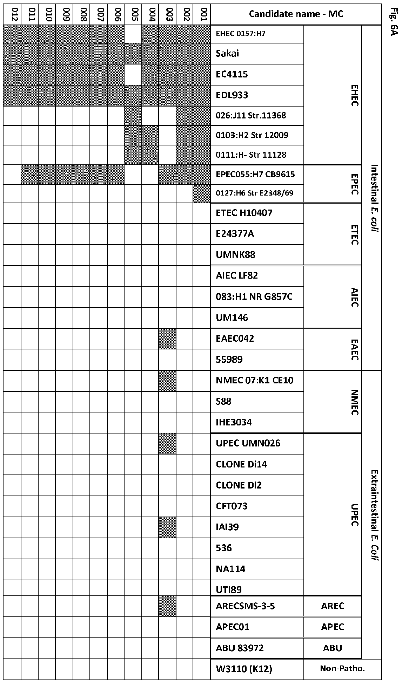

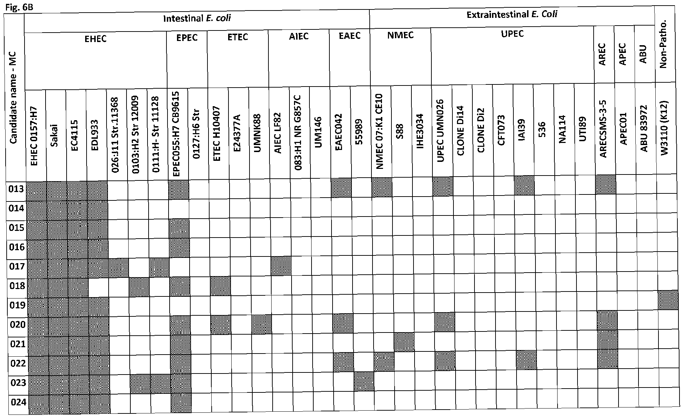

**Figure 6****. The selected candidates are mainly present and conserved among EHEC strains.** The gene distribution analysis (by BLASTP) shows the presence/absence of the antigens in a panel of 47 *E. coli* complete genomes. Black cells represent a sequence identity >= 80% with a query coverage >= 90%, white cells represent gene absence or presence with a sequence identity < 80% and query coverage below 90%. Figure 6A provides analysis for antigen candidates MC001 - MC012; Figure 6B provides analysis for antigen candidates MC013 - MC024.

### EXAMPLES

### Introduction

Enterohemorrhagic *E. coli* (EHEC) are a major cause of large outbreaks mainly affecting developed countries. For instance, from 1982 to 2002, a total of 350 *E. coli* 0157 outbreaks were reported in the United States. EHEC infection causes diarrheal disease often associated with clinical complications like hemorrhagic colitis and hemolytic uremic syndrome (HUS). Antibiotic based therapy is discouraged due to their potential undesirable effect in releasing shiga-toxin from the bacteria. No licensed vaccine specific for human use against EHEC is currently available. In this study, candidate antigens were identified from the EHEC O157:H7 genome and used with the GMMA antigen delivery system to provide new potential vaccine candidates. In particular, one of the candidate antigens (MC001) was able to reduce intestinal bacterial colonization in a mouse model; to our knowledge this study is the first report describing a lipid A deacylase enzyme (LpxR) as an antigen candidate.

### Materials and methods

### Bacterial strains and culture conditions

All bacterial strains were routinely grown in Luria Bertani (LB) media with antibiotic selection pressure if needed, at 37 °C. The *E. coli* MACH1-T1R (Thermofisher) strain was used for cloning while BL21 DE3 (NEB) strain was used to express and purify the antigens as recombinant proteins. EHEC O157:H7 strain EDL933, is the prototype pathotype used in this study for antigen identification. The strain EHEC O157:H7 86-24 was used in the animal challenging experiments.

### Antigen candidate selection

*In silico* antigen candidate identification was performed as follows. The 5675 CDS (coding DNA sequences) of EHEC O157:H7 EDL933 strain annotated genome (GeneBank sequence CP008957.1) were analyzed by PSORT software (Yu et al., 2010) to predict the cellular localization. TMHMM (http://www.cbs.dtu.dk/services/TMHMM/, (Krogh et al., 2001) was used for prediction of transmembrane regions in putative proteins.

RNA-Seq mapping and reads per kilobase per million mapped reads (RPKM) (cutoff >10) calculation was performed using Geneious R9 software (http://www.geneious.com, (Kearse et al., 2012). Distribution and sequence variability analysis into 47 *E. coli* complete annotated genomes was performed BLASTP (Altschul et al., 1997) using a cutoff of ≥90% of query coverage and a ≥80% of sequence protein identity %). Only antigens present in more than 5 intestinal pathogenic *E. coli* strains were selected. Presence of antigen candidates in non-pathogenic *E. coli* K12 and/or BL21 strains was used as exclusion criterion.

**Cloning and recombinant protein production of antigen candidates.** All candidate antigens were cloned and expressed as His-tagged fusion proteins without the predicted signal sequence. Prediction of the signal peptide was performed by Signal P (Nielsen, 2017). All fragments were amplified by PCR using primers listed in Table S4, using genomic DNA of *E. coli* EHEC O157:H7 EDL933 strain. The PCR amplicons were cloned into a pET-15b plasmid (Novagen, EMD Millipore) with a His-tag in the carboxyl-terminus by the polymerase incomplete primer extension (PIPE) method (Klock and Lesley, 2009) or the NEBUILDER HiFi DNA Assembly Master Mix (NEB). Plasmids were transformed in BL21-DE3 (NEB). Briefly, *E. coli* BL21(DE3) harboring pET-15b constructs were grown in ENPRESSO (BioSilta) following the manufacturer suggestions and using Isopropyl β-D-1-thiogalactopyranoside (IPTG) 0.01M (Sigma-Aldrich) for induction. After biomass collection, cultures were lysed by sonication. The suspension obtained was centrifuged and the supernatant passed through a Ni-NTA Agarose chelating column (Qiagen). Proteins were eluted using an immidazol concentration gradient. In the case of insoluble proteins, the purification-immidazol buffer contained urea 6 M. Protein concentrations were measured by PIERCE BCA Protein Assay Kit (Thermofisher).

### Construction of ToIR mutant

The *tolP* mutant in a *E. coli* K12 was constructed by allelic maker exchange using the Lambda red system (Datsenko and Wanner, 2000). The *tolR* was interrupted with a chloramphenicol resistance cassette (*cat*)*.* Briefly, the *cat* cassette was amplified using forward and reverse primers with ≈70-nucleotides tail homologous to the flanking region of *tolR,* Table S4. The PCR product was purified and used to transform *E. coli* K12 recipient cells (carrying the plasmid expressing the recombinase E, pKD46) as described previously (Datsenko and Wanner, 2000). The deletion of the *tolR* gene was confirmed by PCR genomic DNA amplification using primers specifically annealing to the genes upstream *(tolQ)* and downstream (*tolA*) to *tolR.*

### NOMV and GMMA production

For NOMV isolation the *E. coli* K12 MC4100 WT strain was grown at 37°C in liquid Luria-Bertani medium. For GMMA production *E. coli* K12 Δ*tolR*::*cat* was grown at 37°C in liquid Luria-Bertani (LB) medium containing chloramphenicol (20 µg/ml) as previously described (Fantappie et al., 2014; Rossi et al., 2016). Briefly, 75 ml of media were inoculated with *E. coli* K12 WT or Δ*tolR*::*cat* and grown at 37 °C, 150 rpm overnight (≈16 hrs.). To recover the supernatants cultures were centrifuged for 30 min at 8,000g and 0.22 µm filtered. These media were ultracentrifuged using propylene ultracentrifuge tubes (Beckman Coulter) at 32,000 rpm for 2h at 4°C. Pellets were washed with phosphate-buffered saline (PBS). Pellets were resuspended in 2 ml of PBS followed by 0.22-µm filtration. Vesicles were stored at 4°C. To determine the total protein content present in these preparations a quantification was performed by DC protein assay (Bio-Rad) based on the Lowry assay (Rossi et al., 2015)

### Negative-staining transmission electron microscopy

A drop of 10 µL of GMMA or NOMV suspension was placed on copper formvar/carbon-coated grids and adsorbed for 2 minutes. Grids were then washed with few drops of distilled water and blotted with a Whatman filter paper. For negative staining, grids were treated with Uranyless EM stain (Delta Microscopy with Chromalys France) for 1 minute, air-dried and viewed through transmission electron microscope Hitachi H-7650 at 80 kV. Electron micrographs were recorded at a nominal magnification of 120,000x.

### Over-expression of antigens in GMMA

To over express the MC001, MC007 and MC020 candidates in GMMA, the corresponding coding sequence including their own signal peptide was cloned into a pBAD-A plasmid (Thermofisher) using the NEBUILDER HiFi DNA Assembly Master Mix (NEB). Also, a Flag-tag sequence (DYKDDDDK (SEQ ID NO: 116)) was introduced between the signal peptide of each protein and the rest of their sequence. The generated constructs (pBAD-MC001F, pBAD-MC007F, pBAD-MC020F), were transformed into the *E. coli* K12 Δ*tolR*::*cat* mutant and induced with arabinose (0.01% in final concentration). The generated GMMA were named GMMA-MC001, GMMA-MC007 and GMMA-MC020. The GMMA not expressing any antigen and obtained by transforming the empty pBAD-A plasmid were named GMMA-K12.

### Mice immunization and colonization model

Five-weeks old BALB/c mice (Janvier) (10 mice per group) were immunized using either (a) PBS-alum hydroxide as adjuvant (ALHYDROGEL 2%, Invivogen), (b) GMMA- expressing the MC001 candidate (GMMA-MC001) plus adjuvant, (c) GMMA expressing the MC007 candidate (GMMA-MC007) plus adjuvant, (d) GMMA expressing the MC020 candidate (GMMA-MC020) plus adjuvant, or GMMA-K12 (GMMA not expressing any candidate) plus adjuvant. All GMMA preparations were formulated using 2 mg/ml alum hydroxide as adjuvant. Animals were immunized (Day 1) by intraperitoneal injections (i.p.) with 10 µg of GMMA plus alum hydroxide, and at day 21 and day 35 with 5 µg of GMMA plus alum hydroxide. Blood was collected from all the mice prior immunization and two weeks after the third dose. The challenge experiment was performed at day-49 and using the EHEC O157:H7 strain 86-24. Mice were treated with streptomycin 24-hours prior infection. Also, animals received cimetidine 2 hours before infection via i.p. The animals were infected with 5x10⁹ CFU via gavage. Animal monitoring was performed on a daily basis including weight, signs and symptoms surveillance. Mice displaying signals of illness and losing more than the 15% of the total weight were euthanized, collecting the colon and colon organs. Fecal pellets were collected every day from day 1 to day 7 post-infection. At day 7 the remaining mice were euthanized and their colon and colon organs were collected. This animal model was adapted from models previously reported (Mohawk and O'Brien, 2011; Garcia-Angulo et al., 2013; Garcia-Angulo et al., 2014). All animal experiments were reviewed and approved by the Auvergne Committee for Animal Experimentation C2EA (Agreement N°6065-2016071216144325V2).

### Enzyme-linked Immunosorbent Assay (ELISA)

Ninety-six well Maxisorp plates (Nunc, Thermo Fisher Scientific) were coated with 1 mg/ml of GMMA preparations antigens or 1mg/ml of recombinant protein in PBS overnight (O/N) at 4 ºC. Next day, plates were washed 3 times with T-PBS (0.05% Tween 20 in PBS, pH 7.4) and blocked with 100 µl 2% BSA (Sigma-Aldrich) for 1 hour at 37 °C. Every incubation step was followed by triple T-PBS wash. Serum samples were initially diluted 1:200 in 2% BSA in T-PBS, transferred to coated-blocked plates and serially 2-fold diluted followed by 2-hours incubation at 37 ºC. Then 100 µl/well of 1:2,000 diluted alkaline phosphatase-conjugated goat anti-mouse IgG (H+L) (Southern Biotech) were added and incubated for 2 hours at 37 ºC. Bound alkaline phosphatase was visualized by adding SIGMAFAST p-Nitrophenyl phosphate (Sigma-Aldrich) After 30 minutes at room temperature, plates were analyzed at 405 nm in a microplate spectrophotometer. The endpoint titer of a sample is defined as the reciprocal of the highest dilution that has a reading above the cut-off (Frey, 1998).

### Western Blotting

Western blots were carried out on whole cell extracts (wce), recombinant proteins or GMMA preparations. SDS page was performed in MES buffer (Thermofisher) and transferred to iBlot 2 nitrocellulose stacks (iBlot system, Thermofisher). To visualize transferred proteins, the membranes were stained with ponceau red. Then, membranes were blocked with 10% (w/v) blotting-grade blocker (Bio-Rad) in T-PBS. The membranes were later incubated with the respective mouse polyclonal antisera in a 1:1000 dilution in T-PBS-3% blocker 1 hr. at room temperature. Membranes were washed three times with T-PBS and then incubated with goat anti-mouse horseradish peroxidase-conjugated IgG (Dako antibodies) diluted (1:2000) in T-PBS-3% blocker. Colorimetric staining was performed using Opti-4CN Substrate Kit (Bio-Rad) following manufacturer instructions. To detect the FLAG-tag monoclonal ANTI-FLAG M2 secondary antibody was used (Sigma Aldrich).

### Comparative structural modelling

Structural models of MC001 have been obtained by employing three different approaches: the threading/*ab initio* modelling method implemented in the I-TASSER pipeline (Roy et al., 2010), the membrane proteins-specific approach of MEMOIR (Ebejer et al., 2013) and the homology modelling method of SWISS-MODEL (Biasini et al., 2014). The search for suitable modelling templates has been carried out with PSI-BLAST (Position-Specific Iterated BLAST) (Altschul et al., 1997) sequence similarity search against the Protein Data Bank using the amino acid sequence of MC001 as a bait. While MEMOIR does not provide a proper quality assessment of the models, in the case of I-TASSER and SWISS-MODEL, the quality of the final models has been assessed through the parameters C-score and QMEAN4 (Benkert et al., 2009), respectively. The C-score is a confidence score calculated based on the reliability of threading template alignments and the convergence parameters of the structure assembly simulations. C-score values typically range between -5 and 2, higher values characterizing high confidence models and vice-versa. The QMEAN4 score is a linear combination of four statistical potential terms and is typically in the range 0-1, with higher values characterizing better quality models. MC001 models are characterized by a C-score of -5 and a QMEAN4 score of 0.74.

### Statistical analysis.

All of the statistical analyses were done using GraphPad Prism 7 software. Mann-Whitney (unpaired and non-parametric) and Student t test with threshold of P = < 0.05 were used to analyse the data of the bacterial counts from the mouse colonization model and for the IgG antibody response.

### RESULTS

### Identification of antigen candidates

Potential antigens in EHEC O157:H7 EDL933 strain were identified by first analysing the putative cellular localization of the 5675 CDS from the annotated genome of EHEC O157:H7 EDL933 strain using the PSORT algorithm. We focused mainly on chromosome-encoded proteins predicted to be exported, surface associated proteins, outer-membrane-associated proteins, and proteins with an unknown localization. The selection criteria also included proteins greater than 200 amino acids and with less than 3 transmembrane repeats determined by the TMHMM algorithm. As result of this analysis, 329 potential antigen candidates were identified (Table S1). Next, RNA-Seq data available in NCBI Sequence Read Archive (SRA) was used to identify genes that were expressed at transcriptional level. These RNA-Seq dataset were previously generated using EHEC EDL933 strain grown in LB, LB with antibiotics, LB-agar media and cattle faeces (Landstorfer et al., 2014). Reads mapping on EHEC EDL933 resulted in 68 genes showing an absolute index number of ≥ 10 RPKM (reads per kilobase per million mapped reads) in at least one of the four growth conditions analysed (Table S2). Another selection criterion was based on gene variability and distribution analysis of these 68 EHEC EDL933 potential antigens on 47 complete genomes to select those present (query coverage: ≥90%) and conserved (sequence identity ≥80%) in more than 5 different intestinal pathogenic *E. coli* strains. In addition, presence of antigen candidates in non-pathogenic *E. coli* such as K12 was used as exclusion criterion (Figure 6). This *in silico* selection led to the identification of 24 potential antigens which were cloned, expressed and purified as recombinant His-tagged fusion proteins in *E. coli.* Polynucleotide and polypeptide sequences of these 24 antigen candidates are provided in Table S5. Of these, 12 were successfully purified as soluble and 12 as insoluble proteins (Table S3).

The recombinant proteins were then used to immunize mice to produce polyclonal antibodies. These antibodies were subsequently tested in Western Blot analysis to assess the expression level of the corresponding potential candidate in the homologous EHEC O157:H7 strain, leading to the identification of 17 expressed proteins in standard laboratory growth conditions (Table S3). Finally, among these expressed proteins and as proof of concept, we focused on three potential antigens satisfying all the criteria mentioned herein. In particular, these three antigen candidates included an outer membrane protein (MC001), a putative aminopeptidase (MC007) and an autotransporter belonging to the AIDA family (MC020) (Table 1).

**Table 1. Features of the three selected antigen candidates**

| **Selected Candidates** | **Protein ID** | **Annotation feature** | **Localization prediction** | **Pfam Domain** | **Signal- Localization** | **Purified recombinant protein** |
|---|---|---|---|---|---|---|
| MC001 | AIG67060.1 | Putative outer membrane protein | Extracellular | DUF2219 | Non-Cytoplasmic | Insoluble |
| MC007 | AIG66424.1 | Putative aminopeptidase | Unknown | Unknown | Non-Cytoplasmic | Soluble |
| MC020 | AIG69974.1 | Pertactine precursor | Extracellular | AIDA, Pertactine | Unknown | Soluble |

### Antigen delivery in Generalized Modules for Membrane Antigens (GMMA)

To express the three selected candidates in GMMA, we first generated an overblebbing *E. coli* K12 by mutating the *tolP* gene (K12 Δ*tolR*::*cat*)*.* NOMV and GMMA were then isolated by ultracentrifugation from supernatants of *E. coli* K12 WT and the K12 Δ*tolR::cat.* The native OMV (NOMV) released from the wild-type *E. coli* K12 and GMMA preparation produced by the *tolR* mutant were observed by transmission electronic microscopy (TEM). This analysis showed that NOMV from K12 WT strain appeared as closed spherical particles and were homogeneous in shape with a size ranging from 20 to 100 nm (Figure 1A). On the other hand, GMMA from *tolR* mutant were released in higher amount (size ranging from 20 to 200 nm), and some of these GMMA showed an atypical shape characterized by more than one membrane layer (magnification 120,000x)(Figure 1B). SDS-PAGE (4-12% bis-tris polyacrylamide) comparison of membrane vesicles (NONV and GMMA) purified from 75 ml of culture supernatants showed that the *tolR* mutant yielded 25-fold more vesicles than wild-type strain in terms of total protein content (Figure 1C). Total protein content was quantified and 50 ug of GMMA obtained from K12 Δ*tolR::cat* sample was loaded into the gel. An equivalent volumetric amount of NOMV from K12 WT obtained from 75 mL of supernatant was loaded and separated by SDS-PAGE. The *tolR* mutant showed an extensive protein profile in the supernatant compared to wild type.

In order to express the three antigen candidates (MC001, MC007 and MC0021) in GMMA, each of their coding sequences was cloned in pBAD plasmid and the FLAG tag was inserted after their own signal peptide sequence (Figure 2A). The generated constructs (pBAD-MC001F, pBAD-MC007F, pBAD-MC020F), were each independently transformed in the K12*tolR::cat* mutant and induced with arabinose. To test whether these plasmids were expressing the antigen candidates and incorporated into GMMA, we performed a western blot on GMMA recovered from 16h cultures using the anti-FLAG antibody. As shown in Figure 2B all the three antigens were specifically recognized by the anti-Flag. These results indicate that the selected antigen candidates were expressed in GMMA and preparations of these vesicles can be used as an antigen delivery system.

### Immunization with MC001-GMMA reduces EHEC intestinal bacterial colonization in mice

To test the ability of the selected candidate antigens to prevent or reduce bacterial infection, an intestinal colonization model was setup using BALB/c mice. The EHEC O157:H7 86-24 strain was used for its ability to maintain stable intestinal bacterial colonization for 7-days post-infection using 5x10⁹ CFU (data not shown). Groups of ten mice were immunized with GMMA over-expressing the candidate antigens or with empty GMMA-K12 via intraperitoneal delivery at day 1, 21 and 35. At day 49 , mice were infected with EHEC O157:H7 86-24 strain, via gavage. Fecal samples were collected in a daily basis for performing bacterial counts. GMMA-MC001 immunized mice showed a ≈3-log reduction (*P* = 0.0001) in fecal bacteria number compared to PBS-alum immunized mice at day 5 after infection, while a ≈2-log and ≈4-log reduction (*P* value = 0.0033 and 0.0037) was obtained in comparison to empty GMMA-K12 immunized groups at day 6 and 7 respectively (Figure 3A). For ethical reasons, at day 5 most of the PBS-alum immunized mice were euthanized due their weight loss (>15% of initial body weight). By contrast, mice immunized with GMMA-MC007 and GMMA-MC020 preparations did not show significant reduction in fecal bacterial shedding in comparison to PBS-alum and GMMA-K12 immunized mice (Figure 3A).

At day 7 post infection, colon and caecum tissues were collected from all mice groups and bacterial count was performed. The number of bacteria in colon and caecum tissues was significantly reduced (3-logs and 5-logs, *P* = 0.0003 and 0.0006 respectively) in mice immunized with GMMA-MC001 in comparison to mice immunized with GMMA-K12, while GMMA-MC007 immunized mice showed a reduction of ≈2-logs and ≈3-logs for colon and caecum respectively (*P* =0.0068 and 0.0012) (Figure 3B and 3C) compared to the mice immunized with GMMA-K12.

### Immunization with GMMA expressing antigen candidates induces specific antigen antibody response

To assess the immune response induced by GMMA immunization *per se* and the possible contribution in raising specific immune response against the three antigen candidates (MC001, MC007 and MC020) expressed in GMMA, serum antibody levels were determined by ELISA.

To measure the total level of immunoglobulins G (IgG), serum samples were collected from the mice before the first immunization (preimmune sera) and two weeks after the third immunization, before challenging mice. Microtiter plates coated with purified preparations of GMMA-K12 and each of the three GMMA carrying a antigen candidate showed higher total IgG levels in all the immunized groups versus the preimmune sera. A non-significant difference was found among the four GMMA immunized groups (Fig. 5A). To test whether there was an induction of a specific immune response attributable to the antigens expressed in GMMA, we perform ELISA assays using microtitre plates coated with the MC001, MC007 and MC020 recombinant proteins. A significant increase in antibody response was found for GMMA-MC001 (*P* = 0.0076) and GMMA-MC020 (*P* = 0.0075) sera in comparison to GMMA-K12 sera (Fig. 5B). To confirm that the immunization with GMMA expressing these antigen candidates was able to generate antigen specific antibodies, we performed western blot assays, using MC001, MC020 or MC007 recombinant proteins as target. Only MC001 and MC020 recombinant proteins were detected using GMMA-MC001 and GMMA-MC020 sera respectively, while MC007 was not recognized by GMMA-MC007 serum.

### MC001 is homologous to Salmonella typhimurium lipid A deacylase (LpxR)

To obtain more insights about the structural features of the **MC001** antigen candidate an *in-silico* analysis was performed. In order to find proteins with known structure and significant sequence similarity with MC001, its protein sequence was used to run a PSI-BLAST search against the Protein Data Bank (PDB) (Berman et al., 2000). This search retrieved as first hit the sequence of the ***Salmonella** typhimurium* lipid A deacylase (LpxR). Furthermore, a PSI-BLAST search over the non-redundant protein sequences database revealed a high sequence similarity also with LpxR from *Vibrio cholerae, Yersinia enterocolitica* and *Helicobacter pylori.* Structural **MC001** models using the LpxR structure as a template (PDB code: 3FID (Rutten et al., 2009) were built by I-TASSER, MEMOIR and SWISS-MODEL software. All three generated models that were in agreement with each other and showing a pairwise Cα root mean square deviation being in all cases lower than 0.5 Å.

Figure 5A provides a representative MC001 structural model obtained with the SWISS-MODEL The MC001 model was composed of a 12-stranded β-barrel in which the β-strands were arranged in an antiparallel fashion in a structure that is quite common in porins and other cell membrane proteins. The high homology between MC001 and *Salmonella typhimurium* LpxR was confirmed by the presence in the active site of six conserved residues essential for Ca+2 binding and LpxR catalytic activity: (*Salmonella*/*E. coli)* Asn (9/31), Asp (10/32), Thr/Ser (34/56), His (122/144), Gln (118/140) and Glu (128/150) (Figure 5B). Sequence comparison revealed that MC001 has the same length of the LpxR *Salmonella* orthologue (319 amino acids) and shares approximately 74% sequence identity and 93.73% sequence similarity (74% of identity with 93% query coverage) (Figure 5C).

### Discussion

In this study, we identified 329 potential antigen candidates starting from the 5675 CDS of the EHEC O157:H7 EDL933 annotated genome. Further, to determine whether these candidates are expressed during infection or *in vitro* thus having a higher potential to be immunogenic we took into account the expression at the RNA level. Exploiting previously generated RNA-Seq dataset (Landstorfer et al., 2014) we identified antigens expressed in at least one out of four analyzed conditions, and selected potential antigens possibly present and conserved in more than a single intestinal pathotype. Following these criteria, we selected 24 candidate antigens; 17 of these 24 were found to be expressed also at the protein level in standard laboratory growth condition. Among this antigen panel, 12 proteins were predicted to be outer membrane associated proteins, three with extracellular localization and nine unknown.

On the basis of these observations we used GMMA to express and deliver these candidate antigens (Berlanda Scorza et al., 2012; Bartolini et al., 2013; Daleke-Schermerhorn et al., 2014; Fantappie et al., 2014); GMMA is a system that has been successfully used for expression of properly folded membrane associated recombinant antigens and to induce functional immune responses (Bartolini et al., 2013). Also, GMMA can be a useful delivery system because the presence of native proteins on the membrane surface can act as self-adjuvants, helping to elicit an immune response (Kaparakis-Liaskos and Ferrero, 2015). However, in native conditions, blebs are recovered in small quantity and as consequence *E. coli* strains may be genetically modified by deletion of the *tolR* gene to enhance the level of vesicle production (Bernadac et al., 1998; Berlanda Scorza et al., 2012). In a first attempt, we introduced the *tolR* mutation in EHEC in which Stx was deleted to avoid possible release of toxins into the vesicles. Once these purified blebs were used to immunize mice we observed sick animals, showing bristled hair, lethargic behavior and lose of weight (data not shown). For this reason, we constructed a *tolR* mutant in an avirulent *E. coli* K12 which was able to release higher amounts of blebs in comparison to the wild-type K12.A small fraction of these vesicles when observed in TEM showed an atypical shape characterized by more than one membrane layer (double-bilayer) a characteristic observed by Perez-Crutz et *al.,* (Perez-Cruz et al., 2013; Perez-Cruz et al., 2016). In the present study, we used GMMA for antigen expression and delivery. As proof of concept we selected three out of the 24 candidate antigens, including an outer membrane protein (MC001), a putative aminopeptidase (MC007) and an autotransporter (MC020) for expression in GMMA. Two out of the three selected candidate antigens (MC001 and MC020) showed typical features of membrane associated proteins, while for the MC007 the putative cellular localization was unknown. In addition, the MC001 candidate when purified as recombinant protein was obtained as insoluble form. Thus, we reasoned that the expression and delivery of these antigen candidates in GMMA would increase their antigenic potential since they could be presented in their native conformation. Our data showed that all the three candidates expressed by GMMA were specifically recognized by the anti-Flag antibody.

For testing the ability of the selected candidate antigens to prevent or reduce bacterial infection, an intestinal colonization model was setup using BALB/c mice. This animal model was adapted from the previous animal models of infection for EHEC and Garcia-Angulo and colleagues (Mohawk and O'Brien, 2011; Garcia-Angulo et al., 2013). Although mice did not develop the symptoms associated with diarrheal disease as observed in humans, these murine models of *E. coli* O157:H7 infection, based on streptomycin-treated Balb/c mice, are promising for EHEC colonization and candidate vaccine testing (Mohawk and O'Brien, 2011; Garcia-Angulo et al., 2013). Our data indicated that a stable EHEC intestinal bacterial colonization for 7-days post-infection using 5x10⁹ CFU was maintained in the animals, and immunization with GMMA-K12 did not result in toxicity. We showed that intraperitoneal immunization using MC001 was able to significantly reduce EHEC colonization in mice feces (day 6 and 7), colon and caecum tissues (day 7), in comparison to immunization with empty GMMA-K12. For MC007 a less significant reduction was observed in colon and caecum tissues.

Moreover, the immune response of the treated mice not only showed high titers of total IgG in the GMMA-MC001 vaccinated group, but also IgG specific to MC001. In fact, MC001 recombinant protein was recognized by GMMA-MC001 serum indicating this antigen candidate was associated with membrane vesicles and also easily accessible to immune system.

In Western Blot, MC020 recombinant protein was detected by its respective GMMMA serum, and in agreement with the ELISA results showing higher IgG titer in comparison with GMMA-K12; this was not the case for MC007.

As MC001 was indicated by these studies to be a promising antigen candidate, we used bioinformatic pipelines to predict molecular model by homology. BLAST analysis showed that MC001 was homologous to the ***Salmonella** typhimurium* lipid A deacylase (LpxR) and shared similarity also with LpxR from *Vibrio cholerae, Yersinia enterocolitica* and *Helicobacter pylori.* Structural MC001 model using the ***Salmonella** typhimurium* LpxR as a template revealed a structure composed of a 12-stranded β-barrel in which the β-strands were arranged in an antiparallel fashion. The high homology between MC001 and ***Salmonella** typhimurium* LpxR was also confirmed by the presence in the active site of six conserved residues essential for Ca+2 binding and LpxR catalytic activity. In addition, we showed that MC001 was present and conserved among different EHEC strains as Sakai, O26:H11, O103:H2 and EPEC genomes.

Recently, it has been reported that LpxR can play an important role in pathogenesis by removing the 3'-acyloxyacyl group of lipid A (the hydrophobic anchor of lipopolysaccharide, LPS). This modification increases the ability of ***Salmonella** Typhimurium* to evade the innate immune response and promotes survival within macrophages (Kawasaki et al., 2012; Petrone et al., 2014). More recently, a role of LpxR from EHEC Sakai strain was shown in the innate immune response evasion. Experiments with an EHEC O157:H7 *lpxR* mutant strain, reduced the lipid A deacylation and showed an increased inflammatory and phagocytic responses. These effects were attributed to the augmented NF-κB signaling and phosphorylated p38 mitogen protein kinase (MPK), both via TLR4 response. In contrast, LpxR-positive strains, able to modify the lipid A, were capable of attenuating these immune responses. This study also showed the prevalence of LpxR in other LEE positive pathotypes, as EPEC, and as we did in this study (Figure 6). Furthermore, LpxR seems to be under the positive regulation of Ler and Pch -LEE-positive transcriptional regulators- and H-NS -global-negative regulator. Ler and H-NS seem to directly regulate the LpxR expression (as many of both LEE- and non-LEE-encoded genes). In addition, Pch and expression of LEE-virulence genes are involved also in the modulation of LPxR effect on lipid A, more directly by activating these virulence genes for colonization and in the phagocytic regulation response. In this regard, targeting a specific antibody response toward LpxR could potentially avoid the LPS modification and subsequent immune evasion. However, further studies are ongoing to characterize this protein and its role in EHEC pathogenesis and immune modulation in the host.

The present studies on the EHEC O157:H7 genome combined with the GMMA antigen delivery system led us to identify new potential antigen candidates. In particular, one of them (MC001) was able to reduce intestinal bacterial colonization and to our knowledge this study was the first report describing a lipid A deacylase enzyme (LpxR) as an antigen candidate.

### REFERENCES

Altschul, S.F., Madden, T.L., Schaffer, A.A., Zhang, J., Zhang, Z., Miller, W., et al. (1997). Gapped BLAST and PSI-BLAST: a new generation of protein database search programs. Nucleic Acids Res 25(17), 3389-3402.
Bartolini, E., Ianni, E., Frigimelica, E., Petracca, R., Galli, G., Berlanda Scorza, F., et al. (2013). Recombinant outer membrane vesicles carrying Chlamydia muridarum HtrA induce antibodies that neutralize chlamydial infection in vitro. J Extracell Vesicles 2. doi: 10.3402/jev.v2i0.20181.
Benkert, P., Kunzli, M., and Schwede, T. (2009). QMEAN server for protein model quality estimation. Nucleic Acids Res 37(Web Server issue), W510-514. doi: 10.1093/nar/gkp322.
Berlanda Scorza, F., Colucci, A.M., Maggiore, L., Sanzone, S., Rossi, O., Ferlenghi, I., et al. (2012). High yield production process for Shigella outer membrane particles. PLoS One 7(6), e35616. doi: 10.1371/journal.pone.0035616.
Berman, H.M., Westbrook, J., Feng, Z., Gilliland, G., Bhat, T.N., Weissig, H., et al. (2000). The Protein Data Bank. Nucleic Acids Res 28(1), 235-242.
Bernadac, A., Gavioli, M., Lazzaroni, J.C., Raina, S., and Lloubes, R. (1998). Escherichia coli tol-pal mutants form outer membrane vesicles. J Bacteriol 180(18), 4872-4878.
Biasini, M., Bienert, S., Waterhouse, A., Arnold, K., Studer, G., Schmidt, T., et al. (2014). SWISS-MODEL: modelling protein tertiary and quaternary structure using evolutionary information. Nucleic Acids Res 42(Web Server issue), W252-258. doi: 10.1093/nar/gku340.
Croxen, M.A., and Finlay, B.B. (2010). Molecular mechanisms of Escherichia coli pathogenicity. Nat Rev Microbiol 8(1), 26-38. doi: 10.1038/nrmicro2265.
Croxen, M.A., Law, R.J., Scholz, R., Keeney, K.M., Wlodarska, M., and Finlay, B.B. (2013). Recent advances in understanding enteric pathogenic Escherichia coli. Clin Microbiol Rev 26(4), 822-880. doi: 10.1128/CMR.00022-13.
Daleke-Schermerhorn, M.H., Felix, T., Soprova, Z., Ten Hagen-Jongman, C.M., Vikstrom, D., Majlessi, L., et al. (2014). Decoration of outer membrane vesicles with multiple antigens by using an autotransporter approach. Appl Environ Microbiol 80(18), 5854-5865. doi: 10.1128/AEM.01941-14.
Datsenko, K.A., and Wanner, B.L. (2000). One-step inactivation of chromosomal genes in Escherichia coli K-12 using PCR products. Proc Natl Acad Sci U S A 97(12), 6640-6645. doi: 10.1073/pnas.120163297.
De Benedetto, G., Alfini, R., Cescutti, P., Caboni, M., Lanzilao, L., Necchi, F., et al. (2017). Characterization of O-antigen delivered by Generalized Modules for Membrane Antigens (GMMA) vaccine candidates against nontyphoidal Salmonella. Vaccine 35(3), 419-426. doi: 10.1016/j.vaccine.2016.11.089.
Ebejer, J.P., Hill, J.R., Kelm, S., Shi, J., and Deane, C.M. (2013). Memoir: template-based structure prediction for membrane proteins. Nucleic Acids Res 41(Web Server issue), W379-383. doi: 10.1093/nar/gkt331.
Ellis, T.N., and Kuehn, M.J. (2010). Virulence and immunomodulatory roles of bacterial outer membrane vesicles. Microbiol Mol Biol Rev 74(1), 81-94. doi: 10.1128/MMBR.00031-09.
Fantappie, L., de Santis, M., Chiarot, E., Carboni, F., Bensi, G., Jousson, O., et al. (2014). Antibody-mediated immunity induced by engineered Escherichia coli OMVs carrying heterologous antigens in their lumen. J Extracell Vesicles 3. doi: 10.3402/jev.v3.24015.
Garcia-Angulo, V.A., Kalita, A., Kalita, M., Lozano, L., and Torres, A.G. (2014). Comparative genomics and immunoinformatics approach for the identification of vaccine candidates for enterohemorrhagic Escherichia coli O157:H7. Infect Immun 82(5), 2016-2026. doi: 10.1128/IAI.01437-13.
Garcia-Angulo, V.A., Kalita, A., and Torres, A.G. (2013). Advances in the development of enterohemorrhagic Escherichia coli vaccines using murine models of infection. Vaccine 31(32), 3229-3235. doi: 10.1016/j. vaccine.2013.05.013.
Gerke, C., Colucci, A.M., Giannelli, C., Sanzone, S., Vitali, C.G., Sollai, L., et al. (2015). Production of a Shigella sonnei Vaccine Based on Generalized Modules for Membrane Antigens (GMMA), 1790GAHB. PLoS One 10(8), e0134478. doi: 10.1371/journal.pone.0134478.
Goldwater, P.N., and Bettelheim, K.A. (2012). Treatment of enterohemorrhagic Escherichia coli (EHEC) infection and hemolytic uremic syndrome (HUS). BMC Med 10, 12. doi: 10.1186/1741-7015-10-12.
Kaparakis-Liaskos, M., and Ferrero, R.L. (2015). Immune modulation by bacterial outer membrane vesicles. Nat Rev Immunol 15(6), 375-387. doi: 10.1038/nri3837.
Kaper, J.B., Nataro, J.P., and Mobley, H.L. (2004). Pathogenic Escherichia coli. Nat Rev Microbiol 2(2), 123-140. doi: 10.1038/nrmicro818.
Kawasaki, K., Teramoto, M., Tatsui, R., and Amamoto, S. (2012). Lipid A 3'-O-deacylation by Salmonella outer membrane enzyme LpxR modulates the ability of lipid A to stimulate Toll-like receptor 4. Biochem Biophys Res Commun 428(3), 343-347. doi: 10.1016/j.bbrc.2012.10.054.
Kearse, M., Moir, R., Wilson, A., Stones-Havas, S., Cheung, M., Sturrock, S., et al. (2012). Geneious Basic: an integrated and extendable desktop software platform for the organization and analysis of sequence data. Bioinformatics 28(12), 1647-1649. doi: 10.1093/bioinformatics/bts199.
Klock, H.E., and Lesley, S.A. (2009). The Polymerase Incomplete Primer Extension (PIPE) method applied to high-throughput cloning and site-directed mutagenesis. Methods Mol Biol 498, 91-103. doi: 10.1007/978-1-59745-196-3_6.
Krogh, A., Larsson, B., von Heijne, G., and Sonnhammer, E.L. (2001). Predicting transmembrane protein topology with a hidden Markov model: application to complete genomes. J Mol Biol 305(3), 567-580. doi: 10.1006/jmbi.2000.4315.
Landstorfer, R., Simon, S., Schober, S., Keim, D., Scherer, S., and Neuhaus, K. (2014). Comparison of strand-specific transcriptomes of enterohemorrhagic Escherichia coli O157:H7 EDL933 (EHEC) under eleven different environmental conditions including radish sprouts and cattle feces. BMC Genomics 15, 353. doi: 10.1186/1471-2164-15-353.
MacLennan, C.A., and Saul, A. (2014). Vaccines against poverty. Proc Natl Acad Sci USA 111(34), 12307-12312. doi: 10.1073/pnas.1400473111.
Maione, D., Margarit, I., Rinaudo, C.D., Masignani, V., Mora, M., Scarselli, M., et al. (2005). Identification of a universal Group B streptococcus vaccine by multiple genome screen. Science 309(5731), 148-150. doi: 10.1126/science.1109869.
Mohawk, K.L., and O'Brien, A.D. (2011). Mouse models of Escherichia coli O157:H7 infection and shiga toxin injection. J Biomed Biotechnol 2011, 258185. doi: 10.1155/2011/258185.
Mora, M., Donati, C., Medini, D., Covacci, A., and Rappuoli, R. (2006). Microbial genomes and vaccine design: refinements to the classical reverse vaccinology approach. Curr Opin Microbiol 9(5), 532-536. doi: 10.1016/j.mib.2006.07.003.
Mora, M., Veggi, D., Santini, L., Pizza, M., and Rappuoli, R. (2003). Reverse vaccinology. Drug Discov Today 8(10), 459-464.
Moriel, D.G., Bertoldi, I., Spagnuolo, A., Marchi, S., Rosini, R., Nesta, B., et al. (2010). Identification of protective and broadly conserved vaccine antigens from the genome of extraintestinal pathogenic Escherichia coli. Proc Natl Acad Sci U S A 107(20), 9072-9077. doi: 10.1073/pnas.0915077107.
Moriel, D.G., Rosini, R., Seib, K.L., Serino, L., Pizza, M., and Rappuoli, R. (2012). Escherichia coli: great diversity around a common core. MBio 3(3). doi: 10.1128/mBio.00118-12.
Nesta, B., Valeri, M., Spagnuolo, A., Rosini, R., Mora, M., Donato, P., et al. (2014). SslE elicits functional antibodies that impair in vitro mucinase activity and in vivo colonization by both intestinal and extraintestinal Escherichia coli strains. PLoS Pathog 10(5), e1004124. doi: 10.1371/journal.ppat.1004124.
Nielsen, H. (2017). Predicting Secretory Proteins with SignalP. Methods Mol Biol 1611, 59-73. doi: 10.1007/978-1-4939-7015-5_6.
Nieves, W., Petersen, H., Judy, B.M., Blumentritt, C.A., Russell-Lodrigue, K., Roy, C.J., et al. (2014). A Burkholderia pseudomallei outer membrane vesicle vaccine provides protection against lethal sepsis. Clin Vaccine Immunol 21(5), 747-754. doi: 10.1128/CVI.00119-14.
Perez-Cruz, C., Canas, M.A., Gimenez, R., Badia, J., Mercade, E., Baldoma, L., et al. (2016). Membrane Vesicles Released by a hypervesiculating Escherichia coli Nissle 1917 tolR Mutant Are Highly Heterogeneous and Show Reduced Capacity for Epithelial Cell Interaction and Entry. PLoS One 11(12), e0169186. doi: 10.1371/journal.pone.0169186.
Perez-Cruz, C., Carrion, O., Delgado, L., Martinez, G., Lopez-Iglesias, C., and Mercade, E. (2013). New type of outer membrane vesicle produced by the Gram-negative bacterium Shewanella vesiculosa M7T: implications for DNA content. Appl Environ Microbiol 79(6), 1874-1881. doi: 10.1128/AEM.03657-12.
Petersen, H., Nieves, W., Russell-Lodrigue, K., Roy, C.J., and Morici, L.A. (2014). Evaluation of a Burkholderia pseudomallei Outer Membrane Vesicle Vaccine in Nonhuman Primates. Procedia Vaccinol 8, 38-42. doi: 10.1016/j.provac.2014.07.007.
Petrone, B.L., Stringer, A.M., and Wade, J.T. (2014). Identification of HilD-regulated genes in Salmonella enterica serovar Typhimurium. J Bacteriol 196(5), 1094-1101. doi: 10.1128/JB.01449-13.
Pizza, M., Scarlato, V., Masignani, V., Giuliani, M.M., Arico, B., Comanducci, M., et al. (2000). Identification of vaccine candidates against serogroup B meningococcus by whole-genome sequencing. Science 287(5459), 1816-1820.
Rappuoli, R. (2000). Reverse vaccinology. Curr Opin Microbiol 3(5), 445-450.
Rappuoli, R., Pizza, M., Del Giudice, G., and De Gregorio, E. (2014). Vaccines, new opportunities for a new society. Proc Natl Acad Sci U S A 111(34), 12288-12293. doi: 10.1073/pnas.1402981111.
Rivas, M., Chinen, I., and Guth, B.E.C. (2016). "Enterohemorrhagic (Shiga Toxin-Producing) Escherichia coli," in Escherichia coli in the Americas, ed. A.G. Torres. (Cham: Springer International Publishing), 97-123.
Rossi, O., Caboni, M., Negrea, A., Necchi, F., Alfini, R., Micoli, F., et al. (2016). Toll-Like Receptor Activation by Generalized Modules for Membrane Antigens from Lipid A Mutants of Salmonella enterica Serovars Typhimurium and Enteritidis. Clin Vaccine Immunol 23(4), 304-314. doi: 10.1128/CVI.00023-16.
Rossi, O., Maggiore, L., Necchi, F., Koeberling, O., MacLennan, C.A., Saul, A., et al. (2015). Comparison of Colorimetric Assays with Quantitative Amino Acid Analysis for Protein Quantification of Generalized Modules for Membrane Antigens (GMMA). Molecular Biotechnology 57(1), 84-93. doi: 10.1007/s12033-014-9804-7.
Roy, A., Kucukural, A., and Zhang, Y. (2010). I-TASSER: a unified platform for automated protein structure and function prediction. Nat Protoc 5(4), 725-738. doi: 10.1038/nprot.2010.5.
Rutten, L., Mannie, J.P., Stead, C.M., Raetz, C.R., Reynolds, C.M., Bonvin, A.M., et al. (2009). Active-site architecture and catalytic mechanism of the lipid A deacylase LpxR of Salmonella typhimurium. Proc Natl Acad Sci USA 106(6), 1960-1964. doi: 10.1073/pnas.0813064106.
Tapia, D., Ross, B.N., Kalita, A., Kalita, M., Hatcher, C.L., Muruato, L.A., et al. (2016). From In silico Protein Epitope Density Prediction to Testing Escherichia coli O157:H7 Vaccine Candidates in a Murine Model of Colonization. Front Cell Infect Microbiol 6, 94. doi: 10.3389/fcimb.2016.00094.
Tarr, P.I., Gordon, C.A., and Chandler, W.L. (2005). Shiga-toxin-producing Escherichia coli and haemolytic uraemic syndrome. Lancet 365(9464), 1073-1086. doi: 10.1016/S0140-6736(05)71144-2.
Yu, N.Y., Wagner, J.R., Laird, M.R., Melli, G., Rey, S., Lo, R., et al. (2010). PSORTb 3.0: improved protein subcellular localization prediction with refined localization subcategories and predictive capabilities for all prokaryotes. Bioinformatics 26(13), 1608-1615. doi: 10.1093/bioinformatics/btq249.

### Numbered embodiments

1. An isolated polypeptide comprising or consisting of:
   (a) an amino acid sequence selected from the group consisting of SEQ ID NOs: 25, 26, 27, 28, 29, 30, 31, 32, 33, 34, 35, 36, 37, 38, 39, 40, 41, 42, 43, 44, 45, 46, 47 or 48; or
   (b) a variant and/or fragment of (a), for example:
      (i) a variant of (a);
      (ii) a fragment of (a);
      (iii) a variant of a fragment of (a).
2. The isolated polypeptide according to embodiment 1, wherein (a) is selected from the group consisting of SEQ ID NOs: 25, 26 and 27.
3. The isolated polypeptide according to any preceding embodiment, wherein (a) is SEQ ID NO: 25.
4. The isolated polypeptide according to any preceding embodiment, wherein (b) exhibits at least 60% sequence identity to an amino acid sequence listed in (a), for example, at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 99.5% sequence identity to an amino acid sequence listed in (a);
   (i) wherein the at least 60% sequence identity is exhibited over at least 60% of the amino acid sequence listed in (a), for example, a contiguous amino acid sequence spanning at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of the amino acid sequence listed in (a); or
   (ii) wherein the at least 60% sequence identity is exhibited over at least 10 contiguous amino acids of the amino acid sequence listed in (a), for example, at least 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 350, 400, 450, 500, 550, 600, 650, 700, 750, 800, 850, 900, 950, 1000, 1500, 2000, 2500, 2600, 2601, 2602, 2603, 2604, 2605, 2606, 2607, 2608, 2609, 2610, 2611, 2612, 2613, 2614, 2615, 2616, 2617, 2618 or 2619 contiguous amino acids of the amino acid sequence listed in (a).
5. The isolated polypeptide according to any preceding embodiment, wherein the polypeptide comprises or consists of a fragment comprising at least 10 contiguous amino acids, for example, at least 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233 contiguous amino acids, and/or, where present, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 350, 400, 450, 500, 550, 600, 650, 700, 750, 800, 850, 900, 950, 1000, 1500, 2000, 2500 or 2600 contiguous amino acids.
6. The isolated polypeptide according to any preceding embodiment, wherein the polypeptide comprises or consists of a fragment wherein 1, or at least, 1 amino acid, is truncated from the N-terminus with respect to an amino acid sequence listed in (a), for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300 amino acids are truncated from the N-terminus with respect to the amino acid sequence listed in (a).
7. The isolated polypeptide according to any preceding embodiment, wherein the polypeptide comprises or consists of a fragment wherein 1, or at least 1 amino acid, is truncated from the C-terminus with respect to the reference sequence, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300 amino acids are truncated from the C-terminus with respect to the reference sequence.
8. The isolated polypeptide according to any preceding embodiment, wherein the polypeptide is fused to one or more additional polypeptide, for example:
   (a) N-terminal fusion;
   (b) C-terminal fusion; or
   (c) N-terminal and C-terminal fusion.
9. The isolated polypeptide according to any preceding embodiment, wherein the polypeptide is conjugated to one or more additional moiety, for example:
   (a) one or more lipid (to form a lipoprotein);
   (b) one or more saccharide or polysaccharide (to form a glycoprotein);
   (c) one or more phosphate group (to form a phosphoprotein);
   (d) one or more heme group (to form a hemoprotein);
   (e) one or more the flavin adenine dinucleotide (FAD) or flavin mononucleotide (FMN) (to form a flavoprotein); and
   (f) one or more metal ion cofactor (to form a metalloprotein).
10. The isolated polypeptide according to any preceding embodiment, wherein the polypeptide is substantially purified.
11. The isolated polypeptide according to any preceding embodiment, wherein the polypeptide is not naturally occurring.
12. The isolated polypeptide according to any preceding embodiment, wherein the polypeptide is recombinant.
13. The isolated polypeptide according to embodiment 1 wherein the polypeptide is a fragment, variant, fusion and/or derivative capable of inducing a specific immune response to an amino acid sequence listed in (a).
14. The isolated polypeptide according to embodiment 1 wherein the polypeptide is a fragment, variant, fusion and/or derivative capable of inducing an immune response to an amino acid sequence listed in (a).
15. The isolated polypeptide according to embodiment 2, wherein the immune response is an immune activating response.
16. The isolated polypeptide according to embodiment 2, wherein the immune response is a protective immune response.
17. The isolated polypeptide according to embodiment 3, wherein the polypeptide is capable of eliciting an *in vitro* protective immune response.
18. The isolated polypeptide according to embodiment 3, wherein the polypeptide is capable of eliciting an *in vivo* protective immune response.
19. The isolated polypeptide according to embodiment 5, wherein the polypeptide is capable of eliciting an *in vivo* protective immune response in a mammal.
20. The isolated polypeptide according to embodiment 6, wherein the mammal is selected from the group consisting of armadillo *(dasypus novemcinctus),* baboon (*papio anubis; papio cynocephalus);* camel *(camelus bactrianus, camelus dromedarius, camelus ferus)* cat *(felis catus),* dog *(canis lupus familiaris),* horse (*equus ferus caballus*)*,* ferret (*mustela putorius furo),* goat (*capra aegagrus hircus),* guinea pig (*cavia porcellus*)*,* golden hamster *(mesocricetus auratus*)*,* kangeroo *(macropus rufus),* llama (*lama glama*)*,* mouse *(mus musculus),* pig *(sus scrofa domesticus),* rabbit (*oryctolagus cuniculus),* rat *(rattus norvegicus),* rhesus macaque (*macaca mulatto*)*,* sheep *(ovis aries*)*,* non-human primate, and human *(Homo sapiens).*
21. The isolated polypeptide according to embodiment 18, wherein the protective immune response is protective against a disease or condition caused, wholly or in part, by an organism selected from the group consisting of: bacteria, Gram negative bacteria; *proteobacteria, enterobacteriales, enterobacteriaceae* (for example, *Salmonella, Escherichia* [*E. albertii, E. coli, E. fergusonii, E. hermannii, E. marmotae,* and *E. vulneris*]*, Yersinia, Klebsiella, Proteus, Enterobacter, Serratia, and Citrobacter).*
22. The isolated polypeptide according to embodiment 21, wherein disease or condition caused, wholly or in part, by *Escherichia coli,* for example, extraintestinal pathogenic *E. coli* (ExPEC), or intestinal pathogenic *E. coli* (InPEC).
23. The isolated polypeptide according to embodiment 22, wherein the *Escherichia coli* is from a pathotype selected from the group consisting of: (i) enteropathogenic *E. coli* (EPEC); (ii) enterohemorrhagic *E. coli* (EHEC); (iii) enterotoxigenic *E. coli* (ETEC); (iv) enteroaggregative *E. coli* (EAEC); (v) diffusely adherent *E. coli* (DAEC); (vi) enteroinvasive *E. coli* (EIEC); (vii) uropathogenic *E. coli* (UPEC); (viii) neonatal meningitis *E. coli* (NMEC); (ix) Shiga Toxin (Stx) producing enteroaggregative *E. coli* (STEAEC); (x) adherent Invasive *E. coli* (AIEC); (xi) amoxicillin-resistant *E. coli* (AREC); (xii) asymptomatic bacteriuria *E. coli* (ABU); (xiii) Avian pathogenic *E. coli* (APEC).
24. The isolated polypeptide according to embodiment 22 or 23, wherein the *Escherichia coli* is an enterohaemorrhagic *E. coli* (EHEC) selected from the group consisting of: O157:H7 e.g., EHEC O157:H7 EDL933 strain; EHEC O157:H7 Sakai stain; EHEC O26:H11 (e.g., strain 11368); EHEC O103:H2 (e.g., strain 12009); and EHEC O111:H- (e.g., strain 11128).
25. The isolated polypeptide according to embodiment 22 or 23, wherein the *Escherichia coli* is an enteropathogenic *E. coli* (EPEC) selected from the group consisting of: O55:H7 (e.g., CB9615); and O127:H6 (e.g., strain E2348/69).
26. The isolated polypeptide according to embodiment 22 or 23, wherein the *Escherichia coli* is an enterotoxigenic *E. coli* (ETEC) selected from the group consisting of: H10407; E24377A; and Porcine ETEC.
27. The isolated polypeptide according to embodiment 22 or 23, wherein the *Escherichia coli* is an adherent Invasive *E. coli* (AIEC) selected from the group consisting of: LF82; O83:H1 NR G857C; and UM146.
28. The isolated polypeptide according to embodiment 22 or 23, wherein the *Escherichia coli* is an enteroaggregative *E. coli* (EAEC) selected from the group consisting of: 042, and 55989.
29. The isolated polypeptide according to embodiment 22 or 23, wherein the *Escherichia coli* is a neonatal meningitis *E. coli* (NMEC) selected from the group consisting of: O7:K1 CE10, S88, and 1H E3034.
30. The isolated polypeptide according to embodiment 22 or 23, wherein the *Escherichia coli* is an uropathogenic *E. coli* (UPEC) selected from the group consisting of: UMN026, CLONEDi14; CLONE Di2; CFT073; IA139; 536; NA114; and UTI89.
31. The isolated polypeptide according to embodiment 22 or 23, wherein the *Escherichia coli* is AREC SMS-3-5; APEC 01; or ABU 83972.
32. The isolated polypeptide according to embodiment 22, wherein the *Escherichia coli* is a strain with a K antigen selected from the group consisting of K1, K2a/ac, K3, K4, K5, K6, K7 (=K56), K8, K9 (=0104), K10, K11, K12 (K82), K13(=K20 and =K23), K14, K15, K16, K18a, K18ab (=K22), K19,K24, K26, K27, K28, K29, K30, K31, K34, K37, K39, K40, K41,K42, K43, K44, K45, K46, K47, K49 (O46), K50, K51, K52, K53, K54 (=K96), K55, K74, K84, K85ab/ac (=O141), K87 (=O32), K92, K93, K95, K97, K98, K100, K101, K102, K103, KX104, KX105, and KX106).
33. The isolated polypeptide according to embodiment 22 or 32, wherein the *Escherichia coli* is a strain with an O antigen selected from the group consisting of O1 A, O1 A1, O1 B, O1 C, O2, O3, O4, O4, O5 ab, O5 ac, O6, O6, O6, O7, O8, O9, O9 a, O10, O11, 012, 013, 015, 016, 016, 016, 017, 018 A, 018 A1, 018 A1A2, 018 ab, 018 ac, 018 B, 018 B1, 019 ab, O20 ab, O20 ac, 021, O22, O23 A, O24, O25, O26, O27, O28 ab, O28 ac, O29, O30, O32, O33, O34, O35, O36, O37, O38, O39, O40, O41, O42, O43, O44, O45, O45 rel, O46, O48, O49, O50, 051, O52, O53, O54, O55, O55, O56, O58, O59, O60, 061, O62, O63, O64, O65, O66, O68, O69, O70, 071, O73, O73 ab, O74, O75, O76, O77, O78, O79, O80, O81, O82, O83, O84, O85, O86, O86, O86, O87, O88, O89, O90, 091, O92, O95, O96, O97, O98, O99, O100, O101, 0102, O103, O104, O105, O106, O107, O108, O109, O110, O111, 0112 ab, 0112 ac, 0113, 0114, 0115, 0116, 0117, 0118, 0119, 0120, 0121, 0123, 0124, 0125 ab, 0125 ac, 0126, 0126, 0127, 0128 ab, 0128 ab, 0128 ac, 0129, 0130, 0131, 0132, 0133, 0134, 0135, 0136, 0137, 0138, 0139, 0140, 0141, 0142, 0143, 0144, 0145, 0146, 0147, 0148, 0149, 0150, 0151, 0152, 0153, 0154, 0155, 0156, 0157, 0158 ab, 0158 ac, 0159, 0160, 0161, 0163, 0164, 0165, 0166, 0167, 0168, 0169, 0170, 0171, 0172, 0173, 0174 ab, 0174 ac, 0175, 0176, 0177, 0178, 0179, 0180, 0181, 0182, 0183, 0184, 0185, 0186 and 0187.
34. The isolated polypeptide according to embodiment 22, 32 or 33, wherein the *Escherichia coli* is a strain with a H antigen selected from the group consisting of: H1, H2, H3, H4, H5, H6, H7, H8, H9, H10 (=H50), H11, H12, H13, H14, H15, H16, H17, H18, H19, H20, H21, H22, H23, H24, H25, H26, H27, H28, H29, H30, H31, H32, H33, H34, H35, H36, H37, H38, H39, H40, H41, H42, H43, H44, H45, H46, H47, H48, H49, H50 (=H10), H51, H52, H53, H54, H55 and H56.
35. The isolated polypeptide according to any one of embodiments 16-20, wherein the protective immune response is protective against a disease or condition selected from the group consisting of: gastroenteritis; haemolytic uremic syndrome (HUS), urinary tract infection; neonatal meningitis; haemorrhagic colitis; and Crohn's disease.
36. The isolated polypeptide according to any preceding embodiment, wherein the protective immune response is an immune response that results in increased seral cytokine levels; for example, cytokines selected from the group consisting of IL-1α, IL-1β, IL-1RA, IL-18, IL-2, IL-4, IL-7, IL-9, IL-13, IL-15, IL-3, IL-5, GM-CSF, IL-6, IL-11, G-CSF, IL-12, LIF, OSM, IL-10, IL-20, IL-14, IL-16, IL-17, IFN-α, IFN-β, IFN-γ, CD154, LT-β, TNF-α, TNF-β, 4-1BBL, APRIL, CD70, CD153, CD178, GITRL, LIGHT, OX40L, TALL-1, TRAIL, TWEAK, TRANCE, TGF-β1, TGF-β2, TGF-β3, Epo, Tpo, Flt-3L, SCF, M-CSF and MSP.
37. The isolated polypeptide according to any preceding embodiment, wherein the protective immune response is an immune response is an immune response that results in bactericidal activity and/or opsonophagocytosis.
38. An isolated nucleic acid molecule comprising or consisting of:
   (A) a nucleic acid sequence selected from the group consisting of selected from the group consisting of SEQ ID NO: 1, 2, 3, 4, 5, 6, 7, 8, 9, 10, 11, 12, 13, 14, 15, 16, 17, 18, 19, 20, 21, 22, 23 or 24; or
   (B) a fragment, variant and/or fusion of (A).
39. The isolated nucleic acid according to embodiment 38, wherein (A) is selected from the group consisting of SEQ ID NOs: 1, 2 and 3.
40. The isolated nucleic acid according to embodiment 38 or 39, wherein (A) is SEQ ID NO: 1.
41. The isolated nucleic acid according to embodiment 38, wherein (B) exhibits at least 60% sequence identity to a nucleic acid sequence listed in (A), for example, at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to the nucleic acid sequence listed in (a); and
   (I) wherein the at least 60% sequence identity is exhibited over at least 60% of the nucleic acid sequence listed in (a), for example, a contiguous amino acid sequence spanning at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of the nucleic acid sequence listed in (A); or
   (II) wherein the at least 60% sequence identity is exhibited over at least 30 contiguous nucleic acids of the nucleic acid sequence listed in (A), for example, at least 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 350, 400, 450, 500, 550, 600, 650, 700, 750, 800, 850, 900, 950, 1000, 1500, 2000, 2500, 3000, 3500, 4000, 4500, 5000, 5500, 6000, 6500, 7000, or 7500 contiguous amino acids of the nucleic acid sequence listed in (A).
42. The isolated nucleic acid according to any one of embodiments 38-41, wherein the nucleic acid encodes a polypeptide according to embodiments 1-37.
43. A vector comprising a nucleic acid molecule defined in any one of embodiments 38-42.
44. A host cell comprising a nucleic acid molecule defined in any one of embodiments 38-42 or a vector as defined in embodiment 43.
45. A method for producing a polypeptide according to any one of embodiments 1-37:
   comprising or consisting of the steps of culturing a population of host cells according to embodiment 44 under conditions in which the peptide is expressed, and isolating the peptide therefrom.
46. A vesicle comprising one or more polypeptide defined in any one of embodiments 1-37.
47. The vesicle according to embodiment 46, wherein the vesicle is derived from the membrane of a cell, for example, a Gram positive bacteria membrane vesicle or a Gram negative bacteria outer membrane vesicle (OMV).
48. The vesicle according to embodiment 46, wherein the vesicle is a Gram negative bacteria outer membrane vesicle (OMV) of a bacterium defined in any one of embodiments 21-34.
49. The vesicle according to any one of embodiments 46-48, wherein the polypeptide is:
   (a) homologous to the cell; or
   (b) heterologous to the cell.
50. The vesicle according to any one of embodiments 45-49, wherein the polypeptide is:
   (a) located in the lumen of the vesicle (for example, where the vesicle is a cell, in the cytoplasm);
   (b) located in the/a inter-membrane space of the vesicle (for example, where the vesicle is a cell, the periplasm); and/or
   (c) located on the outer surface of the outer membrane (for example, where the vesicle is a cell, the outer membrane).
51. The vesicle according to any one of embodiments 46-50, wherein the polypeptide expressed by the cell.
52. The vesicle according to any one of embodiments 46-50, wherein the polypeptide is chemically conjugated to the vesicle (for example, to a lipid, protein or polysaccharide component of the vesicle membrane).
53. A binding moiety capable of specifically binding to one or more polypeptide defined in any one of embodiments 1-37.
54. The binding moiety according to embodiment 53, wherein the binding moiety is selected from the group consisting of: antibodies; antigen-binding fragments; and antibody mimetics.
55. The binding moiety according to embodiment 53 or 54, wherein the binding moiety is an antibody.
56. The binding moiety according to embodiment 55, wherein the antibody is polyclonal or monoclonal.
57. The binding moiety according to embodiment 55, wherein the binding moiety is an antigen-binding fragment selected from the group consisting of: Fab (fragment antigen binding); F(ab')₂; Fab'; scFv (single chain variable fragment); di-scFv; sdAb (single domain antibody / domain antibody); trifunctional antibody; chemically-linked F(ab')₂; and BiTE (bi-specific T-cell engager).
58. The binding moiety according to embodiment 57, wherein the antibody or antigen binding fragment thereof is an antigen binding fragment selected from the group consisting of affibodies molecules; affilins; affimers; affitins; alphabodies; anticalins; avimers; DARPins; fynomers; kunitz domain peptides; monobodies and nanoCLAMPs.
59. A pharmaceutical composition comprising a polypeptide defined in any one of embodiments 1-37, a nucleic acid molecule defined in any one of embodiments 38-42, a vector as defined in embodiment 43, a host cell as defined in embodiment 46, a vesicle as defined in any one of embodiments 46-52 and/or a binding moiety as defined in any one of embodiments 53-58.
60. A kit comprising or consisting of a polypeptide defined in any one of embodiments 1-37, a nucleic acid molecule defined in any one of embodiments 38-42, a vector as defined in embodiment 43, a host cell as defined in embodiment 44, a vesicle as defined in any one of embodiments 46-52, a binding moiety as defined in any one of embodiments 53-58 and/or a pharmaceutical composition as defined in embodiment 59; and (optionally) instructions for use.
61. A polypeptide defined in any one of embodiments 1-37, a nucleic acid molecule defined in any one of embodiments 38-42, a vector as defined in embodiment 43, a host cell as defined in embodiment 44, a vesicle as defined in any one of embodiments 46-52, a binding moiety as defined in any one of embodiments 53-58 and/or a pharmaceutical composition as defined in embodiment 59, for use in medicine.
62. A polypeptide defined in any one of embodiments 1-37, a nucleic acid molecule defined in any one of embodiments 38-42, a vector as defined in embodiment 43, a host cell as defined in embodiment 44, a vesicle as defined in any one of embodiments 46-52, a binding moiety as defined in any one of embodiments 53-58 and/or a pharmaceutical composition as defined in embodiment 59, for use in preventing or treating bacterial infection and/or symptoms thereof.
63. The polypeptide, nucleic acid molecule, vector, vesicle, host cell, binding moiety and/or pharmaceutical composition for use according to embodiment 62, wherein the bacterial infection is, wholly or in part, infection with one or more bacterium defined in any one of embodiments 21-34.
64. The polypeptide, nucleic acid molecule, vector, vesicle, host cell, binding moiety and/or pharmaceutical composition for use according to embodiment 63, wherein the use comprises the consecutive or concurrent use of:
   (a) a polypeptide defined in embodiments 1-37;
   (b) a nucleic acid molecule defined in embodiments 38-42;
   (c) a vector as defined in embodiment 43;
   (d) a vesicle as defined in any one of embodiments 46-52;
   (e) a host cell as defined in embodiment 44;
   (f) a binding moiety as defined in any one of embodiments 53-58; and/or
   (g) a pharmaceutical composition as defined in embodiment 59.
65. The polypeptide, nucleic acid molecule, vector, vesicle, host cell, binding moiety and/or pharmaceutical composition for use according to embodiment 64, wherein the use comprises the consecutive or concurrent use of a binding moiety as defined in any one of embodiments 53-58 with:
   (a) a nucleic acid molecule defined in any one of embodiments 37-42 and/or a vector as defined in embodiment 43; and/or
   (b) a binding moiety as defined in any one of embodiments 53-58 and/or a vesicle as defined in any one of embodiments 46-52.
66. The use of polypeptide defined in any one of embodiments 1-37, a nucleic acid molecule defined in any one of embodiments 38-42, a vector as defined in embodiment 43, a host cell as defined in embodiment 44, a vesicle as defined in any one of embodiments 46-52, a binding moiety as defined in any one of embodiments 53-58 and/or a pharmaceutical composition as defined in embodiment 59, in preventing or treating bacterial infection and/or symptoms thereof, for example, wherein the bacterial infection is, wholly or in part, infection with one or more bacterium defined in any one of embodiments 21-34.
67. The use of polypeptide defined in any one of embodiments 1-37, a nucleic acid molecule defined in any one of embodiments 38-42, a vector as defined in embodiment 43, a host cell as defined in embodiment 44, a vesicle as defined in any one of embodiments 46-52, a binding moiety as defined in any one of embodiments 53-58 and/or a pharmaceutical composition as defined in embodiment 59, in the manufacture of a medicament for preventing or treating bacterial infection and/or symptoms thereof, for example, wherein the bacterial infection is, wholly or in part, infection with one or more bacterium defined in any one of embodiments 21-34.
68. A method for preventing or treating bacterial infection and/or symptoms thereof comprising or consisting of administering to a subject an effective amount of a polypeptide defined in any one of embodiments 1-37, a nucleic acid molecule defined in any one of embodiments 38-42, a vector as defined in embodiment 43, a host cell as defined in embodiment 44, a vesicle as defined in any one of embodiments 46-52, a binding moiety as defined in any one of embodiments 53-58 and/or a pharmaceutical composition as defined in embodiment 59, for example, wherein the bacterial infection is, wholly or in part, infection with one or more bacterium defined in any one of embodiments 21-34.
69. The use of a nucleic acid molecule defined in any one of embodiments 38-42, or a binding moiety as defined in any one of embodiments 53-58, for detecting the presence of bacteria, for example, wherein the bacteria are one or more bacterium defined in any one of embodiments 21-34.
70. The use according to embodiment 69, wherein the detection is *in vitro* and/or *in vivo.*
71. A polypeptide, nucleic acid molecule, vector, vesicle, host cell, binding moiety, pharmaceutical composition, use or method as described in specification and figures herein.

| **Table S1. Antigen candidates selected by Psort analysis** | | | | | |
|---|---|---|---|---|---|
| **Protein ID** | **Functional annotation** | **Length (bp)** | **PSORT Local.¹** | **PSORT score** | **Mod HMM²** |
| AIG68144.1 | Ferric siderophore transport system, periplasmicbinding protein TonB CDS | 720 | U | 2 | 1 |
| AIG70214.1 | Type III secretion outermembrane pore forming protein (YscC,MxiD,HrcC, InvG) CDS | 1704 | OM | 10 | 1 |
| AIG71811.1 | adherence and invasion outermembrane protein (Inv,enhances Peyer's patches colonization) CDS | 5037 | OM | 9,95 | 0 |
| AIG66227.1 | Putative outer membrane protein CDS | 2451 | OM | 10 | 0 |
| AIG66265.1 | Outer membrane protein Imp, required for envelope biogenesis CDS | 2322 | OM | 10 | 0 |
| AIG66267.1 | hypothetical protein CDS | 753 | U | 2 | 0 |
| AIG66308.1 | Secretion monitor precursor CDS | 588 | U | 6,49 | 1 |
| AIG66347.1 | Fimbrial protein Yad like protein CDS | 1110 | U | 2 | 1 |
| AIG66348.1 | Fimbrial protein YadK CDS | 591 | U | 2 | 0 |
| AIG66349.1 | Fimbrial protein YadL CDS | 606 | U | 2,5 | 1 |
| AIG66351.1 | Outer membrane usher protein HtrE CDS | 2601 | OM | 10 | 0 |
| AIG66353.1 | Fimbrial protein YadN CDS | 597 | Ex | 9,65 | 1 |
| AIG66363.1 | Ferric hydroxamate outer membrane receptor FhuA CDS | 2244 | OM | 10 | 0 |
| AIG66390.1 | Outer membrane protein assembly factor YaeT precursor CDS | 2433 | OM | 10 | 1 |
| AIG66406.1 | Copper homeostasis protein CutF precursor CDS | 711 | OM | 9,92 | 0 |
| AIG66407.1 | putative lipoprotein yaeF precursor CDS | 825 | U | 2,5 | 0 |
| AIG66409.1 | hypothetical protein CDS | 708 | U | 2 | 0 |
| AIG66417.1 | hypothetical protein CDS | 801 | U | 2 | 0 |
| AIG66424.1 | putative aminopeptidase CDS | 801 | U | 2,5 | 1 |
| AIG66428.1 | hypothetical protein CDS | 1410 | U | 2 | 1 |
| AIG66446.1 | core protein CDS | 4215 | U | 2 | 0 |
| AIG66447.1 | hypothetical protein CDS | 612 | U | 2 | 1 |
| AIG66450.1 | core protein CDS | 1761 | Ex | 9,52 | 0 |
| AIG66460.1 | putative exported protein CDS | 741 | U | 2,5 | 1 |
| AIG66464.1 | putative lipoprotein yafL precursor CDS | 774 | U | 4,9 | 1 |
| AIG66480.1 | Outer membrane pore protein E precursor CDS | 1056 | OM | 10 | 1 |
| AIG66490.1 | putative tail fiber protein CDS | 795 | U | 2 | 0 |
| AIG66511.1 | Zinc binding domain protein CDS | 2334 | U | 2 | 0 |
| AIG66518.1 | hypothetical protein CDS | 1137 | U | 2,5 | 1 |
| AIG66526.1 | CFA/I fimbrial chaperone CDS | 711 | U | 2,5 | 1 |
| AIG66527.1 | CFA/I fimbrial minor adhesin CDS | 1644 | Ex | 9,64 | 1 |
| AIG66528.1 | CFA/I fimbrial subunit C usher protein CDS | 2526 | U | 4,69 | 2 |
| AIG66528.1 | Putative adhesin CDS | 4254 | OM | 10 | 0 |
| AIG66545.1 | AidA-I adhesin-like protein CDS | 4050 | U | 5,87 | 0 |
| AIG66568.1 | hypothetical protein CDS | 864 | U | 2 | 0 |
| AIG66574.1 | Putative flagellin structural protein CDS | 2943 | OM | 10 | 0 |
| AIG66636.1 | hypothetical protein CDS | 624 | U | 2 | 0 |
| AIG66640.1 | putative lipoprotein CDS | 1095 | U | 2,5 | 0 |
| AIG66656.1 | Protein YkiA CDS | 2166 | U | 2 | 0 |
| AIG66683.1 | Nucleoside-specific channel-forming protein Tsx precursor CDS | 885 | OM | 10 | 0 |
| AIG66684.1 | putative lipoprotein yajI CDS | 540 | U | 2 | 0 |
| AIG66769.1 | Large repetitive protein CDS | 4386 | U | 6,04 | 1 |
| AIG66781.1 | Putative stomatin/prohibitin-family membrane protease subunit YbbK | 918 | U | 2 | U |
| AIG66819.1 | Oligopeptide ABC transporter, periplasmic oligopeptide-binding protein OppA CDS | 1701 | U | 2 | 0 |
| AIG66819.1 | Outer membrane usher protein SfmD CDS | 2610 | OM | 10 | 2 |
| AIG66854.1 | Agglutination protein CDS | 1356 | OM | 10 | 1 |
| AIG66854.1 | TonB-dependent receptor CDS | 2241 | OM | 10 | 0 |
| AIG66909.1 | Rare lipoprotein A precursor CDS | 1089 | Ex | 9,65 | 0 |
| AIG66920.1 | hypothetical protein CDS | 588 | Ex | 9,71 | 0 |
| AIG66922.1 | hypothetical protein CDS | 708 | U | 2 | 0 |
| AIG66972.1 | core protein CDS | 4200 | U | 2 | 0 |
| AIG66973.1 | orf, hypothetical protein CDS | 543 | U | 2 | 0 |
| AIG66984.1 | hypothetical protein CDS | 1062 | U | 2,5 | 0 |
| AIG66987.1 | putative fimbrial-like protein ygiL precursor CDS | 567 | Ex | 9,72 | 0 |
| AIG67020.1 | TolA protein CDS | 1185 | U | 2 | 2 |
| AIG67060.1 | Putative outer membrane protein CDS | 960 | U | 2,5 | 1 |
| AIG67099.1 | Biotin synthesis protein BioC CDS | 756 | U | 2 | 0 |
| AIG67129.1 | Ferrichrome-iron receptor CDS | 2283 | OM | 10 | 0 |
| AIG67212.1 | Virulence factor VirK CDS | 993 | U | 2 | 0 |
| AIG67245.1 | Urease accessory protein UreF CDS | 675 | U | 2 | 0 |
| AIG67247.1 | Per-activated serine protease autotransporter enterotoxin EspC | 126 | Ex | 9,71 | U |
| AIG67280.1 | Colicin I receptor precursor CDS | 2010 | OM | 10 | 0 |
| AIG67308.1 | putative hemolysin activator protein CDS | 1065 | OM | 8,86 | 1 |
| AIG67367.1 | Putative metalloprotease yggG | 789 | U | 2 | U |
| AIG67384.1 | exported protein CDS | 549 | U | 2,5 | 1 |
| AIG67387.1 | Outer membrane protein F precursor CDS | 1089 | OM | 10 | 1 |
| AIG67397.1 | type 1 fimbriae major subunit FimA CDS | 540 | Ex | 10 | 0 |
| AIG67400.1 | type 1 fimbriae anchoring protein FimD CDS | 1893 | OM | 10 | 0 |
| AIG67407.1 | hypothetical protein CDS | 543 | U | 2 | 0 |
| AIG67412.1 | Paraquat-inducible protein B CDS | 1641 | U | 2 | 1 |
| AIG67413.1 | Paraquat-inducible protein B CDS | 564 | U | 2 | 0 |
| AIG67419.1 | Outer membrane protein A precursor CDS | 1065 | OM | 10 | 0 |
| AIG67426.1 | UPF0319 protein YccT precursor CDS | 663 | U | 2,5 | 1 |
| AIG67448.1 | hypothetical protein CDS | 558 | U | 2 | 0 |
| AIG67453.1 | hypothetical protein CDS | 774 | U | 2 | 0 |
| AIG67464.1 | Hypothetical protein CDS | 1851 | U | 2 | 0 |
| AIG67503.1 | hypothetical protein CDS | 753 | Ex | 9,71 | 0 |
| AIG67516.1 | Putative polysaccharide export protein YccZ precursor CDS | 1140 | OM | 9,92 | 1 |
| AIG67517.1 | Putative outer membrane lipoprotein YmcA CDS | 2097 | OM | 9,52 | 0 |
| AIG67577.1 | Shiga-like toxin II subunit A precursor CDS | 960 | U | 2,5 | 1 |
| AIG67607.1 | hypothetical protein CDS | 618 | U | 2 | 0 |
| AIG67611.1 | hypothetical protein CDS | 657 | U | 2 | 0 |
| AIG67633.1 | hypothetical protein CDS | 1344 | Ex | 9,64 | 0 |
| AIG67644.1 | Biofilm PGA synthesis deacetylase PgaB CDS | 2019 | U | 2 | 1 |
| AIG67645.1 | Biofilm PGA outer membrane secretin PgaA CDS | 2424 | U | 2,5 | 0 |
| AIG67652.1 | Putative exported protein precursor CDS | 1086 | U | 2 | 0 |
| AIG67653.1 | outer membrane fimbrial usher protein CDS | 2523 | OM | 10 | 0 |
| AIG67658.1 | Hemolysin CDS | 3813 | OM | 9,95 | 0 |
| AIG67671.1 | hypothetical protein CDS | 789 | U | 2 | 0 |
| AIG67672.1 | hypothetical protein CDS | 1389 | U | 2 | 0 |
| AIG67699.1 | Per-activated serine protease autotransporter enterotoxin EspC | 126 | Ex | 9,71 | U |
| AIG67732.1 | TonB-dependent receptor CDS | 2010 | OM | 10 | 0 |
| AIG67760.1 | putative hemolysin activator protein CDS | 1065 | OM | 8,86 | 1 |
| AIG67820.1 | Protein ycel precursor CDS | 576 | U | 2,5 | 1 |
| AIG67827.1 | Putative lipoprotein yceB precursor CDS | 561 | U | 2 | 0 |
| AIG67831.1 | Protein of unknown function YceH CDS | 648 | U | 2 | 0 |
| AIG67832.1 | Virulence factor MviM CDS | 924 | U | 2 | 0 |
| AIG67840.1 | Flagellar basal-body rod modification protein FlgD CDS | 696 | Ex | 10 | 0 |
| AIG67841.1 | Flagellar hook protein FlgE CDS | 1206 | Ex | 10 | 0 |
| AIG67843.1 | Flagellar basal-body rod protein FlgG CDS | 783 | Ex | 10 | 0 |
| AIG67844.1 | Flagellar L-ring protein FlgH CDS | 699 | OM | 10 | 1 |
| AIG67847.1 | Flagellar hook-associated protein FlgK CDS | 1644 | Ex | 9,96 | 0 |
| AIG67848.1 | Flagellar hook-associated protein FlgL CDS | 954 | Ex | 10 | 0 |
| AIG67862.1 | YceG like protein CDS | 1023 | U | 6,49 | 1 |
| AIG67867.1 | Putative OMR family iron-siderophore receptor precursor CDS | 2190 | OM | 10 | 0 |
| AIG67871.1 | Lipoprotein YcfM, part of a salvage pathway of unknown substrate CDS | 642 | U | 2 | 0 |
| AIG67876.1 | Putative exported protein CDS | 540 | U | 2,5 | 1 |
| AIG67905.1 | hypothetical protein CDS | 588 | U | 2 | 0 |
| AIG67914.1 | Hypothetical protein CDS | 1938 | U | 2 | 0 |
| AIG67929.1 | hypothetical protein CDS | 1938 | U | 2 | 0 |
| AIG67963.1 | hypothetical protein CDS | 744 | U | 2 | 0 |
| AIG68016.1 | hypothetical protein CDS | 1602 | U | 2 | 0 |
| AIG68033.1 | Attachment invasion locus protein precursor CDS | 600 | OM | 10 | 1 |
| AIG68048.1 | Protease VII (Omptin) precursor CDS | 954 | OM | 10 | 0 |
| AIG68053.1 | Pertactin precursor CDS | 705 | U | 7 | 0 |
| AIG68064.1 | Hemolysin E, chromosomal CDS | 1056 | Ex | 10 | 0 |
| AIG68081.1 | Putative TonB dependent outer membrane receptor CDS | 1971 | OM | 10 | 0 |
| AIG68093.1 | Putative adhesion and penetration protein CDS | 1644 | Ex | 9,65 | 0 |
| AIG68114.1 | Invasin CDS | 1431 | OM | 10 | 0 |
| AIG68125.1 | UPF0028 protein YchK CDS | 945 | Ex | 8,89 | 0 |
| AIG68148.1 | Outer membrane protein W precursor CDS | 639 | OM | 10 | 0 |
| AIG68163.1 | hypothetical protein CDS | 582 | U | 2 | 0 |
| AIG68165.1 | Putative intestinal colonization factor encoded by prophage CP-9330 CDS | 747 | U | 2 | 0 |
| AIG68173.1 | Hypothetical protein CDS | 1854 | U | 2 | 0 |
| AIG68183.1 | hypothetical protein CDS | 1938 | U | 2 | 0 |
| AIG68190.1 | putative major tail subunit CDS | 786 | Ex | 9,64 | 0 |
| AIG68238.1 | Hypothetical protein CDS | 1851 | U | 2 | 0 |
| AIG68271.1 | Attachment invasion locus protein precursor CDS | 600 | OM | 10 | 1 |
| AIG68309.1 | Autoinducer 2 (AI-2) aldolase LsrF CDS | 876 | U | 2 | 0 |
| AIG68316.1 | hypothetical protein CDS | 3894 | U | 5,87 | 0 |
| AIG68317.1 | hypothetical protein CDS | 1401 | OM | 9,83 | 0 |
| AIG68320.1 | type 1 fimbriae major subunit FimA CDS | 564 | Ex | 10 | 1 |
| AIG68322.1 | type 1 fimbriae anchoring protein FimD CDS | 2223 | OM | 10 | 0 |
| AIG68325.1 | mannose-specific adhesin FimH CDS | 915 | U | 2 | 1 |
| AIG68333.1 | hypothetical protein CDS | 2373 | OM | 9,49 | 0 |
| AIG68337.1 | redicted glycoside hydrolase CDS | 1320 | U | 2 | 0 |
| AIG68357.1 | Outer membrane porin protein NmpC precursor CDS | 1101 | OM | 10 | 1 |
| AIG68359.1 | hypothetical protein CDS | 669 | U | 2 | 0 |
| AIG68376.1 | internalin, putative CDS | 1260 | U | 2 | 0 |
| AIG68383.1 | hypothetical protein CDS | 1062 | U | 2,5 | 0 |
| AIG68385.1 | core protein CDS | 4203 | U | 5,48 | 0 |
| AIG68385.1 | putative tonB-dependent receptor yncD precursor CDS | 2019 | OM | 10 | 0 |
| AIG68406.1 | putative membrane lipoprotein clustered with tellurite resistance proteins TehA/TehB CDS | 669 | U | 2 | 0 |
| AIG68433.1 | putative BigA-like protein CDS | 2460 | U | 6,26 | 0 |
| AIG68444.1 | Outer membrane protein N precursor CDS | 753 | OM | 10 | 0 |
| AIG68484.1 | porin, autotransporter (AT) family CDS | 3036 | Ex | 9,65 | 2 |
| AIG68484.1 | Hypothetical protein CDS | 1854 | U | 2 | 0 |
| AIG68547.1 | Outer membrane protein G precursor CDS | 906 | OM | 10 | 0 |
| AIG68579.1 | RND efflux system, outer membrane lipoprotein CmeC CDS | 1374 | OM | 9,98 | 0 |
| AIG68638.1 | Attachment invasion locus protein precursor CDS | 600 | OM | 10 | 1 |
| AIG68679.1 | Hypothetical protein CDS | 1851 | U | 2 | 0 |
| AIG68688.1 | hypothetical protein CDS | 585 | U | 2 | 0 |
| AIG68723.1 | Putative protease ydgD | 822 | U | 2,5 | U |
| AIG68730.1 | Protein ydgH precursor CDS | 945 | U | 2,5 | 0 |
| AIG68784.1 | Putative lipoprotein CDS | 816 | U | 6,49 | 0 |
| AIG68794.1 | putative enzyme CDS | 1257 | U | 7 | 0 |
| AIG68797.1 | hypothetical protein CDS | 813 | U | 2 | 0 |
| AIG68812.1 | Iron-sulfur cluster assembly protein SufB CDS | 1488 | U | 2 | 0 |
| AIG68838.1 | hypothetical protein CDS | 714 | U | 2 | 0 |
| AIG68851.1 | hypothetical protein CDS | 1899 | U | 2 | 0 |
| AIG68853.1 | Putative outer membrane protein CDS | 759 | U | 2,5 | 0 |
| AIG68861.1 | hypothetical protein CDS | 768 | U | 2 | 0 |
| AIG68883.1 | Hypothetical protein YdjY CDS | 678 | U | 2,5 | 0 |
| AIG68886.1 | ABC transporter, periplasmic substrate-binding protein YnjB CDS | 1167 | U | 2,5 | 0 |
| AIG68897.1 | Protein ydjA CDS | 552 | U | 2 | 1 |
| AIG68899.1 | putative lipoprotein CDS | 711 | U | 2 | 1 |
| AIG68918.1 | MltA-interacting protein MipA CDS | 747 | OM | 10 | 0 |
| AIG68945.1 | Starvation lipoprotein Slp-like protein CDS | 582 | OM | 9,92 | 1 |
| AIG68946.1 | Inactive metal-dependent proteases like protein,putative molecular chaperone | 696 | U | 2 | U |
| AIG68978.1 | Paraquat-inducible protein B CDS | 2640 | U | 2 | 2 |
| AIG68989.1 | Protease II | 2061 | U | 5,41 | U |
| AIG69001.1 | Cell wall endopeptidase, family M23/M37 CDS | 1323 | U | 2 | 1 |
| AIG69007.1 | hypothetical protein CDS | 603 | U | 2 | 0 |
| AIG69057.1 | putative membrane protein CDS | 651 | U | 2 | 1 |
| AIG69095.1 | Flagellar biosynthesis protein FliZ CDS | 552 | U | 2 | 0 |
| AIG69098.1 | Flagellar biosynthesis protein FliC CDS | 1758 | Ex | 9,96 | 0 |
| AIG69099.1 | Flagellar hook-associated protein FliD CDS | 1398 | Ex | 10 | 0 |
| AIG69108.1 | invasion plasmid antigen CDS | 1140 | Ex | 8,89 | 0 |
| AIG69110.1 | invasion plasmid antigen CDS | 1140 | Ex | 8,89 | 0 |
| AIG69118.1 | Flagellar hook-length control protein FliK CDS | 1128 | Ex | 10 | 0 |
| AIG69140.1 | Outer membrane protein N precursor CDS | 645 | OM | 10 | 1 |
| AIG69141.1 | Outer membrane protein N precursor CDS | 576 | OM | 10 | 0 |
| AIG69149.1 | putative zinc-binding lipoprotein ZinT CDS | 651 | U | 2,5 | 0 |
| AIG69153.1 | hypothetical protein CDS | 711 | U | 2 | 0 |
| AIG69158.1 | Attachment invasion locus protein precursor CDS | 600 | OM | 10 | 1 |
| AIG69189.1 | Hypothetical protein CDS | 1851 | U | 2 | 0 |
| AIG69198.1 | hypothetical protein CDS | 564 | U | 2 | 0 |
| AIG69216.1 | adherence and invasion outermembrane protein (Inv,enhances Peyer's patches | 7863 | OM | 9,95 | 0 |
| | colonization) CDS | | | | |
| AIG69305.1 | AsmA protein CDS | 1854 | U | 2,5 | 1 |
| AIG69312.1 | Putative chaperonin CDS | 1941 | U | 2 | 2 |
| AIG69314.1 | hypothetical protein CDS | 660 | U | 2 | 0 |
| AIG69346.1 | Uncharacterized protein YehA precursor CDS | 1035 | U | 2 | 0 |
| AIG69347.1 | Fimbriae usher protein StcC CDS | 2481 | OM | 10 | 0 |
| AIG69349.1 | Putative fimbrial-like protein CDS | 543 | U | 2,5 | 1 |
| AIG69361.1 | hypothetical protein CDS | 1530 | U | 2 | 0 |
| AIG69362.1 | hypothetical protein CDS | 732 | U | 2 | 0 |
| AIG69377.1 | Attachment invasion locus protein precursor CDS | 600 | OM | 10 | 1 |
| AIG69390.1 | Minor tail protein Z CDS | 624 | U | 2 | 0 |
| AIG69393.1 | Prophage Clp protease-like protein | 1041 | U | | U |
| AIG69411.1 | Shiga toxin A-chain precursor CDS | 948 | U | 2,5 | 1 |
| AIG69429.1 | putative superinfection exclusion protein CDS | 555 | U | 2 | 2 |
| AIG69485.1 | Colicin I receptor precursor CDS | 1884 | OM | 10 | 0 |
| AIG69508.1 | Lipoprotein spr precursor CDS | 567 | U | 2,5 | 0 |
| AIG69510.1 | ABC transporter, periplasmic substrate-binding protein CDS | 1815 | U | 6,58 | 0 |
| AIG69523.1 | Putative ATP-binding component of a transport system CDS | 2592 | Ex | 9,46 | 0 |
| AIG69547.1 | Outer membrane protein C precursor CDS | 1104 | OM | 10 | 1 |
| AIG69552.1 | hypothetical protein CDS | 777 | U | 2 | 0 |
| AIG69553.1 | Putative membrane protein CDS | 4515 | U | 4,69 | 0 |
| AIG69558.1 | Type V secretory pathway, adhesin AidA CDS | 3705 | OM | 9,83 | 0 |
| AIG69581.1 | Polymyxin resistance protein PmrG CDS | 603 | U | 2,5 | 1 |
| AIG69644.1 | DedD protein CDS | 663 | U | 2 | 1 |
| AIG69664.1 | Uncharacterized protein YadU in stf fimbrial cluster CDS | 843 | U | 2 | 0 |
| AIG69669.1 | Fimbriae usher protein StfC CDS | 2583 | OM | 10 | 0 |
| AIG69676.1 | Long-chain fatty acid transport protein CDS | 1341 | OM | 10 | 0 |
| AIG69679.1 | Lipoprotein CDS | 756 | OM | 9,92 | 2 |
| AIG69706.1 | hypothetical protein CDS | 636 | U | 2,5 | 0 |
| AIG69727.1 | putative virulence protein CDS | 1149 | U | 2 | 0 |
| AIG69790.1 | YpfJ protein, zinc metalloprotease superfamily | 864 | U | 2 | U |
| AIG69792.1 | Outer membrane protein NlpB, lipoprotein component of the protein assembly complex (forms a complex with YaeT, YfiO, and YfgL) CDS | 1035 | OM | 9,93 | 0 |
| AIG69809.1 | Exported zinc metalloprotease YfgC precursor | 1464 | U | 2,5 | U |
| AIG69829.1 | Outer membrane protein YfgL, lipoprotein component of the protein assembly complex (forms a complex with YaeT, YfiO, and NlpB) CDS | 1179 | OM | 9,92 | 0 |
| AIG69833.1 | putative membrane protein CDS | 1014 | U | 2 | 1 |
| AIG69842.1 | Thiosulfate sulfurtransferase, rhodanese CDS | 846 | U | 5,41 | 1 |
| AIG69880.1 | putative alpha helix protein CDS | 636 | U | 2 | 0 |
| AIG69913.1 | hypothetical protein CDS | 564 | U | 2 | 0 |
| AIG69916.1 | hypothetical protein CDS | 732 | U | 2 | 0 |
| AIG69918.1 | putative component of the lipoprotein assembly complex (forms a complex with YaeT, YfgL, and NlpB) CDS | 738 | OM | 10 | 0 |
| AIG69931.1 | Signal recognition particle, subunit Ffh SRP54 CDS | 1362 | U | 5,6 | 0 |
| AIG69958.1 | Hypothetical protein CDS | 1854 | U | 2 | 0 |
| AIG69974.1 | Pertactin precursor CDS | 4308 | U | 5,87 | 0 |
| AIG70043.1 | Coenzyme F420 hydrogenase maturation protease | 471 | U | | U |
| AIG70069.1 | Lipoprotein NlpD CDS | 1140 | OM | 9,93 | 0 |
| AIG70199.1 | Type III secretion bridge between inner and outermembrane lipoprotein (YscJ,HrcJ,EscJ, PscJ) CDS | 735 | OM | 9,92 | 1 |
| AIG70202.1 | Type III secretion protein EprH CDS | 735 | U | 2 | 1 |
| AIG70217.1 | Uncharacterized protein YgeP CDS | 1026 | U | 2 | 0 |
| AIG70219.1 | putative lipoprotein YgeR precursor CDS | 756 | OM | 9,93 | 1 |
| AIG70290.1 | Uridine kinase family protein CDS | 714 | U | 2 | 0 |
| AIG70298.1 | Putative metalloprotease yggG | 759 | U | 2 | U |
| AIG70300.1 | hypothetical protein CDS | 732 | U | 2,5 | 0 |
| AIG70312.1 | UPF0301 protein YqgE CDS | 564 | U | 2 | 0 |
| AIG70323.1 | Uncharacterized protein YggN CDS | 720 | U | 2,5 | 0 |
| AIG70398.1 | Modulator of drug activity B CDS | 582 | U | 2 | 0 |
| AIG70405.1 | Ferrichrome-iron receptor CDS | 2142 | OM | 10 | 0 |
| AIG70411.1 | Type I secretion outer membrane protein, TolC precursor CDS | 1482 | OM | 10 | 0 |
| AIG70414.1 | Uncharacterized protein ygiD CDS | 816 | U | 2 | 0 |
| AIG70516.1 | type 1 fimbriae anchoring protein FimD CDS | 2592 | OM | 10 | 0 |
| AIG70532.1 | Putative lipid carrier protein CDS | 525 | U | 2 | 0 |
| AIG70535.1 | Putative protease | 879 | U | 2 | U |
| AIG70568.1 | putative ABC transporter, auxiliary component YrbC CDS | 636 | U | 2,5 | 0 |
| AIG70569.1 | putative ABC transporter, periplasmic component YrbD CDS | 552 | U | 2,5 | 1 |
| AIG70575.1 | Uncharacterized protein YrbK clustered with lipopolysaccharide transporters CDS | 576 | U | 2 | 1 |
| AIG70604.1 | Outer membrane stress sensor protease DegQ, serine protease | 1368 | P | | U |
| AIG70605.1 | Outer membrane stress sensor protease DegS | 1068 | P | | U |
| AIG70619.1 | Rod shape-determining protein MreC CDS | 1104 | U | 2 | 1 |
| AIG70742.1 | Type IV pilus biogenesis protein PilM CDS | 795 | U | 2 | 0 |
| AIG70779.1 | hypothetical protein CDS | 993 | U | 2,5 | 0 |
| AIG70798.1 | hypothetical protein CDS | 912 | U | 2 | 0 |
| AIG70844.1 | Putative transmembrane protein CDS | 606 | U | 2,5 | 0 |
| AIG70880.1 | hypothetical protein CDS | 753 | U | 2 | 0 |
| AIG70893.1 | TonB-dependent hemin, ferrichrome receptor CDS | 1983 | OM | 10 | 0 |
| AIG70922.1 | Uncharacterized protein YhjG CDS | 2061 | U | 2 | 2 |
| AIG70925.1 | Protein YhjJ, putative peptidase CDS | 1497 | U | 2,5 | 0 |
| AIG70935.1 | hypothetical protein CDS | 1560 | U | 2 | 0 |
| AIG70950.1 | Putative fimbrial protein CDS | 1056 | Ex | 9,65 | 1 |
| AIG70952.1 | Long polar fimbria protein A precursor CDS | 525 | Ex | 9,72 | 0 |
| AIG70954.1 | Putative lipase CDS | 699 | U | 7 | 1 |
| AIG70958.1 | Outer membrane protein A precursor CDS | 660 | OM | 10 | 3 |
| AIG70960.1 | putative exported protein CDS | 711 | U | 2,5 | 0 |
| AIG70976.1 | BAX protein CDS | 573 | U | 2 | 0 |
| AIG70983.1 | Putative outer membrane protein yiaT precursor CDS | 741 | OM | 10 | 1 |
| AIG70995.1 | core protein CDS | 4230 | U | 2 | 0 |
| AIG71007.1 | hypothetical protein CDS | 4767 | OM | 9,95 | 2 |
| AIG71034.1 | Lipopolysaccharide heptosyltransferase III CDS | 1023 | U | 2 | 0 |
| AIG71060.1 | Putative exported protein CDS | 1710 | U | 2 | 1 |
| AIG71083.1 | Secreted protein EspB CDS | 939 | Ex | 10 | 1 |
| AIG71084.1 | Secreted protein EspD CDS | 1125 | Ex | 10 | 3 |
| AIG71087.1 | Type III secretion system EscD protein CDS | 1221 | U | 2 | 1 |
| AIG71088.1 | Intimin CDS | 2805 | OM | 10 | 2 |
| AIG71090.1 | translocated intimin receptor Tir CDS | 1677 | Ex | 10 | 2 |
| AIG71096.1 | SepQ CDS | 918 | U | 2 | 0 |
| AIG71102.1 | Type III secretion bridge between inner and outermembrane lipoprotein (YscJ,HrcJ,EscJ, PscJ) CDS | 573 | OM | 9,93 | 0 |
| AIG71104.1 | Type III secretion outermembrane pore forming protein (YscC,MxiD,HrcC, InvG) CDS | 1539 | OM | 10 | 0 |
| AIG71117.1 | ROrf2 CDS | 1146 | Ex | 10 | 0 |
| AIG71137.1 | hypothetical protein CDS | 624 | U | 2 | 0 |
| AIG71158.1 | Uncharacterized protein YidR CDS | 1251 | U | 2 | 0 |
| AIG71159.1 | Uncharacterized protein YidS CDS | 1083 | U | 2 | 0 |
| AIG71177.1 | hypothetical protein CDS | 750 | U | 2 | 0 |
| AIG71181.1 | hypothetical protein CDS | 2409 | U | 5,48 | 0 |
| AIG71182.1 | hypothetical protein CDS | 672 | U | 2 | 0 |
| AIG71192.1 | Putative fimbrial protein CDS | 1083 | Ex | 10 | 0 |
| AIG71193.1 | Putative fimbrial protein CDS | 1071 | Ex | 9,65 | 0 |
| AIG71194.1 | type 1 fimbriae anchoring protein FimD CDS | 2535 | OM | 9,93 | 1 |
| AIG71272.1 | hypothetical protein CDS | 903 | U | 2,5 | 0 |
| AIG71273.1 | hypothetical protein CDS | 765 | U | 2,5 | 0 |
| AIG71277.1 | hypothetical protein CDS | 849 | OM | 9,49 | 0 |
| AIG71291.1 | Putative carboxymethylenebutenolidase CDS | 816 | U | 2 | 0 |
| AIG71333.1 | Outer membrane sugar transport protein YshA CDS | 693 | OM | 9,93 | 1 |
| AIG71349.1 | hypothetical protein CDS | 915 | U | 2 | 1 |
| AIG71360.1 | hypothetical protein CDS | 1056 | U | 4,9 | 0 |
| AIG71365.1 | Putative glycoporin CDS | 1395 | U | 2,5 | 0 |
| AIG71378.1 | hypothetical protein CDS | 675 | U | 2 | 0 |
| AIG71388.1 | Putative uncharacterized protein YiiQ CDS | 600 | U | 2,5 | 1 |
| AIG71408.1 | core protein CDS | 4185 | U | 2 | 0 |
| AIG71411.1 | hypothetical protein CDS | 609 | U | 2,5 | 0 |
| AIG71419.1 | hypothetical protein CDS | 618 | U | 2,5 | 0 |
| AIG71443.1 | Outer membrane vitamin B12 receptor BtuB CDS | 1845 | OM | 10 | 0 |
| AIG71471.1 | hypothetical protein CDS | 636 | U | 2 | 0 |
| AIG71532.1 | NMN phosphatase CDS | 714 | U | 2,5 | 1 |
| AIG71558.1 | Putative exported protein CDS | 690 | U | 2,5 | 0 |
| AIG71638.1 | Outer membrane lipoprotein Blc CDS | 534 | OM | 10 | 1 |
| AIG71658.1 | HflK protein CDS | 1260 | U | 5,48 | 1 |
| AIG71669.1 | hypothetical protein CDS | 639 | U | 2 | 0 |
| AIG71674.1 | YjfP protein CDS | 750 | U | 2 | 0 |
| AIG71687.1 | Uncharacterized protein yjfZ CDS | 795 | U | 2 | 0 |
| AIG71690.1 | putative virulence protein CDS | 1149 | U | 2 | 0 |
| AIG71691.1 | Putative cell envelope opacity-associated protein A CDS | 639 | U | 2 | 0 |
| AIG71701.1 | Protein ytfj precursor CDS | 555 | U | 4,9 | 0 |
| AIG71707.1 | Uncharacterized protein YtfM precursor CDS | 1734 | OM | 10 | 0 |
| AIG71708.1 | Uncharacterized protein YtfN CDS | 3780 | U | 4,72 | 1 |
| AIG71752.1 | hypothetical protein CDS | 1503 | U | 2 | 0 |
| AIG71763.1 | hypothetical protein CDS | 774 | U | 2,5 | 0 |
| AIG71781.1 | hypothetical protein CDS | 981 | U | 2 | 0 |
| AIG71788.1 | type 1 fimbriae major subunit FimA CDS | 549 | Ex | 10 | 1 |
| AIG71791.1 | type 1 fimbriae anchoring protein FimD CDS | 2637 | OM | 10 | 0 |
| AIG71794.1 | mannose-specific adhesin FimH CDS | 903 | U | 4,65 | 2 |
| AIG71800.1 | Uncharacterized protein YjiC CDS | 831 | U | 2 | 0 |
| AIG71813.1 | hypothetical protein CDS | 843 | U | 4,9 | 1 |
| AIG71857.1 | hypothetical protein CDS | 1074 | U | 2 | 0 |
| AIG68360.1 | internalin,putative_EDL933_2172 | 1260 | U | 2 | 0 |

| | | | | | |
|---|---|---|---|---|---|
| 1. PSORT Localization, where Ex = Extracellular, OM = Outer Membrane, P = Periplasmic, U = Unknown 2. Number of internal helices, where U = unknown | | | | | |

| **Table S2.** Vaccine candidates expressed at transcriptional level (cutoff >10 RPKM) | | | | **Expression (absolute value RPKM)** | | | |
|---|---|---|---|---|---|---|---|
| **Protein ID** | **Locus Tag** | **Functional annotation** | **PSORT score** | **RPKM (LB)** | **RPKM (LB agar)** | **RPKM (LB antibiotics)** | **RPKM (LB Feces)** |
| AIG66267.1 | EDL933_0059 | hypothetical protein CDS | 2 | 32,7 | 65.6 | 75.0 | 0.0 |
| AIG66347.1 | EDL933_0139 | Fimbrial protein Yad like protein CDS | 2 | 192,8 | 327.0 | 101.7 | 347.5 |
| AIG66348.1 | EDL933_0140 | Fimbrial protein YadK CDS | 2 | 32,2 | 37.6 | 0.0 | 171.7 |
| AIG66349.1 | EDL933_0141 | Fimbrial protein YadL CDS | 2,5 | 44,4 | 48.9 | 0.0 | 134.0 |
| AIG66424.1 | EDL933_0216 | putative aminopeptidase CDS | 2,5 | 9,8 | 18.5 | 105.7 | 0.0 |
| AIG66447.1 | EDL933_0241 | hypothetical protein CDS | 2 | 223,3 | 250.2 | 288.3 | 779.5 |
| AIG66490.1 | EDL933_0284 | putative tail fiber protein CDS | 2 | 12 | 0.0 | 1305.2 | 51.1 |
| AIG66511.1 | EDL933_0305 | Zinc binding domain protein CDS | 2 | 11 | 10.6 | 48.4 | 30.4 |
| AIG66518.1 | EDL933_0312 | hypothetical protein CDS | 2,5 | 24,6 | 0.0 | 24.8 | 0.0 |
| AIG66656.1 | EDL933_0452 | Protein YkiA CDS | 2 | 8,3 | 6.8 | 0.0 | 28.1 |
| AIG66781.1 | EDL933_0577 | Putative stomatin/prohibitin-family membrane protease subunit YbbK | 2 | 45,2 | 225.9 | 0.0 | 619.2 |
| AIG66972.1 | EDL933_0770 | core protein CDS | 2 | 6,8 | 12.9 | 6.7 | 4.8 |
| AIG66984.1 | EDL933_0782 | hypothetical protein CDS | 2,5 | 21,6 | 18.6 | 13.3 | 38.2 |
| AIG66987.1 | EDL933_0786 | putative fimbrial-like protein ygiL precursor CDS | 9,72 | 41,5 | 17.4 | 24.9 | 143.2 |
| AIG67020.1 | EDL933_0819 | TolA protein CDS | 2 | 454,8 | 283.4 | 405.1 | 325.5 |
| AIG67060.1 | EDL933_0859 | Putative outer membrane protein CDS | 2,5 | 45,5 | 30.9 | 338.2 | 126.9 |
| AIG67245.1 | EDL933_1046 | Urease accessory protein UreF CDS | 2 | 23,2 | 0.0 | 0.0 | 0.0 |
| AIG67308.1 | EDL933_1109 | putative hemolysin activator protein CDS | 8,86 | 0 | 0.0 | 13.3 | 19.1 |
| AIG67453.1 | EDL933_1258 | hypothetical protein CDS | 2 | 68 | 51.0 | 383.0 | 131.1 |
| AIG67464.1 | EDL933_1259 | Hypothetical protein CDS | 2 | 24,9 | 27.4 | 78.4 | 0.0 |
| AIG67503.1 | EDL933_1308 | hypothetical protein CDS | 9,71 | 35,7 | 45.9 | 0.0 | 53.9 |
| AIG67577.1 | EDL933_1383 | Shiga-like toxin II subunit A precursor CDS | 2,5 | 372,9 | 262.4 | 566.2 | 42.3 |
| AIG67652.1 | EDL933_1458 | Putative exported protein precursor CDS | 2 | 17,5 | 9.1 | 13.0 | 37.4 |
| AIG67653.1 | EDL933_1459 | outer membrane fimbrial usher protein CDS | 10 | 11,5 | 7.8 | 0.0 | 72.4 |
| AIG67671.1 | EDL933_1477 | hypothetical protein CDS | 2 | 2,8 | 0.0 | 0.0 | 25.7 |
| AIG67672.1 | EDL933_1478 | hypothetical protein CDS | 2 | 2,4 | 0.0 | 0.0 | 21.9 |
| AIG67760.1 | EDL933_1570 | putative hemolysin activator protein CDS | 8,86 | 3,2 | 4.6 | 13.3 | 76.2 |
| AIG67905.1 | EDL933_1717 | hypothetical protein CDS | 2 | 32,4 | 8.4 | 144.1 | 138.1 |
| AIG67914.1 | EDL933_1726 | Hypothetical protein CDS | 2 | 3,5 | 0.0 | 0.0 | 10.5 |
| AIG67963.1 | EDL933_1775 | hypothetical protein CDS | 2 | 484,9 | 491.2 | 1537.0 | 2182.8 |
| AIG68016.1 | EDL933_1828 | hypothetical protein CDS | 2 | 3,8 | 0.0 | 8.8 | 12.7 |
| AIG68165.1 | EDL933_1977 | Putative intestinal colonization factor encoded by prophage CP-9330 CDS | 2 | 30 | 171.9 | 0.0 | 135.9 |
| AIG68173.1 | EDL933_1985 | Hypothetical protein CDS | 2 | 70,7 | 40.0 | 7.6 | 10.9 |
| AIG68238.1 | EDL933_2050 | Hypothetical protein CDS | 2 | 53,3 | 26.7 | 45.8 | 0.0 |
| AIG68357.1 | EDL933_2169 | Outer membrane porin protein NmpC precursor CDS | 10 | 16,3 | 22.4 | 0.0 | 110.6 |
| AIG68359.1 | EDL933_2171 | hypothetical protein CDS | 2 | 1,7 | 14.8 | 0.0 | 30.3 |
| AIG68376.1 | EDL933_2189 | internalin, putative CDS | 2 | 6,3 | 2.3 | 10.1 | 19.3 |
| AIG68484.1 | EDL933_2297 | Hypothetical protein CDS | 2 | 8,5 | 8.0 | 30.5 | 10.9 |
| AIG68679.1 | EDL933_2505 | Hypothetical protein CDS | 2 | 27,2 | 13.3 | 15.3 | 21.9 |
| AIG68688.1 | EDL933_2514 | hypothetical protein CDS | 2 | 76,6 | 33.8 | 0.0 | 242.9 |
| AIG69057.1 | EDL933_2888 | putative membrane protein CDS | 2 | 606,7 | 933.1 | 401.2 | 1262.9 |
| AIG69153.1 | EDL933_2986 | hypothetical protein CDS | 2 | 108,7 | 83.4 | 119.1 | 142.8 |
| AIG69189.1 | EDL933_3022 | Hypothetical protein CDS | 2 | 18,8 | 10.7 | 30.5 | 0.0 |
| AIG69198.1 | EDL933_3031 | hypothetical protein CDS | 2 | 37,7 | 78.8 | 25.0 | 180.0 |
| AIG69346.1 | EDL933_3180 | Uncharacterized protein YehA precursor CDS | 2 | 14,1 | 0.0 | 0.0 | 19.6 |
| AIG69349.1 | EDL933_3183 | Putative fimbrial-like protein CDS | 2,5 | 88,7 | 582.1 | 208.0 | 37.4 |
| AIG69390.1 | EDL933_3224 | Minor tail protein Z CDS | 2 | 3,6 | 0.0 | 22.6 | 0.0 |
| AIG69393.1 | EDL933_3227 | Prophage Clp protease-like protein | | 2,2 | 0.0 | 13.6 | 0.0 |
| AIG69411.1 | EDL933_3245 | Shiga toxin A-chain precursor CDS | 2,5 | 654,7 | 1586.3 | 871.2 | 235.5 |
| AIG69664.1 | EDL933_3500 | Uncharacterized protein YadU in stf fimbrial cluster CDS | 2 | 33,2 | 93.7 | 50.2 | 72.2 |
| AIG69958.1 | EDL933_3799 | Hypothetical protein CDS | 2 | 31,4 | 16.0 | 22.8 | 21.9 |
| AIG70298.1 | EDL933_4145 | Putative metalloprotease yggG | 2 | 519,6 | 390.4 | 93.0 | 320.9 |
| AIG70798.1 | EDL933_4653 | hypothetical protein CDS | 2 | 3,7 | 0.0 | 15.5 | 22.3 |
| AIG70844.1 | EDL933_4699 | Putative transmembrane protein CDS | 2,5 | 10,2 | 0.0 | 0.0 | 0.0 |
| AIG71087.1 | EDL933_4946 | Type III secretion system EscD protein CDS | 2 | 55,1 | 44.5 | 46.2 | 83.1 |
| AIG71096.1 | EDL933_4955 | SepQ CDS | 2 | 223,3 | 102.2 | 399.8 | 309.6 |
| AIG71137.1 | EDL933_4996 | hypothetical protein CDS | 2 | 19,8 | 39.6 | 22.6 | 130.1 |
| AIG71181.1 | EDL933_5041 | hypothetical protein CDS | 5,48 | 5,6 | 0.0 | 111.3 | 16.9 |
| AIG71182.1 | EDL933_5042 | hypothetical protein CDS | 2 | 0 | 14.7 | 84.0 | 151.0 |
| AIG71349.1 | EDL933_5210 | hypothetical protein CDS | 2 | 6,1 | 16.2 | 0.0 | 0.0 |
| AIG71763.1 | EDL933_5624 | hypothetical protein CDS | 2,5 | 34,7 | 82.9 | 145.9 | 26.2 |
| AIG71811.1 | EDL933_5672 | adherence and invasion outermembrane protein (Inv,enhances Peyer's patches colonization) CDS | 9,95 | 17,3 | 23.5 | 11.2 | 64.5 |
| AIG66227.1 | EDL933_0019 | Putative outer membrane protein EDL9330019 | 10.00 | 3,2 | 10.1 | 0.0 | 8.3 |
| AIG68053.1 | EDL933_1865 | Pertactin precursor | 7.00 | 36,6 | 42.0 | 40.0 | 28.8 |
| AIG68360.1 | EDL933_2172 | internalin, putative | 2.00 | 8 | 7.8 | 11.2 | 145.0 |
| AIG69216.1 | EDL933_3049 | adherence and invasion outermembrane protein (Inv,enhances Peyer's patches colonization) | 9.95 | 5,8 | 12.6 | 10.8 | 10.3 |
| AIG69974.1 | EDL933_3815 | Pertactin precursor | 5.87 | 12 | 21.8 | 3.3 | 47.1 |
| AIG68899.1 | EDL933_2727 | Putative lipoprotein | 2 | 11 | 6,9 | 0 | 0 |

**Table S3. Features of the 24 antigen candidates expressed as recombinant proteins**

| | | | | | | | | **Expression (absolute value RPKM)** | | | | |
|---|---|---|---|---|---|---|---|---|---|---|---|---|
| **No.** | **Protein ID** | **Desig** | **Functional annotation** | **Sol.** | **kDa** | **Localization** | **Pfam domain** | **LB** | **LB Agar** | **LB Antibiotics** | **Feces** | **WB** |
| 1 | AIG67060.1 | MC001 | Putative outer membrane protein | I | 35 | OM | DUF2219 | 45,5 | 30,9 | 338,2 | 126,9 | ++ |
| 2 | AIG68165.1 | MC002 | Putative intestinal colonization factor encoded by prophage CP-9330 | S | 27 | EC | SBP bac 11 | 30 | 171,9 | 0 | 135,9 | ++ |
| 3 | AIG68357.1 | MC003 | Outer membrane porin protein NmpC precursor | I | 41 | OM | Porin 1 | 16,13 | 22,4 | 0 | 110,6 | + |
| 4 | AIG67577.1 | MC004 | Shiga-like toxin II subunit A precursor | I | 36 | EC | RIP | 372 | 262,4 | 566,2 | 42,3 | + |
| 5 | AIG69411.1 | MC005 | Shiga toxin A-chain precursor | S | 35 | EC | RIP | 654,7 | 1586,3 | 871,2 | 235,5 | - |
| 6 | AIG66347.1 | MC006 | Fimbrial protein Yad like protein | S | 40 | OM | Fimbrial | 192,8 | 327 | 101,7 | 347,5 | - |
| 7 | AIG66424.1 | MC007 | putative aminopeptidase | S | 30 | Unknown | unknown | 9,8 | 18,5 | 105,7 | 0 | ++ |
| 8 | AIG66984.1 | MC008 | hypothetical protein | I | 40 | Fimbrial | Fimbrial | 21,6 | 18,6 | 13,3 | 38,2 | + |
| 9 | AIG67652.1 | MC009 | Putative exported protein precursor | I | 38 | OM | Fimbrial | 17,5 | 9,1 | 13 | 37,4 | ++ |
| 10 | AIG67671.1 | MC010 | hypothetical protein | I | 31 | Unknown | DUF1329 | 2,8 | 0 | 0 | 25,7 | + |
| 11 | AIG67672.1 | MC011 | hypothetical protein | I | 51 | OM | unknown | 2,4 | 0 | 0 | 21,9 | + |
| 12 | AIG68053.1 | MC012 | Pertactin precursor | I | 25 | OM | unknown | 36,6 | 42 | 40 | 28,8 | + |
| 13 | AIG68360.1 | MC013 | internalin, putative | S | 48 | Unknown | unknown | 8 | 7,8 | 11,2 | 145 | + |
| 14 | AIG68899.1 | MC014 | putative lipoprotein | S | 25 | OM | SIMPL | 11 | 6,9 | 0 | 0 | - |
| 15 | AIG69664.1 | MC015 | Uncharacterized protein YadU in stf fimbrial cluster | I | 31 | Unknown | DUF2544 | 33,2 | 93,7 | 50,2 | 72,2 | - |
| 16 | AIG70798.1 | MC016 | hypothetical protein | I | 34 | Unknown | DUF4225 | 3,7 | 0 | 15,5 | 22,3 | - |
| 17 | AIG67308.1 | MC017 | putative hemolysin activator protein 100%ID | I | 37 | OM | Potra 2 | 0 | 0 | 13,3 | 19,1 | + |
| 18 | AIG66972.1 | MC018 | core protein | S | 159 | Unknown | unknown | 6,8 | 12,9 | 6,7 | 4,8 | + |
| 19 | AIG69216.1 | MC019 | adherence and invasion outermembrane protein (Inv,enhances Peyer's patches colonization) | S | 275 | OM | Invasin | 5,8 | 12,6 | 10,8 | 10,3 | - |
| 20 | AIG69974.1 | MC020 | Pertactin precursor | S | 148 | OM | AIDA/Pertectin | 12 | 21,8 | 3,3 | 47,1 | ++ |
| 21 | AIG71811.1 | MC021 | adherence and invasion outermembrane protein (Inv,enhances Peyer's patches colonization) | S | 181 | OM | DUF3442 | 17,3 | 23,5 | 11,2 | 64,5 | - |
| 22 | AIG71181.1 | MC022 | hypothetical protein | S | 93 | Unknown | unknown | 5,6 | 0 | 111,3 | 16,9 | + |
| 23 | AIG66227.1 | MC023 | Putative outer membrane protein | S | 92 | OM | PapC Usher | 3,2 | 10,1 | 0 | 8,3 | + |
| 24 | AIG66656.1 | MC024 | Protein YkiA | I | 83 | Unknown | DUF2773 | 8,3 | 6,8 | 0 | 28,1 | + |

| | | | | | | | | | | | | |
|---|---|---|---|---|---|---|---|---|---|---|---|---|
| S = soluble; I = insoluble OM = Outer membrane; EC = extracellular | | | | | | | | | | | | |

**able S4. Primers**

| **Primer** | **Sequence** | **Product** | **Notes (ID, purpose)** |
|---|---|---|---|
| MCRL-34Fw | CTGTACTTCCAGGGCTTTCAACCAATACTTAATGAT **[SEQ ID NO: 49]** | MC001 | AIG67060.1 |
| MCRL-34Rv | AATTAAGTCGCGTTAAAAAAAGAAGGTGATTGC **[SEQ ID NO: 50]** | | |
| MCRL-24Fw | CTGTACTTCCAGGGCGATATCAATCTGTATGGTCC **[SEQ ID NO: 51]** | MC002 | AIG68165.1 |
| MCRL-24Rv | AATTAAGTCGCGTTAAGTGCCTTTCCTGGT **[SEQ ID NO: 52]** | | |
| MCRL-40Fw | CTGTACTTCCAGGGCGACCTTTATGGCAAGG **[SEQ ID NO: 53]** | MC003 | AIG68357.1 |
| MCRL-40Rv | AATTAAGTCGCGTTAGAACTGATAGGTAATGCC **[SEQ ID NO: 54]** | | |
| MCRL-35Fw | CTGTACTTCCAGGGCGAGTTTACGATAGACTTTTCG **[SEQ ID NO: 55]** | MC004 | AIG67577.1 |
| MCRL-35Rv | AATTAAGTCGCGTTATTTACCCGTTGTATATAAAAAC **[SEQ ID NO: 56]** | | |
| MCRL-32Fw | CTGTACTTCCAGGGCACGTATGTAGATTCGCTG **[SEQ ID NO: 57]** | MC005 | AIG6941 1.1 |
| MCRL-32Rv | AATTAAGTCGCGTTAACTGCTAATAGTTCTGCG **[SEQ ID NO: 58]** | | |
| MCRL-41Fw | CTGTACTTCCAGGGCATGAAGATAAGCTCTACT **[SEQ ID NO: 59]** | MC006 | AIG66347.1 |
| MCRL-41Rv | AATTAAGTCGCGTTATTCGTAGGTAAAGGA **[SEQ ID NO: 60]** | | |
| MCRL-28Fw | CTGTACTTCCAGGGCCAGCAACTGACAGACAA **[SEQ ID NO: 61]** | MC007 | AIG66424.1 |
| MCRL-28Rv | AATTAAGTCGCGTTACTGGAATCGACTCACC [**SEQ ID NO: 62]** | | |
| MCRL-36Fw | CTGTACTTCCAGGGCAACTGCTATTTTGGTACC **[SEQ ID NO: 63]** | MC008 | AIG66984.1 |
| MCRL-36Rv | AATTAAGTCGCGTTAGTTGTAGTTAATTTTGAAAAG **[SEQ ID NO: 64]** | | |
| MCRL-38Fw | CTGTACTTCCAGGGCATGTGGGAATGTGATG **[SEQ ID NO: 65]** | MC009 | AIG67652.1 |
| MCRL-38Rv | AATTAAGTCGCGTTATTGCATTTTCACCAA **[SEQ ID NO: 66]** | | |
| MCRL-26Fw | CTGTACTTCCAGGGCACGCTGACGGTAACGG **[SEQ ID NO: 67]** | MCOlO | AIG67671.1 |
| MCRL-26Rv | AATTAAGTCGCGTTATTTCGCACCTCGCTG **[SEQ ID NO: 68]** | | |
| MCRL-43Fw | CTGTACTTCCAGGGCACGCTTTACGAGCAG **[SEQ ID NO: 69]** | MC011 | AIG67672.1 |
| MCRL-43Rv | AATTAAGTCGCGTTATAGATAGTATTTAAAGCCGT **[SEQ ID NO: 70]** | | |
| MCRL-22Fw | CTGTACTTCCAGGGCGTGGGGATCGACAGC **[SEQ ID NO: 71]** | MC012 | AIG68053.1 |
| MCRL-22Rv | AATTAAGTCGCGTTAGAACGACCAGTTCACAC **[SEQ ID NO: 72]** | | |
| MCRL-42Fw | CTGTACTTCCAGGGCATGAAATTCCCTTCA **[SEQ ID NO: 73]** | MC013 | AIG68360.1 |
| MCRL-42Rv | AATTAAGTCGCGTTAGTGATAAAAAGGCCA **[SEQ ID NO: 74]** | | |
| MCRL-23Fw | CTGTACTTCCAGGGCGGTTATTTTGTTGGCG **[SEQ ID NO: 75]** | MC014 | AIG68899.1 |
| MCRL-23Rv | AATTAAGTCGCGTTAATCCTGCAACGCATA **[SEQ ID NO: 76]** | | |
| MCRL-29Fw | CTGTACTTCCAGGGCATTTATTATGCGATGAAA **[SEQ ID NO: 77]** | MC015 | AIG69664.1 |
| MCRL-29Rv | AATTAAGTCGCGTTACAGTTCATTCAGTACATACTG **[SEQ ID NO: 78]** | | |
| MCRL-30Fw | CTGTACTTCCAGGGCATGGGTACAGCAGCTATA **[SEQ ID NO: 79]** | MC016 | AIG70798.1 |
| MCRL-30Rv | AATTAAGTCGCGTTATTGTATTCCTGCACCA **[SEQ ID NO: 80]** | | |
| MCRL-37Fw | CTGTACTTCCAGGGCGATGTCCGGCGTAGC **[SEQ ID NO: 81]** | MC017 | AIG67308.1 |
| MCRL-37Rv | AATTAAGTCGCGTTACTGCTTTTTACAACCATTC **[SEQ ID NO: 82]** | | |
| MCRL-48Fw | CTGTACTTCCAGGGCATGAGCGGAAAACCG **[SEQ ID NO: 83]** | MC018 | AIG66972.1 |
| MCRL-48Rv | AATTAAGTCGCGTTATTTTCTTATTCCTCTCGATG **[SEQ ID NO: 84]** | | |
| MCRL-52Fw | CTGTACTTCCAGGGCATGGCTGCGGCAGCA **[SEQ ID NO: 85]** | MC019 | AIG69216.1 |
| MCRL-52Rv | AATTAAGTCGCGTTACGCAATATTGACGAT **[SEQ ID NO: 86]** | | |
| MCRL-49Fw | CTGTACTTCCAGGGCGTGGGGCAGTCTAAT **[SEQ ID NO: 87]** | MC020 | AIG69974.1 |
| MCRL-49Rv | AATTAAGTCGCGTTAACTCGCTTTCATTATGTT **[SEQ ID NO: 88]** | | |
| MCRL-51Fw | CTGTACTTCCAGGGCGTGCCTTACACGCTTGGT **[SEQ ID NO: 89]** | MC021 | AIG71811.1 |
| MCRL-51Rv | AATTAAGTCGCGTTAAAGTGATTTACGGCAGGC **[SEQ ID NO: 90]** | | |
| MCRL-45Fw | CTGTACTTCCAGGGCATGGTCGCTAAATTAAAAC **[SEQ ID NO: 91]** | MC022 | AIG71181.1 |
| MCRL-45Rv | AATTAAGTCGCGTTAAGCCTGGGTTATATTAAC **[SEQ ID NO: 92]** | | |
| MCRL-45Fw | CTGTACTTCCAGGGCATGGTCGCTAAATTAAAAC **[SEQ ID NO: 93]** | MC023 | AIG66227.1 |
| MCRL-45Rv | AATTAAGTCGCGTTAAGCCTGGGTTATATTAAC **[SEQ ID NO: 94]** | | |
| MCRL-46Fw | CTGTACTTCCAGGGCAGTTATGGCCGATTT **[SEQ ID NO: 95]** | MC024 | AIG66656.1 |
| MCRL-46Rv | AATTAAGTCGCGTTATGTAAACTGCACATAAGA **[SEQ ID NO: 96]** | | |
| pET-TEV-Fw | TAACGCGACTTAATTCTAGCATAACC **[SEQ ID NO: 97]** | pET15 | pET-15 expression vector |
| pET-TEV-Rv | GCCCTGGAAGTACAGGTTTTC **[SEQ ID NO: 98]** | | |
| TolR-Cat-Fw | TGGAGGTCGATTTGCCAGACGCTACTGAATCACAGGCGGTGAGCAGTAACGATA ATCCGCCAGTGATTGTTGACATATGAATATCCTCCTTAGTTCCTATTCC [SEQ ID NO: 99] | Cat-TolR | *toIR* mutant construction |
| TolR-Cat-Rv | CCTTGAAACGGCTGGACACTTCCGCCACCACCTGCTCTGGTGGTAAACG CTCCAGGCGATCTTTCTCAACCAGTGTAGGCTGGAGCTGCTTCGAAG [SEQ ID NO: 100] | | |
| GMMA-MC001Fw | CAGGAGGAATTAACCATGAAAAAAAGTGTCATCGCTGGC **[SEQ ID NO: 101]** | MC001 with signal sequence | Antigen expression on GMMA |
| GMMA-MC001Rv-NONhis | ATGATGATGATGATGATGAAAAAAGAAGGTGATTGCTCC **[SEQ ID NO: 102]** | | |
| GMMA-MC007Fw | CAGGAGGAATTAACCATGGGGCAGTCTAATAATACCAC **[SEQ ID NO: 103]** | MC007 with signal sequence | Antigen expression on GMMA |
| GMMA-MC007Rv-NONhis | ATGATGATGATGATGATGGAACGACCAGTTCACACCAGC **[SEQ ID NO: 104]** | | |
| GMMA-MC020Fw | CAGGAGGAATTAACCATGGGGCAGTCTAATAATACCACC **[SEQ ID NO: 105]** | MC020 with signal sequence | Antigen expression on GMMA |
| GMMA-MC020Rv-NONhis | ATGATGATGATGATGATGGAACGACCAGTTCACACCAG **[SEQ ID NO: 106]** | | |
| MC001tagFw | GATTACAAAGACGATGATGACAAGGATTACAAAGACGATGATGACAAGA ACAGCCTTGCATTATCATT [SEQ ID NO: 107] | MC001 with signal sequence and Flag Tag | Antigen expression on GMMA with Flag Tag |
| MC001tagRv | CTTGTCATCATCGTCTTTGTAATCCTTGTCATCATCGTCTTTGTAATCC GCGATAGCTGAACACGTGG [SEQ ID NO: 108] | | |
| MC007tagFw | GATTACAAAGACGATGATGACAAGGATTACAAAGACGATGATGACAAGT CTGAATCTTCTATTGATGA [SEQ ID NO: 109] | MC007 with signal sequence and Flag Tag | Antigen expression on GMMA with Flag Tag |
| MC007tagRv | CTTGTCATCATCGTCTTTGTAATCCTTGTCATCATCGTCTTTGTAATCG GCACATCCTGCAAGCAGCG [SEQ ID NO: 110] | | |
| MC020tagFw | GATTACAAAGACGATGATGACAAGGATTACAAAGACGATGATGACAAGG CCTTCACTCCTGATGTTAT [SEQ ID NO: 111] | MC020 with signal sequence and Flag Tag | Antigen expression on GMMA with Flag Tag |
| MC020tagRv | CTTGTCATCATCGTCTTTGTAATCCTTGTCATCATCGTCTTTGTAATCA GCCATCCCGGCGGGGCAT [SEQ ID NO: 112] | | |
| pBADRv | CATGGTTAATTCCTCCTGTTAGCCC **[SEQ ID NO: 113]** | pBAD Vector without His-Tag | Expression of Ag in GMMA |
| pBADFw NonHIS | TGAGTTTAAACGGTCTCCAGCTTGG **[SEQ ID NO: 114]** | | |

**Table 5 - Nucleotide and Protein Sequences of the 24 Antigen Candidates**

| Name | Sequence | Sequence Length |
|---|---|---|
| MC001 | ATGAAAAAAAGTGTCATCGCTGGCGTCTTTATTGCTCTGTCATTTACCACGTGTTCAGCTATCGCGAA CAGCCTTGCATTATCATTAGCAAATGATGATGCAGGGAAGTTTCAACCAATACTTAATGATATTTATG GCAATAAACATGAAAACAGAGATGATTACTCACAAGGCTTATTTCTGGGATATAGCCACGATATCTCA GACTCGAGCCAATTATCTCTCCATATTGCGCAAGATATTTACTCTCCATCAGGCAGTAATAAAAGACA CAACACAGCTGTAACTGGAGACAGAGCTTTTAGTGCATACACTCACACTGGTATTGAATGGAACTCCC TTGCGAATGACTGGATTCGCTATCGATTAGGTACTGACATAGGTGTTGTTGGCCCCGACGCAGGCGGT CAGAAAGTACAAAATAAAGCTCATGAGATTATTGGGGCAGAAAAATATCATGCATGGGATGATCAAAT AGAGAATCGCTACGGTTATACTGTAAAAGGGATGCTATCCATGACACCAAGTATGGATATTTTAGGTG CTAATGTTGGATTATACCCTGAAGTTTCTGCTGTTACTGGAAACTTATTTCAATATGTAGCATATGGC GCAACCATTGCCATTGGTAATGATAAAACCTTCAATTCGGATAATGGCTTTGGTCTGCTGGCTCCCCG TGGTTTAATGCATATGTCCGATACAAGCGGATTCAAATACAAGATTTTTGCAGGTATGGAAAGACGAG ATGTCAATCGCAACTATACTCTCGAAGGAAAAACAATACAGACGAAACAAACAACAGTATCGCTAAAC AAAACTGTTGATGAATATCAAGTTGGCGCAACAATTGGGTATGCACCTGTAGCCTTCACACTAGCATT TAATAAAGTAACATCAGAATTCAAGACAGGGGATGACTATTCATTTATAAATGGAGCAATCACCTTCT TTTTTTAA [SEQ ID NO: 1] | 960 |
| MC001 prot | MKKSVIAGVFIALSFTTCSAIANSLALSLANDDAGKFQPILNDIYGNKHENRDDYSQGLFLGYSHDIS DSSQLSLHIAQDIYSPSGSNKRHNTAVTGDRAFSAYTHTGIEWNSLANDWIRYRLGTDIGVVGPDAGG QKVQNKAHEIIGAEKYHAWDDQIENRYGYTVKGMLSMTPSMDILGANVGLYPEVSAVTGNLFQYVAYG ATIAIGNDKTFNSDNGFGLLAPRGLMHMSDTSGFKYKIFAGMERRDVNRNYTLEGKTIQTKQTTVSLN KTVDEYQVGATIGYAPVAFTLAFNKVTSEFKTGDDYSFINGAITFFF [SEQ ID NO: 25] | 319 |
| MC007 | ATGAATTCAAAAAAGCTTTGTTGCATATGTGTGTTATTCTCGCTGCTTGCAGGATGTGCCTCTGAATCT TCTATTGATGAAAAGAAGAAAAAAGCTCAAGTCACACAAAGTAATATTAATAAAAACACTCCCCAGCAA CTGACAGACAAAGATTTATTCGGTAATGAAACCACTCTGGCCGTATCCGAAGAGGATATTCAAGCAGCG CTTGATGGAGATGAGTTCCGCGTTCCCCTTAATTCTCCTGTAATACTTGTTCAATCCGGCAACCGCGCA CCGGAAACCATTATGCAGGAGGAGATGCGAAAATATTATACTGTTTCCACATTCTCTGGTATCCCGGAC AGGCAAAAGCCTCTGACTTGTAACAAAAACAAAGATAAAAATGAGAACGAGGATGTTGCCCGTGCTGAG AATATGAACTGGATGCAGGCACTGCGTTTTGTGGCTGCAAAAGGACATCAGAAAGCGATTATTGTTTAC CAGGATATGTTGCAGACAGGAAAATATGACTCTGCGCTGAAATCAACAGTATGGTCCGACTATAAAAAT GAAAAACTCACTGACGCTATCTCCCTGCGCTACCTGGTACGTTTCACGCTGGTGGATGTGGCAACAGGT GAGTGGGCTACCTGGTCGCCGGTGAATTATGAATCGAGAGTAATTTTTCCGCCGGCTGGAATAACAAAA ACGAGCAATAAAGAACTATCAAATGACCATGTTACGGAAGCGCAACTGTTTAATTTGAAACAAAAAACA | 801 |
| | TATGTATCGATGGTGAAAGACTTGGTGAGTCGATTCCAGTAA [SEQ ID NO: 2] | |
| MC007 prot | MNSKKLCCICVLFSLLAGCASESSIDEKKKKAQVTQSNINKNTPQQLTDKDLFGNETTLAVSEEDIQAA LDGDEFRVPLNSPVILVQSGNRAPETIMQEEMRKYYTVSTFSGIPDRQKPLTCNKNKDKNENEDVARAE NMNWMQALRFVAAKGHQKAIIVYQDMLQTGKYDSALKSTVWSDYKNEKLTDAISLRYLVRFTLVDVATG EWATWSPVNYESRVIFPPAGITKTSNKELSNDHVTEAQLFNLKQKTYVSMVKDLVSRFQ [SEQ ID NO: 26] | 266 |
| MC020 | ATGAACAGGACCAGTCCCTATTATTGTCGTCGCTCAGTACTTTCCTTATTGATATCTGCCTTGATATAT GCCCCGCCCGGGATGGCTGCCTTCACTCCTGATGTTATTGGTGTGGTAAACGATGAGACTGTAGATGGC AGCCAACGAGTAGATGAACGAGGTACAACAAATAACACTCATATTATCAACCATGGCCAGCAGAATGTT TATGGCGGGGTATCTAATGGAAGTCTTATTGAATCTGGTGGATATCAAGATGTAGGAAGGCATAACAAT TATGTGGGGCAGTCTAATAATACCACCATTAACGGGGGCAGACAGTCAATTCATGACGGGGGTATTTCC ACAGGTACGATAATCGAGAGTGGCAATCAGGACGTTTATAAAGGGGGTATCAGCAATGGAACGACAATT AAGGGCGGTGCTTCACGCGTAGAGGGAGGGAGTGCGAATGGAACACTCATTGATGGTGGTAGCCAGATA GTAAAAGTTCAAGGGCATGCTGATGGTACAACGATAAATAAGTCTGGCTCTCAGGACGTAGTACAAGGA AGTCTGGCAACGAACACAACCATAAATGGTGGTCGACAGTATGTTGAACAGAGCACAGTAGAAACAACC ACCATCAAAAATGGCGGTGAGCAAAGAGTATATGAGAGCCGTGCGCTGGACACGACGATTGAAGGCGGA ACTCAGTCTCTGAATAGTAAGTCAACGGCAAAAAATACTCAGATCTATTCTGGTGGTACGCAAATTATT GATAACACCAGCTCCTCGGATGTTATTGAAGTTTATTCCGGTGGCGTGCTTGATGTTAGTGGTGGTACG GCAACAAATGTTACCCAGCACGATGGTGCAATTTTAAAAACTAACACTAACGGTACGACGGTGAGCGGT ACGAATAGTGAAGGTGCATTCTCCATCCACAATCACGTGGCAGACAATGTGTTGCTGGAAAACGGTGGT CATTTAGACATAAACGCATATGGTTCGGCAAACAAGACGATTATTAAAGATAAAGGAACAATGTCAGTT TTAACCAATGCTAAAGCTGATGCGACCCGAATAGATAATGGCGGGGTTATGGATGTTGCAGGAAACGCG ACAAATACCATAATTAATGGTGGCACACAGAATATTAATAATTATGGCATAGCCACAGGCACCAATATC AACAGCGGAACGCAAAATATCAAAAGCGGCGGGAAAGCTGACACAACAATTATATCCTCCGGGAGCCGG CAGGTTGTTGAGAAAGATGGTACGGCAATTGGCAGCAATATTAGCGCCGGAGGCTCGCTGATTGTCTAT ACCGGCGGTATTGCACATGGGGTTAACCAGGAGACGGGCAGTGCTTTAGTTGCCAACACGGGTGCAGGG ACTGATATCGAAGGATACAACAAGCTCTCTCACTTCACTATTACCGGAGGGGAGGCTAATTATGTTGTG CTGGAAAATACCGGCGAACTGACGGTAGTGGCTAAAACCTCGGCGAAAAATACTACCATTGATGCTGGC GGTAAGCTGATTGTCCAGAAGGAGGCTAAAACAGATAGCACCAGACTTAATAATGGCGGCGTTCTGGAG GTTCAGGACGGTGGTGAGGCTAAGCATGTTGAGCAACAATCCGGCGGCGCATTAATTGCTTCCACGACC TCCGGAACACTTATCGAAGGAACCAACAGTTATGGTGATGCTTTCTACATCAGGAATTCAGAAGCTAAA AATGTAGTGCTGGAAAACGCTGGCTCATTAACAGTCGTCACTGGTTCCCGGGCAGTTGACACGATTATT AATGCCAACGGCAAAATGGATGTTTATGGAAAAGATGTTGGCACTGTACTCAATAGTGCTGGCACCCAA ACAATATATGCCAGTGCCACTTCTGATAAAGCAAATATCAAAGGTGGCAAGCAAACGGTATATGGTTTA GCCACTGAAGCAAATATCGAAAGTGGTGAACAAATTGTTGATGGTGGGTCAACAGAGAAAACACACATC AATGGTGGCACGCAAACCGTTCAGAATTATGGTAAGGCGATCAATACCGATATCGTCTCTGGCCTACAA | 4587 |
| | CAAATTATGGCAAACGGACAGCGGAAGGTTCCATTATTAATGGCGGTTCACAGATAGTTAATGAGGGC GGTCTGGCTGAAAACTCGGTGCTTAATGATGGCGGCACACTCGATGTGCGGGAGAAAGGCAGCGCAACG GGGATACAGCAGAGTAGCCAGGGCGCGTTGGTTGCAACCACCAGGGCGACGCGGGTCACAGGAACACGC GCGGATGGCGTCGCGTTCAGCATCGAGCAGGGTGCGGCGAACAATATCCTGCTGGCAAATGGCGGAGTG TTAACCGTGGAGTCAGACACCTCTTCTGACAAAACACAGGTCAATACGGGCGGACGGGAGATCGTCAAA ACAAAAGCCACTGCGACAGGCACGACGCTCACCGGCGGTGAACAAATTGTCGAGGGTGTGGCGAATGAG ACAACAATTAACGACGGCGGAATACAAACAGTTTCAGCTAACGGAGAGGCAATAAAAACAACGATCAAT GAAGGCGGTACGCTGACAGTCAACGATAATGGCAAAGCGACAGATATCGTCCAGAACAGCGGTGCCGCT CTCCAGACGAGCACGGCTAACGGTATTGAAATCAGCGGTACTCACCAGTACGGCACTTTTTCCATTTCC GGCAATTTAGCGACCAATATGTTGCTGGAAAATGGCGGTAATTTATTGGTATTAGCAGGTACCGAAGCT CGCGACTCCACGGTTGGCAAGGGGGGGGCAATGCAAAACCAGGGTCAGGACTCCGCCACAAAGGTTAAC TCTGGTGGGCAATATACCCTTGGGCGGTCAAAAGATGAGTTTCAGGCTCTGGCCCGGGCAGAAGATCTC CAGGTTGCTGGCGGGACAGCAATCGTCTACGCAGGTACGCTGGCGGATGCATCGGTCAGTGGCGCGACA GGAAGCCTGTCGTTAATGACGCCACGGGATAATGTTACGCCAGTTAAACTCGAAGGGGCGATCCGGATT ACCGATAGCGCGACATTAACTATCGGCAATGGCGTTGATACGACGCTTGCCGACCTGACGGCTGCCAGC CGGGGCAGTGTCTGGCTTAACAGCAATAATTCCTGTGCAGGCACCAGCAACTGCGAGTATAGAGTAAAC AGTTTGCTACTTAACGACGGTAATGTTTATTTATCAGCACAAACAGCAGCGCCTGCCACAACTAACGGT ATATACAATACGCTGACAACCAATGAACTTTCCGGTAGCGGTAATTTCTACCTGCATACCAACGTTGCA GGCTCTCGGGGCGATCAACTGGTCGTCAACAACAACGCCACTGGTAATTTTAAAATCTTTGTTCAGGAT ACCGGCGTCAGTCCTCAGTCTGACGACGCGATGACGCTGGTGAAAACAGGGGGAGGGGATGCTTCGTTT TCGCTGGGCAATACTGGCGGTTTCGTTGATCTTGGGACCTATGAGTATGTCCTGAAAAGCGATGGCAAC AGCAACTGGAACCTGACCAATGATGTCAAACCCAACCCGGATCCCAACCCAAATCCCAACCCAAATCCG AAGCCGGATCCAAAACCAGACCCAAAACCGGATCCGAAACCAGACCCGACTCCCGAGCCAACGCCGACA CCCGTTCCGGAGAAACGCATCACGCCTTCTACCGCAGCCGTACTCAATATGGCAGCAACATTACCGTTG GTATTTGATGCTGAGCTAAACAGTATTCGCGAGCGGTTGAACATAATGAAAGCGAGTCCACACAACAAT AATGTCTGGGGGGCGACGTATAACACCCGTAATAATGTCACCACCGATGCGGGGGCCGGGTTTGAGCAG ACGCTGACCGGAATGACAGTGGGGATCGACAGCCCTAATGATATTCCTGAGGGGATTGCGACGCTGGGC GCTTTTATGGGTTATTCCCATTCACATATCGGTTTTGATCGCGGAGGACATGGCAGTGTGGGCAGTTAT TCTCTGGGCGGCTATGCCAGTTGGGAACATGAAAGTGGTTTCTATCTGGACGGTGTCGTGAAGCTGAAC CGTTTTGAAAGTAACGTAGCCGGTAAAATGAGCAGCGGTGGAGCCGCCAATGGCAGTTACCACAGCAAC GGGCTGGGCGGTCACATTGAAACCGGGATGCGATTTACCGATGGTAACTGGAACCTGACGCCGTATGCA TCGTTAACGGGGTTCACCGCTGATAACCCCGAATATCATTTATCCAATGGCATGGAATCGAAATCAGTC GATACCCGCAGTATATATCGTGAACTGGGCGCAACGCTGAGTTACAACATGCGTCTGGGGAACGGTATG GAAATTGAGCCGTGGCTGAAGGCGGCTGTGCGCAAAGAATTTGTCGATGATAACCGGGTGAAGGTGAAT AATGACGGTAATTTCGTCAATGATTTGTCGGGCAGACGTGGAATATACCAGGCAGGTATTAAAGCCTCA TTCAGCAGTACGTTAAGCGGGCATCTTGGGGTGGGGTATAGCCATGGTGCCGGTGTGGAATCCCCGTGG AACGCGGTAGCTGGTGTGAACTGGTCGTTCTGA [SEQ ID NO: 3] | |
| MC020 prot | MNRTSPYYCRRSVLSLLISALIYAPPGMAAFTPDVIGVVNDETVDGSQRVDERGTTNNTHIINHGQQNV YGGVSNGSLIESGGYQDVGRHNNYVGQSNNTTINGGRQSIHDGGISTGTIIESGNQDVYKGGISNGTTI KGGASRVEGGSANGTLIDGGSQIVKVQGHADGTTINKSGSQDVVQGSLATNTTINGGRQYVEQSTVETT TIKNGGEQRVYESRALDTTIEGGTQSLNSKSTAKNTQIYSGGTQIIDNTSSSDVIEVYSGGVLDVSGGT ATNVTQHDGAILKTNTNGTTVSGTNSEGAFSIHNHVADNVLLENGGHLDINAYGSANKTIIKDKGTMSV LTNAKADATRIDNGGVMDVAGNATNTIINGGTQNINNYGIATGTNINSGTQNIKSGGKADTTIISSGSR QVVEKDGTAIGSNISAGGSLIVYTGGIAHGVNQETGSALVANTGAGTDIEGYNKLSHFTITGGEANYVV LENTGELTVVAKTSAKNTTIDAGGKLIVQKEAKTDSTRLNNGGVLEVQDGGEAKHVEQQSGGALIASTT SGTLIEGTNSYGDAFYIRNSEAKNVVLENAGSLTVVTGSRAVDTIINANGKMDVYGKDVGTVLNSAGTQ TIYASATSDKANIKGGKQTVYGLATEANIESGEQIVDGGSTEKTHINGGTQTVQNYGKAINTDIVSGLQ QIMANGTAEGSIINGGSQIVNEGGLAENSVLNDGGTLDVREKGSATGIQQSSQGALVATTRATRVTGTR ADGVAFSIEQGAANNILLANGGVLTVESDTSSDKTQVNTGGREIVKTKATATGTTLTGGEQIVEGVANE TTINDGGIQTVSANGEAIKTTINEGGTLTVNDNGKATDIVQNSGAALQTSTANGIEISGTHQYGTFSIS GNLATNMLLENGGNLLVLAGTEARDSTVGKGGAMQNQGQDSATKVNSGGQYTLGRSKDEFQALARAEDL QVAGGTAIVYAGTLADASVSGATGSLSLMTPRDNVTPVKLEGAIRITDSATLTIGNGVDTTLADLTAAS RGSVWLNSNNSCAGTSNCEYRVNSLLLNDGNVYLSAQTAAPATTNGIYNTLTTNELSGSGNFYLHTNVA GSRGDQLVVNNNATGNFKIFVQDTGVSPQSDDAMTLVKTGGGDASFSLGNTGGFVDLGTYEYVLKSDGN SNWNLTNDVKPNPDPNPNPNPNPKPDPKPDPKPDPKPDPTPEPTPTPVPEKRITPSTAAVLNMAATLPL VFDAELNSIRERLNIMKASPHNNNVWGATYNTRNNVTTDAGAGFEQTLTGMTVGIDSPNDIPEGIATLG AFMGYSHSHIGFDRGGHGSVGSYSLGGYASWEHESGFYLDGVVKLNRFESNVAGKMSSGGAANGSYHSN GLGGHIETGMRFTDGNWNLTPYASLTGFTADNPEYHLSNGMESKSVDTRSIYRELGATLSYNMRLGNGM EIEPWLKAAVRKEFVDDNRVKVNNDGNFVNDLSGRRGIYQAGIKASFSSTLSGHLGVGYSHGAGVESPW NAVAGVNWSF [SEQ ID NO: 27] | 1528 |
| MC002 | ATGGCAGGTTTTTTAATATTCCTGTCTTCTGCTGCTTATGCTGATATCAATCTGTATGGTCCTGGTGG CCCGCATACAGCCTTGCTTGATGCAGCCAAACTTTATGCCGAAAAAACAGGTATTATAGTGAACGTTC ATTACGGCCCACAGAACAAATGGAATGAAGATGCCAAAAAAAATGCAGATATCTTGTTTGGCGCATCA GAACAATCTGCTCTGGCTATCATTCGGGACCATAAAGACAGCTTCAGTGAAAAAGATATTCAGCCTCT TTATCTGCGAAAAAGTATTTTACTGGTAAAGAAAGGTAATCCTAAAAATATCCGGAGTATTGACGACC TGACCAGACCTGGGATTGGCGTAATTGTTAATGATGGTGGTGGTACCAGTAATACATCAGGCACTGGC GTCTGGGAAGATATTGCCGGACGTAAAGGGAATATAGAAACTGTCGCCGCAATCCGAAAAAATATTAT TTTATATGCGCCCAATAGCGGAACTGCACGTAAGGCTCTTGAGAATCAGCCTGGAGCAGATGTCTGGA TAACCTGGGCTGACTGGGCAGCCAGTAATCCAGAAATTGGTGATGTCGTGGAAATAGCGCCAGACTAC GTGATATGGCGTGATATGAACATTACAGTACGTCAGGATGCAAATGATGAAACCCGTCGATTTGCAGA ATGGCTACAAACCGATGAAGCGGCGCCTGCATTCAAAAAATATGGCTGGACCAGGAAAGGCACTTGA [SEQ ID NO: 4] | 747 |
| MC002 prot | MAGFLIFLSSAAYADINLYGPGGPHTALLDAAKLYAEKTGIIVNVHYGPQNKWNEDAKKNADILFGAS EQSALAIIRDHKDSFSEKDIQPLYLRKSILLVKKGNPKNIRSIDDLTRPGIGVIVNDGGGTSNTSGTG VWEDIAGRKGNIETVAAIRKNIILYAPNSGTARKALENQPGADVWITWADWAASNPEIGDVVEIAPDY | 248 |
| | VIWRDMNITVRQDANDETRRFAEWLQTDEAAPAFKKYGWTRKGT [SEQ ID NO: 28] | |
| MC003 | ATGAAATTAAAAATAGTTGCGGTGGTTGTAACTGGTTTGTTAGCTGCGAACGTAGCACACGCTGCCGA AGTCTATAACAAGGATGGTAATAAACTCGACCTTTATGGCAAGGTTACCGCTCTACGTTATTTTACTG ATGATAAGCGTGACGATGGTGATAAAACTTATGCCCGTCTCGGCTTTAAAGGAGAAACGCAAATCAAT GATCAAATGATTGGTTTTGGTCACTGGGAATATGATTTTAAAGGCTATAACGATGAAGCCAACGGCTC GCGCGACAACAAGACCCGTCTGGCCTATGCTGGTTTAAAAATTAGTGAATTTGGCTCTCTGGACTATG GCCGTAACTACGGTGTCGGCTATGACATTGGTTCATGGACTGATATGTTGCCAGAATTTGGTGGCGATA CCTGGAGTCAGAAAGATGTCTTCATGACATACCGTACCACCGGTGTAGCAACCTATCGCAACTACGATT TCTTTGGCTTAATTGAAGGGCTGAACTTTGCCGCGCAATATCAAGGCAAAAATGAACGTACTGACAACA GTCATCTTTATGGTGCTGACTACACGCGTGCCAACGGTGACGGTTTCGGTATCTCCTCAACTTATGTTT ATGATGGCTTTGGTATCGGAGCGGTGTATACCAAATCCGATCGGACAAATGCGCAGGAAAGAGCCGCTG CTAATCCTCTCAATGCCTCCGGTAAGAATGCAGAACTGTGGGCTACAGGTATAAAATATGATGCCAACA ACATCTACTTTGCAGCTAATTACGCTGAAACATTAAACATGACCACCTATGGCGATGGTTATATTTCTA ACAAAGCACAAAGTTTTGAAGTGGTGGCGCAATATCAATTCGACTTCGGCTTGCGCCCCTCACTCGCTT ACCTGAAATCGAAAGGCATAGATCTGGGCCGCTACGGCGATCAGGACATGATTGAGTATATCGACGTTG GTGCGACGTATTTCTTCAACAAAAATATGTCGACCTATGTTGATTATAAAATCAACCTGATTGATGAAA GCGACTTTACCCGTGCCGTAGATATTCGCACCGATAACATCGTCGCTACGGGCATTACCTATCAGTTCT AA [SEQ ID NO: 5] | 1101 |
| MC003 prot | MKLKIVAVVVTGLLAANVAHAAEVYNKDGNKLDLYGKVTALRYFTDDKRDDGDKTYARLGFKGETQIND QMIGFGHWEYDFKGYNDEANGSRDNKTRLAYAGLKISEFGSLDYGRNYGVGYDIGSWTDMLPEFGGDTW SQKDVFMTYRTTGVATYRNYDFFGLIEGLNFAAQYQGKNERTDNSHLYGADYTRANGDGFGISSTYVYD GFGIGAVYTKSDRTNAQERAAANPLNASGKNAELWATGIKYDANNIYFAANYAETLNMTTYGDGYISNK AQSFEVVAQYQFDFGLRPSLAYLKSKGIDLGRYGDQDMIEYIDVGATYFFNKNMSTYVDYKINLIDESD FTRAVDIRTDNIVATGITYQF [SEQ ID NO: 29] | 366 |
| MC004 | ATGAAGTGTATATTATTTAAATGGGTACTGTGCCTGTTACTGGGTTTTTCTTCGGTATCCTATTCCCGG GAGTTTACGATAGACTTTTCGACCCAACAAAGTTATGTCTCTTCGTTAAATAGTATACGGACAGAGATA TCGACCCCTCTTGAACATATATCTCAGGGGACCACATCGGTGTCTGTTATTAACCACACCCCACCGGGC AGTTATTTTGCTGTGGATATACGAGGGCTTGATGTCTATCAGGCGCGTTTTGACCATCTTCGTCTGATT ATTGAGCAAAATAATTTATATGTGGCCGGGTTCGTTAATACGGCAACAAATACTTTCTACCGTTTTTCA GATTTTACACATATATCAGTGCCCGGTGTGACAACGGTTTCCATGACAACGGACAGCAGTTATACCACT CTGCAACGTGTCGCAGCGCTGGAACGTTCCGGAATGCAAATCAGTCGTCACTCACTGGTTTCATCATAT CTGGCGTTAATGGAGTTCAGTGGTAATACAATGACCAGAGATGCATCCAGAGCAGTTCTGCGTTTTGTC ACTGTCACAGCAGAAGCCTTACGCTTCAGGCAGATACAGAGAGAATTTCGTCAGGCACTGTCTGAAACT GCTCCTGTGTATACGATGACGCCGGGAGACGTGGACCTCACTCTGAACTGGGGGCGAATCAGCAATGTG CTTCCGGAGTATCGGGGAGAGGATGGTGTCAGAGTGGGGAGAATATCCTTTAATAATATATCAGCGATA CTGGGGACTGTGGCCGTTATACTGAATTGCCATCATCAGGGGGCGCGTTCTGTTCGCGCCGTGAATGAA GAGAGTCAACCAGAATGTCAGATAACTGGCGACAGGCCTGTTATAAAAATAAACAATACATTATGGGAA | 960 |
| | AGTAATACAGCTGCAGCGTTTCTGAACAGAAAGTCACAGTTTTTATATACAACGGGTAAATAA [SEQ ID NO: 6] | |
| MC004 prot | MKCILFKWVLCLLLGFSSVSYSREFTIDFSTQQSYVSSLNSIRTEISTPLEHISQGTTSVSVINHTPPG SYFAVDIRGLDVYQARFDHLRLIIEQNNLYVAGFVNTATNTFYRFSDFTHISVPGVTTVSMTTDSSYTT LQRVAALERSGMQISRHSLVSSYLALMEFSGNTMTRDASRAVLRFVTVTAEALRFRQIQREFRQALSET APVYTMTPGDVDLTLNWGRISNVLPEYRGEDGVRVGRISFNNISAILGTVAVILNCHHQGARSVRAVNE ESQPECQITGDRPVIKINNTLWESNTAAAFLNRKSQFLYTTGK [SEQ ID NO: 30] | 319 |
| MC005 | ATGAAAATAATTATTTTAGAGTGCTAACTTTTTCTTTGTTATCTTTTCAGTTAATGTGGTTGCGAAG GAATTTACCTTAGACTTCTCGACTGCAAAGACGTATGTAGATTCGCTGAATGTCATTCGCTCTGCAATA GGTACTCCATTACAGACTATTTCATCAGGAGGTACGTCTTTACTGATGATTGATAGTGGCACAGGGGAT AATTTGTTTGCAGTTGATGTCAGAGGGATAGATCCAGAGGAAGGGCGGTTTAATAATCTACGGCTTATT GTTGAACGAAATAATTTATATGTGACAGGATTTGTTAACAGGACAAATAATGTTTTTTATCGCTTTGCT GATTTTTCACATGTTACCTTTCCAGGTACAACAGCGGTTACATTGTCTGGTGACAGTAGCTATACCACG TTACAGCGTGTTGCAGGGATCAGTCGTACGGGGATGCAGATAAATCGCCATTCGTTGACTACTTCTTAT CTGGATTTAATGTCGCATAGTGGAACCTCACTGACGCAGTCTGTGGCAAGAGCGATGTTACGGTTTGTT ACTGTGACAGCTGAAGCTTTACGTTTTCGGCAAATACAGAGGGGATTTCGTACAACACTGGATGATCTC AGTGGGCGTTCTTATGTAATGACTGCTGAAGATGTTGATCTTACATTGAACTGGGGAAGGTTGAGTAGT GTCCTGCCTGATTATCATGGACAAGACTCTGTTCGTGTAGGAAGAATTTCTTTTGGAAGCATTAATGCA ATTCTGGGAAGCGTGGCATTAATACTGAATTGTCATCATCATGCATCGCGAGTTGCCAGAATGGCATCT GATGAGTTTCCTTCTATGTGTCCGGCAGATGGAAGAGTCCGTGGGATTACGCACAATAAAATATTGTGG GATTCATCCACTCTGGGGGCAATTCTGATGCGCAGAACTATTAGCAGTTGA [SEQ ID NO: 7] | 948 |
| MC005 prot |  [SEQ ID NO: 31] | 315 |
| MC006 | ATGCAAAGGAAAGGCAATAAACTGTTGATTCAGTTATGCAGTGTGATACTGCTATTTTTACCACATCC TGGTATGCATTGGCGAATGAATGTTATATAGAGAGAAATGCTGAAGGGGATTATCACATGAAGATAAGC TCTACTCAGCTTAGTCTGGCGTCACAAATGGTCGAGGTTCCGACAGAAATAGCCGAAGCTACATGGGAT GTAAATATTCAACTAAGAGGCGATGCCATAGGGTGTAAATCTCTTGGGGATAGTAAGGCAGTTCACTTT CTTAATACAGCTGACCCAAGTTTAATATCCACGTACACCACAACGAATGGCGCAGCGTTATTAAAAACA ACTGTTCCAGGCATTGTGTATTCTGTCGAGTTATTATGCCTTAGTTGTGGTGCCGCAGATGAACTTGAT TTATGGCTACCTGCACAAAGTGGCGCAGATAACTTCATACCAAGCACCCAGACGAAATGGGCCTATGAG TACAGTGATCAAAGTTGGTATTTACGTTTTCGCTTATTCATAACTCCTGAATTTAAACCCAAGAATGGT GTTTCCAGCGGAACAACGATAGCAGGAAAGATTGCGTCATGGTATATAGGTACCAATGACCAGCCGTGG ATCAACTTTTACATTGACAATGACTCTTTAAAGTTTTTCGTCGATGAACCGACCTGTGCAACAGTTGCC CTGGCACAAGATCAGGGCAACGTCAGTGGCAATCAGGTAACGCTTGGGAACAGCTATGTTTCGGAAGTG | 1110 |
| | AAAAATGGGCTTACGCGGAAATCCCTTTTTCTATCCGTGCTGAATACTGTTATGCCAGTAAAATTACG GTTAAGTTGAAAGCGGCAAATAAACCCAGCGATGCCACACTGGTGGGTAAAACGACTGGCTCGGCTTCA GGCGTGGCTGTAAAAGTAAATTCAACTTATGACAATAGCAAAGTATTGTTAAAAGCAGATGGTAGCAAC ACGGTTGACTACAACTTCGCCGCCTGGTCAAACAACCTGCTGTTTTTACCTTTTACGGCGCAGCTGGTA CCGGATGGTAGCGGTAATGCTGTCGGTGTTGGAACATTTTCAGGTAACGCGACCTTCTCCTTTACCTAC GAATAA [SEQ ID NO: 8] | |
| MC006 prot | MQRKGNKLLIQLCSVILLFFTTSWYALANECYIERNAEGDYHMKISSTQLSLASQMVEVPTEIAEATWD VNIQLRGDAIGCKSLGDSKAVHFLNTADPSLISTYTTTNGAALLKTTVPGIVYSVELLCLSCGAADELD LWLPAQSGADNFIPSTQTKWAYEYSDQSWYLRFRLFITPEFKPKNGVSSGTTIAGKIASWYIGTNDQPW INFYIDNDSLKFFVDEPTCATVALAQDQGNVSGNQVTLGNSYVSEVKNGLTREIPFSIRAEYCYASKIT VKLKAANKPSDATLVGKTTGSASGVAVKVNSTYDNSKVLLKADGSNTVDYNFAAWSNNLLFLPFTAQLV PDGSGNAVGVGTFSGNATFSFTYE [SEQ ID NO: 32] | 369 |
| MC008 | ATGAGATTCGCCAAAGGCGTGTTGCTCGCCATCTGTCTGATTTTTTTGCCGCTAAAAGCGGCGCTGGCG CTGAACTGCTATTTTGGTACCGCAAACGGCGCTGTAGAAAAATCGGAAGCTATTATGCCCTTCGCCGTT CCGGCCAATTCCAAACCCGGCGATAAAATCTGGGAATCTGACGATATAAAAATTCCGGTCTACTGTGAC AACAACACAAACGGTAATTTTGAAAGCGAGCACGTCTACGCCTGGGTAAACCCTTACCCCGGTATTCAG GATCCCTATTATCAATTGGGCGTAACCTATGAAGGCGTGGACTATGACGCAAGCCTTGGGAAAAGCCGC ATCGACACGAATCAGTGCATCGACAGTAAGAATATTGATATTTACACCCCGGAGCAGATCATTGCGATG GGCTGGCAAAATAAACTTTGCTCCGGCGATCCAACCGTTATGCACAAATCGCGGACGTTTGTGGCGCGT ATGCGGCTTTACGTTAAAGTTCGCGCCATGCCGCCCCATGATTATCAGAGCAAACTCAGCGATTACATC GTCGTACAGTTTGATGGCGCAGGCAGCGTGAACGAAGACCCTACCGCGAAGAATCTGAAATATCACATT ACCGGTCTGGAAAATATCCGCGTGCTGGATTGCAGCGTCAACTTTGCTATCTCACCTGAAACCCAGGTC GTTGATTTTGGCCGATTTAACGTGCTGGATATTCGTCGCCATACGATGTCGCAGCAATTTAAAATAACT ACAACCAAATCACAAAACGACCAGTGTACCGACGGTTTTAAAGTCAGCTCTTCGTTTTATACCGATGAA ACCCTTATCGACGAAGATAAATCACTGCTCATTGGCAATGGCTTGAAGCTTCGATTGTTGGATGAAAAT GCCTCACCCTATACTTTCAACAAATACAGTGAGTATGCCGATTTTACCAGCGACCTGTTAGTGTATGAA AAAACCTACACGGCAGAATTGTCATCCACGCCGGGTACGCCCATCGACGTTGGTCCCTTTGATACAGTG GTACTTTTCAAAATTAACTACAACTGA [SEQ ID NO: 9] | 1062 |
| MC008 prot | MRFAKGVLLAICLIFLPLKAALALNCYFGTANGAVEKSEAIMPFAVPANSKPGDKIWESDDIKIPVYCD NNTNGNFESEHVYAWVNPYPGIQDPYYQLGVTYEGVDYDASLGKSRIDTNQCIDSKNIDIYTPEQIIAM GWQNKLCSGDPTVMHKSRTFVARMRLYVKVRAMPPHDYQSKLSDYIVVQFDGAGSVNEDPTAKNLKYHI TGLENIRVLDCSVNFAISPETQVVDFGRFNVLDIRRHTMSQQFKITTTKSQNDQCTDGFKVSSSFYTDE TLIDEDKSLLIGNGLKLRLLDENASPYTFNKYSEYADFTSDLLVYEKTYTAELSSTPGTPIDVGPFDTV VLFKINYN [SEQ ID NO: 33] | 353 |
| MC009 | TTGTGGGAATGTGATGCAACAGATTTGCCAAACATCTATTTCTGGTCGCTACCAATGGCGACGACCGC GTGGGGGGCTTTACGATGCAGGCGGGCCTGATGGTCTGAGTGATGTCTATGCCACCTGGTTCGCTTTT GTCGGTCTCAAGCAGACCATGGCGGGCGTGACGCTTGGTCGTTACTGGAAGAAAGTGCCCATCACCAGT | 1086 |
| | TATGCCACTCAGGGAACTAAAATCCAGATTCGCTTACAGGATATCCCTCCTCTTCATGCTGAGCTTTAT CGCATCAGTACGCTACCTGATACATCAGCAACAACAAGTTGGTGCGGTAATAATAATACAGATAGTAGT GGAGTCGGATTCGCAAAACCTTCCGGTACAATCTATAACTGTGTTCAGCCCAATGCCTATATTCAGCTT TCCGGTACCAGCGGCATTTTATTTGGTCATGATGAGCCCGGCGAAGATAGTTCTGTTCATTGGGATTTC TGGGGTGCTGATAATGGTTTTGGTTACGGAATGCGTTCGGCCAATCGACTCTACAACAATGCCACCTGC GTTGCCCGCAGCGCCACGCCGTTAGTATTGCTGCCGACAATTGCAGAAGCACAACTGAATGCGGGCATG GAAAGTACCGGTAATTTTAATGTCCGCGTCGAGTGTAGTAACTCGGTTCAATCAGGGATTAGCGATACT CAGACAGCATTAGGAATCCAGGTGTCTGAAGGTGCATATACAGCGGCGCAAAAACTGGGGATTATCAAT AGCAACGGCGGCGTCAGCGCCCTGGTCTCTGATAATTATGACGCAGCAGAGATGGCAAAGGGCGTTGGG ATCTACATTTCTAACAGTGCTCACCCCGATACGGCGATGACGCTGGTTGGTCAACCGGGCATCGCGAAG TTAACCCCCGGAGGAAATGCAGCGGGGTGGTATCCTGTATTTGAAGGGGCAACATTAGAAGGTGCGACT CACCCCGGATACTCCAGCTATAGTTACTCTTTTATCGCCCGGTTGAAGAAACTGCCAAATCAGACAGTC AGTGCGGGAAAAGTGCGGGCAACGGCTTATATATTGGTGAAAATGCAATGA [SEQ ID NO: 10] | |
| MC009 prot | MWECDATDLPNIYFLVATNGDDRVGGFYDAGGPDGLSDVYATWFAFVGLKQTMAGVTLGRYWKKVPITS YATQGTKIQIRLQDIPPLHAELYRISTLPDTSATTSWCGNNNTDSSGVGFAKPSGTIYNCVQPNAYIQL SGTSGILFGHDEPGEDSSVHWDFWGADNGFGYGMRSANRLYNNATCVARSATPLVLLPTIAEAQLNAGM ESTGNFNVRVECSNSVQSGISDTQTALGIQVSEGAYTAAQKLGIINSNGGVSALVSDNYDAAEMAKGVG IYISNSAHPDTAMTLVGQPGIAKLTPGGNAAGWYPVFEGATLEGATHPGYSSYSYSFIARLKKLPNQTV SAGKVRATAYILVKMQ [SEQ ID NO: 34] | 361 |
| MC010 | ATGAATATGGCTTTTTACGGCAAGTGGTTTGCTTGCCTGTGGCTCGCTACGTCGTGCGTTCAGGCGGCG AGCACAGATAATAAGGCGCTGGAGATAATTCGTCGCGCTGACGAAATTCGTTCGCCGAATAAACCGTTT CGTTACACGCTGACGGTAACGGAATATAAAGCGGGCGCAACCCAGCCAGAGAATAAACAGGTCCTCGAT ATTTCCATGCGCTTTATGAAACCGCAGGGTAATGAAAAAGCGGATGCACGCTCGCTGGTGCGTTTTATT TATCCGCCGCGAGATAAAGGAAAAATCATGCTTTCCGACTGGTACGATTTATGGTTTTACACGCCGGAA CTGCGACGTCCTATGCCTATTTCACGGCAACAAAGATTAATTGGGCAAATCTCTAATGGCGATGTCATT GTCACTAACTTTGAATATGCCTATGACTCGACCCTGATGGGGGAAGTCACGTGTGCTGAAAAGCAGTGT TACAAGCTGGCGCTGGTGCGTAAATCGGCGGATATCACCTGGCCGAAGGTTATCTATTACGTTGAAAAA GACGGTGATAATCGCCCGTGGAAGGCGGCCTATTATTCGCAGGATGACCAGTTAATAAAAGAAGTGTTG TATCAGGATTTCCAGCCGGTGTTGGGGAAAACGCGACCCATGAAAATAACCGTTACCGATGTTCGTCAC GGCAATAACTATTCAGTGATGGAGTACAGCGATGTTCGTCTGGAATCGCTGCCGGAGTTTCATTTCACC AAAGAGTATATTCAGCGAGGTGCGAAATGA [SEQ ID NO: 11] | 789 |
| MC010 prot | MNMAFYGKWFACLWLATSCVQAASTDNKALEIIRRADEIRSPNKPFRYTLTVTEYKAGATQPENKQVLD ISMRFMKPQGNEKADARSLVRFIYPPRDKGKIMLSDWYDLWFYTPELRRPMPISRQQRLIGQISNGDVI VTNFEYAYDSTLMGEVTCAEKQCYKLALVRKSADITWPKVIYYVEKDGDNRPWKAAYYSQDDQLIKEVL YQDFQPVLGKTRPMKITVTDVRHGNNYSVMEYSDVRLESLPEFHFTKEYIQRGAK [SEQ ID NO: 35] | 262 |
| MC011 | ATGAAACGTACGCTGAAGATCTCTTCGTTGCTGTGTGTTGCGCTGCCGTTGACGGTACAGGCGGATTGC | 1389 |
| | CTGTCAGGTGACGAGGTGGCACAAAATAGCGATGTGACGCTTTACGAGCAGGTGTCTTATGTCAATAAA CAGGCTTCGGCCTGGCAGATTGCAGGCAAAAATCCCTACACCCGTCACAACGGTTATCAGGAGGCGGGG ATTGGCATCAATAGCGGTTGTTCGATAATCGACAATACGCTGGACCTTAAGCTCAATCTGTATGGAATA AACGAATATGCGCTTAAACCCGCAGGGAAATTCGAAACGGATGATAGCCGCACGCGTGCGTTAATTAAC CGCCTCAGTCTGGTCTATAGCGCCTCGGACAGCGTTCAATTTGAAGCAGGCAAATTTGCCGCGCCGTCA GGAATGTTCTTTTTGCGTTCGCCTTCTGATTTGCAAACGCATTATTACACCGGATTCCAGTCGACGCGT TTGCACGATCCCAAAATGACATCGGCGTATCAGGCGTCATCGTGGGGCGCAAAAATGAGCGTGGATACA CGCGATTATGCGTTTTCGGCGAGTGTCATACCGAAGTTAGCCACCATCGACAAACGCTATGTGACGTCT GGCAACTGGTCGGCTAATCAGCAGGGAAACAGCGACGAAGCCTATCTGCTGAGCTTCAGCGATCATCGC TTTGGTGAGCACACGCCGACAGTCAATGTGCGGCTCGGACCATCGCCGTCACTCGCGCTGTCTGACAGT TATCACTACACCCCGCAGCTTACGCTGAATGTCGACGCAGCCTACCACCGCTCCCAGCAATGGCGGCAT CTTTCCCACCGTGAGACGGCGCAGGTCGAGGAATATCAGTTTCCGGATTCGCTCTATGAAACGAAAGAT GAAAGCGGCGTAGAGCTGGCGCTGGGTGGGCAGTACACCAGTGATAATTTCAGCGTGTTTGGCGTTGAG TATTACTTTCAGAGCGAGGGTTATTCGCGCGCTGAACAGCGTCAGCAGCGCGAGTTAATTGACTTCCTC AATACCACAACCGGCTATGCGCCACTGGATCAGGCATTTGATTCTTACAAATACCTGATGGCTTCAGAA ATCAGCAATACCGCCAATCAGGGGATGCTGCAGGGTAAGCACTATCTGAACGCCTGGGCCAGTCTGCCG CTGGCGGGAGAGTCGACGCTGCAGCCTTCGCTGGTGGTGAATCTCGTTGATGGCAGCACGTTGCTGGGC CTGCATTATTCCACGCCGCTCAGCGCTATAAGTAACCAGCTTGAAGCCTATGCCGGTGGCTATAGCGCG CTGGGAAGTCGGTACTCCGAATTTGCGCTTTTTGGCGACACGTTAGGCCTTTATTTGGGCTTTAAATAC TATCTATAA [SEQ ID NO: 12] | |
| MC011 prot | MKRTLKISSLLCVALPLTVQADCLSGDEVAQNSDVTLYEQVSYVNKQASAWQIAGKNPYTRHNGYQEAG IGINSGCSIIDNTLDLKLNLYGINEYALKPAGKFETDDSRTRALINRLSLVYSASDSVQFEAGKFAAPS GMFFLRSPSDLQTHYYTGFQSTRLHDPKMTSAYQASSWGAKMSVDTRDYAFSASVIPKLATIDKRYVTS GNWSANQQGNSDEAYLLSFSDHRFGEHTPTVNVRLGPSPSLALSDSYHYTPQLTLNVDAAYHRSQQWRH LSHRETAQVEEYQFPDSLYETKDESGVELALGGQYTSDNFSVFGVEYYFQSEGYSRAEQRQQRELIDFL NTTTGYAPLDQAFDSYKYLMASEISNTANQGMLQGKHYLNAWASLPLAGESTLQPSLVVNLVDGSTLLG LHYSTPLSAISNQLEAYAGGYSALGSRYSEFALFGDTLGLYLGFKYYL [SEQ ID NO: 36] | 462 |
| MC012 | GTGGGGATCGACAGCCGTAATGATATTCCTGAGGGGATTGCGACGCTGGGCGCTTTTATGGGTTATTCC CATTCACATATCGGTTTTGATCGTGGAGGACATGGCAGTGTGGACAGTTATTCTCTGGGCGGCTATGCC AGTTGGGAACATGAAAGTGGTTTCTATCTGGACGGTGTCGTGAAGCTGAACCGTTTTGAAAGTAACGTA GCCGGTAAAATGAGCAGCGGTGGAGCCGCCAATGGCAGTTACCATAGCAACGGGCTGGGCGGTCACATT GAAACCGGGATGCGATTTACCGATGGTAACTGGAACCTGACGCCGTATGCCTCGTTAACGGGGTTCACC GCTGATAACCCCGAATATCATTTATCCAATGGCATGGAATCGAAATCAGTCGATACCCGCAGTATATAT CGTGAACTGGGTGCAACGCTGAGTTACAACATGCGTCTGGGGAACGGTATGGAAGTTGAGCCGTGGCTG AAGGCGGCTGTGCGCAAAGAATTTGTCGATGATAACCGGGTGAAAGTGAATAGTGACGGTAATTTCGTC AATGATTTGTCGGGCAGACGTGGAATATACCAGGCAGGTATTAAAGCCTCATTCAGCAGTACGTTAAGC GGGCATCTTGGGGTGGGGTATAGCAACGGTGCTGGTATGGAATCCCCGTGGAACGCGGTGGCTGGTGTG AACTGGTCGTTCTGA [SEQ ID NO: 13] | 705 |
| MC012 prot | MGIDSRNDIPEGIATLGAFMGYSHSHIGFDRGGHGSVDSYSLGGYASWEHESGFYLDGVVKLNRFESNV AGKMSSGGAANGSYHSNGLGGHIETGMRFTDGNWNLTPYASLTGFTADNPEYHLSNGMESKSVDTRSIY RELGATLSYNMRLGNGMEVEPWLKAAVRKEFVDDNRVKVNSDGNFVNDLSGRRGIYQAGIKASFSSTLS GHLGVGYSNGAGMESPWNAVAGVNWSF [SEQ ID NO: 37] | 234 |
| MC013 | ATGAAATTCCCTTCAATATTTAACAAAATAAAACCACAATCCATACAGCAACATCCAGAAAAAAATCAA CTTAACTGGATGCTCGAATTAAATAAATGGAAAGAAGAACGTATACTTACAGGTGAAATCCATCGTCCG GAATGTCGAAACGAAGCCGCTAAAAGGATAAACTGTGCTTTTTTGTCGAAACAGAATGACATTGATTTA TCAGGACTTAATTTATCTACTCAACCACCAGGGCTGCAAAACTTCACCTCTATCAATCTTGATAATAAC CAACTCACACATTTTGATGCAACCAACTACGATAGACTCGTAAAACTTAGTCTGAATAGTAACACTCTT GAGTCAATAAATATTCATCAAGGCAGAAATGTAAGCATTACACATATATCTATGAATAATAATTGTCTC AGAAATATTGATATAGATAGGCTTTCATCAATTACTTATTTAGTGCGGCACATAATAAACTAGAGTTT GTGCAATTAGAATCTTGCGAATGGCTGCAATACCTGAATCTCAGCCATAATCAATTAACTGATATTGTT ACAGGAAATAAAGAAGAACTCTTACTGCTGGATCTATCCCATAATAAACTAGCAAGTTTACACAATGCC TTATTTCCCAACTTAAATACGTTACTTATCAACAACAACTTGCTTTCTGAAATTAAAATGTTTTATAGC AACTTCTGCAAAGTTCAGACATTAAACGCTGCTAACAATCAGTTGGAAAAAATAAACCTTCATTTCCTG ACTTATCTTTCATCTATCAAAAGTTTAAGGCTGGACAATAATAAAATAACTCGCATTGATACTGAGAAC ACATCCGATATTAGAAGTTTATTCCCCATAATAAAGAAGAGCGAAAGCTTAAATTTTTTAAATATTTCT GGCGAGAACAATTGCCCTACTATCCAGCTCATGTTATTTAATTTGTTTTCCCCAGCACTTAAGCTTAAT ACTGGCCTGGCAATTCTTTCGCCTGGTGCATTTGAAGATCACTCTGACGGATTAGATGTGGATAACGAA TTGTTTCACTATACTATTAATAAAGCATATACCCCATATAATATACATACTTATAAAACAGAAGAAGTT GTAAACCAGAGGAATATAAAAATTAAAAATATGACCTTAGATGAAATAAACAATACTTATTGTAATAAC GATTATTACAATGAGGCAATAAGAGAGGAACCGATAGACTTTCTGGACAGATCGTTTTCCTCCAGCTCA TGGCCTTTTTATCACTAA **[SEQ ID NO: 14]** | 1260 |
| MC013 prot | MKFPSIFNKIKPQSIQQHPEKNQLNWMLELNKWKEERILTGEIHRPECRNEAAKRINCAFLSKQNDIDL SGLNLSTQPPGLQNFTSINLDNNQLTHFDATNYDRLVKLSLNSNTLESINIHQGRNVSITHISMNNNCL RNIDIDRLSSITYFSAAHNKLEFVQLESCEWLQYLNLSHNQLTDIVTGNKEELLLLDLSHNKLASLHNA LFPNLNTLLINNNLLSEIKMFYSNFCKVQTLNAANNQLEKINLHFLTYLSSIKSLRLDNNKITRIDTEN TSDIRSLFPIIKKSESLNFLNISGENNCPTIQLMLFNLFSPALKLNTGLAILSPGAFEDHSDGLDVDNE LFHYTINKAYTPYNIHTYKTEEVVNQRNIKIKNMTLDEINNTYCNNDYYNEAIREEPIDFLDRSFSSSS WPFYH **[SEQ ID NO: 38]** | 419 |
| MC014 | GTGAACAAGGCCCCCTCTTTAATTGCCGCTATCGTCCTCGGACTGGGGATTAGCGCCTGTGGTTATTTT GTTGGCGATGGTGTTAAACATTTAAAAACCAACAACCGTTATGTCAATGTGCGCGGGCTTTCTGAAAAA GAAGTTCGCGCAGATACGGCGGAATTAACAATTGCGATTAACTTTAAGGGTAATGTTCCCGGCGAACTG TTCCCGAAACTGGAAGAAGCGCAGAAAAAAATTGTTGCCGAACTCAATGCTCAGGGGATTAACGAGAAA GAGATAATTCTGGGCCAATGGACAAGCAAGCGAACAGATTCCTTTTATCTGAAAGATGATCCCACAATG CCACGCTATAACGCAGACGGGTCAGTGACGATAAAAACCCATAATGTTGCTGCTGTCGAAAAAGTCGTC GCGAAATTAAACGAACTGCAAGTCGCTACGGATGGCGCTATCGCTGAGAGCAAAGTCGCCTATCGCTTT AATGGTATTGGCGCACTGCGCGCTGAGATGATTGCTGCCGCCACCAAAGATGCACGCAACGCCGCACTA | 711 |
| | CAATTTGCAACGGACTCAGGTAGCCAGGTGGGGTCAATTAGCGATGCCTCACAAGGCGTATTCCAGATC TTTGCCAGTGGCAGTGATGAAGATGATCCTACGGCGATTAATAAAACGGTACGCGTAGTCACGACCGTA ACCTATGCGTTGCAGGATTAA [SEQ ID NO: 15] | |
| MC014 prot | MNKAPSLIAAIVLGLGISACGYFVGDGVKHLKTNNRYVNVRGLSEKEVRADTAELTIAINFKGNVPGEL FPKLEEAQKKIVAELNAQGINEKEIILGQWTSKRTDSFYLKDDPTMPRYNADGSVTIKTHNVAAVEKVV AKLNELQVATDGAIAESKVAYRFNGIGALRAEMIAAATKDARNAALQFATDSGSQVGSISDASQGVFQI FASGSDEDDPTAINKTVRVVTTVTYALQD [SEQ ID NO: 39] | 236 |
| MC015 | ATGAAAAAATGGACGATATTTTTGACAAGTCTGATATTACTTGCTCTCAGTTTAGAAACACCAAAGTGC TATGCCGGTGATAAACTCATGAGTGCAAGCTTTTCCAGTACTAAAATTTATTATGCGATGAAAAACGTT ACTGCTTCCGGCAGTTTATATTTCTATGTCACGGTAGTGACGCCGGGAGAAGTAAGCTACGGACAATAC AACTCCAATGCAAGAAAAGGCGACACCCTCAAGTTAATATCCTGGAGCGGATCTGGCCCGGCACCGACA CTGGTTTTGACTGATTATAGACGTACGGACACCTCAAATTGCCCTGGTATCAATACTCGTGTCTTTGTC TGTGCATATATGACTTTTAACGTCACTGTAGAATCTGATAATTATGGTTGTCCGTGGATTGCCTCATTT TATTCAGTATCTGAGGCATTTGGATTTGGTACGTATACAAGCCCAACAGTTCATGATTCTATTTGCCCG ACAATTCCCGTCGCTTCGTTTGATATATCCTGGAGTGATAATTATGTTAGCCATAATAAAGCGCTGCGA TTGCAATCCGATGGTTCGACAATCACGACTACGCTTTCGACCTACCTGATGGAAAGCGGCAAACTCTGC GATGGCAGTATTTTTGATTCCAGAGGGGCATATTGTCGTGCTGTTTCTGACTTATTAACCTTTACCTCG TATGGTTGTGATAATGCAAAGGTGACAGTCACCCCCTCACGTCAACCATTGACGGATAGAAAGCTACAC GACATTGTGGTACAGGTGAATACCAGTAGCCGTGAGCCAATTGATTCTACATGCCGTTTCCAGTATGTA CTGAATGAACTGTAG [SEQ ID NO: 16] | 843 |
| MC015 prot | MKKWTIFLTSLILLALSLETPKCYAGDKLMSASFSSTKIYYAMKNVTASGSLYFYVTVVTPGEVSYGQY NSNARKGDTLKLISWSGSGPAPTLVLTDYRRTDTSNCPGINTRVFVCAYMTFNVTVESDNYGCPWIASF YSVSEAFGFGTYTSPTVHDSICPTIPVASFDISWSDNYVSHNKALRLQSDGSTITTTLSTYLMESGKLC DGSIFDSRGAYCRAVSDLLTFTSYGCDNAKVTVTPSRQPLTDRKLHDIVVQVNTSSREPIDSTCRFQYV LNEL [SEQ ID NO: 40] | 280 |
| MC016 | ATGGGTACAGCAGCTATAAGTCATTTACGATATGATCTTAACAAATATGCCCTTTCTTTACGCAAAACC GCAACACTTGCTTCAACATTTTTTATTGAGTCCCCATTAGTTAGATTCGAGTATCTGCAAGAAATAGAA AATACTATTAATGATATTACTCACAGATTCAATTCTTCATATGACATCAATGAAAAAGCACGACTTATC AATGAATTAAAAATGGAGTCAGAAACAGCAAGAAAAGAATATCAACTTTTCAGACAAGGAAATTATGAT AAATATATCACTACTGATATTTTCGAAGAACATGGTTTGATAAAATATGTGAACTTAAGTTTCGATATT GTTGCATCTGTAGGACAGGTCGTAGGCGGGGTTGGCGCGTTGAAATTTGGTAAGGTAGTGCATTCAAAC CGAATAAAAGGTATAGGCGTCACATTAGTTGCTCATGGGGCGAATAATTTTTATGAAAGCTTATCACCT TTATTTTTTAACGAGTATGATTCAGGTCCAATACGTGAACTCTACAGAGTAATAGCAAAAAAAATGGGA GGTGATATTAATTCTGCTGATTATGCATACAGCATCGTGGACTTTTCGATTACTGCATATGGTGGATAT TCAGGCATGAAAATCGTACCTAAGTATAATAGATTGATTCGTCCTTCATTAGGAAATAGACCTGGTACA GGACGATTATTTCATTACACATCAGTTGATTTTAAAAATAAATTCTCATTAAAACCTACCCCTTTAAAA ATCATACAAATTAGTTCTACTGTAAAAAAATTCAAGGTTACATTTTATGATGAAAATACAAATTTGAGG | 912 |
| | ATAAAACATGGCCTCAATAATCATATGCCACATCAATTATTAATGAATATATCAGGAACTACAACTGGT GCAGGAATACAATAA [SEQ ID NO: 17] | |
| MC016 prot | MGTAAISHLRYDLNKYALSLRKTATLASTFFIESPLVRFEYLQEIENTINDITHRFNSSYDINEKARLI NELKMESETARKEYQLFRQGNYDKYITTDIFEEHGLIKYVNLSFDIVASVGQVVGGVGALKFGKVVHSN RIKGIGVTLVAHGANNFYESLSPLFFNEYDSGPIRELYRVIAKKMGGDINSADYAYSIVDFSITAYGGY SGMKIVPKYNRLIRPSLGNRPGTGRLFHYTSVDFKNKFSLKPTPLKIIQISSTVKKFKVTFYDENTNLR IKHGLNNHMPHQLLMNISGTTTGAGIQ [SEQ ID NO: 41] | 303 |
| MC017 | TTGCTGTCACTGACACTGATGTCTGCTTTATTATCGCCTTTATCTCTTCAGGCAGCGGATGTCCGGCGT AGCGGAGATGAAGCATTTATCATTCAGCAGCAGCGTCAGGAAGCCCTTGAGCAACAACTGACGCCTTCA GCCCCTGATGTTCGCCTTTCTGCACCTGGCTCTTTTGCCCATAAGATTAATTTTCCTGTTGAAACGCCC TGTTTTCAGATTAAACAGACGGAACTGAAGGGGGCTGATGCGTTACCACACTGGCTGCCTTTACAAAAA ATCGCCAACGGGGCGGTCGGGCATTGCCTGGGGGCGAAAGGAATTAATCTGCTGATGAGTACATTGCAG AACCGTCTGGTCGATCATGGTTATGTCACCACCCGTGTTCTGGCACCTTCGCAGGATTTAAAAAGCGGT ATCCTCCGGCTGGTTATTATTCCCGGTGTTGTGCGACATGTGCGTCTGACACCGGACAGTGATGACTAT ATTCAGTTGTATTCCTCATTCCCGGCACACGAAGGTTCTCTGCTGGATTTACGGGACATTGAGCAGGGG CTGGATTTAGGTAACAGCCGGATACAGGGACAACATACTGAGCTGAATGCAACCAGTGGAAATCTGTCT ACACAGAATGCGCAACTGAGTGCCGATACGCTTTCCGCCCGGACTGCCGGGCAGTTCAGCAGTAATGGC GGTACGATAAATGCCGACACACTGCAGATATCGGCACAAAGCCTGTCAAATCGTAAAGGCAGTCTGATT CAGACGGGAACAGGGGATTTTTCGCTGAGTCTGCCGGGAAGCGTGGATAACCGGGAAGGGCTGCTTGCG GCAAATGGCGCGGTGCGTCTGGATGCACTGAGCCTTGATAATCGCAAGGGGAAAGTGCAGGCGGAGCAG TCACCCTCCCTTCAGAAATCCCCGCCCACGTTTCTGAAACCGTTTGTGGCTGGTGTCTGTGCGGCATTG CTGGCGGTCAGCGTGGCTATTCCGGGATGGCAGTTTCTGACACAGCCATCACCGGAGGAGCAGCATTTT ACCTGGGGGAATGGTTGTAAAAAGCAGTGA [SEQ ID NO: 18] | 1065 |
| MC017 prot | MLSLTLMSALLSPLSLQAADVRRSGDEAFIIQQQRQEALEQQLTPSAPDVRLSAPGSFAHKINFPVETP CFQIKQTELKGADALPHWLPLQKIANGAVGHCLGAKGINLLMSTLQNRLVDHGYVTTRVLAPSQDLKSG ILRLVIIPGVVRHVRLTPDSDDYIQLYSSFPAHEGSLLDLRDIEQGLDLGNSRIQGQHTELNATSGNLS TQNAQLSADTLSARTAGQFSSNGGTINADTLQISAQSLSNRKGSLIQTGTGDFSLSLPGSVDNREGLLA ANGAVRLDALSLDNRKGKVQAEQSPSLQKSPPTFLKPFVAGVCAALLAVSVAIPGWQFLTQPSPEEQHF TWGNGCKKQ [SEQ ID NO: 42] | 354 |
| MC018 | ATGAGCGGAAAACCGGCGGCGCGTCAGGGCGACATGACGCAGTATGGCGGTAGCATTGTTCAGGGTTCA GCCGGGGTGCGCATTGGTGCCCCCACCGGCGTGGCCTGTTCGGTGTGCCCCGGCGGAGTGACGTCCGGC CATCCGGTCAATCCCCTGCTCGGTGCAAAGGTCCTTCCCGGTGAAACCGACATCGCCCTGCCCGGCCCG CTGCCGTTCATCCTCTCCCGCACCTACAGCAGTTACCGGACAAAAACGCCCGCGCCGGTGGGGAGCCTC GGCCCCGGCTGGAAAATGCCTGCGGATATCCGCTTACAGCTGCGCGATAACACACTGATACTCAGTGAT AACGGCGGCAGAAGCCTGTATTTTGAGCACCTGTTTCCCGGTGAGGACGGTTACAGCCGCAGCGAGTCA CTCTGGCTGGTGCGCGGCGGCGTGGCGAAACTGGATGAAGGTCACCGGCTGGCCGCACTCTGGCAGGCG CTGCCGGAAGAACTCCGCTTAAGTCCGCATCGTTATCTGGCGACAAACAGTCCGCAGGGGCCGTGGTGG | 4200 |
| | CTGCTCGGCTGGTGTGAGCGGGTGCCGGAAGCGGATGAGGTGCTGCCTGCGCCGCTGCCGCCGTACCGG GTACTGACCGGGCTGGTGGACCGCTTCGGGCGCACACAGACGTTCCACCGCGAAGCCGCCGGTGAATTC AGCGGCGAAATCACCGGCGTGACGGATGGTGCCGGGCGTCACTTCCGGCTGGTACTGACCACGCAGGCG CAGCGGGCAGAAGAAGCCCGGCAGCAGGCCATTTCCGGCGGGACGGAACCGTCCGCTTTTCCTGATACC CTGCCGGGTTACACCGAATATGGCCGGGACAACGGCATCCGTCTGTCTGCCGTGTGGCTGACGCACGAC CCGGAATACCCGGAGAATTTACCTGCCGCGCCGCTGGTGCGCTATGGCTGGACGCCGCGCGGCGAACTG GCGGTGGTGTATGACCGTAGTGGCAAACAGGTGCGCAGCTTTACTTACGATGATAAATACCGGGGCCGG ATGGTGGCGCACCGTCACACGGGCCGGCCGGAAATCCGTTACCGTTACGACAGCGACGGGCGGGTGACA GAACAGCTAAACCCGGCAGGCTTAAGCTACACGTATCAGTATGAGAAAGACCGCATCACCATCACCGAC AGCCTGAACCGCCGTGAAGTCCTGCACACGCAGGGTGAAGGCGGGCTGAAGCGGGTGGTGAAAAAGGAA CACGCGGACGGCAGCGTCACGCAGAGTCAGTTTGACGCGGTGGGCAGGCTCAGGGCACAGACGGATGCC GCAGGCAGGACAACAGAATACAGCCCGGATGTGGTGACGGGCCTCATCACGCGCATCACCACGCCGGAT GGCAGGGCATCGGCGTTTTACTATAACCACCACAGCCAGTTAACGTCAGCCACCGGGCCTGACGGGCTG GAAATACGCCGGGAATATGATGAATGGGGCCGTCTGATTCAGGAAACTGCCCCTGACGGCGATATCACC CGCTACCGTTATGATAATCCACACAGTGACTTACCCTGCGCAACGGAAGATGCCACCGGCAGCCGGAAA ACCATGACGTGGAGCCGTTACGGTCAGTTGCTGAGCTTCACCGACTGTTCCGGTTATGTAACCCGTTAT GACCATGACCGCTTCGGGCAGATGACGGCGGTGCACCGCGAGGAAGGGCTGAGTCAGTACCGCGCATAC GACAGCCGTGGACAGTTAATTGCCGTGAAAGACACGCAGGGCCATGAAACGCGGTATGAATACAACGCC GCCGGTGACCTGACCACCGTCATTGCCCCGGACGGCAGCAGAAACGGGACACAGTACGATGCGTGGGGA AAAGCCATCTGTACCACGCAGGGCGGTCTGACGCGCAGTATGGAATACGATGCTGCCGGACGGGTCATC CGCCTGACCAGTGAAAACGGCAGCCACACCACCTTCCGTTACGATGTACTCGACCGGCTGATACAGGAA ACCGGCTTTGACGGCCGCACACAGCGTTATCACCACGACCTGACCGGCAAACTTATCCGCAGCGAGGAT GAGGGGCTGGTCACCCACTGGCACTATGACGAAGCAGACCGCCTCACGCACCGCACCGTGAAGGGTGAA ACCGCAGAGCGCTGGCAGTATGACGAACGCGGCTGGCTGACAGACATCAGCCATATCAGCGAAGGGCAC CGGGTGACGGTGCATTACGGGTATGATGAGAAAGGCCGGCTGACCGGTGAGCGTCAGACGGTGCATCAC CCGCAGACGGAAGCACTGCTCTGGCAGCATGAGACCAGACACGCTTACAACGCGCAGGGGCTGGCGAAC CGCTGTATACCGGACAGCCTGCCCGCCGTGGAATGGCTGACCTATGGCAGCGGCTGGCTGGCAGGCATG AAGCTCGGCGACACACCGCTGGTGGATTTCACCCGCGACCGCCTGCACCGGAAAACGCTGCGCAGATTC GGCCGTTATGAACTCACCACCGCTTATACCCCTGCCGGGCAGTTACAGAGCCAGCACCTGAACAGCCTG CAGTATGACCGCGATTACACCTGGAACGACAACGGCGAACTCATCCGCATCAGCAGCCCGCGCCAGACC CGGAGTTACAGCTACAGCGACTCCGGCAGGCTGACCGGCGTTCACACCACCGCAGCGAATCTGGATATC CGCATCCCGTATGCCACGGACCCGGCAGGTAACCGCCTGCCCGACCCGGAGCTGCACCCGGACAGCACC CTCAGCATGTGGCCGGATAACCGTATCGCCCGTGACGCGCACTATCTTTACCGGTATGACCGTCACGGC AGGCTGACAGAGAAAACCGACCTCATCCCGGAAGGGGTTATCCGCACGGATGATGAGCGGACTCACCGG TACCATTACGACAGTCAGCACCGGCTGGTGCACTACACGCGGACACAATATGAAGAGCCGCTGGTCGAA AGCCGCTATCTTTACGACCCGCTGGGCCGCAGGGTGGCAAAACGGGTGTGGCGACGTGAACGGGACCTG ACGGGCTGGATGTCGCTGTCACGGAAACCGCAAGTGACCTGGTACGGCTGGGACGGCGACCGGCTGACC ACAATACAGAACGACAGAACCCGCATCCAGACGATTTATCAGCCGGGGAGCTTCACGCCACTCATCAGG | |
| | GTTGAAACCGCCACCGGTGAGCTGGCGAAAACGCAGCGCCGCAGCCTGGCGGATGCGCTTCAGCAGTCC GGCGGCGAAGACGGTGGCAGTGTGGTGTTCCCGCCGGTGCTGGTGCAGATGCTCGACCGGCTGGAAAGT GAAATCCTGGCTGACCGGGTGAGTGAGGAAAGCCGCCGCTGGCTGGCATCGTGCGGCCTGACTGTGGCG CAGATGCAAAGCCAGATGGACCCGGTATACACGCCGGCGCGAAAAATCCACCTGTACCACTGCGACCAT CGCGGCCTGCCGCTGGCCCTTATCAGTAAGGAAGGGGCAACAGAATGGTGCGCAGAATACGATGAGTGG GGCAACCTGCTGAATGAAGAGAACCCGCATCAGCTGCAGCAGCTTATCCGCCTGCCGGGGCAGCAGTAT GATGAGGAGTCCGGCCTGTATTACAACCGCCACCGCTATTATGACCCGCTGCAGGGGAGGTATATCACT CAGGATCCGATTGGACTGAAGGGGGGATGGAATTTTTATCAGTATCCGTTGAATCCGGTTCAGTATATA GATTCAATGGGACTGGCATCAAAATATGGACACTTAAATAATGGCGGATATGGAGCGAGACCCAACAAA CCGCCTACGCCCGATCCAAGTAAATTTCCAGACATAGCGAAACAATTAAGACTGCCATATCCTATTGAC CAGGCCAGTAGTGCGCCTAATGTTTTCAAAACATTCTTCAGAGCATTAAGCCCTTACGACTACACACTG TATTGCAGGAAGTGGGTAAAACCAAATCTGACTTGTACGCCACAGGATGATCCCCAGTATCCAGGGATG GATACAAAGACAGCAAGTGATTACCTGCCACAGACAAATTGGCCAACAACTCAATTACCACCAGGATAT ACTTGTGCAGAACCCTATTTATTCCCTGACATTAATAAACCCGATGGGCCAGCAACAGCAGGGATAGAT GATTTGGGTGAAATTTTAGCTAAAATGAAACAGAGAACATCGAGAGGAATAAGAAAATGA [SEQ ID NO: 19] | |
| MC018 prot | MSGKPAARQGDMTQYGGSIVQGSAGVRIGAPTGVACSVCPGGVTSGHPVNPLLGAKVLPGETDIALPGP LPFILSRTYSSYRTKTPAPVGSLGPGWKMPADIRLQLRDNTLILSDNGGRSLYFEHLFPGEDGYSRSES LWLVRGGVAKLDEGHRLAALWQALPEELRLSPHRYLATNSPQGPWWLLGWCERVPEADEVLPAPLPPYR VLTGLVDRFGRTQTFHREAAGEFSGEITGVTDGAGRHFRLVLTTQAQRAEEARQQAISGGTEPSAFPDT LPGYTEYGRDNGIRLSAVWLTHDPEYPENLPAAPLVRYGWTPRGELAVVYDRSGKQVRSFTYDDKYRGR MVAHRHTGRPEIRYRYDSDGRVTEQLNPAGLSYTYQYEKDRITITDSLNRREVLHTQGEGGLKRVVKKE HADGSVTQSQFDAVGRLRAQTDAAGRTTEYSPDVVTGLITRITTPDGRASAFYYNHHSQLTSATGPDGL EIRREYDEWGRLIQETAPDGDITRYRYDNPHSDLPCATEDATGSRKTMTWSRYGQLLSFTDCSGYVTRY DHDRFGQMTAVHREEGLSQYRAYDSRGQLIAVKDTQGHETRYEYNAAGDLTTVIAPDGSRNGTQYDAWG KAICTTQGGLTRSMEYDAAGRVIRLTSENGSHTTFRYDVLDRLIQETGFDGRTQRYHHDLTGKLIRSED EGLVTHWHYDEADRLTHRTVKGETAERWQYDERGWLTDISHISEGHRVTVHYGYDEKGRLTGERQTVHH PQTEALLWQHETRHAYNAQGLANRCIPDSLPAVEWLTYGSGWLAGMKLGDTPLVDFTRDRLHRKTLRRF GRYELTTAYTPAGQLQSQHLNSLQYDRDYTWNDNGELIRISSPRQTRSYSYSDSGRLTGVHTTAANLDI RIPYATDPAGNRLPDPELHPDSTLSMWPDNRIARDAHYLYRYDRHGRLTEKTDLIPEGVIRTDDERTHR YHYDSQHRLVHYTRTQYEEPLVESRYLYDPLGRRVAKRVWRRERDLTGWMSLSRKPQVTWYGWDGDRLT TIQNDRTRIQTIYQPGSFTPLIRVETATGELAKTQRRSLADALQQSGGEDGGSVVFPPVLVQMLDRLES EILADRVSEESRRWLASCGLTVAQMQSQMDPVYTPARKIHLYHCDHRGLPLALISKEGATEWCAEYDEW GNLLNEENPHQLQQLIRLPGQQYDEESGLYYNRHRYYDPLQGRYITQDPIGLKGGWNFYQYPLNPVQYI DSMGLASKYGHLNNGGYGARPNKPPTPDPSKFPDIAKQLRLPYPIDQASSAPNVFKTFFRALSPYDYTL YCRKWVKPNLTCTPQDDPQYPGMDTKTASDYLPQTNWPTTQLPPGYTCAEPYLFPDINKPDGPATAGID DLGEILAKMKQRTSRGIRK [SEQ ID NO: 43] | 1399 |
| MC019 | ATGGCTGCGGCAGCACAAGGTGTGGTAAACGCCGCAACCCAACAACCAGTTCCTGCACAAATTGCCATT GCAAATGCCAATACGGTGCCCTACACCCTTGGAGCGCTGGAATCGGCCCAAAGCGTTGCCGAACGTTTC GGTATTTCGGTGGCTGAGTTACGCAAACTCAACCAGTTTCGTACGTTTGCTCGAGGTTTTGATAATGTC CGCCAGGGTGATGAACTGGATGTCCCGGCACAAGTTAGTGAAAATAATTTAACCCCGCCACCGGGTAAT AGCAGTGGCAACCTTGAGCAACAGATAGCCAGTACTTCACAGCAAATCGGGTCTCTGCTCGCCGAGGAT ATGAACAGCGAGCAAGCGGCAAATATGGCGCGTGGATGGGCCTCTTCTCAGGCTTCAGGCGCAATGACA GACTGGTTAAGCCGCTTCGGTACCGCAAGAATCACGCTGGGCGTGGATGAAGATTTTAGCCTGAAGAAC TCCCAGTTCGATTTTCTCCATCCGTGGTATGAAACGCCTGATAATCTCTTTTTCAGTCAGCATACTCTC CATCGTACTGACGAGCGTACGCAGATTAACAACGGCTTGGGTTGGCGTCATTTCACTCCCACATGGATG TCGGGCATCAACTTCTTTTTCGACCACGATCTTAGCCGTTACCACTCCCGCGCCGGCATTGGCGCGGAG TACTGGCGCGACTATCTAAAATTAAGCAGTAACGGCTATTTGCGACTGACCAACTGGCGCAGCGCACCT GAACTGGACAACGATTATGAAGCACGCCCGGCCAATGGCTGGGATGTACGCGCAGAAGGCTGGCTACCC GCCTGGCCGCACCTTGGCGGTAAACTGGTCTATGAACAGTATTATGGCGATGAAGTGGCCCTGTTCGAT AAAGATGATCGGCAAAGTAATCCTCATGCCATAACCGCTGGACTTAACTATACCCCCTTCCCGCTGATG ACCTTCAGCGCGGAGCAACGCCAGGGTAAACAGGGCGAAAATGACACCCGTTTTGCCGTCGATTTTACC TGGCAACCTGGAAGCGCGATGCAGAAACAGCTTGACCCGAATGAAGTCGATGCACGGCGTAGCCTTGCA GGCAGCCGTTTTGATCTGGTGGATCGCAACAACAACATCGTTCTGGAATATCGCAAAAAAGAACTGGTT CGCCTGACCCTGACAGACCCCGTGACAGGGAAGTCAGGAGAAGTGAAATCACTGGTTTCGTCGCTACAA ACCAAATATGCCCTGAAAGGCTATAACGTCGAAGCCACCGCTCTGGAAGCTGCCGGTGGTAAAGTGGTT ACAACGGGTAAAGATATTCTGGTTACCCTGCCGGCGTACCGGTTCACCAGTACGCCAGAAACCGATAAC ACCTGGCCGATTGAAGTCACCGCTGAAGATGTCAAAGGCAATTTTTCGAATCGTGAACAGAGCATGGTA GTCGTTCAGGCTCCTACGCTAAGCCAGAAAGATTCCTCGGTATCGTTAAGTAGCCAGACGTTGAGCGCG GATTCCCATTCAACCGCCACACTGACTTTTATTGCGCATGATGCAGCAGGTAATCCTGTTATCGGGCTG GTGCTCTCGACGCGTCACGAAGGTGTTCAGGACATCACCCTTTCTGACTGGAAAGATAATGGTGACGGA AGCTATACCCAGATCCTGACCACAGGAGCGATGTCTGGCACGCTGACGCTGATGCCACAGCTGAACGGT GTGGATGCGGCTAAAGCCCCCGCCGTGGTGAATATCATTTCTGTTTCGTCATCCCGGACTCACTCGTCA ATTAAGATTGATAAGGACCGTTATCTCTCCGGGAATCCTATCGAGGTGACGGTAGAACTGAGAGATGAA AATGACAAACCTGTTAAGGAGCAAAAACAGCAACTGAATACCGCAGTCAGCATCGACAACGTGAAACCT GGTGTCACTACAGACTGGAAAGAAACCGCAGATGGCGTCTATAAGGCAACCTATACCGCCTATACCAAA GGCAGTGGGCTTACTGCGAAGCTGTTAATGCAAAACTGGAATGAAGATTTGCATACCGCTGGATTTATC ATCGACGCCAACCCGCAGTCAGCGAAAATTGCGACATTATCTGCCAGCAATAATGGTGTGCTCGCCAAT GAGAATGCAGCAAACACCGTCTCGGTCAATGTCGCTGATGAAGGAAGCAACCCAATCAATGATCATACC GTCACGTTTGCGGTATTAAGCGGATCGGCAACTTCCTTTAACAATCAAAACACCGCAAAAACGGATGTT AATGGTCTGGCGACTTTTGATCTGAAAAGTAGTAAGCAGGAAGACAACACGGTTGAAGTCACCCTTGAA AATGGCGTGAAACAAACGTTAATCGTCAGTTTTGTCGGCGACTCGAGTACCGCGCAGGTTGATCTGCAG AAGTCGAAAAATGAAGTGGTCGCTGACGGCAATGACAGTGCCACAATGACCGCGACAGTTCGGGATGCA AAAGGCAACCTGCTCAATGACGTCAAGGTCACCTTCAATGTCAATTCAGCAGCAGCGAAACTGAGCCAA ACCGAAGTGAATAGCCACGACGGGATCGCCACAGCTACGCTGACCAGTTTGAAAAATGGTGATTATACG | 7863 |
| | GTTACGGCCTCTGTGAGCTCTGGTTCTCAGGCTAATCAACAGGTGATTTTTATCGGTGATCAAAGTACT GCTGCCCTGACCCTCAGTGTGCCTTCAGGTGATATCACCGTCACCAACACAGCTCCGCTACATATGACT GCAACCTTGCAGGATAAAAATGGCAATCCACTAAAAGATAAAGAAATCACCTTCTCTGTGCCAAACGAC GTCGCAAGTCGGTTCTCGATTAGCAACAGCGGAAAAGGCATGACGGATAGCAACGGGACTGCAATCGCC TCCCTGACCGGCACGTTAGCGGGCACGCATATGATCACGGCTCGTCTGGCTAACAGCAATGTCAGCGAT ACACAGCCAATGACGTTTGTGGCGGATAAAGACAGAGCGGTTGTCGTTCTGCAAACATCGAAAGCGGAA ATCATTGGGAATGGCGTGGATGAGACGACTCTGACAGCAACAGTTAAAGATCCTTTTGATAACGTGGTT AAAAATCTTTCAGTAGTCTTCCGCACCTCCCCCGCAGACACGCAACTGAGTCTGAACGCGCGTAATACT AATGAGAACGGTATTGCCGAAGTTACCCTTAAGGGCACGGTTTTGGGTGTTCATACAGCCGAAGCCATA CTGCTTAACGGCAACAGAGATACGAAAATCGTCAATATTGCGCCCGATGCCAGCAACGCGCAGGTCACC CTGAACATCCCTGCACAACAGGTGGTGACGAATAACAGTGACAGCGTGCAGCTGACGGCGACGGTGAAA GACCCGTCGAATCATCCGGTGGCGGGAATAACGGTGAACTTCACCATGCCACAGGACGTGGCGGCAAAC TTTACCCTTGAAAATAACGGTATTGCCATCACTCAGGCCAATGGCGAAGCGCATGTCACCCTCAAAGGC AAAAAAGCGGGCACGCATACTGTGACCGCCACGCTGGGTAACAATAATGCCAGCGATGCGCAACCAGTC ACCTTCGTGGCGGATAAGGACAGCGCGGTTGTCGTTCTGCAAACATCGAAAGCGGAAATCATTGGGAAT GGCGTGGATGAGACGACTCTGACGGCAACAGTGAAAGATCCTTTTGATAACGCAGTAAAAGATCTACAG GTCACCTTCAGTACCAACCCCGCAGATACTCAACTTAGTCAGAGCAAAAGCAATACTAACGACAGTGGT GTGGCCGAAGTTACCTTTAAGGGCACGGTTTTGGGTGTTCATACAGCCGAAGCCACACTGCCTAACGGC AACAACGATACGAAGATAGTCAATATTGCGCCCGATGCCAGCAACGCGCAGGTTACGCTGAACATCCCT GCTCAACAGGTGGTGACGAATAACAGCGACAGCGTGCAGCTGACGGCGACGGTGAAAGATCCGTCGAAT CATCCGGTGGCGGGAATAACGGTGAACTTCACCATGCCACAGGACGTGGCGGCAAACTTTACCCTCGAA AATAACGGTATTGCCATCACCCAGGCCAATGGGGAAGCGCATGTCACGCTCAAAGGTAAAAAAGCGGGT ACGCATACGGTTACCGCAACGCTGAGTAATAACAATACCAGTGATTCACAGCCGGTAACGTTTGTGGCG GACAAAACCTCGGCTCTGGTTGTTCTTCAGATATCAAAAAATGAGATCACAGGTAATGGCGTCGATAGC GCAACGCTAACTGCAACGGTCAAAGATCAGTTCGACAATGAGGTGAACAATCTTCCGGTAACATTCAGC ACAGCTTCTTCAGGCCTCACCCTGACCCCAGGGGAAAGTAATACCAATGAGTCTGGCATCGCGCAGGCC ACTCTCGCAGGCGTTGCCTTTGGTGAGCAGACGGTCACTGCATCACTGGCTAATAATGGTGCCAGCGAC AACAAAACTGTGCATTTTATTGGCGACACAGCGGCGGCAAAAATTATCGAGTTGACGCCTGTCCCAGAC AGCATAATCGCAGGTACCCCGCAGAACAGCTCCGGCAGCGTCATCACCGCCACAGTCGTTGATAATAAT GGCTTTCCGGTGAAAGGTGTGACTGTGAACTTCACCAGCAACGCAGCGACAGCCGAAATGACGAATGGC GGTCAAGCCGTGACGAACGAACAGGGTAAGGCTACCGTCACTTATACCAATACCCGCTCCTCGATAGAA TCAGGAGCGAGACCGGATACCGTTGAGGCCAGTCTGGAAAATGGTAGCTCCACGCTTAGCACATCAATT AATGTCAACGCTGATGCGTCTACGGCACATCTCACCTTGCTACAGGCACTTTTTGATACAGTCTCCGCA GGCGACACTACCAATCTGTATATTGAGGTGAAGGATAATTACGGCAACGGAGTACCCCAGCAGGAGGTA ACCCTCAGCGTTTCACCAAGTGAAGGTGTGACCCCCAGTAATAACGCTATATATACGACCAATCACGAC GGCAATTTTTACGCAAGCTTTACCGCTACAAAAGCCGGGGTATACCAAGTGACGGCAACCCTCGAAAAT GGCGATTCGATGCAACAAACAGTGACCTATGTGCCGAACGTAGCGAATGCTGAAATCTCGCTGGCAGCC TCGAAGGATCCGGTAATTGCCAACAATAACGATCTCACGACACTAACAGCAACAGTCGCTGATACAGAG | |
| | GGCAATGCGATAGCCAACAGTGAGGTAACATTTACTCTGCCGGAAGATGTGAGGGCGAACTTCACGCTG GGCGATGGCGGTAAAGTGGTTACTGATACTGAAGGCAAAGCGAAAGTCACGCTGAAAGGTACAAAAGCA GGCGCTCATACTGTTACAGCATCGATGGCTGGCGGTAAGAGTGAGCAGTTGGTGGTGAACTTTATTGCG GATACACTCACTGCGCAGGTTAATCTTAACGTTACCGAGGACAATTTTATCGCTAATAACGTCGGGATG ACCAGGCTGCAGGCAACAGTGACTGATGGAAACGGCAACCCGTTAGCCAATGAGGCGGTGACATTCACG CTACCGGCAGATGTGAGCGCAAGCTTTACTCTCGGACAAGGCGGTTCCGCCATTACTGACATCAACGGC AAGGCTGAAGTTACACTGAGCGGTACAAAATCCGGCACCTACCCCGTGACAGTTAGCGTGAACAATTAT GGTGTCAGTGATACGAAACAGGTGACTTTGATTGCCGATGCTGGTACCGCAAAACTAGCCTCCTTAACC TCTGTATACTCATTCGTCGTCAGCACGACCGAGGGCGCGACCATGACTGCAAGCGTCACTGACGCTAAC GGCAACCCGGTAGAAGGTATAAAAGTTAATTTCCGCGGAACTTCCGTCACGCTAAGCAGCACCAGCGTT GAAACGGATGATCGGGGTTTCGCTGAAATTCTTGTGACAAGCACCGAGGTCGGACTGAAAACAGTTTCA GCCTCTCTGGCAGATAAACCTACTGAAGTCATCTCGCGATTACTGAATGCAAAAGCAGATATTAATTCT GCAACGATTACCAGTCTGGAGATACCTGAAGGTCAGGTCATGGTCGCACAAGACGTAGCAGTTAAAGCT CACGTCAACGACCAGTTTGGCAATCCGATTCTTAATGAATCTGTAACATTCAGTGCAGAACCACCAGAG CACATGACCATCAGCCAAAATATTGTCTCTACTGATACGCATGGTATAGCCGAGGTCACTATGACGCCC GAAAGAAACGGTTCGTATATGGTGAAAGCATCCCTGGCGAATGGATCCTCTTATGAGAAGGATCTGGTG GTAATCGATCAAAAACTGACACTCTCGGCGTCCAGCCCGCTTATCGGTGTCAATTCCCCAACAGGTGCA ACTCTGACGGCAACGCTAACTTCTGCAAATGGCACTCCAGTGGAGGGTCAGGTCATCAACTTTAGCGTA ACGCCAGAAGGTGCGACGTTAAGTGGCGGAAAAGTGAGAACCAACTCTTCAGGTCAGGCTCCAGTCGTT CTGACCAGCAATAAAGTCGGTACATATACGGTGACTGCATCGTTCCATAACGGCGTAACAATACAGACA CAGACAATCGTGAAAGTCACTGGCAACTCAAGCACCGCCCATGTTGCTAGCTTTATCGCTGATCCATCG ACTATAGCCGCCACCAACAGTGATTTAAGTACCTTAAAGGCAACGGTTGAGGATGGCAGTGGTAACCTG ATCGAAGGTCTCACTGTGTACTTCGCCTTAAAAAGCGGCTCTGCCACATTAACGTCATTAACAGCGGTG ACAGATCAAAACGGAATCGCGACAACAAGCGTGAGAGGAGCGATAACGGGGAGCGTCACGGTAAGCGCA GTCACGACCGCTGGTGGAATGCAAACAGTAGATATAACGCTGGTGGCAGGCCCGGCAGACGCCTCGCAG TCCGTCCTTAAGAACAATCGGTCATCATTGAAAGGAGACTTTACCGATAGTGCTGAGCTACATCTTGTT CTGCACGATATATCAGGCAATCCGATCAAAGTTTCTGAAGGGCTGGAATTTGTGCAGTCAGGTACCAAC GCGCCCTATGTGCAAGTTAGTGCAATTGACTACAGTAAAAATTTCTCAGGCGAGTACAAAGCCACTGTT ACAGGCGGCGGAGAGGGTATCGCAACGCTGATCCCTGTATTGAATGGTGTTCATCAAGCGGGTCTGAGT ACCACAATACAATTCACTCGCGCAGAAGACAAAATAATGAGCGGTACAGTGTTAGTCAATGGTGCTAAC CTACCGACAACTACATTCCCTTCGCAGGGGTTCACTGGGGCGTATTATCAGTTGAATAATGACAACTTT GCCCCAGGAAAAACGGCGGCTGATTATGAGTTTTCAAGCTCTGCCTCCTGGGTTGATGTTGATGCTACC GGTAAAGTGACATTTAAAAATGTCGGCAGCAAATGGGAGAGGATTACGGCGACGCCAAAAACAGGCGGC CCTAGCTATATATACGAAATCCGAGTGAAGAGTTGGTGGGTGAACGCCGGCGATGCTTTCATGATATAC AGCCTTGCTGAAAATTTTTGCAGTAGCAATGGCTACACACTTCCCCTTGGAGACCATTTAAACCATAGT CGTTCCCGAGGCATCGGGTCACTGTACAGTGAATGGGGAGATATGGGGCATTACACGACTGAAGCTGGT TTTCATTCAAATATGTATTGGTCATCGAGTCCCGCAAACTCAAACGAACAATACGTAGTTTCCCTGGCA ACAGGTGATCAAAGCGTATTTGAAAAGCTTGGGTTTGCTTATGCGACATGTTATAAAAACCTCTGA | |
| | [SEQ ID NO: 20] | |
| MC019 prot | MAAAAQGVVNAATQQPVPAQIAIANANTVPYTLGALESAQSVAERFGISVAELRKLNQFRTFARGFDNV RQGDELDVPAQVSENNLTPPPGNSSGNLEQQIASTSQQIGSLLAEDMNSEQAANMARGWASSQASGAMT DWLSRFGTARITLGVDEDFSLKNSQFDFLHPWYETPDNLFFSQHTLHRTDERTQINNGLGWRHFTPTWM SGINFFFDHDLSRYHSRAGIGAEYWRDYLKLSSNGYLRLTNWRSAPELDNDYEARPANGWDVRAEGWLP AWPHLGGKLVYEQYYGDEVALFDKDDRQSNPHAITAGLNYTPFPLMTFSAEQRQGKQGENDTRFAVDFT WQPGSAMQKQLDPNEVDARRSLAGSRFDLVDRNNNIVLEYRKKELVRLTLTDPVTGKSGEVKSLVSSLQ TKYALKGYNVEATALEAAGGKVVTTGKDILVTLPAYRFTSTPETDNTWPIEVTAEDVKGNFSNREQSMV VVQAPTLSQKDSSVSLSSQTLSADSHSTATLTFIAHDAAGNPVIGLVLSTRHEGVQDITLSDWKDNGDG SYTQILTTGAMSGTLTLMPQLNGVDAAKAPAVVNIISVSSSRTHSSIKIDKDRYLSGNPIEVTVELRDE NDKPVKEQKQQLNTAVSIDNVKPGVTTDWKETADGVYKATYTAYTKGSGLTAKLLMQNWNEDLHTAGFI IDANPQSAKIATLSASNNGVLANENAANTVSVNVADEGSNPINDHTVTFAVLSGSATSFNNQNTAKTDV NGLATFDLKSSKQEDNTVEVTLENGVKQTLIVSFVGDSSTAQVDLQKSKNEVVADGNDSATMTATVRDA KGNLLNDVKVTFNVNSAAAKLSQTEVNSHDGIATATLTSLKNGDYTVTASVSSGSQANQQVIFIGDQST AALTLSVPSGDITVTNTAPLHMTATLQDKNGNPLKDKEITFSVPNDVASRFSISNSGKGMTDSNGTAIA SLTGTLAGTHMITARLANSNVSDTQPMTFVADKDRAVVVLQTSKAEIIGNGVDETTLTATVKDPFDNVV KNLSVVFRTSPADTQLSLNARNTNENGIAEVTLKGTVLGVHTAEAILLNGNRDTKIVNIAPDASNAQVT LNIPAQQVVTNNSDSVQLTATVKDPSNHPVAGITVNFTMPQDVAANFTLENNGIAITQANGEAHVTLKG KKAGTHTVTATLGNNNASDAQPVTFVADKDSAVVVLQTSKAEIIGNGVDETTLTATVKDPFDNAVKDLQ VTFSTNPADTQLSQSKSNTNDSGVAEVTFKGTVLGVHTAEATLPNGNNDTKIVNIAPDASNAQVTLNIP AQQVVTNNSDSVQLTATVKDPSNHPVAGITVNFTMPQDVAANFTLENNGIAITQANGEAHVTLKGKKAG THTVTATLSNNNTSDSQPVTFVADKTSALVVLQISKNEITGNGVDSATLTATVKDQFDNEVNNLPVTFS TASSGLTLTPGESNTNESGIAQATLAGVAFGEQTVTASLANNGASDNKTVHFIGDTAAAKIIELTPVPD SIIAGTPQNSSGSVITATVVDNNGFPVKGVTVNFTSNAATAEMTNGGQAVTNEQGKATVTYTNTRSSIE SGARPDTVEASLENGSSTLSTSINVNADASTAHLTLLQALFDTVSAGDTTNLYIEVKDNYGNGVPQQEV TLSVSPSEGVTPSNNAIYTTNHDGNFYASFTATKAGVYQVTATLENGDSMQQTVTYVPNVANAEISLAA SKDPVIANNNDLTTLTATVADTEGNAIANSEVTFTLPEDVRANFTLGDGGKVVTDTEGKAKVTLKGTKA GAHTVTASMAGGKSEQLVVNFIADTLTAQVNLNVTEDNFIANNVGMTRLQATVTDGNGNPLANEAVTFT LPADVSASFTLGQGGSAITDINGKAEVTLSGTKSGTYPVTVSVNNYGVSDTKQVTLIADAGTAKLASLT SVYSFVVSTTEGATMTASVTDANGNPVEGIKVNFRGTSVTLSSTSVETDDRGFAEILVTSTEVGLKTVS ASLADKPTEVISRLLNAKADINSATITSLEIPEGQVMVAQDVAVKAHVNDQFGNPILNESVTFSAEPPE HMTISQNIVSTDTHGIAEVTMTPERNGSYMVKASLANGSSYEKDLVVIDQKLTLSASSPLIGVNSPTGA TLTATLTSANGTPVEGQVINFSVTPEGATLSGGKVRTNSSGQAPVVLTSNKVGTYTVTASFHNGVTIQT QTIVKVTGNSSTAHVASFIADPSTIAATNSDLSTLKATVEDGSGNLIEGLTVYFALKSGSATLTSLTAV TDQNGIATTSVRGAITGSVTVSAVTTAGGMQTVDITLVAGPADASQSVLKNNRSSLKGDFTDSAELHLV LHDISGNPIKVSEGLEFVQSGTNAPYVQVSAIDYSKNFSGEYKATVTGGGEGIATLIPVLNGVHQAGLS TTIQFTRAEDKIMSGTVLVNGANLPTTTFPSQGFTGAYYQLNNDNFAPGKTAADYEFSSSASWVDVDAT | 2620 |
| | GKVTFKNVGSKWERITATPKTGGPSYIYEIRVKSWWVNAGDAFMIYSLAENFCSSNGYTLPLGDHLNHS RSRGIGSLYSEWGDMGHYTTEAGFHSNMYWSSSPANSNEQYVVSLATGDQSVFEKLGFAYATCYKNL [SEQ ID NO: 44] | |
| MC021 | GTGCCTTACACGCTTGGTGCGCTGGAATCAGCGCAAAGCGTTGCTAATCGCTTCGGTATTTCACTGGAG GAGCTTCGTCGTCTTAATCAGTTCCGTACTTTTGCTCGCGGCTTTGATAACGTGCGCCAGGGTGAAGAA CTGGATGTTCCGGCAACAACCTCGCAGAAAAGCCATGAGCAACAAAATGCCGTACCGCCTGCGAATGGC GAAAACACGCTGGAGAATCAAATAGCCAGCACCTCGCAGCGAGTTGGCACTCTGCTTTCACAAGATATG AATAGTGAGCAGGCCAGCGGCATGGCGCGTGGTTGGGCGTCTTCAGAAGCCTCAGGCGCGATGACTGAT TGGTTAAATAACTTTGGTACTGCGAGAATCTCTCTGGGTGTGGATGAAGATTTTAGCCTGAAAAATTCG CAATTCGACTTCCTGCATCCGTGGTATGACACACCTGATTATCTGCTCTTCAGCCAGCATACCCTTCAC CGAACAGACGATCGTACCCAGATCAACACCGGTTTGGGCTGGCGTCATTTCACCTCCAGCTGGATGTCA GGCATCAACCTTTTTTTTGACCACGACCTGAGCCGCTATCACTCCCGCGCAGGGCTTGGCGCAGAATAC TGGCGTGATTATCTGAAGTTGAGCAGCAACGCTTATATCGGCCTGACCGGCTGGCGTAGCGCACCAGAA TTGGATAACGACTTCGAAGCCCGCCCGGCCAACGGCTGGGATTTACGCGCGGAAGGCTGGTTACCTGCC TGGCCACAACTGGGGGGAAAACTGGTCTATGAACAATACTATGGCGATGAAGTGGCGCTGTTTGACAAG AATGATCGTCAAAGTAACCCCCATGCTATTACCGCAGGCCTCAACTATACCCCCTTCCCGCTTCTGACT CTCAGTGCGGAACAGCGTCAGGGGAAGCAAGGTGAAAATGACACACGTTTTGCCGTTGATCTGACCTGG CAACCCAGCAGTTCAATGCAGAAACAGCTTAATCCGGACGAAGTGGCCGGACGGCGCAGTCTGGCCGGT AGTCGTTATGACCTGATTGATCGCAACAACAACATCGTTCTGGAATACCGCAAGAAAGAGCTGATTCGC CTGAGTCTGCTGGATCCGGTGAAAGGGAAGTCTGGAGAAATAAAACCGCTGGTTTCCTCGCTACAGACC AAATATGCCCTTAAAGGCTATAACATCGAAGCCGCTGCGCTGGAAGCTGCCGGAGGTAAAGTCAGCACG TCTGGAAAAGATATCACGGTCACGCTGCCAGGTTACCGCTTCACTAACACCCCAGAAACCGATAATACA TGGTCGATAGACGTTACCGCCGAGGATGTAAAAGGTAACCTGTCACGGCATGAACAAAGCATGGTAGTT ATTCAGGCTCCGACATTAAGCCAGAAAGATTCTCTGTTATCCGTCAATCCGCTAACCGTGGCTGCAGAT AAAAAATCGACGACCACATTGACCGTTACTGCGCACGATTCCGACGGAACTCCGGTGCCGGGGCTGGCG CTGCAAACCCGCAGTGAAGGCGTTCAGGATATCACCCTGTCTGACTGGACAGATAACGGTGATGGTAGT TACACACAGATACTGACCGCCGGAACGACATCAGGTTCAGTAACACTGACGCCGCAAATTAACGGTGAG AGTGCGGTAAAAGAATCCATCGTCGTTAATATCGTCCCTGTTGTCTCATCCCGCGACCATTCATCAATA ACAATTGATAACGTATCGTATTATGCCGGAGACGACATCAAGGTTAGGGTGGAACTGAAAGACGATAGC AATCAACCGGTTGCATATCAAAAAGAGGAATTGGTAAAAGCCGTTACTGTCGAAAACAGCAAACCTGGC GCCACGATTGTCTGGCACGAAGAGCAGCCGGGGGTTTATGCCGCGAATTATCCGGCCTATAAGCAAGGG ACTGCACTAAGGGCACAACTTAGCCTTCACAACTGGAATGCTCCACTGCAATCGCATATTTATAACATT GAGGCAAACCAGAATAAGGCTCGCGTTGCCACATTATCAGCGACAAATAATGACGTTTACGCCGATAAA AAGACATTTAATACCCTCACGATCAACGTCACTGATGAGAGTGATAATCCCCTGACAAATCATCAGGTC ACCTTTAAGAATGAAAAAGGAAGCGCGGAGTTTGTCGAACCGCCGCAGCAAAATACGGATGCATATGGT GTTGCCACAATAAACATGGTAAGTCAGGTTGCGGAAGAAAATACGATTAGCGCCACGCTGCCAAATGGT TTTTCACAACGGATAATTGCGAAATTCGTTAGCGATTCGAGTACGCCAAAATTCAAACAACTGGTTGCC GATCCAGATACCATTATTGCTGGCAACAGCCAGGGCAGTACTCTGACCGCCATCATCACAGACTTTCAT | 5037 |
| | AACAACCCGTTAAAAGATATGAAAGTGAATTTTGTGGCACCTGGTGGCTCGCAACTGGACAACACGACC GCCACAACAGACCAGTCCGGTATTGTGCGGGTGCACCTGACCAGTTCAAAAGCTGGTAGCTATTCCGTC GATGCCTCGCTTGAGGTGGATAAAAATATTCACCAGTCGGTCACGATCACCGTGGTCCCAAACAGGGAA CAATCGGTAATGACCTTGAATGCCGGGTCGGCAGTGCGATCGCTAACAATACAAATATCGTTACCCTG ACTGCCAGTGTGAAAGATGTTTATGGACACCCGTTGCCGGATGAGGATGTGAAATTTACCTTGCCAGCC TCCATGACCGGGAACTTCACGCTAAGTAGTGAAACCGCCCGCACCGATGCAAACGGTGATGCCGTGGTC ACATTGCGAGGCACAAAAGCGGGTGAGTTTACAGTTACGGCGACGCTGACCAGAAATAATACCGTTGCT TATCAGCAAGTCACTTTTATTGGGGATACAAACAGTGCGCAGCTCCAGCCGCTGACTGCCTCATTAAAT TCCATTGTTGCGGGTAACAGTACGGGGAGTACCCTGACGGCAACGATCCTGGACGCTTACCAAAATCCG CTTAAAGACCAGTTGGTCACTTTCCAGAGTAACGATGTCACTCTAAGCGAAACAGAAGTCACCACCAAT ACGCTGGGTCAGGCGACGGTAACAATGACCAGCAATATTGCCGGACAACATAACGTCGTGGTGAGCCGG AAAGCGCAAGCTTCCGATAATAAAACGTTTAGTTTATCAGTGCTACCGGATGAAAGTTCGGCGAAGGTA ATAAGTATAACCGGAGCCGAAAAAACGATAACGGTGGGCGAAAACATCACGCTACGGATACTCGTCCAG GACGCGTTTAACAATGTAATCGCGGGTCAACGCGTCAGATTAAGTGCGCAGCCAACAACTAACATTACG ATAGGCGATACGGCTTACACCGATAATAACGGTTATGCGTACGTTAACCTTCTCAGCACCCAACCTGGG GTTTATCAGGTGACGGCAACGCTGGACAATAACAGTAGTAGTAAGGTTGACGTGAATGTGGCAAATGGC AAACTCGAGTTAACATCATCGAAACCAGAAACTACGGTCCATAATAGTGAGGGTATTACGCTGACCGCA ACGGCGAGAAATGCGCGGGGTGAATTGATGCCAGGGCAAATTATCACCTTTAGCGTAACGCCTGAAGGT GCAACGCTAAGCAATACAGGGGAAGTCCTTACTGACCAGTCAGGTCAGGCCAAAGTGACGCTGACCAGT GACAAAGTGAATGTCTATACCGTTACGGCCATAATGGGCAAAGATGTTCCCGTTCAGAGCCAGGTAACG GTTGCGGTTAAGGCAGATGCTAAAACGGCACATGTTGTGAGCGTCGTGGCTTCTCCTGACACCATCACC GCCGACGGCATCGATAGCAGCACCATCACTTCACGAGTAGAAGATGATTACGGATTCCCGGTTGAAGGT GTCGATATTAGTCATGGCTTAGACACCAAAGGCAGCCCGGTAGTTAATATTCCAACTACGCGTACCGAT CAGTCCGGGCAAGTCACGGCGACAATAACCAGTACATTGGCAGAAACCTTAACAGTCAATGTGCAAGTT CCTGGCACAGCCAACCAATCCGCAACCATTACATTGGTTGCCGGCACGGCCGATGAAAGTAAGTCAATT TTGAAATCCGATGTTGACACTCTGAAGGCTGACTACCAGCAGAGCGCAAAACTTACGCTAACATTGCAA GACAAGTACGGTAACCCGATAGTGACGTCTGATCATCTGGAATTTGTCCAGTCAGGCCCCTTCGTGAAC TTTCTCAAGTTGAGCGATATTGATTACAGCCAAAGAAATTATGGCGAGTACACCGTGACTGTCACTGGC GGAAAAGAGGGAACAGCGACACTCATTCCCATGCTGAACGGGGTTCATCAGGCAAACTTAAGCATATCG CTGAATCTCATCCAATCGATAAAAGAAATGTCCGGTCATGTCACTGCAAACAACCATACCTTCTCCACG GCTAAATTCCCGAGCGAAGGCTTTGCAGGAGCGTATTACACACTCAACAATGATAACTTTGAAGCGGGT AAAACCGTTGATGATTATATGTTTTCAAGTTCACAGGGTTGGGTGTCTGTCGATGCTTCGGGTAAAGTT TCTTTCGCAAATATCGGCGATCAAACGTCAGTCACAATAAGCGCTGTTCCCCGACAAGGAGGTACAACC TACCAGACCTTAATTAAGCTGAAAGGCTGGTGGGTGAATAATGGAAATCATACCAATATCTGGCTAGCT GCCAATGCGCTCTGTCATGCTAAAAATGATGGATATAATCTTCCTGGCATCACACATTTGACGTCTGGC GAAAACAAACGCACGCAGGGATCACTGTATGGTGAATGGGGGAACGTTGGAGCGTTTTCCAGTAATTCG CAATTTACACCGGGAGCTTACTGGACAAGTGAATCTGATGATTACAGTCGGCACTACTATGTGCAGATG CTAACCGGTATGACCGGAAGCGACGCTGATTCCAGCCCCCAACTGACCGCCTGCCGTAAATCACTTTAA | |
| | [SEQ ID NO: 21] | |
| MC021 prot | MPYTLGALESAQSVANRFGISLEELRRLNQFRTFARGFDNVRQGEELDVPATTSQKSHEQQNAVPPANG ENTLENQIASTSQRVGTLLSQDMNSEQASGMARGWASSEASGAMTDWLNNFGTARISLGVDEDFSLKNS QFDFLHPWYDTPDYLLFSQHTLHRTDDRTQINTGLGWRHFTSSWMSGINLFFDHDLSRYHSRAGLGAEY WRDYLKLSSNAYIGLTGWRSAPELDNDFEARPANGWDLRAEGWLPAWPQLGGKLVYEQYYGDEVALFDK NDRQSNPHAITAGLNYTPFPLLTLSAEQRQGKQGENDTRFAVDLTWQPSSSMQKQLNPDEVAGRRSLAG SRYDLIDRNNNIVLEYRKKELIRLSLLDPVKGKSGEIKPLVSSLQTKYALKGYNIEAAALEAAGGKVST SGKDITVTLPGYRFTNTPETDNTWSIDVTAEDVKGNLSRHEQSMVVIQAPTLSQKDSLLSVNPLTVAAD KKSTTTLTVTAHDSDGTPVPGLALQTRSEGVQDITLSDWTDNGDGSYTQILTAGTTSGSVTLTPQINGE SAVKESIVVNIVPVVSSRDHSSITIDNVSYYAGDDIKVRVELKDDSNQPVAYQKEELVKAVTVENSKPG ATIVWHEEQPGVYAANYPAYKQGTALRAQLSLHNWNAPLQSHIYNIEANQNKARVATLSATNNDVYADK KTFNTLTINVTDESDNPLTNHQVTFKNEKGSAEFVEPPQQNTDAYGVATINMVSQVAEENTISATLPNG FSQRIIAKFVSDSSTPKFKQLVADPDTIIAGNSQGSTLTAIITDFHNNPLKDMKVNFVAPGGSQLDNTT ATTDQSGIVRVHLTSSKAGSYSVDASLEVDKNIHQSVTITVVPNREQSVMTLNAGSGSAIANNTNIVTL TASVKDVYGHPLPDEDVKFTLPASMTGNFTLSSETARTDANGDAVVTLRGTKAGEFTVTATLTRNNTVA YQQVTFIGDTNSAQLQPLTASLNSIVAGNSTGSTLTATILDAYQNPLKDQLVTFQSNDVTLSETEVTTN TLGQATVTMTSNIAGQHNVVVSRKAQASDNKTFSLSVLPDESSAKVISITGAEKTITVGENITLRILVQ DAFNNVIAGQRVRLSAQPTTNITIGDTAYTDNNGYAYVNLLSTQPGVYQVTATLDNNSSSKVDVNVANG KLELTSSKPETTVHNSEGITLTATARNARGELMPGQIITFSVTPEGATLSNTGEVLTDQSGQAKVTLTS DKVNVYTVTAIMGKDVPVQSQVTVAVKADAKTAHVVSVVASPDTITADGIDSSTITSRVEDDYGFPVEG VDISHGLDTKGSPVVNIPTTRTDQSGQVTATITSTLAETLTVNVQVPGTANQSATITLVAGTADESKSI LKSDVDTLKADYQQSAKLTLTLQDKYGNPIVTSDHLEFVQSGPFVNFLKLSDIDYSQRNYGEYTVTVTG GKEGTATLIPMLNGVHQANLSISLNLIQSIKEMSGHVTANNHTFSTAKFPSEGFAGAYYTLNNDNFEAG KTVDDYMFSSSQGWVSVDASGKVSFANIGDQTSVTISAVPRQGGTTYQTLIKLKGWWVNNGNHTNIWLA ANALCHAKNDGYNLPGITHLTSGENKRTQGSLYGEWGNVGAFSSNSQFTPGAYWTSESDDYSRHYYVQM LTGMTGSDADSSPQLTACRKSL [SEQ ID NO: 45] | 1678 |
| MC022 | ATGGTCGCTAAATTAAAACCAGATGTTTTTGTTAATACTAATCCTTTTTTAGAAGCAATGTATAAGGAG AGGCTTAGTCATAAAGGCTATTCTGATAAAATTGCTTTAAGTATTAATGATAAGAAATATAATGTAAAC TCTAAGGATATTGAAAATATTCTTGATGGCAAAGGGGATTTATTTAAAAAACGTACACTGTGGGAGTTT GTTCGCGATCTTTTTCCTGGCTCTCATATTAAAGAAGTCAAAGGCTTAATTTACGAATTTGTCACGAAA GTTGATAATAAAGCCGAGGTGTTCGATAAGATTAAATCTCTGGCAAAAAAAGAACAACAATGGCGATTT AGTACTAAGACAGACTTTACCACAAATGAAAATAATGAAGTTATTGTGAGTAGATCCTTTAATCTCTAC ACGGGGGCTACTTCGAATGATAACGAAAAAAAACAGGTTTCTTCAGAGAGATTAACGCTTGATAATTAT CTCGATGATTTACATTTTGATAATTCTCCCTTGGTGAGATTAACGTTTGATAATTATTCTGTAAAACTT GCGACGCTGATTAAAAATAAAATTCCTATTATTAATACAACAATCAATTTATCATCGTTGTCTAAGGAT GTTTTAAATTCATTAAAATATTGTAGTTTTAAGAATGTAATTTTTTCAGGAGTGATAAACAGCCCTAAC CTTGAAGGTCCGGTTTTTGAAAATTGCTATTTCGATGATTGTAAATTCAACAACATACAGCTTTATGAG | 2409 |
| | ACTAATGATGATACTGTGGAATCAGGAAATAAAAAACCAATAAAAGGTATGTTTAAAGGTTGTTTTATT TCTAAATGCAAAATTGAAAATTATAGATGTGAGACGTCTAAAATCTACACCGTTACACAACCTGATAGT ATTCAAGAAAAATTAGGTTCTTATCTCTTTATGCAGTCTTTCGTTCAGGATTGTATAATCCAGGGAGGA TGTTGTCCTGGCTCGAGTATACTTCTGAGTCATTTTTATAACTGTAATATAATAGGGCTTGATGCTCAT GGAATGGATTTTTTGGCAAATTCGTTCAACAAGTCTAATTATGATGAAATCAGGGGTCCGGATACGGGG ACTGTGTTTTATAATTGTAATTTGAAGCATATTAAAATAAACGATGGTCTTCGTGAAGATGGTTCAGAT AGAACTATGTTTAACCCCAAAGAGTATTTTTCTGATTACATTGAAAGAAGATCGAGAAATCTTAAGTTA GATGATAATTTTTCGAAATGCGTAGAGAGTATAAAGAAAGGGACCGATTCTTCATTTAGCAAGAAAAAT ACAGAGAACAATAATGTCTTCTTTAAAAATAGTAATTTGATTGGGGCAAATTTAGGCTATTATTACAGT GGAGATAGGTGCAAAGACTGTGCTATTGATCCAAATACAAATTATTCAAAAGATGGTCTTACAGCGCAA AAATACATGTATATGGATCTGGACGGAGCTATAAGTAAAGAACAAATACCTGATTTCTTAAAAAAGGTA AGTGCTATCAATATCGATGTAATAAATTTTTATAATTCCGAGTCAAAGACTAAGGAAAAATATAAAAAC GCATTTTTAGAGTTAGAGTCGTTTTTATCAACACTTTATGTTGAAAATAAATCGTACGAGGAAAAATAT CATTTTGATAATAGTAGGGTTGAGTTTTTTATTTTTAAAGATATGCAGGAAAATGCACAAAATATCATT AACAATATGATAGAAAGTGACCGGATTAAGTTTGTTGAATCTATAATAAATAAAATGATTCCGCCGCCA GATGGAACAATACTTACTGAAAATCCAAAAGAATATGTCCAAAAGCAAATAAAGGAGTCTCATAAAGAA TCTGCTACAAATGTAACATTTGATTATAAGGAACTTGCACCCATTTTTGAAGGTGTTGAAGAAAAACAA ATTAAAGCACTATCAAACCAACTGGAACATATAAAATCGTTTAAGACAGATTACGACTCAAGGCTCAAT AAGTATGCTCGCGATTTTTACTATTTTTCCAGTGCATTTGCAATTAACTGGGAGGATTTACTAAAAAAC TATCAGGAAATAAGAGCTTCCATTAAAGATTACGATGACTTATTGCGAGAAATTAAAGAGTTAATCATA AGTCGTAATAAATCATTAGAGGATAAAAGGACTTTATTTGATAACAAAAGGGACAATTGGAATAGTATA GAAGTGCAGGATGAAGTAAATGCATTGAATGCGAAAATTGTCGATTGTGATGATAAAATACGTTCTAAG CTTACTATTGTTCGAAATAATAGACTTGAGAATCAATATAAAGACGATAAAAACATCAGTGACGCGATG CGTAATATTCTGGATTGGTTCGAACGGTATCCCGATATTGTACAAAATATAACCCAGGCTTAA [SEQ ID NO: 22] | |
| MC022 prot | MVAKLKPDVFVNTNPFLEAMYKERLSHKGYSDKIALSINDKKYNVNSKDIENILDGKGDLFKKRTLWEF VRDLFPGSHIKEVKGLIYEFVTKVDNKAEVFDKIKSLAKKEQQWRFSTKTDFTTNENNEVIVSRSFNLY TGATSNDNEKKQVSSERLTLDNYLDDLHFDNSPLVRLTFDNYSVKLATLIKNKIPIINTTINLSSLSKD VLNSLKYCSFKNVIFSGVINSPNLEGPVFENCYFDDCKFNNIQLYETNDDTVESGNKKPIKGMFKGCFI SKCKIENYRCETSKIYTVTQPDSIQEKLGSYLFMQSFVQDCIIQGGCCPGSSILLSHFYNCNIIGLDAH GMDFLANSFNKSNYDEIRGPDTGTVFYNCNLKHIKINDGLREDGSDRTMFNPKEYFSDYIERRSRNLKL DDNFSKCVESIKKGTDSSFSKKNTENNNVFFKNSNLIGANLGYYYSGDRCKDCAIDPNTNYSKDGLTAQ KYMYMDLDGAISKEQIPDFLKKVSAINIDVINFYNSESKTKEKYKNAFLELESFLSTLYVENKSYEEKY HFDNSRVEFFIFKDMQENAQNIINNMIESDRIKFVESIINKMIPPPDGTILTENPKEYVQKQIKESHKE SATNVTFDYKELAPIFEGVEEKQIKALSNQLEHIKSFKTDYDSRLNKYARDFYYFSSAFAINWEDLLKN YQEIRASIKDYDDLLREIKELIISRNKSLEDKRTLFDNKRDNWNSIEVQDEVNALNAKIVDCDDKIRSK LTIVRNNRLENQYKDDKNISDAMRNILDWFERYPDIVQNITQA [SEQ ID NO: 46] | 802 |
| MC023 | ATGTTAAGGACGACACGATGGGTCGCTGCTATTATTTTTTTGTATTCTTTTCCGGGTTATGCTGAAGAA ACTTTTGATACGCACTTTATGATCGGCGGCATGAAAGGAGAAAAAGTTCCGAATATCATTTCGATAAT AAACAACCGCTTCCAGGAAACTACGAACTTGATTTTTATGTAAATAACCAGTGGCGTGGAAAACAAGAT ATCACGATCCCAGAGTCACCAGTCAAACCATGTCTGCCTAAGGTGCTACTAACCAAACTGGGGGTAAAG ACCGGTAACCTCAATACAGAAGATAATTGTATTTTACTGGATAAAGCGGTTCATGGTGGACAATATCAG TGGGATATCAGTGAACACCGACTAAATTTAACAGTACCACAGGCGTATATCAATGAACTTGAAAGAGGT TACGTTCCCCCGGAAAGTTGGGATCGCGGCATTGATGCTTTTTATACCTCCTATAATTTAAGTCAGTAT CGCTCTTATGACAGCAATAATAACAGTAATACAGCCAGTTATGGCCGATTTAACAGTGGATTAAATTTA TTTAGTTGGCAGTTACACTCTGATGCCAGCTATAGCAAACCCGATGATATGAAAGGCACATGGCAAAGT AATACTCTTTACCTGGAACATGGCTGGTCGCAGATATTAAGCACAGTTCAGATAGGAGAAAATTACACA TCCTCACTCATATTCGACTCTCTGAGATTTAGCGGAATACGCTTATTTCGCGATATGCAAATGCTACCT GATTCTATGCAGAGCTTTACACCTTTAGTACAAGGTGTTGCGCAAAGTAATGCCTTGATAACGGTTTCG CAAAATGGCTACATCATTTACCAAAAAGAGGTTCCCCCGGGACCATTTACTATCGCAGATTTGCAACTC TCGGGCAGCGGCTCTGATCTCGATGTCAGCATAAAGGAAGCAGATGGCAGTGTTCGTTCCTTTTTGGTG CCCTACTCTTCCGTGCCTAACATGCTGCAACCTGGTATTTCAAATTTCGATTTTATCGCCGGACGCAGT AAGATATATGGTGTAAAGAATCAGGAGGACTTTCTCGAGGCAAATTATATATATGGCCTCAACAATCTT TTGACACTTTATGGCGGTACGATTTTATCCGATAACTATAATGCTATAACTCTGGGAAATGGTTGGAAT ACACCGCTGGGTGCCATCTCTTTTGACGCTACACGATCGAGTAGTAAGTTAAATAATGATATAACACAC GAAGGTACGAGCTATCAGGTCGCCTATAATAAATATCTAGTACAAACGGCCACTCGATTTAGCGTGGCC GCCTGGCGTTATGCTTCGCAGGATTACAGGACATTCAGCGACCATCTTTACGAAAATGATAAAATAAAT CATCAGAGTGACTATGACGATTTTTATGATATTGGCAGAAAAAATAGCCTTTCTGCCAATATCATGCAA CCTTTATCCAATAATCTGGGAAATGTATCATTAAGTGCGCTTTGGCGGAATTACTGGGGGCGAAGTGGA AATGCTAAAGATTACCAATTCAGTTATTCCAATAGCTGGCAACGCATCAGTTATACTTTCTCTGCCAGC CAATCCTATGATGAAAACGACAAAGAAGAGGAGCGTTTTAATCTGTTTATCTCCATTCCTTTCTACTGG GGGGATGATATTGCCAAAACACGTCACCAAATTAACTTATCGAATTCGACCTCATTTTCCAAAGATGGC TATTCCTCCAACAATACTGGAATTACTGGCATAGCCGGTGAACATGATCAGTTAAATTATGGTATATAT GTTAATCAGCAACAACAAAATAATGATACATCGCTTGGTACGAATTTAAGCTGGAGAACTCCCATCGCC ACAATAGATGGCAGCTATAGCCATTCTAAAAACGCCTGGCAAAGTGGTGGAAGTATTAGTAGTGGATTA GTTGTCTGGCCCGGTGGTATTAATATCACTAACCAGCTATCCGATACATTTGCAATTCTGGATGCGCCT GGGTTAGAAGGCGCGCATATTAATGGACAAAAATACAACCGAACAAACAGCAAAGGCCAGGTTGTTTAC GACCTGATGATACCTCATCGTGAAAACCATCTGGTACTTGATACAGCAAACAGTGAAAGTGAAACAGAA TTGCAGGGCAATCGTCAAATTATTGCGCCTTACCGTGGAGCAGTTTCTTATGTGCAGTTTACAACTGAC CAACGTAAGCCCTGGTATATACAGGCACTGCGTCCCGATGGTTCGCCATTAACTTTTGGCTATGACGTA CTGGATCTCCAGGAAAACAATATTGGAGTCGTTGGCCAGGGTAGTCGCCTTTTTATTCGCGTAGATGAA ATTCCAACTGGCATAAAAGTTGCTCTCAATGATGAACAGAATTTATTCTGTACTATTACTTTTCAACAC GTTATCGATGAAAACAAAACATATATATGCCAGTAA [SEQ ID NO: 23] | 2451 |
| MC023 | MLRTTRWVAAIIFLYSFPGYAEETFDTHFMIGGMKGEKVSEYHFDNKQPLPGNYELDFYVNNQWRGKQD ITIPESPVKPCLPKVLLTKLGVKTGNLNTEDNCILLDKAVHGGQYQWDISEHRLNLTVPQAYINELERG | 816 |
| prot | YVPPESWDRGIDAFYTSYNLSQYRSYDSNNNSNTASYGRFNSGLNLFSWQLHSDASYSKPDDMKGTWQS NTLYLEHGWSQILSTVQIGENYTSSLIFDSLRFSGIRLFRDMQMLPDSMQSFTPLVQGVAQSNALITVS QNGYIIYQKEVPPGPFTIADLQLSGSGSDLDVSIKEADGSVRSFLVPYSSVPNMLQPGISNFDFIAGRS KIYGVKNQEDFLEANYIYGLNNLLTLYGGTILSDNYNAITLGNGWNTPLGAISFDATRSSSKLNNDITH EGTSYQVAYNKYLVQTATRFSVAAWRYASQDYRTFSDHLYENDKINHQSDYDDFYDIGRKNSLSANIMQ PLSNNLGNVSLSALWRNYWGRSGNAKDYQFSYSNSWQRISYTFSASQSYDENDKEEERFNLFISIPFYW GDDIAKTRHQINLSNSTSFSKDGYSSNNTGITGIAGEHDQLNYGIYVNQQQQNNDTSLGTNLSWRTPIA TIDGSYSHSKNAWQSGGSISSGLVVWPGGINITNQLSDTFAILDAPGLEGAHINGQKYNRTNSKGQVVY DLMIPHRENHLVLDTANSESETELQGNRQIIAPYRGAVSYVQFTTDQRKPWYIQALRPDGSPLTFGYDV LDLQENNIGVVGQGSRLFIRVDEIPTGIKVALNDEQNLFCTITFQHVIDENKTYICQ **[SEQ ID NO: 47]** | |
| MC024 | ATGACGGCAGATGAGCGCAACGTGGTTAAAGCGGCAACCGACGATAGTATGGAAGCTGCTTATATGCTA AAAGATAATATCCGCTGGTATTATCATAATGGTGACCTATCATTACCCGCTAACTTCAGCAATAAAAAT AAACTGGTGGTGAACGGTAATTTAACGATTTCCGGCGATTATGATGATTATCTGTCAGGTAATGGTCAT CTGATTGTATTAGGTAATGTTATTGTCGATAACTTTATTAATCATGATTTCGCGTATGTCAAAGGACAG ATGACAGCGAAAGGTTTAGTTTATGCTGATTACAATGATCATAACTTTGAAGTGATGAAGGGAATTAGT GCGCGAGGCATTATTGTTTCCGATAAAGCAACGCAATTTGAAGTCATTAAAGCTGAATTTTATATCAAT GAAGATGGAAGTGGTGAAGGATATAACTGGGATGAGAATATTCAAAAAGCCTATTCACTGGTGACTGCC GATCTTTATGATCACACTGAAATCGAAACCGATAACATTTCCAATGCGTATCCTGACTATGATAGCGTC GCAGACAACATCGTTCAGGGTTTACCGCTTTTTCGAGATAAAGCCGCTCCTGAAATTAATGAGAAATTA AAATGGATTGAAACGGGGAAACTCGATAATTTCCCAGCGAATAAAATAAAGCATCAAGATCCACTGGTG GCACGTTTTCTGACTCATACAGAGAGTTTATCACCCGCCGTTATGCTGCAACTGTTACAGCACCCCGAT GACCAGACGCGTGAATCGATGGCGCAGTCCTGGCCCGCTCAACAGATGCATTGGCTTACCGATGAATTA ATTAAAGATGAGGCTGTTGCCAGAGGGCTGGTTAAAAACAGTAATATTTCCGCCGATGTGAATAAAAAA TTAATGTCTGTACCCGTCGAGAGCGTACAGCTGGAGCAGGCCAGACAGGATAATCTTTCTCCAGATATT GTTGCGTCGTTAAGTCATAGCCCCTTTCTCAGTGTACGCAAGACATTACTCAGCCATTACGATTACGCC TGGCTGGTTCCGACTGCAGTGGCCGATGAGTTGATAAATAACGAAGACCCGGAGTTACGTGAAAGAATT ACCGGAGCAGATTTAACCGCACAACAAGCAGTGATGTTGAGTAAAGATAAATCACTGAAAGTTCGTGAA GCATTAGCCCGGACGCTTACTGAATTAAAAATTACCCAACTGTCAGCAACCCTGCGTACAGAGGATATT GAACGTATCGCAGAACAAATGTATCTGGATAATAAAGAAAATAAAAATATCGTAAAGGTGTTATTGATA GCGTTGCCGGAGATGCGTCAACTGTCTCTGGCAAAAGAAGATGTGCACAATTTGCGCGAAGGTGCGCGT TATCTGACATCGAAGGACGTGATTAGTTATCTATTGACTCAGCATGATGTTCCCACCGTCTGGGATGAG CTGGCACGCGATAAATTATTGCCGCTGGAATATAAGAAACAGTTATGGCAACGCACATTAAATTTAATG ATGAGCAAACGGCAAGAAGATCAAGAGCAAGCCTACGAAGTACAGCTGGCGCTGATTGATAATGGTGTT GTTGACGAAGAGATGTTAAATAATGCCATTGATTTACTGGTTGATTTACCCGCTGAATACCGCTATCGA ATGCGTAACCAATTATTTGATAATAAAGAATTACCTTCCGGAATTATCAACAAACTCGATCAACAGTAT CGCTTCAACTCTGACTGGGCGCTGGCTGTTGTCAGTATGAAAAATAGCACCCGTAGACAAAGTGAAAGA GGATTACACCGCTGGAACCATGAGGATAGCGATATTTTTGCGGAACTGGCGACGATAAAAGATAAATCA | 2166 |
| | GATGATGAATGGTGGCGTGCATTGCTGCAAAGTCGCAATGATCATTTGCGCCAAACGGCATTACGCAAC GCACATACCCCGGCGTCGTTGTTAACGACATTGACTGAGTCTCAGGATCGGTCGCTGGCTATTAATAAT CCACAGCTGGCTGCCGATGTGAAAACGGTGTGGCTAAAAGAGGATCCATCATTACTTTTATTGTCGAT AAACCCGATCTTTCGCAGTTACGGGATTTAGTGAAAACCGGGGCAACGCGGAAAATTCGCAATGAAGCG CGTCACCGGCTTGAGGAAAAACAATGA [SEQ ID NO: 24] | |
| MC024 prot | MTADERNVVKAATDDSMEAAYMLKDNIRWYYHNGDLSLPANFSNKNKLVVNGNLTISGDYDDYLSGNGH LIVLGNVIVDNFINHDFAYVKGQMTAKGLVYADYNDHNFEVMKGISARGIIVSDKATQFEVIKAEFYIN EDGSGEGYNWDENIQKAYSLVTADLYDHTEIETDNISNAYPDYDSVADNIVQGLPLFRDKAAPEINEKL KWIETGKLDNFPANKIKHQDPLVARFLTHTESLSPAVMLQLLQHPDDQTRESMAQSWPAQQMHWLTDEL IKDEAVARGLVKNSNISADVNKKLMSVPVESVQLEQARQDNLSPDIVASLSHSPFLSVRKTLLSHYDYA WLVPTAVADELINNEDPELRERITGADLTAQQAVMLSKDKSLKVREALARTLTELKITQLSATLRTEDI ERIAEQMYLDNKENKNIVKVLLIALPEMRQLSLAKEDVHNLREGARYLTSKDVISYLLTQHDVPTVWDE LARDKLLPLEYKKQLWQRTLNLMMSKRQEDQEQAYEVQLALIDNGVVDEEMLNNAIDLLVDLPAEYRYR MRNQLFDNKELPSGIINKLDQQYRFNSDWALAVVSMKNSTRRQSERGLHRWNHEDSDIFAELATIKDKS DDEWWRALLQSRNDHLRQTALRNAHTPASLLTTLTESQDRSLAINNPQLAADVKTVWLKEDPSLLLFVD KPDLSQLRDLVKTGATRKIRNEARHRLEEKQ [SEQ ID NO: 48] | 721 |

Confidential client-attorney work product

## Claims

1. An isolated polypeptide comprising or consisting of:
(a) an amino acid sequence selected from the group consisting of SEQ ID NOs: 25, 26, 27, 28, 29, 30, 31, 32, 33, 34, 35, 36, 37, 38, 39, 40, 41, 42, 43, 44, 45, 46, 47 or 48; or
(b) a variant and/or fragment of (a), for example:
(i) a variant of (a);
(ii) a fragment of (a);
(iii) a variant of a fragment of (a).

2. The isolated polypeptide according to claim 1, wherein (a) is selected from the group consisting of SEQ ID NOs: 25, 26 and 27.

3. The isolated polypeptide according to any preceding claim, wherein (b) exhibits at least 60% sequence identity to an amino acid sequence listed in (a), for example, at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 99.5% sequence identity to an amino acid sequence listed in (a);
(iii) wherein the at least 60% sequence identity is exhibited over at least 60% of the amino acid sequence listed in (a), for example, a contiguous amino acid sequence spanning at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of the amino acid sequence listed in (a); or
(iv) wherein the at least 60% sequence identity is exhibited over at least 10 contiguous amino acids of the amino acid sequence listed in (a), for example, at least 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 350, 400, 450, 500, 550, 600, 650, 700, 750, 800, 850, 900, 950, 1000, 1500, 2000, 2500, 2600, 2601, 2602, 2603, 2604, 2605, 2606, 2607, 2608, 2609, 2610, 2611, 2612, 2613, 2614, 2615, 2616, 2617, 2618 or 2619 contiguous amino acids of the amino acid sequence listed in (a).

4. An isolated nucleic acid molecule comprising or consisting of:
(C) a nucleic acid sequence selected from the group consisting of selected from the group consisting of SEQ ID NO: 1, 2, 3, 4, 5, 6, 7, 8, 9, 10, 11, 12, 13, 14, 15, 16, 17, 18, 19, 20, 21, 22, 23 or 24; or
(D) a fragment, variant and/or fusion of (A).

5. A vector comprising a nucleic acid molecule defined in claim 4.

6. A host cell comprising a nucleic acid molecule defined in claim 4 or a vector as defined in claim 5.

7. A method for producing a polypeptide according to any one of claims 1-3:
comprising or consisting of the steps of culturing a population of host cells according to claim 6 under conditions in which the peptide is expressed, and isolating the peptide therefrom.

8. A vesicle comprising one or more polypeptide defined in claim 1.

9. The vesicle according to claim 8, wherein the vesicle is derived from the membrane of a cell, for example, a Gram positive bacteria membrane vesicle or a Gram negative bacteria outer membrane vesicle (OMV).

10. A binding moiety capable of specifically binding to one or more polypeptide defined in claim 1.

11. A pharmaceutical composition comprising a polypeptide defined in claim 1, a nucleic acid molecule defined in claim 4, a vector as defined in claim 5, a host cell as defined in claim 6, a vesicle as defined in claim 8 and/or a binding moiety as defined in claim 10.

12. A kit comprising or consisting of a polypeptide defined in claim 1, a nucleic acid molecule defined in claim 4, a vector as defined in claim 5, a host cell as defined in claim 6, a vesicle as defined in claim 8, a binding moiety as defined in claim 10 and/or a pharmaceutical composition as defined in claim 11; and (optionally) instructions for use.

13. A polypeptide defined in claim 1, a nucleic acid molecule defined in claim 4, a vector as defined in claim 5, a host cell as defined in claim 6, a vesicle as defined in claim 8, a binding moiety as defined in claim 10 and/or a pharmaceutical composition as defined in claim 11, for use in medicine.

14. A polypeptide defined in claim 1, a nucleic acid molecule defined in claim 4, a vector as defined in claim 5, a host cell as defined in claim 6, a vesicle as defined in claim 8, a binding moiety as defined in claim 10 and/or a pharmaceutical composition as defined in claim 11, for use in preventing or treating bacterial infection and/or symptoms thereof.

15. The use of a nucleic acid molecule defined in claim 4, or a binding moiety as defined in claim 10, for detecting the presence of bacteria.
